(19)

**Europäisches Patentamt**
**European Patent Office**
**Office européen des brevets**

(11) **EP 4 559 410 A1**

(12) **EUROPEAN PATENT APPLICATION**

(43) Date of publication:
**28.05.2025 Bulletin 2025/22**

(21) Application number: **24214878.1**

(22) Date of filing: **22.11.2024**

(51) International Patent Classification (IPC):
**A61B 17/04** (2006.01)   **A61B 17/00** (2006.01)

(52) Cooperative Patent Classification (CPC):
**A61B 17/0401; A61B 17/06166;** A61B 17/064;
A61B 17/068; A61B 17/0682; A61B 17/10;
A61B 17/1604; A61B 17/846; A61B 17/8897;
A61B 17/92; A61B 50/20; A61B 50/33;
A61B 2017/00004; A61B 2017/00429;
A61B 2017/00526;                     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.11.2023 US 202318518272**

(71) Applicant: **Anika Therapeutics, Inc.
Bedford, MA 01730 (US)**

(72) Inventors:
• **CHAO, Nam**
  **Marlborough, MA (US)**
• **EK, Steven W.**
  **Bolton, MA (US)**
• **BRIGHTMAN, Timothy H.**
  **Franklin, MA (US)**
• **LEDUC, Daniel Adam**
  **Carver, MA (US)**
• **BAKER, Christopher E.**
  **Wesley Chapel, FL (US)**

• **AGNELLO, Stefano**
  **Grotte (IT)**
• **AHN, Edward**
  **Grover, MA (US)**
• **CENTOLA, Matteo**
  **Padua (IT)**
• **KENNEDY, Stephan J.**
  **Hudson, NH (US)**
• **MANNARINO, Matthew M.**
  **Burlington, MA (US)**
• **MARCHETTO, Elvira**
  **Albano Terme (IT)**
• **PURI, Sonali**
  **Ashland, MA (US)**
• **RICHARD, Robert**
  **Wakefield, RI (US)**
• **SIMONCIONI, Sara**
  **Senigallia (IT)**
• **GORALTCHOUK, Alexei**
  **Cambridge, MA (US)**

(74) Representative: **Evans, Sophie Elizabeth et al
Kilburn & Strode LLP
Lacon London
84 Theobalds Road
London WC1X 8NL (GB)**

(54) **SURGICAL TOOLS AND SYSTEMS**

(57)    In one aspect, a system for delivery of an implant to a targeted area of a subject, the system includes the implant includes a plurality of bioresorbable hyaluronan-based fibers and a plurality of non-resorbable fibers, a bone fastener configured to couple the implant to bone, a soft-tissue fastener configured to couple the implant to soft tissue, a soft-tissue fastener tool (SFT) configured to couple with and deliver the soft-tissue fastener through the implant and soft-tissue, a bone fastener tool (BFT) configured to couple with and deliver the bone fastener and the implant. The BFT may further be configured to facilitate positioning of at least a portion of the implant over the targeted area after delivery thereto, and a cannula having a cannula passage. The cannula passage may be configured to receive the BFT coupled with the bone fastener and implant therethrough to secure the implant about the targeted site.

FIG. 25

EP 4 559 410 A1

(52) Cooperative Patent Classification (CPC): (Cont.)
   A61B 2017/00893; A61B 2017/00898;
   A61B 2017/00902; A61B 2017/00964;
   A61B 2017/0404; A61B 2017/0406;
   A61B 2017/0409; A61B 2017/0414;
   A61B 2017/0416; A61B 2017/0417;
   A61B 2017/044; A61B 2017/0495;
   A61B 2017/0647; A61B 2017/3458;
   A61B 2017/3484; A61B 2050/0059;
   A61B 2050/3008; A61B 2090/08021; A61F 2/0063;
   A61F 2/0095; A61F 2/08; A61F 2002/0072

**Description**

CROSS-REFERENCE TO RELATED APPLICATIONS

This application is a continuation-in-part of U.S. Patent Application Serial No. 17/663,651, filed May 16, 2022, which claims the benefit of priority to U.S. Provisional Application No. 63/189,400, filed May 17, 2021, each of which is hereby incorporated by reference in its entirety.

This application is a continuation-in-part of U.S. Patent Application Serial No. 17/658,817, filed April 11, 2022, which claims the benefit of priority to U.S. Provisional Application No. 63/173,623, filed April 12, 2021, and to U.S. Provisional Application No. 63/189,351, filed May 17, 2021 each of which is hereby incorporated by reference in its entirety.

This application is a continuation-in-part of U.S. Patent Application Serial No. 17/456,901, filed November 30, 2021, which claims the benefit of priority to U.S. Provisional Application No. 63/120,232, filed December 2, 2020, each of which is hereby incorporated by reference in its entirety.

This application is a continuation-in-part of U.S. Patent Application Serial No. 17/357,345, filed June 24, 2021, which claims the benefit of priority to U.S. Provisional Application No. 63/043,517, filed June 24, 2020, each of which is hereby incorporated by reference in its entirety.

This application is a continuation-in-part of U.S. Patent Application Serial No. 18/487,601, filed October 16, 2023, which is a continuation of U.S. Patent Application Serial No. 15/330,819, filed November 7, 2016, which is a continuation of PCT Application Serial No. PCT/US2015/029792, filed May 7, 2015, which claims the benefit of priority to U.S. Provisional Application No. 61/989,899, filed May 7, 2014, each of which is hereby incorporated by reference in its entirety.

This application claims the benefit of priority to U.S. Provisional Application No. 63/511,530, filed June 30, 2023, which is hereby incorporated by reference in its entirety

FIELD OF INVENTION

For example, in some embodiments, implants, (e.g., including bioabsorbable textiles) of the invention can be used in conjunction with multiple types of fixation devices, known in the field of orthopedics for repairing damaged soft tissue. These fixation devices can be bone screws, bone screws with washers, fasteners (as described herein), staples (as described herein), suture anchors, suture buttons and the like; used to create, repair or augment tissue connection sites relevant to orthopedic procedures. These connection sites can be comprised of bone-to-bone tissue connections, as typically seen in ligament injuries; tissue-to-bone connections, as typically seen in tendon or ligament injuries; and tissue-to-tissue connections, as typically seen within tendon, ligament, or muscle injuries. The medical textiles (e.g., implants) used in conjunction with these fixation devices may take the form of sutures, suture tapes of different constructions and sizes, patches (as described herein), meshes, etc.

Embodiments of the present invention relate generally to ,implants, fasteners, tools, and methods for surgical repair of anatomical structures, such as tendons (e.g., the supraspinatus tendon, commonly known as the rotator cuff). The implants may incorporate bioabsorbable compositions. The methods may include simultaneous or substantially simultaneous delivery of a fastener and implant.

The present invention relates generally to surgical fasteners, more particularly, but not by way of limitation, to fasteners, tools, and methods for surgical repair of anatomical structures, such as tendons (e.g., the supraspinatus tendon, commonly known as the rotator cuff).

The medical textiles (e.g., implants) of the invention can be fashioned so that they are packaged and provided to the surgeon user directly coupled to these fixation devices, or they can be fashioned using splicing or knotting techniques within the textile so that they could be packaged and provided to the surgeon user for use with separately packaged fixation devices.

BACKGROUND

Medical textiles such as sutures are ubiquitous in the field of surgery. Sutures are typically made from non-resorbable materials such as ultra- high molecular weight polyester (UHMWPE) fibers, as well as polypropylene, nylon, and derivatives thereof. While providing the requisite biocompatibility and strength, these materials must be removed or will remain in place in the patient owing to their non-resorbable nature.

Hyaluronic acid is a naturally occurring non-sulphated glycosaminoglycan consisting of a linear sequence of D-glucuronic acid and *N*-acetyl-D-glucosamine. It is present in connective tissue, in the synovial fluid of articular joints and in the vitreous humor of the eye. Hyaluronic acid is important in many biological processes such as tissue hydration, cell differentiation, cell behavior and tissue repair. In recent years a hyaluronan polymer-based scaffold has shown surprising properties in the field of tissue engineering. See US 5,939,323 "Hyaluronan Based Biodegradable Scaffolds for Tissue Repair' issued August 17, 1999, and US 6,872,819 "Biomaterials Containing Hyaluronic Acid Derivatives in the Form of Three-Dimensional Structures Free From Cellular Components or Products Thereof for the In Vivo Regeneration of Tissue Cells" issued Mar. 29, 2005. A semisynthetic insoluble polymer has been obtained by the hyaluronic acid esterification with benzyl alcohol. See PCT/EP97/04684 published as WO98/08876 on March 5, 1998 (Fidia Advanced Biopolymers - F.A.B Abano Terme, Italy). The disclosures of these documents are incorporated fully by reference.

Products including hyaluronic acid esterified with benzyl alcohol are sold under the trademark HYAFF11[®] and are manufactured by Anika Therapeutics S.R.L., Padua Italy. The HYAFF11[®] composition is biocompatible, completely biodegradable, soluble in dimethylsulfoxide (DMSO), exhibits good stability to hydrolysis, forms contact angle measurements and presents a strong ability to interact with polar molecules. HYAFF 11[®] fibers comprise partially-to- fully esterified hyaluronic acid pendent polymer. This process increases the hydrophobicity of the hyaluronic acid such that it can be produced into non-tissue soluble fiber with a controlled rate of bioresorption. A variety of different chemical compositions of this material can be produced to affect residence time, hydrophilicity, and strength.

Various surgical fasteners are known, such as for securing an implant, for example for repair of a tear in the supraspinatus tendon (which may be commonly referred to as a rotator cuff tear, and which may be referred to herein as a targeted area). For example, United States Patent Application No. US 2008/0188936 discloses certain examples of fasteners and implants for such repairs.

In reading the present application, the practitioner of ordinary skill in the art will appreciate that the terms "substantial" and "substantially" are intended to convey having a detectable amount and more than a negligible amount or degree of the attribute modified by the terms; the term "flexible" is intended to convey having the ability to be actively deformed and restored to its spatial condition and configuration with a minimum of persistent distortion, at least over some number of cycles, whereas the term "elastic" conveys having the characteristic of being flexible as well as having a tendency to self-restore to its original condition or configuration and, potentially, to urge other components and/or features to return to an earlier configuration. The term "elongate" is intended to convey the sense of a three dimensional object where one of the three dimensions is substantially longer than the other two dimensions.

Sutures have been used in the treatment of wounds for thousands of years. Over this time many important improvements have been made to compositions, apparatus and techniques for the use of sutures. One might think that such an extended development time would have allowed the technology to reach stasis. Nevertheless, the application of creativity and diligent effort continues to yield beneficial improvements such as those presented in the present application.

Rotator cuff acts to stabilize the shoulder. Rotator cuff tears are a common source of debilitating pain, weakness and reduced shoulder function, which can lead to degenerative changes in the glenohumeral joint over time (Lehman, 1995). Rotator cuff tears affect up to 22% of the general population, with the incidence increasing as the population ages (Yamamoto, 2010; Minagawa, 2013). Full-thickness tears alone are estimated to have a prevalence of 28% for >60 years-old patients and 50% for >80 years-old patients (Tashjian, 2012).

The complex anatomy of the shoulder, the extended range of motion of this joint, as well as the hypovascularity of tendons and the relative weakening associated with tissue degeneration contribute to the impaired healing of these tears and impose significant challenges in the treatment of these disorders (Ratcliffe, 2015).

In some cases, partial- and full-thickness tears can be treated non-operatively with conservative therapies; however, the vast majority are treated operatively, by surgical repair involving sutures, anchors and staples.

Multiple studies have documented significant re-tear rates between 5% (Bishop, 2006) and 90% (Galatz, 2004) in arthroscopically-managed tears, with those of larger size being more at risk.

Generally, the defective healing of the damaged rotator cuff tendon(s) is the main reason for repair failure. Patients who re-tear have reduced functional outcome and fewer treatment options available, often involving the unwanted and unpleasant scenario of a second surgical intervention that is often the shoulder arthroplasty (Smith, 2016).

A method of improving the mechanical integrity of rotator cuff tendon repairs is the application of a reinforcement device or scaffold. Tendon repair can be mechanically-augmented using a device grafted to the lesion area and fixed to the native tissue(s) with additional sutures and anchors, overall indicated as an augmentation technique (Zhang, 2012).

Scaffolds can be broadly categorized into 3 different categories: biological, synthetic, and a combination thereof (biosynthetic).

Biological devices (allografts or xenografts) are typically derived from decellularized extracellular matrix (ECM) of human, porcine, bovine and equine origin. The most common tissue sources are small intestine submucosa (SIS), dermis and pericardium, which are processed through cascade steps, to remove any non-collagen components (Longo, 2010). Common disadvantages of this category are the possibility of immune reaction, including the potential for graft rejection, the persistence of chronic inflammation and the risk of disease transmission due to the presence of genetic or infectious material in their composition (Chen, 2009).

Moreover, there is a high product variability and the need for long preparation time (hydration protocol) before entering in the operating room, which is never a preferred option for the end-users. Furthermore, these biological devices inherently possess significant safety risks linked to possible residual xenogenic DNA and to a strong foreign body reaction following the implantation.

Considering the strong disadvantages associated to the biological devices, an increasing interest has been focused in the development and use of synthetic devices. However, these devices are also associated to some drawbacks, such as: (i) the slow degradation time (>2 years), eventually associated with long-term problems related to long-term fatigue of the materials, (ii) poor support for tissue ingrowth and new tissue formation, (iii) poor integration into native tissue, and (iv) persistent low- level inflammatory response.

Generally, the synthetic augmentation devices are optimized around one single feature that is intrinsically associated to the raw material comprising a given device. For example, X-Repair (Synthasome), a poly-L-lactide-based woven scaffold was specifically designed with mechanical properties that are the most similar to human tendon among the commercially available augmentation scaffolds; however it is not able to support an effective biological healing and may be associated with a high risk of long-term inflammation associated to the slow degradation time of polylactic acid (Derwin 2009).

Even though more than 20 devices have been commercially available for the past 3 decades, few have demonstrated clinical efficacy in the context of surgical rotator cuff repair

(Smith, 2017) and none have been implemented into routine clinical practice (Thangarajah, 2015). The inadequacy of existing scaffolds is partly due to the fact that they have not been specifically designed for rotator cuff repair, but for other specific uses, such as for anterior cruciate ligament (ACL) replacement, diabetic wound or Achilles tendon repair, or for a broad range of surgical repair applications.

None of the commercially-available devices have demonstrated the ability to effectively restore the highly hierarchical histological architecture of the native tendon tissue (Tong, 2012; Kishore, 2012; Younesi, 2014).

In conclusion, none of the developed devices aimed at repairing, reinforcing and/or augmenting rotator cuff tendons achieve the desired outcomes and this explains the low adoption rate of the rotator cuff augmentation technique many years after the technique was invented. Therefore, the scientific and the orthopedic community still demands viable, durable and effective solutions to provide patients with a valid therapeutic option, which is not yet available in the market.

No commercially-available devices meet the requirements for functional joint tissue healing, and this explains the low adoption rate that the surgical techniques aimed at preserving and not replacing the joint tissue(s) have shown to date in clinical practice. Thus, the orthopedic and, more importantly, the patient community still demands viable, durable and effective therapeutic solutions, alternative to the more invasive total joint arthroplasties or autografts that become less and less favorable considering their irreversible shortcomings.

An ideal device for functional and integrative joint restoration must first meet the physiological demand of the native tissue by promoting host cell-mediated healing while also providing a mechanical augmentation. Furthermore, the device should be biodegradable in order to be gradually replaced by new tissue.

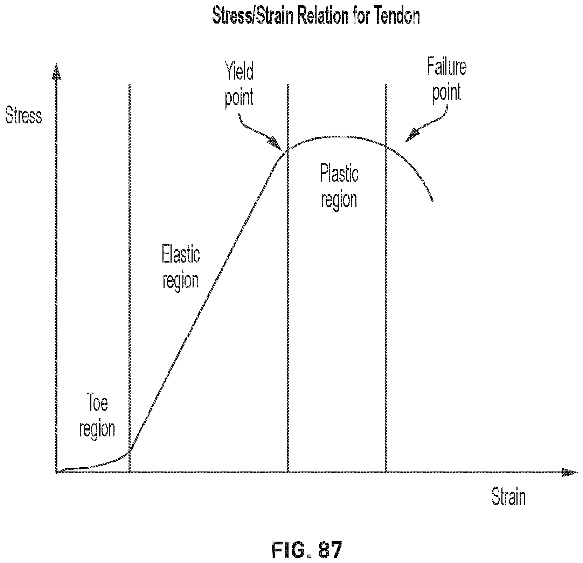

More specifically, a typical tensile stress/strain relation for tendon is showed in FIG. 87 The mechanical properties of an ideal rotator cuff augmentation device should be similar to those of tendon to minimize complications due to compliance mis-match.

The mechanical design inputs for such devices are (Funakoshi, 2005): Strength greater than the peak in vivo loads experienced by the repair tissue and preferably similar to the tissue itself, therefore ensuring that the device will not fail under physiological loads (FIG. 87). Stiffness that allows for substantial load sharing, providing reinforcement and at the same time ensuring some load will be applied across the repair site, required for optimal biologic repair (FIG. 87). Strain within the toe region of a load-displacement curve rather than experiencing loads in the linear stiffness region (FIG. 87).

A device for tendon repair should reach a maximum load as high as possible in the range of 50-800 N and within a strain at ultimate tensile stress of 0.5-8%. Suture retention strength is an additional fundamental property for the device. The suture pull-out strength should be high enough to resist functional loading, moreover, the elongation required for this load should be very low.

Additional requirements for an ideal device aimed at restoring joint function are biocompatibility and tolerability of both the intact device and its degradation products, as well as ease of handling and fitting for possible arthroscopic application (Moffat, 2009).

On the other hand, the biological properties of a device for joint tissue restoration can be enhanced with surface properties biomimicking the native structure. Hence, biologically relevant design inputs include high surface area to volume ratio, low density, high porosity, variable pore size, pore morphology, fiber orientation and microscale mechanical properties approximating those of the native tendon tissue. All these structural characteristics have been shown to significantly influence cell behaviors such as adhesion, growth, and differentiation, and neo-tissue formation. For example, *in vitro* studies have revealed that scaffold topography and fiber orientation, roughly recapitulating the architecture of the collagen-rich joint tissue matrix, positively regulate cell response thereby improving likelihood for a successful joint tissue repair (Kishore, 2012). In particular, the alignment of scaffold fibers promoted cell orientation along the longitudinal axis of the fibers, mimicking the physiological mature tenocytes disposition in native tendon; the matrix stiffness instead was found to influence cell differentiation and new collagen deposition (Grier, 2017).

Lastly, the device must integrate with the host joint tissue as well as with all the other surrounding tissues in the joint (typically bone) by promoting the regeneration of the native tissue interface.

Only an unprecedented, non-obvious, tuned and integrated combination of the above-listed features will result in a clinically-effective solution for treating joint tears, ultimately offering to the surgeons and the patients a viable and long-lasting regenerative solution that preserves the shoulder joint and avoid or delay a total arthroplasty intervention.

A possible solution to the above-mentioned demands may be offered by the development and/or by the use of novel biocompatible and degradable biomaterials with mechanical and biological properties that are fine-tuned to match specific requirements (Funakoshi, 2005; Caliari, 2011).

Various surgical fasteners and delivery tools are known, such as for securing an implant, for example for repair of a tear in the supraspinatus tendon (which may be commonly referred to as a rotator cuff tear). For example, United States Patent Application No. US 2008/0188936 discloses certain examples of fasteners and implants for such repair

The present invention aims at providing a device satisfying all the above listed needs, by also overcoming the disadvantages of the prior art devices.

BRIEF SUMMARY

Disclosed herein, in some aspects is a system for delivery of an implant to a targeted area of a subject, the system comprising: the implant comprising a plurality of bioresorbable hyaluronan-based fibers and a plurality of non-resorbable fibers; a bone fastener configured to couple the implant to bone, the bone fastener having a first elongated shaft extending from a first enlarged head, and one or more first barbs at least partially extending laterally from the first elongated shaft; a soft-tissue fastener configured to couple the implant to soft tissue, the soft-tissue fastener having a second elongated shaft extending from a second enlarged head, and one or more second barbs at least partially extending laterally from the second elongated shaft; a soft-tissue fastener tool (SFT) configured to couple with and deliver the soft-tissue fastener through the implant and soft-tissue; a bone fastener tool (BFT) configured to couple with and deliver the bone fastener and the implant, the BFT further configured to facilitate positioning of at least a portion of the implant over the targeted area after delivery thereto; and a cannula comprising a cannula distal end, a cannula proximal end, and a cannula passage extending through and between the cannula proximal end and the cannula distal end, the cannula passage configured to receive the BFT coupled with the bone fastener and implant therethrough, such that the BFT is configured to secure the implant about the targeted area via passing through the cannula distal end to insert the bone fastener to a bone about the targeted area with the implant located therebetween.

In some embodiments, the implant comprises a nonwoven medical textile, a woven medical textile, a braided construction, a weft-knit medical textile, a warp knit medical textile, or a combination thereof. In some embodiments, the implant comprises a woven layer and a nonwoven layer coupled to the woven layer. In some embodiments, the implant comprises a first end edge, a second edge, and a pair of lateral edges that each extend between the first and second edges. In some embodiments, the implant comprises a substantially planar configuration when in a flattened state, optionally in which the first end edge is separated from the second end edge under a first tension of 5 Newtons (N) applied perpendicular to each of the end edges, and optionally in which the lateral edges are separated from each other under a tension of 5 N applied perpendicular to each of the lateral edges.

In some embodiments, the bioresorbable hyaluronan-based fibers and the non-resorbable fibers are joined together by at least one selected from the group consisting of braided, knitted, adhered, intermeshed, weaved, interlocked, twisted, and heat set. In some embodiments, the hyaluronan-based fibers comprise at least one selected from the group consisting of hyaluronic acid, sodium hyaluronate, and esters of hyaluronic acid. In some embodiments, the esters of hyaluronic acid comprise benzyl esters of hyaluronic acid.

In some embodiments, the implant comprises from 10% to 98% non-resorbable fibers, based on the weight of the non-resorbable fibers to the total weight of the non-resorbable fibers and bioresorbable hyaluronan-based fibers. In some embodiments, the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton. In some embodiments, the implant further comprises bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxybutyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

In some embodiments, the hyaluronan-based bioresorbable fibers are prepared by at least one selected from the group consisting of ring spinning, air-jet spinning, open-end spinning, mule spinning, wet spinning, dry spinning, electrospinning, pneumatospinning, pultrusion, and extrusion. In some embodiments, the extrusion comprises at least one selected from the group consisting of solvent exchange extrusion, precipitation extrusion, phase exchange extrusion, and phase change extrusion. In some embodiments, the bioresorbable hyaluronan-based fibers comprise at least one selected from the group consisting of mono- and multifilaments.

In some embodiments, the system further comprises at least one active agent. In some embodiments, the active agent is an antimicrobial agent. In some embodiments, the antimicrobial agent comprises at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

In some embodiments, the active agent is an analgesic. In some embodiments, the analgesic comprises at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine. In some embodiments, the active agent comprises a vasoconstrictive agent. In some embodiments, the vasoconstrictive agent comprises at least one selected from the group consisting of epinephrine, alpha-adrenoreceptor antagonists, vasopressin analogues, norepinephrine, phenylephrine, dopamine, dobutamine, serotonin agonists, and triptans.

In some embodiments, at least one selected from the group consisting of the non-resorbable fibers and the bioresorbable hyaluronan-based fibers are wetted with the active agent. In some embodiments, at least one selected from the group consisting of the non-resorbable fibers and the bioresorbable hyaluronan-based fibers are coated with the active agent. In some embodiments, at least one selected from the group consisting of the non-resorbable fibers and the bioresorbable hyaluronan-based fibers have the active agent embedded within the fiber.

In some embodiments, the BFT comprises: an elongated body extending from a body proximal end to a body distal end, the body defining a body proximal opening through the body proximal end, a body recess in a peripheral surface of the elongated body at point that is offset from the body distal end, and a body passage extending between the body proximal opening and a body distal opening within the body recess; and one or more spikes each having a spike proximal end coupled to the body distal end, and a spike distal end extending from the body distal end and configured to be at least partially received within the first elongated shaft of the bone fastener, body distal end having a dimension larger than a transverse dimension of the one or more spikes, such that a shoulder is defined about the body distal end that is configured to abut the first enlarged head of the bone fastener; and a handle coupled to the body proximal end.

In some embodiments, the one or more spikes comprise a pair of spikes, where each spike of the pair of spikes are parallel to and laterally spaced from the other. In some embodiments, the BFT further comprises: a hub having a hub proximal end and a hub distal end, the hub configured to receive the proximal end of the elongated body through the hub distal end; an pusher having a pusher proximal end and a pusher distal end, the pusher disposed in the body passage such that the pusher distal end is disposed beyond the body distal opening; a slider coupled to the pusher proximal end, the slider configured to move longitudinally relative to the handle from a retracted position to a deployed position to move the pusher distal end longitudinally away from the body distal opening and laterally outward from the elongated body.

In some embodiments, the hub is coupled in fixed relation to the elongated body, and the handle is rotatable relative to the hub to cause the pusher distal end to rotate relative to the elongate body. In some embodiments, the body recess defines a sloped surface spaced from the body distal opening, and where the apparatus is configured such that, when the slider is moved from the retracted position to the deployed position, a flexible portion of the pusher contacts the sloped surface to cause the pusher distal end to extend laterally outward from the body. In some embodiments, the pusher comprises a roller coupled to the pusher distal end.

In some embodiments, the system further comprises a seal assembly, comprising: an annular seal body having a seal proximal end and a seal distal end, the seal body defining a seal passage extending through and between the seal proximal end and the seal distal end, and a tapered distal surface disposed around the seal passage at the seal distal end; and a resilient membrane disposed at least around an interior periphery of the seal passage, the resilient membrane configured to cooperate with the elongated body of the BFT to substantially seal the seal passage when the elongated body of the BFT extends through the resilient membrane and the seal passage. In some embodiments, the elongated body of the BFT is disposed through the resilient membrane of the seal and the seal passage with the seal distal end facing the body distal end.

In some embodiments, the cannula proximal end is configured to be coupled to the seal body, the cannula further defining a cannula proximal surface tapered distally and inward toward the cannula passage, the cannula proximal surface configured to cooperate with the seal distal surface to provide a seal between the cannula proximal surface and the seal distal surface. In some embodiments, an outer surface of the cannula includes one or more threads that are configured to engage soft tissue as the cannula is rotated. In some embodiments, a proximal end of the cannula includes an enlarged portion that is configured to be engaged by a user to vary the depth and/or direction of the cannula. In some embodiments, the cannula distal end defines a notch configured to permit a portion of the pusher to deflect radially outward through the notch.

In some embodiments, the system further comprises: an obturator having an obturator body with a obturator proximal end and a tapered obturator distal end, the obturator body configured to extend through the cannula such that the obturator distal end extends distally beyond the cannula distal end to facilitate insertion of the cannula through soft tissue of a patient.

In some embodiments, the system further comprises a caddy system comprising: a base defining one or more bone fastener recesses; and a lid coupled to the base and configured to be rotated relative to the base to selectively cover or expose the bone fastener recess(es). In some embodiments, the base further defines one or more implant recesses each overlapping at least one of the one or more bone fastener recesses, where the lid is configured to be rotated relative to the base to selectively cover or expose the implant. In some embodiments, the one or more implant recesses includes a substantially planar lower surface offset below a substantially planar upper surface of the base. In some embodiments, the lid is rotatable, relative to the base, over at least one of the bone fastener recesses. In some embodiments, the base further defines one or more soft tissue fastener recesses, where the lid is configured to be rotated relative to the base to selectively cover or expose the soft fastener recess(es).

In some embodiments, the bone fastener comprises a third elongated shaft extending from the first enlarged head, and one or more third barbs at least partially extending laterally from the third elongated shaft. In some embodiments, the bone fastener further comprises a plurality of fourth and fifth barbs extending at least partially laterally from the first elongated shaft and the third elongated shaft respectively, each of the first, third, fourth, and fifth barbs having a leading edge and a trailing edge. In some embodiments, the first and third elongated shaft of the bone fastener is configured to extend through the implant such that the trailing edge of each of the fourth and fifth barbs are disposed on a first side of the implant, and at least a portion of the leading edges of each of the first barbs and third barbs are disposed on a second side of the implant.

Disclosed herein, in other aspects, is a method for delivering an implant to a targeted area in a subject, the method comprising: coupling a bone fastener with an implant by passing the bone fastener therethrough; inserting the bone fastener at least partially into a bone at the targeted area, thereby securing the implant to said bone; manipulating a portion of the implant not secured via the bone fastener so as to facilitate placement of said portion of the implant about a soft tissue of the targeted area; and securing said portion of the implant to the soft tissue by inserting one or more soft tissue fasteners through the implant and the soft tissue; wherein the implant comprises a plurality of bioresorbable hyaluronan-based fibers and a plurality of non-resorbable fibers.

Disclosed herein, in yet other aspects, is a method for delivering the implant according to any implant described herein to the targeted area using any system described herein, the method comprising: using the BFT, coupling the bone fastener with the implant by passing the bone fastener therethrough; aligning the cannula passage about the targeted area; inserting the BFT through the cannula passage so as to insert the bone fastener at least partially into the bone at the targeted area, thereby securing the implant to said bone; moving the slider from the retracted position to the deployed position, thereby causing the distal end of the pusher to move distally and radially so as to position a portion of the implant about the soft tissue at the targeted area; and securing said portion of the implant to the soft tissue by inserting the soft tissue fastener through the implant and the soft tissue.

In some embodiments, wherein aligning the cannula passage about the targeted area comprises using the obturator to facilitate insertion of the cannula within the subject. In some embodiments, the BFT retrieves the bone fastener from the caddy system by inserting the one or more spikes through the first and third elongated shafts of the bone fastener. In some embodiments, coupling the bone fastener with the implant comprises placing the implant on the implant recess on the caddy system prior to passing the bone fastener through the implant. In some embodiments, inserting the soft tissue fastener through the implant and the soft tissue is via the SFT. In some embodiments, the method further comprises inserting the SFT through the cannula passage for insertion of the soft tissue fastener. In some embodiments, the method further comprises rotating the handle of the BFT so as to cause the pusher to sweep clockwise and/or counterclockwise to further facilitate positioning of the implant about the targeted area.

A composite medical textile includes a plurality of bioresorbable hyaluronan-based fibers and a plurality of non-resorbable fibers. The bioresorbable hyaluronan-based fibers and the non-resorbable fibers can be joined together by at least one selected from the group consisting of braided, knitted, adhered, intermeshed, weaved, interlocked, twisted, and heat set. The medical textile can include from 10 % to 98 % non-resorbable fibers, based on the weight of the non-resorbable fibers to the total weight of the non-resorbable suture fibers and bioresorbable hyaluronan-based polymer fibers.

The hyaluronan-based fibers can include at least one selected from the group consisting of hyaluronic acid, sodium hyaluronate, and esters of hyaluronic acid. The esters of hyaluronic acid can include benzyl esters of hyaluronic acid.

The non-resorbable fibers can include at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

The medical textile can further include bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxybutyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

The hyaluronan-based bioresorbable polymer fibers can be prepared by at least one selected from the group consisting of ring spinning, air-jet spinning, open-end spinning, mule spinning, wet spinning, dry spinning, electrospinning, pneumatospinning, pultrusion, and extrusion. The extrusion can be at least one selected from the group consisting of solvent exchange extrusion, precipitation extrusion, phase exchange extrusion, and phase change extrusion.

The bioresorbable hyaluronan-based fiber can include at least one selected from the group consisting of mono- and multifilaments. The composite medical textile can have a diameter of from 25 $\mu$m to about 5000 $\mu$m.

The composite medical textile can include at least one active agent. The active agent can be an antimicrobial agent. The antimicrobial agent can include at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

The active agent can be an analgesic. The analgesic can include at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine.

The active agent can include a vasoconstrictive agent. The vasoconstrictive agent can include at least one selected from the group consisting of epinephrine, alpha-adrenoreceptor antagonists, vasopressin analogues, norepinephrine, phenylephrine, dopamine, dobutamine, serotonin agonists, and triptans.

At least one of the non-resorbable fibers and the bioresorbable hyaluronan-based fibers can be wetted with the active agent. At least one of the non-resorbable fibers and the bioresorbable hyaluronan-based fibers can be coated with the active agent. At least one of the non-resorbable fibers and the bioresorbable hyaluronan-based fibers can have the active agent embedded within the fibers.

The composite medical textile can be circular in cross section. The composite medical textile can be a tape and can have a cross section comprising at least one selected from the group consisting of rounded rectangular, marquise, oblong and oval.

A method of making a medical textile can include the steps of providing a plurality of non-resorbable fibers, and providing a plurality of bioresorbable hyaluronan-based fibers. The non-resorbable fibers and the bioresorbable hyaluronan-based fibers are joined into a composite medical textile. The medical textile can include from 20 % to 80 % bioresorbable hyaluronan-based fibers, based on the total number of non-resorbable fibers and bioresorbable hyaluronan-based fibers.

The composite medical textile can further comprise an active agent, the active agent comprising at least one selected from the group consisting of antimicrobial agents, analgesic agents, and vasoconstrictive agents. The method can include the step of coating the active agent onto at least one selected from the group consisting of the bioresorbable hyaluronan-based fibers and the non-resorbable fibers. The coating step can include at least one selected from the group consisting of a dip, brush, spray, or curtain coating process.

The active agent can be impregnated into at least one selected from the group consisting of the bioresorbable hyaluronan-based fibers and the non-resorbable fibers by passing the extruded fiber through a solution containing the active agent. The active agent can be impregnated into at least one selected from the group consisting of the bioresorbable hyaluronan-based fibers and the non-resorbable fibers by co-extrusion, wherein a fiber precursor and the active agent are combined into a homogeneous mixture and co-extruded into a fiber. At least one of the bioresorbable hyaluronan-based fibers and the non-resorbable fibers can be wetted with the active agent.

A method for repairing a portion of a mammalian body can include the steps of providing a composite medical textile which comprises a plurality of non-resorbable fibers and a plurality of bioresorbable hyaluronan-based fibers, and connecting the textile between two tissue portions of the mammalian body. The tissue portions can include at least one selected from the group consisting of bony tissue and soft tissue. The medical textile can be a suture, and the method can further include the step of manipulating the suture in a suturing process to suture the tissue portions of the mammalian body. The method can also include the step of seeding the medical textile with stem cells.

A medical device according to the invention can include a plurality of bioresorbable hyaluronan-based fibers and a plurality of non-resorbable fibers. The medical device can be an orthopedic attachment system which comprises at least one flexible connector comprising bioresorbable hyaluronan-based fibers joined with a plurality of non-resorbable fibers, and at least one orthopedic attachment device. The medical device can be tubes, membranes, nonwoven fabrics, gauzes, sponges, and/or sutures. The medical device can be a tissue scaffold.

This disclosure includes fasteners for coupling an implant to tissue (e.g., soft-tissue and/or bone), fabric-like implants, and assemblies with fasteners pre-loaded with implants. The present fasteners generally include at least one barbed shaft and an enlarged head spaced from a distal end of the shaft. Some of the present fasteners include two barbed shafts and an enlarged head spanning the two shafts. The present implants generally comprise at least one flexible, fibrous layer that is substantially planar in a flattened state. This disclosure also includes kits that comprise a plurality of fasteners pre-loaded with implants. Some of the present kits also include one or more of the present fastener-delivery apparatuses or tools; for example, a plurality of pre-loaded fasteners with a single, reloadable tool; a plurality of tools each pre-loaded with a fastener that is pre-loaded with an implant; and/or a plurality of cartridges each pre-loaded with a fastener that is pre-loaded with an implant, and a common tool for use with the cartridges. In some of the present embodiments, the implant comprises a woven layer, and a nonwoven layer coupled to the woven layer (e.g., via stitches). In some configurations of the present implants, the implant further comprises at least one suture leg (e.g., two suture legs each) coupled to the implant at a point that is closer to the second end edge than to the first end edge, the suture leg having a free portion that is configured to extend beyond the second end edge.

In some embodiments of the present assemblies for delivery of a fastener, the assembly comprises fastener cartridge, a fibrous implant wrapped around a portion of the cartridge, a fastener extending through the implant, and an elongated shield disposed around the implant and the cartridge such that the implant is retained between the cartridge and the shield. The implant is flexible and has a first end edge, a second end edge, and a pair of lateral edges extending between the first and second end edges, where the implant is substantially planar when in a flattened state (e.g., in which the first end edge is separated from the second end edge under a first tension of 5 Newtons (N) applied perpendicular to each of the end edges, and in which the lateral edges are separated from each other under a tension of 5 N applied perpendicular to each of the lateral edges).

In some embodiments of such assemblies, the cartridge includes a body with a proximal end, and a distal end, the proximal end configured to be removably coupled to a distal end of an apparatus to deliver a fastener coupled to the cartridge, and the cartridge comprises: a spike having a spike proximal end coupled to the distal end of the cartridge, and a spike distal end extending from the distal end of the cartridge, the spike proximal end having a transverse dimension that is smaller than a transverse dimension of the distal end of the cartridge such that a shoulder is defined at the spike proximal end, the shoulder configured to abut a head of a fastener received over the spike. In other embodiments of such assemblies, the cartridge includes a body with a proximal end, and a distal end, the proximal end configured to be removably coupled to a distal end of an apparatus to deliver a fastener coupled to the cartridge, and the cartridge comprises: a pair of spikes each having a spike proximal end coupled to the distal end of the cartridge, and a spike distal end extending from the distal end of the cartridge, the spike proximal end having a transverse dimension that is smaller than a corresponding transverse dimension of the distal end of the cartridge body such that a shoulder is defined at the spike proximal end, the shoulder configured to abut a head of a fastener received over the spikes.

The fastener is received over the spike(s) of the cartridge. In some embodiments in which the cartridge has a single spike, the fastener comprises: an elongated shaft having a shaft length extending from a shaft proximal end to a shaft distal end, and defining an internal passage extending along the shaft length; an enlarged head coupled to the proximal end of the shaft and abutting the shoulder of the cartridge; a plurality of outriggers extending from the enlarged head in a direction away from the internal passage; and a plurality of first barbs each extending along a portion of the shaft length, each first barb having a leading edge and trailing edge spaced from the proximal end of the shaft, the leading edge of each first barb being disposed between the trailing edge of that first barb and the distal end of the shaft; where the shaft, head, outriggers, and first and second barbs are defined by a unitary piece of polymer.

In other embodiments in which the cartridge has two spikes, the fastener comprises: a first elongated shaft having a first shaft length extending from a first shaft proximal end to a first shaft distal end, and defining an internal first passage extending along the first shaft length; a second elongated shaft having a second shaft length extending from a second shaft proximal end to a second shaft distal end, and defining an internal second passage extending along the second shaft length; an enlarged head coupled to the proximal ends of the first shaft and the second shaft, the had abutting the shoulder of the cartridge; a plurality of first barbs each extending along a portion of the first shaft length, each first barb having a leading edge and trailing edge spaced from the proximal end of the first shaft, the leading edge of each first barb being disposed between the trailing edge of that first barb and the first shaft distal end; a plurality of second barbs each extending along a portion of the second shaft length, each second barb having a leading edge and trailing edge spaced from the proximal end of the second shaft, the leading edge of each second barb being disposed between the trailing edge of that second barb and the second shaft distal end; and where the shafts, head, and barbs are defined by a unitary piece of polymer.

The elongated, tubular shield of the present assemblies is disposed over the implant, at least a portion of the fastener, and at least a portion of the cartridge, and the shaft of the fastener extends through the implant at a point that is closer to the first end edge than to the second end edge, a first portion of the implant extends proximally around at least a portion of the cartridge, and the first portion of the implant is disposed between the cartridge and the shield.

Some implementations of the present methods utilize one of the present assemblies and an apparatus having a distal end coupled to a proximal end of the fastener body, and the method comprises: inserting the shaft(s) of the fastener into a first portion of tissue (e.g., bone) of a patient; retracting the shield to a position that permits the second end edge of the implant to exit the shield; spreading the implant along a second portion of tissue (e.g., soft tissue such as tendon) of the patient; coupling the implant to the second portion of tissue; and decoupling the cartridge from the fastener such that the spike of the cartridge is removed from the internal passage of the shaft of the fastener. In some implementations, spreading the implant comprises pulling the first and/or second suture leg of the implant laterally away from the fastener.

Some embodiments of the present kits comprise a plurality of the present assemblies, for example, that are sterile and sealed in a package (e.g., a tray).

Described herein, in other embodiments (e.g. for an implant described herein) are sutures, including composite sutures. Historically, sutures have been made of many different materials and it remains desirable to use a particular suture material and/or configuration in a specific application. For example, sutures are sometimes used in conjunction with fixturing devices (commonly referred to as suture anchors) that are now available for locating sutures in proximity to bone tissue so as to allow a coupling of soft tissue adjacent to the bone.

Described herein, in some aspects, is a system for delivery of an implant, the system comprising: the implant comprising a plurality of bioresorbable hyaluronan-based fibers and a plurality of non-resorbable fibers; a bone fastener configured to couple the implant to bone, the bone fastener having a first elongated shaft, a first enlarged head, and one or more first barbs at least partially extending laterally from the first elongated shaft; a soft-tissue fastener configured to couple the implant to soft tissue the soft-tissue fastener having a second elongated shaft, a second enlarged head, and one or more second barbs at least partially extending laterally from the second elongated shaft; a soft-tissue fastener tool (SFT) configured to couple with and deliver the soft-tissue fastener through the implant and soft-tissue; a bone fastener tool (BFT) configured to couple with and deliver the bone fastener and the implant, the BFT further configured to at least partially spread the implant over a targeted area in the subject after delivery; and a implant delivery tool comprising a shaft a channel therein, the channel configured to receive the BFT coupled with the bone fastener and implant therethrough, such that the BFT is configured to secure the implant about the targeted area via passing through a distal opening of the implant delivery tool to insert the bone fastener to a bone with the implant located therebetween. In some embodiments, the system further comprises a caddy configured to store one or both of the soft-tissue fastener and the bone fastener, wherein the caddy is configured to receive the implant to have the bone fastener passed therethrough. In one embodiment, the invention includes a multicomponent suture that includes a first substantially elongate suture portion with a first cross-sectional profile, where the first substantially elongate suture portion includes a first substantially bioabsorbable material. The multicomponent suture also includes a second substantially elongate suture portion with a second cross-sectional profile. The second substantially elongate suture portion includes a second substantially non-bioabsorbable material. The first and second substantially elongate suture portions are coupled to one another at respective mutually adjacent ends so as to a form an integrated suture with a first region that is substantially bioabsorbable and a second region that is substantially non-bioabsorbable.

The following description is provided to enable any person skilled in the art to make and use the disclosed inventions and sets forth the best modes presently contemplated by the inventor of carrying out his inventions. In the following description, for purposes of explanation, numerous specific details are set forth in order to provide a thorough understanding of the present invention. It will be apparent, however, to one skilled in the art that the present invention may be practiced without these specific details. In other instances, well-known structures and devices are shown in block diagram form in order to avoid unnecessarily obscuring the substance disclosed. These and other advantages and features of the invention will be more readily understood in relation to the following detailed description of the invention, which is provided in conjunction with the accompanying drawings.

It should be noted that, while the various figures show respective aspects of the invention, no one figure is intended to show the entire invention. Rather, the figures together illustrate the invention in its various aspects and principles. As such, it should not be presumed that any particular figure is exclusively related to a discrete aspect or species of the invention. To the contrary, one of skill in the art would appreciate that the figures taken together reflect various embodiments exemplifying the invention.

Correspondingly, references throughout the specification to "one embodiment" or "an embodiment" means that a particular feature, structure, or characteristic described in connection with the embodiment is included in at least one embodiment of the present invention. Thus, the appearance of the phrases "in one embodiment" or "in an embodiment" in various places throughout the specification are not necessarily all referring to the same embodiment. Furthermore, the particular features, structures, or characteristics may be combined in any suitable manner in one or more embodiments.

In some embodiments, systems and methods described herein relate to a bioabsorbable textile for joint function restoration, wherein the bioabsorbable textile comprises polymeric yarns, wherein said bioabsorbable textile provides a combined mechanical and biological augmentation in the target joint tissue, and wherein at least one of said polymeric yarns comprises a hyaluronic acid derivative.

In one embodiment, said hyaluronic acid derivative is an ester, more preferably a benzyl ester. When compared to all the other solutions so far proposed to solve the problem, the present invention is the only one able to lead to a functional joint tissue restoration due to the combination of all the ideal features a soft tissue healing requires, such as (i) augmenting the mechanical properties to immediately match the behavior of the native joint tissue, (ii) simultaneously promoting host cell-mediated healing by mimicking the peculiar ultrastructural organization of the native joint tissues (iii) slowly resorbing to allow gradual replacement by new tissue, and (iv) finally integrating with the host joint tissue by promoting the regeneration of the native tissue.

Certain aspects of systems and methods described herein was made possible by the unexpected and surprising finding that it is possible to create mechanically-robust, bioabsorbable textiles from hyaluronic acid (HA) derivatives, thereby leveraging their unique properties that make them perfectly suitable for the target application - i.e.restoring joint tissue function.

HA derivatives and their advantages, with a particular relevance for benzyl esters of HA and more in particular for full benzyl esters of HA have already been described elsewhere (US4965353, WO93/11805, WO94/03212, US6482231, WO99/61080 and WO99/65534). For the purpose of this invention, it is important to note how HA needs to be derivatized in a hydrophobic form to make it workable as a solid with a plurality of technologies, such as spinning, weaving, knitting, embroidery, etc.

The present invention is capable to meet all the above-mentioned design inputs so representing an innovative solution to effectively treat joint tissue tears.

In some cases, the applicant has unexpectedly found a tailored combination of device design and raw material that are capable to provide (i) an immediate augmentation of the mechanical reinforcement of the lesion site, (ii) a temporary structure mimicking the native architecture of the target joint tissues, (iii) a sustained pro-regenerative environment during the joint tissue healing, and (iv) an *in vivo* degradation matching the new tissue formation.

Therefore, some aspects of the present invention relates a bioabsorbable textile for joint function restoration, wherein the bioabsorbable textile comprises polymeric yarns, wherein said bioabsorbable textile provides a combined mechanical and biological augmentation in the target joint tissue, and wherein at least one of said polymeric yarns comprises a hyaluronic acid derivative.

In some embodiments, said hyaluronic acid derivative is an ester, more preferably a benzyl ester. In another aspect the invention relates to the bioabsorbable textile of the invention for use in restoring joint function affected by partial thickness tears, small to medium full-thickness tears, large to massive full-thickness tears, acute and chronic/degenerative tears.

In a still another aspect the invention relates to the bioabsorbable textile of the invention for use in surgery in combination with fixation tools during open, mini-open or arthroscopic repair/augmentation procedures of joint tissue tears.

Without being bound to any theory the inventors deem that the present textile allows a biological and mechanical augmentation in the joint target tissue due not only to its polymeric nature but also to the configuration designs as it will be evident from the experimental part.

In an advantageous embodiment the preferred leno-weave or knitted configurations combined together with the use of a hyaluronic acid derivative, preferably benzyl ester of hyaluronic acid, make the bioabsorbable textile especially suitable for mechanical augmentation of the target joint.

The textile of the invention could be also considered as a new biomaterial in view of its mechanical and biological properties conferred to the target joint.

In a further aspect the invention relates to a multilayer structure for joint function restoration comprising at least one bioabsorbable textile according to the invention.

It is known that an appropriate mechanical reinforcement of the lesion site not coupled with a proper device degradation may result either in long-term safety issues, should the device be present *in vivo* too long (e.g. >6 months) or in implant failure (re-tear), should the device be present *in vivo* too short (e.g. <1 month). It is known that an appropriate mechanical reinforcement of the lesion site not coupled with a remodeled and mature tissue formation may result in the formation of sub-optimal fibrotic tissue that will ultimately lead to the implant failure (re-tear).

On the other hand, it is known that a sustained tissue regeneration not coupled with a matching device degradation may result either in long-term safety issues and/or in histologically-unacceptable outcome, should the device be present *in vivo* too long (e.g. >6 months) or in implant failure (re-tear), should the device be present *in vivo* too short (e.g. <1 month).

It appears evident how the only, though not obvious, solution associated with the highest chance to succeed clinically would be provided by a novel device capable to offer all these features at the same time.

This disclosure includes various apparatuses, kits, systems, and methods for simultaneous delivery of a fastener and an implant.

In some configurations of the present apparatuses for simultaneous delivery of a fastener and an implant, the apparatus comprises: an elongated body, one or more spikes, a hub, a handle, an elongated pusher, and a slider. In some such configurations, the elongated body extends from a body proximal end to a body distal end, the body defines a body proximal opening through the body proximal end, a body recess in a peripheral surface of the body at point that is offset from the body distal end, and a body passage extending between the body proximal opening and a body distal opening within the body recess. In some such configurations, the one or more spikes each have a spike proximal end coupled to the body distal end, and a spike distal end extending from the body distal end, and the spike proximal end has a transverse dimension that is smaller than a transverse dimension of the body distal end such that a shoulder is defined at the spike proximal end, where the shoulder is configured to abut a head of a fastener received over the spike. In some such configurations, the hub has a hub proximal end and a hub distal end, and the hub configured to receive the proximal end of the body through the hub distal end, and the handle si coupled to the hub proximal end. In some such configurations, the elongated pusher has a pusher proximal end and a pusher distal end, and the pusher is disposed in the body passage such that the pusher distal end is disposed beyond the body distal opening. In some such configurations, the slider is coupled to the pusher proximal end, and is configured to move longitudinally relative to the handle from a retracted position to a deployed position to move the pusher distal end longitudinally away from the body distal opening and laterally outward from the body.

In some configurations of the present apparatuses, the one or more spikes comprise a pair of spikes, where each of the pair of spikes are parallel to and laterally spaced from the other of the pair of spikes.

In some configurations of the present apparatuses, the hub is coupled in fixed relation to the body, and the handle is rotatable relative to the hub to cause the pusher distal end to rotate relative to the body. In some such configurations, apparatus is configured such that moving the slider longitudinally relative to the handle, or rotating the handle relative to the hub, will move the distal end of the pusher independent of the body distal end.

In some configurations of the present apparatuses, the body recess defines a sloped surface spaced from the body distal opening, and where the apparatus is configured such that, when the slider is moved from the retracted position to the deployed position, a flexible portion of the pusher contacts the sloped surface to cause the pusher distal end to extend laterally outward from the body.

In some configurations of the present apparatuses, the pusher comprises a roller coupled to the pusher distal end.

Some configurations of the present apparatuses further comprise a seal assembly comprising: an annular seal body and a resilient membrane. In some such configurations, the annular seal body having a seal proximal end and a seal distal end, the seal body defines a seal passage extending through and between the seal proximal end and the seal distal end, and a tapered distal surface disposed around the seal passage at the seal distal end. In some such configurations, the resilient membrane is disposed at least around an interior periphery of the seal passage, and is configured to cooperate with the body of the apparatus to substantially seal the seal passage when the body of the apparatus extends through the resilient membrane and the seal passage. In some such configurations, the elongated body of the apparatus is disposed through the resilient membrane of the seal and the seal passage with the seal distal end facing the body distal end.

In some configurations of the present systems, the system comprises: one or more of the present apparatuses comprising a seal assembly, and a cannula. In some such configurations, the cannula comprises a cannula distal end, a cannula proximal end configured to be coupled to the seal body, and a cannula passage extending through and between the cannula proximal end and the cannula distal end, the cannula further defining a cannula proximal surface tapered distally and inward toward the cannula passage, the cannula proximal surface configured to cooperate with the seal distal surface to provide a seal between the cannula proximal surface and the seal distal surface. In some such configurations, an outer surface of the cannula includes one or more threads that are configured to engage soft tissue as the cannula is rotated.

In some configurations of the present systems, a proximal end of the cannula includes an enlarged portion that is configured to be engaged by a user to vary the depth and/or direction of the cannula.

In some configurations of the present systems, the cannula distal end defines a notch configured to permit a portion of the pusher to deflect radially outward through the notch.

Some configurations of the present systems further comprise: an obturator having an elongated body with a obturator proximal end and a tapered obturator distal end, the obturator body configured to extend through the cannula such that the obturator distal end extends distally beyond the cannula distal end to facilitate insertion of the cannula through soft tissue of a patient.

In some configurations of the present caddy systems, the caddy system comprises: a base defining one or more first fastener recesses; and a lid coupled to the base and configured to be rotated relative to the base to selectively cover or expose the first fastener recess(es).

In some configurations of the present caddy systems, the base further defines one or more implant recesses each overlapping at least one of the one or more first fastener recesses, where the lid is configured to be rotated relative to the base to selectively cover or expose the implant. In some such configurations, the one or more implant recesses includes a substantially planar lower surface offset below a substantially planar upper surface of the base.

Some configurations of the present caddy systems further comprise: a flexible, fibrous implant disposed over at least one of the first fastener recesses; and a first fastener extending through the implant and into the at least one of the first fastener recesses; where the lid is rotatable, relative to the base, over the first fastener and at least a portion of the implant.

In some configurations of the present caddy systems, the body further defines one or more second fastener recesses, where the lid is configured to be rotated relative to the base to selectively cover or expose the second fastener recess(es). Some such configurations further comprise: a second fastener extending into at least one of the second fastener recesses; where the lid is rotatable, relative to the base, over the second fastener.

In some implementations of the present methods, the method comprises: disposing a shield distal end of one of the present apparatuses apparatus adjacent a bone of a patient, where the slider is in the retracted position, where a fastener is disposed over the one or more spikes, and the fastener extends through a flexible, fibrous implant; inserting the fastener into the bone to secure a first portion of the implant to the bone; and moving the slider from the retracted position toward the deployed position to urge a second portion of the implant away from the body of the apparatus and toward a soft tissue structure of the patient.

In other implementations of the present methods, the method comprises: inserting the elongated body of one of the present apparatuses through a cannula that is disposed through soft tissue of a patient, the cannula comprising a cannula distal end, a cannula proximal end configured to be coupled to the seal body, and a cannula passage extending through and between the cannula proximal end and the cannula distal end, the cannula further defining a cannula proximal surface tapered distally and inward toward the cannula passage, the cannula proximal surface configured to cooperate with the seal distal surface to provide a seal between the cannula proximal surface and the seal distal surface. In some such implementations, during insertion, a seal assembly is disposed around the elongated body of the apparatus, the seal assembly comprising: an annular seal body having a seal proximal end and a seal distal end, the seal body defining a seal passage extending through and between the seal proximal end and the seal distal end, and a tapered distal surface disposed around the seal passage at the seal distal end; and a resilient membrane disposed at least around an interior periphery of the seal passage, the resilient membrane configured to cooperate with the body of the apparatus to substantially seal the seal passage when the body of the apparatus extends through the resilient membrane and the seal passage. Some such implementations further comprise: coupling the seal body to the proximal end of the cannula such that the seal distal surface cooperates with the cannula proximal surface to provide a seal between the cannula proximal surface and the seal distal surface. Some such implementations further comprise: after moving the slider from the retracted position toward the deployed position, rotating the handle relative to the hub to urge one or both of two lateral edges of the second portion of the implant toward the soft tissue structure. Some such implementations further comprise: inserting a fastener through the second portion of the implant and into the soft tissue structure to couple the second portion of the implant to the soft tissue structure.

In other implementations of the present methods, the method comprises: inserting the elongated body of one of the present apparatuses through the resilient membrane and the seal passage of one of the present seals such that the seal distal end faces the body distal end. Some such implementations further comprise: inserting, while the elongated body is disposed through the resilient membrane and seal passage, a fastener disposed over the one or more spikes through a flexible, fibrous implant. Some such implementations further comprise: inserting the spikes of the apparatus through a fastener that is disposed through a flexible, fibrous implant.

In some configurations of the present kits, the kit comprises: one or more of the present apparatuses and/or one or more of the present systems; and one or more of the present caddy systems.

Described herein, in yet other aspects, is a multicomponent suture comprising first and second substantially elongate suture portions with respective first and second cross-sectional profiles, and further including respective first and second materials, one of said materials being bioabsorbable and a second of said materials being non-bioabsorbable. The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any embodiment of the present apparatuses, kits, and methods, the term "substantially" may be substituted with "within [a percentage] of' what is specified, where the percentage includes 0.1, 1, 5, and/or 10 percent.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, an apparatus or kit that "comprises," "has," "includes" or "contains" one or more elements possesses those one or more elements, but is not limited to possessing only those elements. Likewise, a method that "comprises," "has," "includes" or "contains" one or more steps possesses those one or more steps, but is not limited to possessing only those one or more steps.

Further, an apparatus, device or system that is configured in a certain way is configured in at least that way, but it can also be configured in other ways than those specifically described.

Any embodiment of any of the present apparatuses and methods can consist of or consist essentially of - rather than comprise/include/contain/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

BRIEF DESCRIPTION OF THE SEVERAL VIEWS OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiment depicted in the figures.

FIG. 1A depicts a perspective view of a first embodiment of the present soft-tissue fasteners.

FIG. 1B depicts a distal end view of the fastener of FIG. 1A.

FIG. 1C depicts a first side view of the fastener of FIG. 1A.

FIG. 1D depicts a second side view of the fastener of FIG. 1A.

FIG. 1E depicts a proximal end view of the fastener of FIG. 1A.

FIG. 1F depicts the side cross-sectional view of the fastener of FIG. 1A, along the plane F-F of FIG. 1E.

FIG. 1G depicts the side cross-sectional view of the fastener of FIG. 1A, along the plane G-G of FIG. 1E.

FIG. 1H depicts the side cross-sectional view of the fastener of FIG. 1A, along the plane H-H of FIG. 1E.

FIG. 2A depicts a perspective view of a first embodiment of the present bone fasteners.

FIG. 2B depicts a distal end view of the fastener of FIG. 2A

FIG. 2C depicts a side view of the fastener of FIG. 2A.

FIG. 2D depicts proximal end view of the fastener of FIG. 2A.

FIG. 2E depicts a side cross-sectional view along the plane D-D of FIG. 2C.

FIG. 2F depicts a side view of a variation of the fastener of FIG. 2A.

FIG. 3A depicts a perspective view of a first embodiment of a tool for delivery of a fastener of FIG. 1A or FIG. 2A, with a fastener of FIG. 2A coupled to a distal end of the tool.

FIG. 3B depicts a side view of the tool of FIG. 3A, shown without the fastener of FIG. 2A.

FIG. 3C depicts a side cross-sectional view of the tool of FIG. 3A, along the plane C-C of FIG. 3B.

FIG. 3D depicts a side view of the tool of FIG. 3A with a shield of the tool omitted.

FIG. 3E depicts the tool of FIG. 3A, with the shield included and disposed in an extended position covering the fastener.

FIG. 4A depicts a perspective view of a second embodiment of a tool for delivery of a fastener of FIG. 1A or FIG. 2A, with a fastener of FIG. 2A coupled to a distal end of the tool.

FIG. 4B depicts a side view of the tool of FIG. 4A, shown without the fastener of FIG. 2A.

FIG. 4C depicts a side cross-sectional view of the tool of FIG. 4A, along the plane C-C of FIG. 4B.

FIG. 4D depicts the tool of FIG. 4A, with the shield disposed in an extended position covering the fastener.

FIG. 5A depicts a perspective view of a third embodiment of a tool for delivery of a fastener of FIG. 1A or FIG. 2A via a cartridge, with a fastener of FIG. 2A coupled to a distal end of the cartridge and the cartridge coupled to a distal end of the tool.

FIG. 5B depicts a side view of the tool of FIG. 5A, shown without the fastener of FIG. 2A.

FIG. 5C depicts a side cross-sectional view of the tool of FIG. 5A, along the plane C-C of FIG. 5B.

FIG. 5D depicts a perspective view of the distal end of the tool, without the cartridge but with a guidewire and a trocar.

FIG. 5E depicts an enlarged side view of a fastener cartridge of the tool of FIG. 5A with a shield of the tool omitted.

FIG. 5F depicts an enlarged lower perspective view of the fastener cartridge of FIG. 5E.

FIG. 5G depicts a cutaway perspective view of the fastener cartridge of FIG. 5E.

FIG. 6A depicts a perspective view of a second embodiment of the present soft-tissue fasteners.

FIG. 6B depicts a side view of the fastener of FIG. 6A.

FIG. 6C depicts a distal end view of the fastener of FIG. 6A.

FIG. 6D depicts a proximal end view of the fastener of FIG. 6A.

FIG. 6E depicts side cross-sectional views of the fastener of FIG. 6A, along the planes E-E of FIG. 6D.

FIG. 6F depicts side cross-sectional views of the fastener of FIG. 6A, along the planes F-F of FIG. 6D.

FIG. 7A depicts a perspective view of an embodiment of a tool for delivery of a fastener of FIG. 6A, with a fastener of FIG. 6A coupled to a distal end of the tool.

FIG. 7B depicts a side view of the tool of FIG. 7A.

FIG. 7C depicts a side cross-sectional view of the tool of FIG. 7A, along the plane C-C of FIG. 7B.

FIG. 7D depicts an additional side view of the tool of FIG. 3A.

FIG. 8A depicts a perspective view of a second embodiment of the present bone fasteners.

FIG. 8B depicts a distal end view of the fastener of FIG. 8A

FIG. 8C depicts a proximal end view of the fastener of FIG. 8A.

FIG. 8D depicts a first side view of the fastener of FIG. 8A.

FIG. 8E depicts a second side view of the fastener of FIG. 8A.

FIG. 9A depicts a perspective view of an embodiment of a tool for delivery of a fastener of FIG. 8A via a cartridge, with a fastener of FIG. 8A coupled to a distal end of the cartridge, and the cartridge coupled to a distal end of the tool.

FIG. 9B depicts a side view of the tool of FIG. 9A.

FIG. 9C depicts a side cross-sectional view of the tool of FIG. 9A, along the plane C-C of FIG. 9B.

FIG. 9D depicts an enlarged first side view of a fastener cartridge of the tool of FIG. 9A.

FIG. 9E depicts an enlarged second side view of the fastener cartridge of the tool of FIG. 9A.

FIG. 9F depicts a distal end view of the tool of FIG. 9A, with the bone fastener coupled to the cartridge.

FIG. 9G depicts a distal end view of the tool of FIG. 9A, with the bone fastener omitted.

FIG. 10A depicts an exemplary step for a sequence of deployment of a fastener of FIG. 8A via the tool of FIG. 9A.

FIG. 10B depicts an exemplary step for a sequence of deployment of a fastener of FIG. 8A via the tool of FIG. 9A.

FIG. 10C depicts an exemplary step for a sequence of deployment of a fastener of FIG. 8A via the tool of FIG. 9A.

FIG. 10D depicts an exemplary step for a sequence of deployment of a fastener of FIG. 8A via the tool of FIG. 9A.

FIG. 10E depicts an exemplary step for a sequence of deployment of a fastener of FIG. 8A via the tool of FIG. 9A.

FIG. 10F depicts an exemplary step for a sequence of deployment of a fastener of FIG. 8A via the tool of FIG. 9A.

FIG. 10G depicts an exemplary step for a sequence of deployment of a fastener of FIG. 8A via the tool of FIG. 9A.

FIG. 10H depicts an exemplary step for a sequence of deployment of a fastener of FIG. 8A via the tool of FIG. 9A.

FIG. 10I depict an exemplary step for a sequence of deployment of a fastener of FIG. 8A via the tool of FIG. 9A.

FIG. 10J depicts an exemplary step for a sequence of deployment of a fastener of FIG. 8A via the tool of FIG. 9A.

FIG. 10K depicts an exemplary step for a sequence of deployment of a fastener of FIG. 8A via the tool of FIG. 9A.

FIG. 11 depicts a perspective view of a number of examples of trays or caddies each configured to hold a plurality of the present fasteners.

FIG. 12A depicts a perspective, cutaway view of the connection of a damaged supraspinatus tendon to the humerus in a human shoulder.

FIG. 12B depicts a perspective, cutaway view of the connection of a damaged supraspinatus tendon to the humerus in a human shoulder, showing a repair in accordance with an example of the present methods.

FIG. 13A depicts a side view of a third embodiment of the present soft-tissue fasteners.

FIG. 13B depicts a side cross-sectional view of the fastener of FIG. 13A, taken along the plane B-B of FIG. 13A.

FIG. 14A depicts a side view of a fourth embodiment of the present soft-tissue fasteners.

FIG. 14B depicts a distal end view of the fastener of FIG. 14A.

FIG. 15A depicts a side view of a fifth embodiment of the present soft-tissue fasteners.

FIG. 15B depicts a distal end view of the fastener of FIG. 15A.

FIG. 16A depicts a side view of a sixth embodiment of the present soft-tissue fasteners.

FIG. 16B depicts a distal end view of the fastener of FIG. 16A.

FIG. 17A depicts a side view of a seventh embodiment of the present soft-tissue fasteners.

FIG. 17B depicts a distal end view of the fastener of FIG. 16A.

FIG. 17C depicts a side cross-sectional view of the fastener of FIG. 19A, taken along the plane C-C of FIG. 17A.

FIG. 18A depicts a side view of an eighth embodiment of the present soft-tissue fasteners.

FIG. 18B depicts a distal end view of the fastener of FIG. 18A.

FIG. 19A depicts a side view of a ninth embodiment of the present soft-tissue fasteners.

FIG. 19B depicts a distal end view of the fastener of FIG. 19A.

FIG. 20 depicts a side view of a tenth embodiment of the present soft-tissue fasteners.

FIG. 21 depicts a side view of an eleventh embodiment of the present soft-tissue fasteners.

FIG. 22 depicts a side view of a twelfth embodiment of the present soft-tissue fasteners.

FIG. 23A depicts a perspective view of a fibrous, fabric-like implant for use with the present fasteners.

FIG. 23B depicts an end view of a fibrous, fabric-like implant for use with the present fasteners.

FIG. 23C depicts a plan view of a fibrous, fabric-like implant for use with the present fasteners.

FIG. 24 depicts one of the present implants in combination with a fastener of FIG. 8A-FIG. 8E.

FIG. 25 depicts one of the present implants in combination with a fastener of FIG. 2A-FIG. 2E.

FIG. 26A depicts a first side view of one of the present assemblies with the fastener and implant of FIG. 24 preloaded onto a cartridge of FIG. 9D - FIG. 9G with a shield around the implant and cartridge.

FIG. 26B depicts a second side view of one of the present assemblies with the fastener and implant of FIG. 24 preloaded onto a cartridge of FIG. 9D - FIG. 9G with a shield around the implant and cartridge.

FIG. 27 depicts the assembly of FIG. 26A and FIG. 26B coupled to an apparatus of FIG. 9A- FIG. 9C.

FIG. 28A depicts the deployment of a fastener from the assembly of FIG. 26A and FIG. 26B with the fastener not inserted into tissue for illustrative purposes.

FIG. 28B depicts the deployment of a fastener from the assembly of FIG. 26A and FIG. 26B with the fastener not inserted into tissue for illustrative purposes.

FIG. 28C depicts the deployment of a fastener from the assembly of FIG. 26A and FIG. 26B with the fastener not inserted into tissue for illustrative purposes.

FIG. 29A depicts the deployment of a fastener from the assembly of FIG. 26A and FIG. 26B.

FIG. 29B depicts the deployment of a fastener from the assembly of FIG. 26A and FIG. 26B.

FIG. 29C depicts the deployment of a fastener from the assembly of FIG. 26A and FIG. 26B.

FIG. 30 is a perspective view of a first embodiment of the present systems, including a cannula, an obturator, a seal assembly, and a delivery tool in a first configuration.

FIG. 31 is the cannula and the obturator of FIG. 30.

FIG. 32 is a proximal perspective view of the cannula of FIG. 30. FIG. 32 shows a proximal perspective view of cannula alone.

FIG. 33A is a proximal perspective view of the seal assembly of FIG. 30. FIG. 33A shows a proximal perspective view of seal assembly.

FIG. 33B is a distal perspective view of the seal assembly of FIG. 30. FIG. 33B shows a distal perspective view of seal assembly.

FIG. 34A is a perspective view of the delivery tool and seal assembly of FIG. 30, with the delivery tool in the a configuration.

FIG. 34B is a distal end view of the delivery tool and seal assembly of FIG. 34A.

FIG. 34C is a side cross-sectional view of the delivery tool and seal assembly of FIG. 34A.

FIG. 35 is a partially exploded, cross-sectional side view of the delivery tool of FIG. 30.

FIG. 36 is a side cross-sectional view of the delivery tool of FIGs. 34A-34C, shown in a second configuration.

FIG. 37A is a upper perspective view of a first embodiment of the present caddy systems shown pre-loaded with implants and fasteners.

FIG. 37B is a lower perspective view of a first embodiment of the present caddy systems shown pre-loaded with implants and fasteners.

FIG. 38 is a top view of a caddy base of the caddy system of FIGs. 35A-35B.

FIG. 39A is a top view of the caddy system of FIGs. 37A-37B with a lid of the caddy system in a first configuration relative to the base.

FIG. 39B is a side cross-sectional view of the caddy system of FIG. 39A. FIG. 39B depicts a side cross-sectional view of caddy system with the lid in the first configuration.

FIG. 40A is a top view of the caddy system of FIGs. 37A-37B with a lid of the caddy system in a second configuration relative to the base.

FIG. 40B is an exploded, side cross-sectional view of the caddy system of FIG. 40A. FIG. 40B depicts an exploded, side cross-sectional view of caddy system.

FIG. 41 is a perspective view of the delivery tool of FIG. 30 engaging a fastener of the caddy system of FIG. 37A - FIG. 37B . FIG. 41 -FIG. 43 depict an example of a method of deploying an implant and fastener from caddy system to a bone of a patient.

FIG. 42 is a perspective view of the delivery tool of FIG. 30 deploying a fastener to a bone of a patient to secure a first portion of an implant relative to the bone.

FIG. 43 is a perspective view of the delivery tool of FIG. 30, in the second configuration, deploying a second portion of the implant toward soft tissue.

FIG. 44A depicts a perspective view of various configurations of a distal end of a pusher of the delivery tool.

FIG. 44B depicts a perspective view of various configurations of a distal end of a pusher of the delivery tool.

FIG. 44C depicts a perspective view of various configurations of a distal end of a pusher of the delivery tool.

FIG. 44D depicts a perspective view of various configurations of a distal end of a pusher of the delivery tool.

FIG. 44E depicts a perspective view of various configurations of a distal end of a pusher of the delivery tool.

FIG. 44F depicts a perspective view of various configurations of a distal end of a pusher of the delivery tool.

FIG. 44G depicts a perspective view of various configurations of a distal end of a pusher of the delivery tool.

FIG. 44H depicts a perspective view of various configurations of a distal end of a pusher of the delivery tool.

FIG. 45A is a sequence of side views of a distal end of the delivery tool with a further embodiment of the pusher, illustrating the deployment of an implant.

FIG. 45B is a side view of a distal end of the delivery tool with a further embodiment of the pusher.

FIG. 46 is a perspective view of a further embodiment of the obturator of FIG. 30 and FIG. 31.

FIG. 47 is a distal perspective view of a further embodiment of the seal assembly of FIG. 30, FIG. 33A, and FIG. 33B.

FIG. 48 is a distal perspective view of a further embodiment of the seal assembly of FIG. 30, FIG. 33A, FIG. 33B , FIG. 45A, and FIG. 45B.

FIG. 49 is a distal perspective view of a further embodiment of the cannula of FIG. 30, FIG. 31, and FIG. 32.

FIG. 50 is a distal perspective view of a further embodiment of the cannula of FIG. 30 - FIG. 32 and FIG. 47.

FIG. 51A is an upper perspective view of a further embodiment of the caddy system of FIG. 37A - FIG. 40B.

FIG. 51B is an upper perspective view of a further embodiment of the caddy system of FIG. 37A - FIG. 40B.

FIG. 52A is an upper perspective view of a further embodiment of the caddy system of FIG. 51A and FIG. 51B.

FIG. 52B is an upper perspective view of a further embodiment of the caddy system of FIG. 51A and FIG. 51B.

FIG. 53 shows, in schematic form, a portion of a suture prepared according to principles of the invention.

FIG. 54A shows in schematic form, a portion of a suture prepared according to principles of the application in various states or stages of application, and illustrate a method according to the same.

FIG. 54B shows in schematic form, a portion of a suture prepared according to principles of the application in various states or stages of application, and illustrate a method according to the same.

FIG. 54C shows in schematic form, a portion of a suture prepared according to principles of the application in various states or stages of application, and illustrate a method according to the same.

FIG. 55 shows, in schematic form, a portion of a suture including three components, prepared according to principles of the invention.

FIG. 56A shows, in schematic form, a cross-sectional profile of a suture prepared according to principles of the invention.

FIG. 56B shows, in schematic form, a cross-sectional profile of a suture prepared according to principles of the invention.

FIG. 56C shows, in schematic form, a cross-sectional profile of a suture prepared according to principles of the invention.

FIG. 56D shows, in schematic form, a cross-sectional profile of a suture prepared according to principles of the invention.

FIG. 56E shows, in schematic form, a cross-sectional profile of a suture prepared according to principles of the invention.

FIG. 56F illustrates an aspect of the subject matter in accordance with one embodiment.

FIG. 56G shows, in schematic form, a cross-sectional profile of a suture prepared according to principles of the invention.

FIG. 56H shows, in schematic form, a cross-sectional profile of a suture prepared according to principles of the invention.

FIG. 56I shows, in schematic form, a cross-sectional profile of a suture prepared according to principles of the invention.

FIG. 56J shows, in schematic form, a cross-sectional profile of a suture prepared according to principles of the invention.

FIG. 56K shows, in schematic form, a cross-sectional profile of a suture prepared according to principles of the invention.

FIG. 56L shows, in schematic form, a cross-sectional profile of a suture prepared according to principles of the invention.

FIG. 56M shows, in schematic form, a cross-sectional profile of a suture prepared according to principles of the invention.

FIG. 56N shows, in schematic form, a cross-sectional profile of a suture prepared according to principles of the invention.

FIG. 56O shows, in schematic form, a cross-sectional profile of a suture prepared according to principles of the invention.

FIG. 56P shows, in schematic form, a cross-sectional profile of a suture prepared according to principles of the invention.

FIG. 57A shows, in schematic form, a respective cross-section of a portion of a respective suture prepared according to principles of the invention.

FIG. 57B shows, in schematic form, a respective cross-section of a portion of a respective suture prepared according to principles of the invention.

FIG. 57C shows, in schematic form, a respective cross-section of a portion of a respective suture prepared according to principles of the invention.

FIG. 57D shows, in schematic form, a respective cross-section of a portion of a respective suture prepared according to principles of the invention.

FIG. 57E shows, in schematic form, a respective cross-section of a portion of a respective suture prepared according to principles of the invention.

FIG. 58 shows, in schematic form, a portion of a suture prepared according to principles of the invention

FIG. 59 shows, in schematic cutaway form, a coupling portion of a suture prepared according to principles of the invention.

FIG. 60 shows, in schematic cutaway form, a coupling portion of a suture prepared according to principles of the invention.

FIG. 61 shows, in schematic cutaway form, a coupling portion of a suture prepared according to principles of the invention.

FIG. 62 shows, in schematic cutaway form, a coupling portion of a suture prepared according to principles of the invention.

FIG. 63 shows, in schematic form, a portion of a suture prepared according to principles of the invention including a suture anchor portion.

FIG. 64 shows, in schematic form, a portion of a suture prepared according to principles of the invention including a suture anchor portion.

FIG. 65 shows, in schematic form, a portion of a suture prepared according to principles of the invention including a needle portion.

FIG. 66A is a perspective schematic diagram of a suture anchor with a composite suture according to the invention.

FIG. 66B is a perspective schematic diagram of the suture anchor with a composite suture of indefinite length according to the invention.

FIG. 67A is a schematic diagrams of untwisted filament yarn.

FIG. 67B is a schematic diagrams of twisted filament yarn.

FIG. 67C is a schematic diagrams of high bulk filament yarn.

FIG. 68A is schematic diagram of a nonwoven medical textile according to the invention.

FIG. 68B is schematic diagram of a woven medical textile according to the invention.

FIG. 68C is schematic diagram of a braided medical textile according to the invention.

FIG. 68D is schematic diagram of a weft-kniwmedical textile according to the invention.

FIG. 68E is schematic diagram of a warp knit medical textile according to the invention.

FIG. 69A is a perspective view of a soft tissue anchor assembly according to the invention.

FIG. 69B is an expanded view of area 69B in FIG. 69A.

FIG. 69C is an expanded view of area 69C in FIG. 69A.

FIG. 70A is a perspective view of a rigid bone anchor assembly according to the invention, in a first mode of operation.

FIG. 70B is an expanded view of area 70B in FIG. 70A.

FIG. 71A is a perspective view of the rigid bone anchor assembly of FIG. 70 in a second mode of operation.

FIG. 71B is an expanded view of area 71B in FIG. 71A.

FIG. 72A is a side elevation of a joint repair assembly according to the invention.

FIG. 72B is an enlarged view of area 72B in FIG. 72A.

FIG. 73A is a side elevation of a tissue anchor according to the invention.

FIG. 73B is an enlarged view of area 73B in FIG. 73A.

FIG. 73C is an enlarged view of area 73C in FIG. 73A.

FIG. 74 is a side elevation of the tissue anchor of FIG. 73A, in a deployed mode of operation.

FIG. 75A is a side elevation of a braided medical textile according to the invention.

FIG. 75B is a cross-section taken along line 75B-75B in FIG. 75A.

FIG. 76 is an enlarged side elevation view of area FIG. 76 in FIG. 75A.

FIG. 77 is a schematic cross-section of a medical textile according to the invention in an initial mode of operation.

FIG. 78 is a schematic cross-section of the medical textile of FIG. 77 showing degradation of bioresorbable fibers and cell growth.

FIG. 79 is a schematic cross-section of a medical textile with a mixed array of hyaluronan-based bioresorbable fibers and non-resorbable fibers.

FIG. 80 is a schematic cross-section of the medical textile with an array of hyaluronan-based bioresorbable fibers surrounding non-resorbable fibers.

FIG. 81 is a schematic cross-section of a medical textile with an array of hyaluronan-based bioresorbable fibers surrounded by non-resorbable fibers.

FIG. 82 is a schematic cross-section of a medical textile in which hyaluronan based bioresorbable fibers of a larger diameter are mixed with resorbable fibers of a smaller diameter.

FIG. 83 is a schematic cross-section of a medical textile in which hyaluronan based bioresorbable fibers and non-resorbable fibers are mixed with another fiber which can be bioresorbable or non-resorbable.

FIG. 84 is a schematic cross-section of the medical textile surrounded by a coating of an active material.

FIG. 85 is a schematic cross-section of a medical textile in which one or both of the hyaluronan based bioresorbable fibers or non-resorbable fibers are coated with an active material.

FIG. 86 is a plot of knot pull strength (N) versus time (days) for a suture according to the invention and two prior art sutures.

FIG. 87 shows a typical tensile stress/strain relation for tendon/ligament.

FIG. 88A shows an embodiment of the bioabsorbable textile of the invention (leno-woven bioabsorbable textile), wherein the weft yarns equally outdistanced throughout the textile length with the pore length.

FIG. 88B shows another embodiment of the bioabsorbable textile of the invention (leno-woven bioabsorbable textile), wherein the weft yarns are equally distanced in the central region of the bioabsorbable textile.

FIG. 88C contains four schematic representations of embodiments of the textile of the of warp-knitted bioabsorbable textile Figure 88D is a schematic representation of preferred embodiments of the bioabsorbable textiles with single or a plurality of layers superimposed on each other, where each layer comprises textiles as described in Figure 88A and in Figure 88B or nonwoven pad.

FIG. 88D illustrates an aspect of the subject matter in accordance with one embodiment.

FIG. 89A shows the swelling behavior of four preferred embodiments of the textile of the invention, with reference to thickness as explained in Example 5.

FIG. 89B shows the swelling behavior of four preferred embodiments of the textile of the invention, with reference to volume as explained in Example 5.

FIG. 89C shows the swelling behavior of four preferred embodiments of the textile of the invention, with reference to percentage as explained in Example 5.

FIG. 90A shows the tensile behavior comparison between four preferred embodiments of the textile of the invention as explained in Example 7.

FIG. 90B shows the tensile behavior comparison between four preferred embodiments of the textile of the invention as explained in Example 7.

FIG. 90C shows the tensile behavior comparison between four preferred embodiments of the textile of the invention as explained in Example 7.

FIG. 90D shows the tensile behavior comparison between four preferred embodiments of the textile of the invention as explained in Example 7.

FIG. 91 shows the Maximum Load comparison among different textile configurations of the invention and two comparative devices as explained in Example 7.

FIG. 92A shows the tensile behavior comparison among four preferred embodiments of the textile of the invention and two comparative devices as explained in Example 7.

FIG. 92B shows the tensile behavior comparison among four preferred embodiments of the textile of the invention and two comparative devices as explained in Example 7.

FIG. 92C shows the tensile behavior comparison among four preferred embodiments of the textile of the invention and two comparative devices as explained in Example 7.

FIG. 92D shows the tensile behavior comparison among four preferred embodiments of the textile of the invention and two comparative devices as explained in Example 7.

FIG. 92E shows the tensile behavior comparison among four preferred embodiments of the textile of the invention and two comparative devices as explained in Example 7.

FIG. 92F shows the tensile behavior comparison among four preferred embodiments of the textile of the invention and two comparative devices as explained in Example 7.

FIG. 93A shows the fatigue behavior comparison between four preferred embodiments of the textile of the invention as explained in Example 8.

FIG. 93B shows the fatigue behavior comparison between four preferred embodiments of the textile of the invention as explained in Example 8.

FIG. 93C shows the fatigue behavior comparison between four preferred embodiments of the textile of the invention as explained in Example 8.

FIG. 93D shows the fatigue behavior comparison between four preferred embodiments of the textile of the invention as explained in Example 8.

FIG. 94A shows in suture technique on Design 1 and Design 2 (not folded) as explained in Example 9.

FIG. 94B shows in suture technique on Design 1 and Design 2 (folded) as explained in Example 9.

FIG. 94C shows suture technique on Design 3 and Design 4 as explained in Example 9.

FIG. 94D shows in suture technique on HD1* as explained in Example 9.

FIG. 95 shows a suture pull-out strength comparison among the preferred embodiments of the textile of the invention in different configurations as explained in Example 9.

FIG. 96A shows the *in vitro* degradation results, mechanical results as explained in Example 13.

FIG. 96B shows the *in vitro* degradation results, % of mass loss during the time as explained in Example 13.

FIG. 96C shows the *in vitro* degradation results % of Hyaff 11 esterification during the time as explained in Example 13.

FIG. 97 shows the results of the *in vitro* quantitative assessment of collagen production of Example 15, specifically the quantity of total collagen after 1 and 2 weeks of cell culture. Error bars represent standard deviation.

FIG. 98 shows the results of the *in vitro* quantitative assessment of collagen production of Example 15, specifically the quantity of insoluble, mature collagen after 1 and 2 weeks of cell culture. Error bars represent standard deviation.

FIG. 99 shows the results of the preliminary evaluation of the efficacy and local tissue response in a sheep model, specifically an intraoperative picture of the bioabsorbable textile (Design 1) implanted in an ovine infraspinatus tendon model.

FIG. 100 shows the results of the preliminary evaluation of the efficacy and local tissue response in a sheep model, specifically histological pictures of Group 2 at 6 weeks (A. Masson Trichrome and B. Picrosirius red under polarized light) and at 12 weeks (C. Masson Trichrome and D. Picrosirius red under polarized light). 20X Magnification. Scale bar: 50 μm.

FIG. 101A shows the results of the preliminary evaluation of the efficacy and local tissue response in a sheep model, specifically an intraoperative picture of the bioabsorbable textile implanted in an ovine infraspinatus tendon disruption model.

FIG. 101B shows the results of the preliminary evaluation of the efficacy and local tissue response in a sheep model, specifically an intraoperative picture of the Regeneten implanted in an ovine infraspinatus tendon disruption model.

FIG. 101C shows the results of the preliminary evaluation of the efficacy and local tissue response in a sheep model, specifically an intraoperative picture of the no augmentation (Control) in an ovine infraspinatus tendon disruption model.

FIG. 102 shows the results of the preliminary evaluation of the efficacy and local tissue response in a sheep model, specifically the tendon thickness analysis at 6 weeks.

FIG. 103A shows the results of the preliminary evaluation of the efficacy and local tissue response in a sheep model, specifically the Peak Stress (8% Global Strain) analysis at 12 weeks.

FIG. 103B shows the results of the preliminary evaluation of the efficacy and local tissue response in a sheep model, specifically the Equilibrium Stress (8% Global Strain) analysis at 12 weeks.

FIG. 104A shows the results of the preliminary evaluation of the efficacy and local tissue response in a sheep model, specifically the histological Overview after Regeneten in Ovine Tendon Disruption Model after 12 weeks.

FIG. 104B shows the results of the preliminary evaluation of the efficacy and local tissue response in a sheep model, specifically the histological Overview after Treatment -1 in Ovine Tendon Disruption Model after 12 weeks.

FIG. 104C shows the results of the preliminary evaluation of the efficacy and local tissue response in a sheep model, specifically the histological Overview after Treatment -2 in Ovine Tendon Disruption Model after 12 weeks.

## DETAILED DESCRIPTION

Disclosed herein, in some embodiments, are systems and methods for providing an implant about a tissue defect (e.g., an injured tendon) in a subject. In some embodiments, the implant provides an augmentation layer over the tissue defect. In some embodiments, the implant comprises an at least partially bioabsorbable textile, configured to provide a combined mechanical and biological augmentation about the tissue defect (which may include, for example, a lesion site) aimed at favoring the formation of a mature, hierarchically- organized joint tissue. In some embodiments, the at least partially bioabsorbable textile comprises a patch, mesh, or other implantable fabric known in the art.

In some embodiments, described herein are systems and methods for delivering the implant. In some embodiments, the implant is delivered using one or more delivery tools. In some embodiments, the implant is configured to be secured about the tissue defect to bone using one or more bone fasteners. In some embodiments, the implant is further configured to be secured about the tissue defect to soft-tissue using one or more soft-tissue fasteners.

Described herein, in some aspects, are apparatuses for simultaneous delivery of a fastener and an implant; systems including at least one of the present apparatuses, a seal assembly, and a cannula; caddy systems configured to receive a fastener and/or an implant for use with the present apparatuses; kits including at least one of the present apparatuses or systems and at least one of the present caddies; and methods of simultaneously delivering a fastener and an implant with the present apparatuses. The present apparatuses include an elongated body, one or more spikes at a body distal end of the body, a hub configured to receive a proximal portion of the body, and a handle coupled to the hub. The body defining a body recess in a peripheral surface of the body at point that is offset from the body distal end, and a body passage extending between a body proximal end and a body distal opening within the body recess. The apparatus further includes an elongated pusher and a slider coupled to a proximal end of the pusher, the slider configured to move longitudinally relative to the handle from a retracted position to a deployed position to move the pusher distal end longitudinally away from the body distal opening and laterally outward from the body.

Described herein, in other aspects, is a bioabsorbable textile for the restoration of the joint function whereas the joint is affected by partial thickness tears, small to medium full-thickness tears, large to massive full-thickness tears, acute and chronic/degenerative tears. The bioabsorbable textile may comprise polymeric yams interconnected to form a weave or knitted configuration, wherein said bioabsorbable textile provides a combined mechanical and biological augmentation in the target joint tissue. The bioabsorbable textile may be implanted in combination with fixation tools during open, mini-open or arthroscopic repair/augmentation procedures of joint tissue tears.

Described herein, in yet other aspects, is a multicomponent suture comprising first and second substantially elongate suture portions with respective first and second cross-sectional profiles, and further including respective first and second materials, one of said materials being bioabsorbable and a second of said materials being non-bioabsorbable.

Described herein, in yet other aspects, are fasteners for coupling an implant to tissue (e.g., soft-tissue and/or bone), fabric-like implants, and assemblies with fasteners pre-loaded with implants. The present implants generally comprise at least one flexible, fibrous layer that is substantially planar in a flattened state. In some embodiments of the present assemblies for delivery of a fastener, the assembly comprises fastener cartridge, a fibrous implant wrapped around a portion of the cartridge, a fastener extending through the implant, and an elongated shield disposed around the implant and the cartridge such that the implant is retained between the cartridge and the shield. Kits comprise a plurality of fasteners pre-loaded with implants. Some of the present kits also include one or more of the present fastener-delivery apparatuses or tools; for example, a plurality of pre-loaded fasteners with a single, reloadable tool; a plurality of tools each pre-loaded with a fastener that is pre-loaded with an implant; and/or a plurality of cartridges each pre-loaded with a fastener that is pre-loaded with an implant, and a common tool for use with the cartridges.

Implant

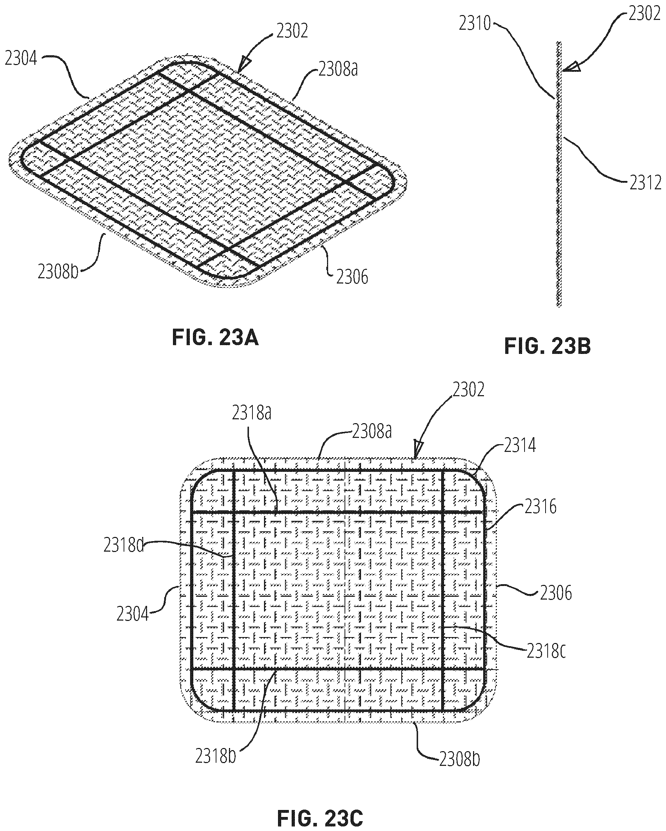

FIG. 23A depicts a perspective view of an exemplary implant as described herein, specifically an exemplary fibrous, fabric-like implant. More specifically, FIGs. 23A, 23B, and 23C depict perspective, end, and plan views, respectively, of a flexible, fibrous, fabric-like implant 2302. In some embodiments, the implant 2302 has a first end edge 2306, a second end edge 2304, and a pair of lateral edges 2308a, 2308b that extend between the first and second end edges. In some embodiments, implant 2302 comprises a woven layer 2310 and a nonwoven layer 2312 coupled to the woven layer. More particularly, in some embodiments, nonwoven layer 2312 is stitched to the woven layer 2310 with a (mono- or multi-filament) thread 2314 along a peripheral path 2316 that is offset inward from the peripheral edges (2306, 2304, 2308a, 2308b) of the implant, as well as internal cross paths 2318a, 2318b, 2318c, 2318d that are offset further inwardly of each of the peripheral edges of the implant.

As described herein, in some embodiments, the invention relates to a an implant comprising a bioabsorbable textile. For example, in some embodiments, both of layers 2310 and 2312 (of implant 2302) comprise a biocompatible polymer. In some cases, the degradation kinetics of the bioabsorbable textile will match the *de novo* tissue formation timelines so that the textile will only be present *in situ* for the time where augmentation (e.g., as an augmentation layer, as described herein) is really needed, ultimately maximizing the efficacy of the treatment as well as the safety profile of the textile.

In some casethethe present invention (e.g, providing an implant as described herein) provides a novel treatment option for patients suffering with joint pain and lack of joint function restoration in the knee, hip, shoulder, ankle, fingers, toes, wrist, elbow and vertebrae. Preferred embodiments of the inventive treatment augment the rotator cuff tendons via mechanical reinforcement and induction of tissue regeneration by reinforcing the suture- and/or anchor-based surgical rotator cuff repair in order to prevent further re-tears and alleviate the corresponding pain in the joint.

In some embodiments, the implant comprises a composite medical textile, such as a suture, a patch (e.g., see implant 2302 in FIG. 23A), etc., wherein, according to an embodiment described herein, the implant includes a plurality of bioresorbable hyaluronan-based fibers interlocked with a plurality of non-resorbable fibers. In some embodiments, the implant as a patch (e.g., implant 2302), comprises a plurality of such composite sutures (e.g., comprising bioresorbable hyaluronan-based fibers interlocked with a plurality of non-resorbable fibers) interlocked together.

In some embodiments, a plurality of different kinds of hyaluronan-based fibers can be used with a plurality of different kinds of bioresorbable fibers. The term "hyaluronan-based" as used herein encompasses fiber materials that can include at least one selected from the group consisting of hyaluronic acid, sodium hyaluronate, and benzyl esters of hyaluronic acid, such as but not limited to the benzyl ester derivative of hyaluronic acid sold as HYAFF®. Other esters of hyaluronic acid with aliphatic, araliphatic, cycloaliphatic or heterocyclic alcohols, in which are esterified all (so-called "total esters") or only a part (so-called "partial esters") of the carboxylic groups of the hyaluronic acid are also possible. Crosslinked hyaluronic acid-based fibers that are cross-linked with such compounds as bis(ethylcarbodiimide)(BCDI), formaldehyde and other aldehydes, (1-Ethyl-3-(3- dimethylaminopropyl)carbodiimide)(EDC), dicyclohexylcarbodiimide) (DCC) or other carbodiimide crosslinking agents, and click chemistry, can also be used.

All of the above are hyaluronan-based materials as used herein. Combinations of hyaluronan-based materials are also possible.

The hyaluronan-based material can be the sodium salt of hyaluronic acid, sodium hyaluronate, but the acid will usually also be present to some degree. Sometimes up to 3% or more of the acid or conjugate is present. The acid will also be present to some degree with HYAFF® materials. The degree of substitution of HYAFF® materials varies between approximately 50 and approximately 100%. The degree of substitution of the hyaluronic acid can be 50,55, 60, 65, 70, 75, 80, 85, 90, 95, 96, 97, 98, 99 and 100 %, and can be within a range of any high value and low value selected from these values. The remaining functional groups are either all acid, all base, or a mix of acid and base. Blends of fibers having different degrees of substitution are also possible, and crosslinked hyaluronic acid. The total benzyl esterified form, named HYAFF11®p100, can be used. HYAFF11® p80 and HYAFF11® p75 is partially esterified and can also be used(Anika Therapeutics S.R.L.,Padua Italy).

The individual fiber dimensions can vary. The deniers(D, g/9000 m) of the non-resorbable and hyaluronan-based bioresorbable fibers can vary from 250 to 100,000 D. The denier range can be from 500 to 15,000 D. Some bioresorbable fibers are from 540 to 720 D. The deniers of the non-resorbable fibers and the hyaluronan-based bioresorbable fibers can be, independently, 250, 300,400,500,750, 1000, 2000, 3000, 4000, 5000, 6000, 7000, 8000, 9000, 10000, 11000, 12000, 13000, 14000, 15000, 16000, 17000, 18000, 19000, 20000, 30000, 40000, 50000, 60000, 70000, 80000, 90000, and 100000 D, and can be within a range of any high value and low value selected from these values. Individual fibers of specific deniers can be combined in a composite suture arrangement of multi-filament, multi-denier bundles.

The diameter of the individual non-resorbable and hyaluronan-based bioresorbable fibers can be from 15-250 $\mu$m. The diameter can be from 50-60 $\mu$m. The diameter of the non-resorbable and hyaluronan-based bioresorbable fibers can be, independently, 15, 20, 25, 30, 40, 50, 60, 70, 80, 90, 100, 110, 120, 130, 140, 150, 160, 170, 180, 190, 200, 210, 220, 230, 240, and 250 $\mu$m, and can be within a range of any high value and low value selected from these values. Individual fibers of specific diameters can be combined in a composite suture arrangement of multi-diameter fibers.

The weight proportion of non-resorbable fibers to the total weight of the non-resorbable fibers and the bioresorbable hyaluronan-based fibers can be 10%-98%. The weight proportion of non-resorbable fibers to the total weight of the non-resorbable fibers and the bioresorbable hyaluronan-based fibers can be from 30%-80%. The weight proportion of non-resorbable fibers to the total weight of the non-resorbable fibers and the bioresorbable hyaluronan-based fibers can be 10, 15, 20, 25, 30, 35, 40, 45, 50, 55, 60, 65, 70, 75, 80, 85, 90, 95, 96, 97, and 98%, and can be within a range of any high value and low value selected from these values.

As described herein, in some embodiments, the implant comprises one or more composite sutures. In some embodiments, the one or more composite sutures are configured as a patch (e.g., see shape of implant 2302 in FIG. 23A, but which made comprised of different materials than as described in FIG. 23A). In some embodiments, a composite suture made from the medical textile of the invention comprising both the non-resorbable fibers and the bioresorbable hyaluronan- based fibers can have different dimensions. Typical suture is commonly from United States Pharmacopeia (USP) sizes USP 3-0 to USP 5. The composite suture can have a size range of from 100 to 5000 $\mu$m, more specifically 25 to 750 $\mu$m. A common size is from 50 to 65 $\mu$m. Some special use suture can be up to 5000 $\mu$m. The composite suture can have a diameter of 25, 50, 75, 100, 200, 300, 400, 500, 600,700, 800,900, 1000, 1250, 1500, 1750, 2000, 2250, 2500, 2750, 3000, 3250, 3500, 3750, 4000, 4250, 4500, 4750, and 5000 $\mu$m, and can be within a range of any high value and low value selected from these values. It is common that suture dimensions will vary slightly due to small variations in fiber dimensions and assembly conditions, and accordingly the above sizes can be considered as averages.

The number of fibers in the suture composite can vary. The suture composite can have 25 to 10,000 total individual fibers, including both the bioresorbable fiber strands and the hyaluronan-based bioresorbable fiber strands. The number of total fiber strands can be from 150 to 6000 individual fiber strands. The total number of fiber strands can be 25, 50, 75, 100, 200,

300, 400, 500, 600, 700, 800, 900, 1000, 2000, 3000,4000, 5000, 6000, 7000, 8000, 9000, and 10000 strands, and can be within a range of any high value and low value selected from these values.

The number proportion of non-resorbable suture fibers to bioresorbable hyaluronan-based polymer fibers in the composite suture, and/or any implant described herein can vary. The composite suture and/or any implant described herein (including implant 2302 from FIG. 23A) can include from 20 % to 80 % bioresorbable hyaluronan- based polymer fibers, based on the total number of non-resorbable suture fibers and bioresorbable hyaluronan-based polymer fibers. The composite suture and/or any implant described herein can include 20%, 25%, 30%, 35%, 40%, 45%, 50%, 55%, 60%, 65%, 70%, 75%, or 80% bioresorbable hyaluronan-based polymer fibers, based on the total number of non-resorbable suture fibers and bioresorbable hyaluronan-based polymer fibers. The number proportion of bioresorbable hyaluronan-based polymer fibers, based on the total number of non-resorbable suture fibers and bioresorbable hyaluronan-based polymer fibers, can be within a range of any high value and low value selected from these values.

The medical textile (e.g., including any implant described herein) can have a straight tensile strength of from 15 to 800 N. The medical textile can have a straight tensile strength of 15, 20, 25, 30, 35, 40, 60, 80, 100, 120, 140, 160, 180, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, and 800 N, and can be within a range of any high value and low value selected from these values.

The medical textile when formed as a suture can have a knot pull strength of from 40 to 300 N. The knot pull strength can be 40, 50, 60, 70, 80, 90, 100, 110, 120, 130, 140, 150, 160, 170, 180, 190, 200, 210, 220, 230, 240, 250, 260, 270, 280, 290, and 300 N, and can be within a range of any high value and low value selected from these values.

The bioresorbable fibers (for any implant described herein) can resorb within about 2 - 8 months. The time for resorption of the bioresorbable fibers can be 2, 3, 4, 5, 6, 7, or 8 months, or within a range of any high value and low value selected from these values.

The non-resorbable fibers can be formed from different materials. The non-resorbable fibers can comprise ultra-high molecular weight polyethylene (UHMWPE). The material of the non-resorbable suture fiber can also include polypropylene, polyethylene terephthalate, polyethylene, polytetrafluoroethylene (Teflon), Dacron, steel, polybutester, polyamide, polyester, polyurethane, nylon and its derivatives, silk and cotton. Combinations of non-resorbable materials are also possible.

The hyaluronan-based bioresorbable polymer fibers can be prepared by different processes. The hyaluronan-based bioresorbable fibers can prepared by at least one selected from the group consisting of ring spinning, air- jet spinning, open-end spinning, mule spinning, wet spinning, dry spinning, electrospinning, pneumatospinning, pultrusion, and extrusion. The extrusion process can be at least one selected from the group consisting of solvent exchange extrusion, precipitation extrusion, phase exchange extrusion, and phase change extrusion. Other processes are possible. The hyaluronan-based bioresorbable fibers can be monofilaments, and can be multifilaments.

Additional bioresorbable fibers can be used with the hyaluronan- based bioresorbable fibers. Such additional bioresorbable fibers include bioresorbable polymer fiber comprising at least one of collagen, polylactic acid (PLA), polyglycolic acid (PGA), polylactic-co-glycolic acid (PLGA), polycaprolactone(PCL), polydioxanone (PDO), polyhydroxybutyrate (PHB), polyhydroxyvalerate (PHV), Poly(3-hydroxybutyrate-co-3-hydroxyvalerate (PHBV), alginate, chitosan, chitin, polylysine, fibrin, pectin, dextran, carrageenan, chondroitin sulfate, agar, gelatin, gellan gum, silk, and/or butyric acid.

The composite suture and/or any implant described herein can also include at least one active agent. Many different kinds of active agents are possible. The active agent can be an antimicrobial agent. The antimicrobial agent can include at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

The active agent can be an analgesic. The analgesic can be at least one selected from the group consisting of Lidocaine, Bupivacaine, amylocaine, articaine, aspirin, benzocaine, benzonatate, butacaine, butanilicaine, chloroprocaine, clopidogrel, cinchocaine, cyclomethycaine, eucaine, ibuprofen, naproxen, paclitaxel, warfarin, heparin, tetracaine, dexamethasone, and ropivocaine.

The active agent can be a vasoconstrictive agent. The vasoconstrictive agent can include at least one selected from the group consisting of epinephrine, alpha-adrenoreceptor antagonists, vasopressin analogues, norepinephrine, phenylephrine, dopamine, dobutamine, serotonin agonists, and triptans.

The active agent can be included in the composite suture by different processes. The active agent can be applied to at least one of the non- resorbable fibers and the bioresorbable hyaluronan-based fibers by wetting with the active agent. The active agent can be applied to at least one of the non- resorbable fibers and the bioresorbable hyaluronan-based fibers by coating with the active agent. The active agent can be applied to at least one of the non- resorbable fibers and the bioresorbable hyaluronan-based fibers by embedding the active agent into the fiber during the extrusion of the fiber. The active agent can be chemically or ionically crosslinked or grafted to the surface of at least one of the non-resorbable fibers and the bioresorbable hyaluronan-based fibers. The coating step can be performed by any suitable process. The coating step can be at least one selected from the group consisting of a dip, brush, spray, or curtain coating process.

The active agent can be impregnated into at least one selected from the group consisting of the bioresorbable hyaluronan-based fibers and the non- resorbable fibers by passing the extruded fiber through a solution containing the active agent. The impregnating step can be by co-extrusion, wherein a fiber precursor and the active agent are combined into a homogeneous mixture and co-extruded into a fiber.

The composite suture (which may be used as part of any implant described herein) can have different cross-sectional shapes. The composite suture can be circular in cross section. The composite suture can be a tape and have a cross section comprising at least one selected from the group consisting of rounded rectangular, marquise, oblong and oval. A method of making a suture fiber can include the step of providing a plurality of non- resorbable fibers and a plurality of bioresorbable hyaluronan-based fibers. The non-resorbable fibers and the bioresorbable hyaluronan-based fibers are interlocked into a composite suture. The joining of the bioresorbable hyaluronan- based fibers and the non-resorbable fibers can be by any suitable process.

Examples of suitable processes include twisting, weaving, braiding, knitting, adhering and heat treating.

The medical textile of the invention, such as any implant described herein (which may include a patch formed of resorbable and non-resorbable based fibers, a patch formed of one or more composite sutures, and/or a composite suture) can take many different textile forms. The bioresorbable hyaluronan-based fibers and non-resorbable fibers can be knitted, twisted, braided, non-woven or other forms of textile construction for combining diverse fibers. Braided suture can for example contain bioresorbable hyaluronan-based fibers, non-resorbable fibers, and one or more additional fibers, which can be bioresorbable fibers and/or non-resorbable fibers. Braided suture can contain a core filament such as bioresorbable hyaluronan-based fibers and an outer braided sheath of one or more polymers. Braided suture can be coated with a bioresorbable hyaluronan-based film. Braided or woven suture can contain bioresorbable hyaluronan-based fibers and at least one other fiber, which can be another bioresorbable hyaluronan-based fiber and/or a non-resorbable fiber. Other medical textile constructions of bioresorbable hyaluronan- based fibers and non-resorbable fibers are possible.

The active agent can be included in the composite suture by different processes. The active agent can be applied to at least one of the non- resorbable fibers and the bioresorbable hyaluronan-based fibers by wetting with the active agent. The active agent can be applied to at least one of the non- resorbable fibers and the bioresorbable hyaluronan-based fibers by coating with the active agent. The active agent can be applied to at least one of the non- resorbable fibers and the bioresorbable hyaluronan-based fibers by embedding the active agent into the fiber during the extrusion of the fiber. The active agent can be chemically or ionically crosslinked or grafted to the surface of at least one of the non-resorbable fibers and the bioresorbable hyaluronan-based fibers. The coating step can be performed by any suitable process. The coating step can be at least one selected from the group consisting of a dip, brush, spray, or curtain coating process.

The active agent can be impregnated into at least one selected from the group consisting of the bioresorbable hyaluronan-based fibers and the non- resorbable fibers by passing the extruded fiber through a solution containing the active agent. The impregnating step can be by co-extrusion, wherein a fiber precursor and the active agent are combined into a homogeneous mixture and co-extruded into a fiber.

The composite suture (which may be used as part of any implant described herein) can have different cross-sectional shapes. The composite suture can be circular in cross section. The composite suture can be a tape and have a cross section comprising at least one selected from the group consisting of rounded rectangular, marquise, oblong and oval. A method of making a suture fiber can include the step of providing a plurality of non- resorbable fibers and a plurality of bioresorbable hyaluronan-based fibers. The non-resorbable fibers and the bioresorbable hyaluronan-based fibers are interlocked into a composite suture. The joining of the bioresorbable hyaluronan- based fibers and the non-resorbable fibers can be by any suitable process.

Examples of suitable processes include twisting, weaving, braiding, knitting, adhering and heat treating.

The medical textile of the invention, such as any implant described herein (which may include a patch formed of resorbable and non-resorbable based fibers, a patch formed of one or more composite sutures, and/or a composite suture) can take many different textile forms. The bioresorbable hyaluronan-based fibers and non-resorbable fibers can be knitted, twisted, braided, non-woven or other forms of textile construction for combining diverse fibers. Braided suture can for example contain bioresorbable hyaluronan-based fibers, non-resorbable fibers, and one or more additional fibers, which can be bioresorbable fibers and/or non-resorbable fibers. Braided suture can contain a core filament such as bioresorbable hyaluronan-based fibers and an outer braided sheath of one or more polymers. Braided suture can be coated with a bioresorbable hyaluronan-based film. Braided or woven suture can contain bioresorbable hyaluronan-based fibers and at least one other fiber, which can be another bioresorbable hyaluronan-based fiber and/or a non-resorbable fiber. Other medical textile constructions of bioresorbable hyaluronan- based fibers and non-resorbable fibers are possible.

The medical textile can also be used to make scaffolds. Scaffolds made with the medical textiles of the invention will provide requisite initial and long-term strength by the presence of both hyaluronan-based bioresorbable fibers and non-resorbable fibers and will allow for cell growth into and around the scaffold. Scaffold devices include tubes, membranes, non-woven fabrics, gauzes, and sponges.

Braided surgical sutures have been shown to cause more trauma (abrasion, tearing, cutting, etc.) to soft tissue than monofilament suture.

Kowalsky MS, Dellenbaugh SG, Erlichman DB, et al., "Evaluation of suture abrasion against rotator cuff tendon and proximal humerus bone" Arthroscopy. 2008;24:329-334. It is also known in the medical field that a lubricant coating on braided sutures reduces the friction as well as the tissue drag which can lead to tearing and trauma on soft tissue; however many lubricant coatings are either short-lived in vivo or are composed of potentially toxic materials. The use of conventional resorbable polymers (e.g., PLA,PGA, PCL, PDO, PHBV, etc.) in a composite braided suture structure will degrade by hydrolysis into smaller molecules and/or their respective monomers. The use of esterified hyaluronic acid such as HYAFF11® (Anika Therapeutics S.R.L., Padua Italy) in a composite braided structure will degrade into benzyl alcohol and high molecular weight hyaluronic acid (HA) over time in vivo. HA is a naturally occurring polysaccharide with unique viscoelastic and rheological properties; it serves as a lubricating agent on articular tissues and prevents mechanical damage. Its viscoelasticity has been shown to be responsible for protecting, lubricating, and stabilizing cells and tissue layers during joint movement. Goa, Karen L., and Paul Benfield. "Hyaluronic acid." Drugs 47.3

(1994): 536-566. Furthermore, high molecular weight HA has been shown to providean antiinflammatory response in addition to improved lubrication properties in tendon lesions. Necas, J. B. L. B. P., et al. "Hyaluronic acid (hyaluronan): a review." Veterinarni medicina 53.8 (2008):397- 411.

In the embodiment of a composite braided, woven, or twisted medical textile such as suture or suture tape comprising of esterified HA (such as HYAFF11® p100 Anika Therapeutics S.R.L., Padua Italy) fibers and non- absorbable polymer (e.g.,UHMWPE) fibers, the Hyaff material will provide a slow release of a self-lubrication agent (high MW HA) to the braided suture structure.

The release of HA over time will provide a self-replenishing supply of lubricant that will reduce abrasion and soft tissue trauma as well as reduce the inflammatory response, which could improve the healing response of the repaired soft tissue.

Hyaluronic acid (HA) is a naturally occurring polysaccharide that is an important component of extra-cellular matrix (ECM); it interacts with binding proteins, proteoglycan, growth factors and other active molecules and contributes to the regulation of water balance. HA is present in all adult joint tissues, including synovial fluid and its rheological properties in solution make it an ideal a lubricant for protecting articular cartilage. Furthermore, HA acts as scavenger molecule for free radicals, inhibits leukocyte and macrophage migration, and helps regulate fibroblast proliferation. Besides all these properties, HA is recognized by some cell receptors such as CD44, regulating the adhesion, differentiation, angiogenesis and modulating the inflammation. For these and other biological properties, HA represents an optimal candidate as a biomaterial to produce scaffolds; however, its water solubility, rapid resorption, and short residence time in the tissue, limit its possible application. Campoccia D. et al., Biomaterials, 19

(1998) 2101-2127. Different HA chemical modification techniques have been used to improve the physical and mechanical properties. One of the most promising materials for tissue engineering and regenerative medicine is the benzyl ester derivative of hyaluronan (HYAFF11®). One of the main advantages of HYAFF11®based scaffold is the degree of cell adhesiveness even without any coating or treatment with different molecules, generally required by other biomaterials such as polyglycolide and polylactide. The total benzyl esterified form of HYAFF11®p100 is sufficiently stable in an aqueous environment, maintaining its structural integrity, so it is easy to handle and does not contract as some collagen-based materials. Campoccia D. et al., Biomaterials, 19 (1998)2101-2127. HYAFF11®p100 can be processed to obtain several types of devices such as tubes, membranes, non-woven fabrics, gauzes, and sponges. All these scaffolds are highly biocompatible. In the human body they do not elicit any adverse reactions and are resorbed by the host tissues. Vindigni V. et al., Int. J. Mol. Sci., 10, (2009)2972-2985.

HYAFF11® scaffolds have been shown to be suitable supports for the attachment, growth, and proliferation of mammalian cells. Human preadipocytes can be successfully and reproducibly inoculated and cultured on HYAFF11®-based three-dimensional scaffolds where there was clear evidence that adipocyte precursor cells placed on this material were able to undergo full maturation into adipocytes. Halbleib, M. et al., Biomaterials, 24, (2003) 3125-3132. Additionally, human hepatocytes, dermal fibroblasts and keratinocytes, chondrocytes, Schwann cells, bone marrow derived mesenchymal stem cells and adipose tissue derived mesenchymal stem cells have been successfully cultured in HYAFF11® meshes. Vindigni V. et al., Int. J. Mol. Sci., 10, (2009) 2972-2985. Furthermore, HYAFF11® scaffolds support the adhesion, migration and proliferation of rMSCs, as well as the synthesis and delivery of extracellular matrix components under static culture conditions without any chemical induction. The high retention rate and viability of the seeded cells as well as their fine modality of interaction with the substrate suggest that such scaffolds could be potentially useful when wide tissue defects are to be repaired as in the case of cartilage repair, wound healing and large vessel replacement. Pasquinelli G. et al., J. Anat., 213 (2008) 520-530.

Delivery Systems

As described herein, implants may be placed about a tissue defect using a delivery system, which may include one or more fixation devices (e.g., fasteners, as described herein, or other fixation members), as described herein, and one or more tools to deliver the implant to the tissue defect location, and secure thereto using the one or more fasteners.

For example, in some embodiments, implants, (e.g., including bioabsorbable textiles) of the invention can be used in conjunction with multiple types of fixation devices, known in the field of orthopedics for repairing damaged soft tissue. These fixation devices can be bone screws, bone screws with washers, fasteners (as described herein), staples (as described herein), suture anchors, suture buttons and the like; used to create, repair or augment tissue connection sites relevant to orthopedic procedures. These connection sites can be comprised of bone-to-bone tissue connections, as typically seen in ligament injuries; tissue-to-bone connections, as typically seen in tendon or ligament injuries; and tissue-to-tissue connections, as typically seen within tendon, ligament, or muscle injuries. The medical textiles (e.g., implants) used in conjunction with these fixation devices may take the form of sutures, suture tapes of different constructions and sizes, patches (as described herein), meshes, etc.

The medical textiles (e.g., implants) of the invention can be fashioned so that they are packaged and provided to the surgeon user directly coupled to these fixation devices, or they can be fashioned using splicing or knotting techniques within the textile so that they could be packaged and provided to the surgeon user for use with separately packaged fixation devices.

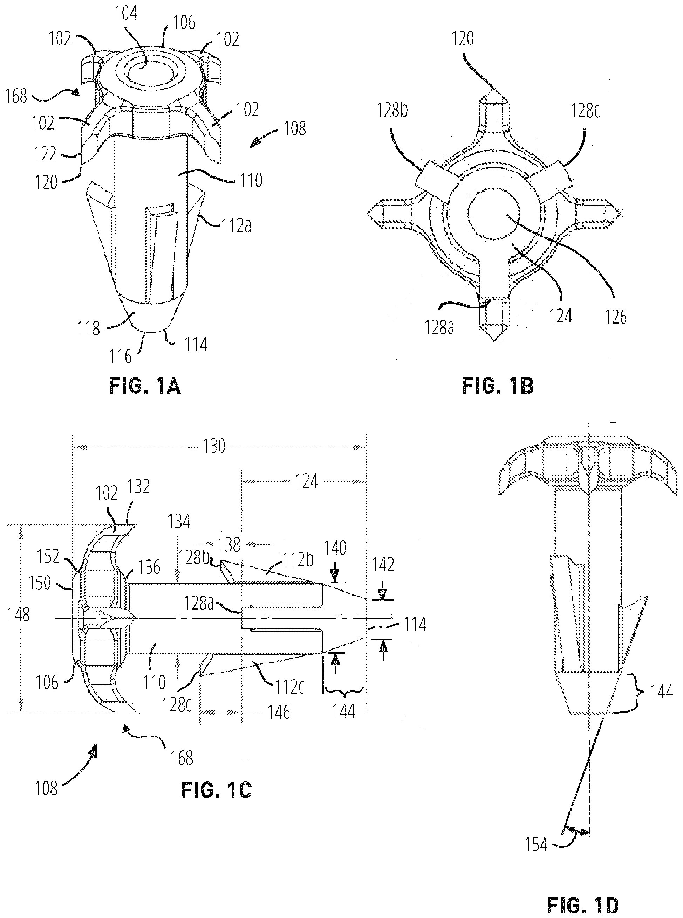

FIG. 1A depicts a perspective view of a first embodiment of exemplary soft-tissue fasteners.

Referring now to the drawings, and more particularly to FIG. 1A -FIG. 1H, shown therein and designated by the reference numeral 108 is one embodiment of the present soft-tissue fasteners. In the depicted example, fastener 108 is configured for coupling an implant (as described herein) to soft tissue, such as a tendon (e.g., the supraspinatus tendon or rotator cuff). As shown, fastener 108 comprises an elongated shaft 110, an enlarged head 106, a plurality of outriggers 102, and a plurality of barbs 112a, 112b, 112c.

Shaft 110 extends from a shaft proximal end 150 (and enlarged head 106) to a shaft distal end 114. Shaft distal end 114 is configured to be inserted (along with at least one of barbs 112a, 112b, 112c) into soft tissue of a patient such that the barbs resist removal of the fastener from the soft tissue. For example, in at least some uses, the distal end can first be inserted through an implant such that inserting the distal end into soft tissue couples the implant to the soft tissue. To that end, outriggers 102 each extend outward from head 106 to resist removal of such an implant over the head. Outriggers 102 can be particularly advantageous when the fastener is used in conjunction with a fibrous (e.g., woven) or fabric-like implant, the flexibility of which may otherwise (without outriggers) be more-susceptible to slipping over the head.

FIG. 1B depicts a distal end view of the fastener of FIG. 1A.

In some embodiments, it can be advantageous for at least one of the barbs to not be radially aligned with any of the outriggers, such that if a barb cuts a slot or creates a tear in an implant when the distal end is inserted through the implant, then at least one outrigger is more likely to not be aligned with that slot or tear and thus reduce the changes of the slot or tear slipping over or around head 106 and outriggers 102. For example, as shown in FIG. 1B, only barb 112a is radially aligned with an outrigger 102, and therefore two of the three barbs (112b, 112c) are not radially aligned with any of the outriggers. Additionally, in some embodiments, the outriggers extend farther outward radially than the barbs, such that- even if all of the barbs cut or tear an implant through which the fastener is inserted-the outriggers (and particularly points 120) are more likely to engage portions of the implant that are outside those cuts or tears.

To further facilitate insertion through an implant and into soft tissue, an outer surface 118 of the shaft defines a tapered section 144 adjacent shaft distal end 114. Tapered section 144 extends from a first outer transverse dimension 142 at shaft distal end 114 and increases in size in a proximal direction to a larger outer transverse dimension 140 that may, as in the depicted embodiment, be substantially equal to an outer transverse dimension along a remainder of the length of the shaft between the tapered section and the head (106). In the embodiment shown, tapered section 144 is tapered linearly at a taper angle 154 relative to axis 156, but in other embodiments may be tapered in a non-linear fashion (e.g., along a curved path). Angle 154 may in some embodiments be equal to angle 164a, and/or may be between 15 and 30 degrees, for example, between any two of: 15, 17.5, 20, 22.5, and/or 25 degrees (e.g., between 17.5 and 22.5 degrees). For example, in the depicted embodiment, angle 154 is between 19 and 20 degrees and is equal to angle 164a such that tapered section 144 and leading edge 160a follow a continuous, linear path.

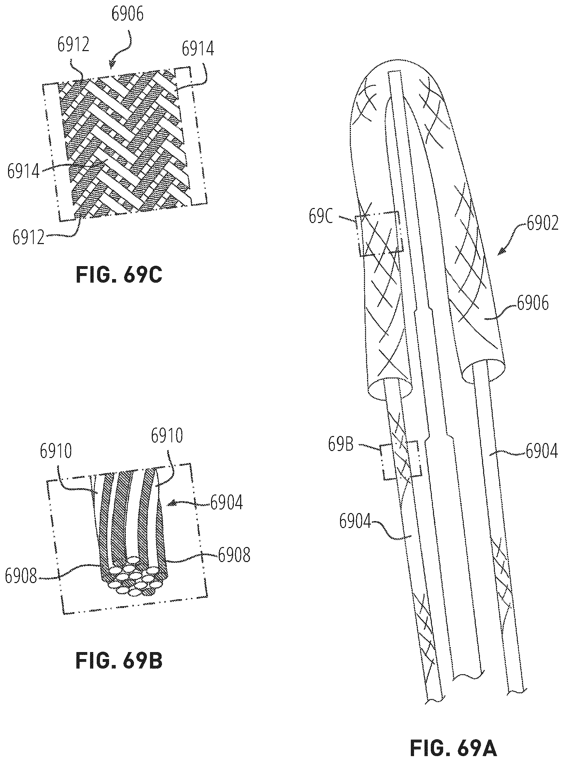

FIG. 69C is an expanded view of area 69C in FIG. 69A. In the embodiment shown, the shaft (110) is also configured to receive a portion of a delivery tool (described below). In particular, an internal surface 104 of the shaft defines an internal passage 126 that extends through the length of the shaft along axis 156 and through both of proximal end 150 and distal end 114. Passage 126 is configured to receive a portion of a tool (e.g., a spike or trocar) such that that the tool extends through passage 126 and beyond distal end 114 to permit the tool to form a pilot hole in the tissue into which the tool (or a user of the tool) can simultaneously drive the fastener and, after the fastener is inserted into the tissue, the tool can thereafter be removed via the passage (126). In some embodiments, passage 126 has a first inner transverse dimension at the shaft proximal end (150) and a smaller second inner transverse dimension at the shaft distal end (114), such that the passage tapers (e.g., linearly) from smaller at the shaft distal end to larger as the passage approaches the shaft proximal end. For example, in the embodiment shown, passage 126 tapers linearly at an angle 166 of one degree. Such a taper can be advantageous in reducing the resistance to removal of a tool after insertion of the fastener. For example, during insertion of the fastener, soft tissue will be displaced and tend to exert an inward force around the perimeter of a tool in the passage. The provision of a taper, such that a proximal portion of the passage is larger than a distal portion of the passage, typically helps mitigate the compressive forces on a tool in the passage during insertion of the fastener and thereby typically reduces resistance to removal of the tool from the passage after such insertion. In some embodiments, such as the one shown, inner surface 104 and outer surface 118 meet at shaft distal end 114 to form an edge 116. Such an edge at the distal end of the shaft minimizes the cross-sectional area of fastener at the distal end to facilitate insertion of the fastener into tissue.

In the embodiment of FIGs. 1A-1H, shaft 110 has a circular cross-sectional shape, such that an outer transverse dimension 134 is the diameter of a circle that defines the outer perimeter of that shape. In some embodiments, transverse dimension 134 is between any two of: 1.25 mm, 1.35 mm, 1.45 mm, 1.55 mm, 1.65 mm, 1.75 mm, 1.85 mm, 1.95 mm, and/or 2.05 mm (e.g., between 1.55 mm and 1.75 mm). For example, in the embodiment shown, diameter 134 is between 1.6 and 1.7 mm. In other embodiments, the shaft can have other cross-sectional shapes, such as, for example, square, hexagonal, octagonal, or the like.

As described above, the barbs of the fastener extend outwardly from the shaft and therefore span a maximum transverse dimension that is larger than that of the shaft, such as, for example, the barbs extend radially outward to an imaginary circle (perpendicular to axis 156) having a maximum transverse dimension that is between any two of: 130%, 140%, 150%, 160%, 170%, 180%, 190%, 200%, 210%, 220%, and/or 230% of the maximum transverse dimension of the shaft (e.g., between 170% and 190% of the diameter of the shaft). For example, in the embodiment shown, barbs 112a, 112b, 112c each extend radially outward to a point between 1.4 and 1.5 mm from axis 156 (i.e., each of point/edge 128a, 128b, 128c is between 1.4 and 1.5 mm from axis 156).

As also described above, outriggers 102 extend outwardly from enlarged head 168 beyond the lateral extend of the barbs and therefore span a maximum transverse dimension 148 that is larger than that of the barbs, such as, for example, a maximum transverse dimension that is between any two of: 200%, 220%, 240%, 260%, 280%, and/or 300% of the maximum transverse dimension of the shaft (e.g., between 260% and 280% of the maximum transverse dimension of that shaft). For example, in the embodiment shown, outriggers 102 each extend radially outward to a point that is between 2.2 mm and 2.3 mm from axis 156.

In some embodiments, the overall length of the fastener is between 5 mm and 9 mm, such as, for example, between any two of 5 mm, 5.5 mm, 6 mm, 6.5 mm, 7 mm, 7.5 mm, 8 mm, and/or 8.5 mm (e.g., between 5.5 mm and 6.5 mm, between 6.5 mm and 7.5 mm, or between 7.5 mm and 8.5 mm). For example, in the embodiment shown, length 130 is nominally 7 mm, and therefore between 6.5 mm and 7.5 mm. In other embodiments, length 130 can be selected for a particular use. For example, when using a soft-tissue fastener with a tendon that overlies and must move relative to other anatomical structure, it is generally desirable that the distal end of the fastener not extend into the underlying anatomical structures. As an example of one type of tendon, for use with a relatively smaller person with a relatively thinner supraspinatus tendon, a fastener with nominal length of 6 mm may be appropriate; while, for use with a relatively larger person with a relatively thicker supraspinatus tendon, a fastener with a nominal length of 8 mm may be appropriate.

In at least some embodiments, the fastener (i.e., shaft 110, head 106, outriggers 102, and barbs 112a, 112b) are defined by a unitary piece of material. For example, it is sometimes desirable for soft-tissue fasteners to be bioresorbable and, in such instances, the fastener can be molded of a bioresorbable polymer such as poly lactic-co-glycolic acid (PLGA) or polylactic acid (PLA or PLLA). In some uses, it is desirable for complete resorption to occur in less than 9 months (e.g., between 3 months and 9 months).

FIG. 1C depicts a first side view of the fastener of FIG. 1A.

As shown, head 106 includes a head proximal side 152, and a head distal side 136. In this configuration, at least a portion of the head distal side is longitudinally aligned with shaft proximal end 150, for example as shown in FIG. 1C.

Additionally, in the depicted embodiment, the trailing edges of the barbs are staggered along the length of shaft to increase the likelihood that at least one of the barbs will securely engage soft tissue. For example, when used with certain tendons (e.g., the supraspinatus tendon), the tendon may have different thicknesses in different regions or may have partial-thickness tears, such that barbs disposed at different distances from the head of the fastener make it more likely that at least one barb will encounter and engage soft tissue of sufficient integrity to resist removal of the fastener. More specifically, in the depicted embodiment, the barbs extend outward from shaft 110 by the same distance and, as a result of their different leading edge angles 164a, 164b, 164c, therefore have different lengths-i.e., trailing edges 162a, 162b, 162c are disposed at different points along the length of the shaft. For example, trailing edge 162b of barb 112b is closer to shaft proximal end 150 than is trailing edge 162a of barb 112a, and trailing edge 162c of barb 112c is closer to shaft proximal end 150 than is trailing edge 162b of barb 112b. As indicated in FIG. 1C, a proximal-most point or edge 128a of barb 112a is a distance 124 from shaft distal end 114, a proximal-most point or edge 128b of barb 112b is a distance 138 proximal of point or edge 128a, and a proximal-most point or edge 128c of barb 112c is a greater distance 146 proximal of point or edge 128a.

In the embodiment shown, barbs 112a, 112b, 112c are disposed at equiangular intervals around the shaft (i.e., around the cross-sectional perimeter of the shaft). Specifically, three barbs are disposed at angular intervals of 120 degrees. In other embodiments with a different number of barb and equiangular intervals, the equiangular intervals will necessarily also vary (e.g., four barbs would be disposed at angular intervals of 90 degrees. In other embodiments, barbs may be disposed at different angular intervals around the shaft; for example, three barbs could be disposed at 90 degree intervals, such that two of the three barbs would be spaced by 180 degrees.

FIG. 1D depicts a second side view of the fastener of FIG. 1A.

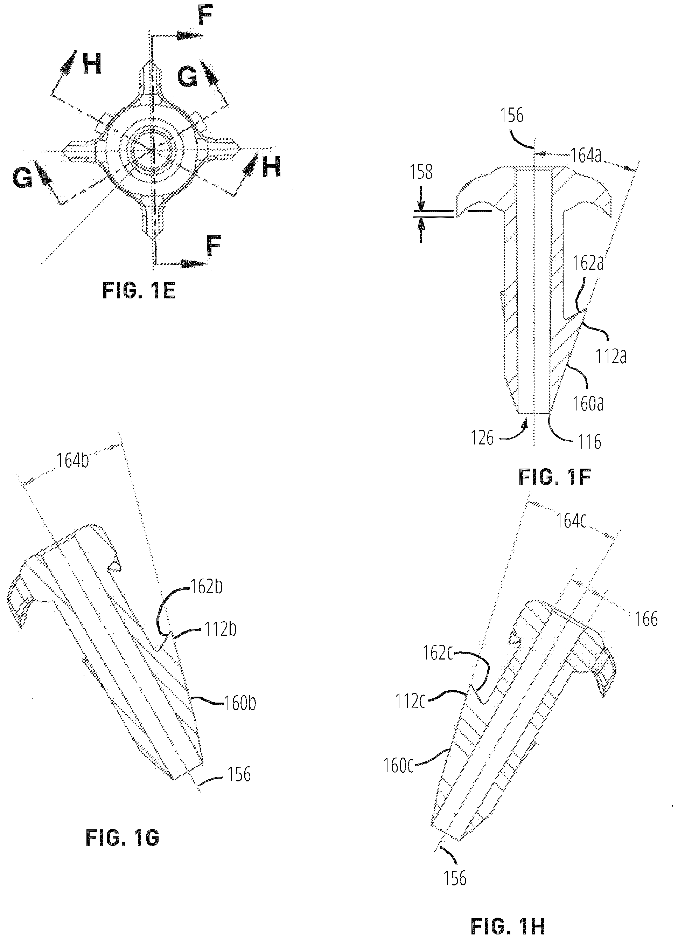

FIG. 1E depicts a proximal end view of the fastener of FIG. 1A.

FIG. 1F depicts the side cross-sectional view of the fastener of FIG. 1A, along the plane F-F of FIG. 1E.

In some embodiments, such as the one shown, a distal end 132 of each outrigger 102 tapers to an edge 122 and a point 120, which point is configured to assist with engaging an implant (as described herein) that is secured by fastener 108, for example by extending partially into or through the implant (e.g., between fibers of a fibrous implant). To further improve engagement with an underlying implant, outriggers 102 of the depicted embodiment are configured such that the respective distal ends-points 120-extend distally of the distal side

(136) of head 106 by a distance 158 (FIG. 1F). Stated another way, when measured parallel to a central, longitudinal axis 156 of the shaft, each of the outrigger distal ends (points 120) is closer to shaft distal end 114 than is at least a portion of distal side 114 of head 106. Other embodiments omit edge 122 but retain point 120 and/or, instead of point 120, may include a rounded, flattened, or roughened distal end (e.g., which may still extend distally of head distal side 136). In the embodiment shown, outriggers 102 are disposed at equiangular intervals around the head. Specifically, four outriggers are disposed at angular intervals of 90 degrees. In other embodiments with a different number of outriggers and equiangular intervals, the equiangular intervals will necessarily also vary (e.g., three outriggers would be disposed at angular intervals of 120 degrees. In other embodiments, outriggers may be disposed at different angular intervals around the head; for example, if one outrigger 102 were omitted from fastener 108, it would leave three outriggers at 90 degree intervals. By way of example, a fastener so modified may be advantageous for use near an edge of an implant (e.g., so the two opposing outriggers separated by an angular interval of 180 degrees could be parallel to the implant edge and the third outrigger could extend inward away from the implant edge).

As shown, each of barbs 112a, 112b, 112c extends longitudinally along a portion of a length 130 of the shaft. In this configuration, each barb extends linearly along the shaft, but in other embodiments, the barbs may be helical or otherwise curved or angled along the shaft. Each of the barbs has a respective distal leading edge 160a, 160b, 160c, and a respective proximal trailing edge 162a, 162b, 162c that is spaced from shaft proximal end 150. For each barb, the leading edge faces distally (toward shaft distal end 114) and is disposed between the shaft distal end and the respective trailing edge. As shown, each of the leading edges is disposed at an acute angle relative to longitudinal axis 156 to facilitate insertion into tissue and/or an implant, and each of the trailing edges is also disposed at an acute angle relative to longitudinal axis 156 to resist removal of the fastener once inserted. In some embodiments, the leading edges of the barbs are disposed at different angles. For example, leading edge 160a of barb 112a is disposed at a first angle 164a, leading edge 160b of barb 112b is disposed at a second angle 164b that is smaller than first angle 164a, and leading edge 160c of barb 112c is disposed at a third angle 164c that is smaller than second angle 164b. These angles (164a, 164b, and 164c) can be selected to manage the force required for insertion and minimize damage to tissue and/or an implant, while maintaining sufficient resistance to removal. For example, for a given barb length, as the angle of the leading edge increases, so does the distance the barb extends from the shaft and the resistance to removal. However, the farther the barb extends outward from the shaft, the greater the chances of tissue fibers being damaged by insertion of the fastener instead of simply permitting the barb to slip past those tissue fibers so they can be engaged by the barb's trailing edge. As such, in the depicted embodiment, the angles of the leading edges decrease with the length of the barbs. In particular, barb 112a has the shortest length (parallel to axis 156) and the greatest leading edge angle 164a, barb 112c has the longest length and the smallest leading edge angle 164c, and barb 112c has a length and a leading edge angle 164b between those of barbs 112a and 112c. In some embodiments, angle 164a is between 15 and 30 degrees, for example, between any two of: 15, 17.5, 20, 22.5, and/or 25 degrees (e.g., between 17.5 and 22.5 degrees); angle 164b is between 10 and 20 degrees, for example, between any two of 10, 12.5, 15, 17.5, and/or 20 degrees (e.g., between 12.5 and 17.5 degrees); and angle 164c is between 7.5 and 17.5 degrees, for example, between any two of 7.5, 10, 12.5, 15, and/or 17.5 degrees (e.g., between 10 and 15 degrees). In the depicted embodiment, angle 164a is between 19 and 20 degrees, angle 164b is between 15 and 16 degrees, and angle 164c is between 12 and 13 degrees.

FIG. 1G depicts the side cross-sectional view of the fastener of FIG. 1A, along the plane G-G of FIG. 1E.

FIG. 1H depicts the side cross-sectional view of the fastener of FIG. 1A, along the plane H-H of FIG. 1E.

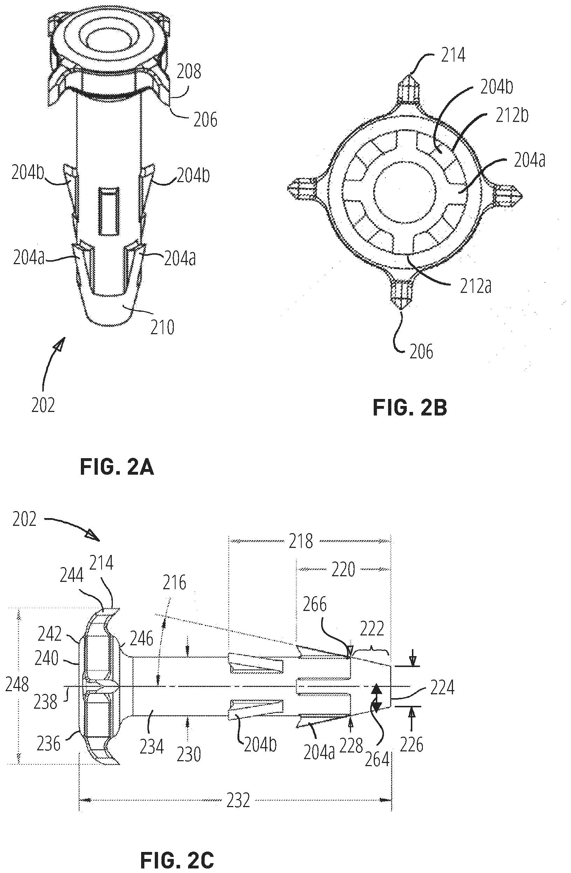

FIG. 2A depicts a perspective view of a first embodiment of the present bone fasteners.

Referring next to FIGs. 2A-2D, a first embodiment of the exemplary fasteners 202 is shown. In the depicted example, fastener 202 is configured for coupling an implant (as described herein) to bone, such as the humerus (e.g., to repair the supraspinatus tendon or rotator cuff). As shown, fastener 202 comprises an elongated shaft 234, an enlarged head 236, a plurality of outriggers 244, a plurality of first barbs 204a, and a plurality of second barbs 204b.

Shaft 234 extends from a shaft proximal end 240 (and enlarged head 236) to a shaft distal end 224. Shaft distal end 224 is configured to be inserted (along with at least first barbs 204a) into bone of a patient such that the barbs resist removal of the fastener from the bone. For example, in at least some uses, the distal end can first be inserted through an implant such that inserting the distal end into bone couples the implant to the bone. To that end, outriggers 244 each extend outward from head 236 to resist removal of such an implant over the head. Outriggers 244 can be particularly advantageous when the fastener is used in conjunction with a fibrous (e.g., woven) or fabric-like implant, the flexibility of which may otherwise (without outriggers) be more-susceptible to slipping over the head.

FIG. 2B depicts a distal end view of the fastener of FIG. 2A.

In some embodiments, it can be advantageous for at least the first barbs or the second barbs to not be radially aligned with any of the outriggers, such that if a barb cuts a slot or creates a tear in an implant when the distal end is inserted through the implant, then the outriggers are more likely to not be aligned with that slot or tear and thus reduce the changes of the slot or tear slipping over or around head 236 and outriggers 244. For example, as shown in FIG. 2B, only first barbs 204a are radially aligned with outriggers 244, and second barbs 112b are not radially aligned with any of the outriggers. Additionally, in some embodiments, the outriggers extend farther outward radially than the barbs, such that-even if all of the barbs cut or tear an implant through which the fastener is inserted-the outriggers (and particularly points 206) are more likely to engage portions of the implant that are outside those cuts or tears. In other embodiments, all of the first and second barbs can be rotated relative to the outriggers such that none of the barbs are radially aligned with the outriggers.

FIG. 2C depicts a side view of the fastener of FIG. 2A.

As shown, head 236 includes a head proximal side 242, and a head distal side 246. In this configuration, at least a portion of the head distal side is longitudinally aligned with shaft proximal end 240, for example as shown in FIG. 2C.

In some embodiments, such as the one shown, a distal end 214 of each outrigger 244 tapers to an edge 208 and a point 206, which point is configured to assist with engaging an implant that is secured by fastener 202, for example by extending partially into or through the implant (e.g., between fibers of a fibrous implant). To further improve engagement with an underlying implant, outriggers 244 of the depicted embodiment are configured such that the respective distal ends-points 206-extend distally of another part of the distal side of the respective outrigger. For example, a distal surface of each outrigger is curved to from a concave surface. In this embodiment, points 206 are longitudinally even with distal side 246 of head 236; however, in other embodiments, points 206 may extend distally of the distal side (246) of head 236 by a distance (e.g., similar to outriggers 102 of fastener 108). Other embodiments omit edge 208 but retain point 206 and/or, instead of point 206, may include a rounded, flattened, or roughened distal end (e.g., which may still extend distally of head distal side 246). In the embodiment shown, outriggers 244 are disposed at equiangular intervals around the head. Specifically, four outriggers are disposed at angular intervals of 90 degrees. In other embodiments with a different number of outriggers and equiangular intervals, the equiangular intervals will necessarily also vary (e.g., three outriggers would be disposed at angular intervals of 120 degrees. In other embodiments, outriggers may be disposed at different angular intervals around the head; for example, if one outrigger 244 were omitted from fastener 202, it would leave three outriggers at 90 degree intervals. By way of example, a fastener so modified may be advantageous for use near an edge of an implant (e.g., so the two opposing outriggers separated by an angular interval of 180 degrees could be parallel to the implant edge and the third outrigger could extend inward away from the implant edge).

As shown, each of the first and second barbs (204a, 204b) extends longitudinally along a portion of a length 232 of the shaft. In this configuration, each barb extends linearly along the shaft, but in other embodiments, the barbs may be helical or otherwise curved or angled along the shaft. Each of the barbs has a respective distal leading edge 252a, 252b, and a respective proximal trailing edge 250a, 250b that is spaced from shaft proximal end 240. For each barb, the leading edge faces distally (toward shaft distal end 224) and is disposed between the shaft distal end and the respective trailing edge. As shown, each of the leading edges is disposed at an acute angle relative to longitudinal axis 238 to facilitate insertion into tissue, and each of the trailing edges is also disposed at an acute angle relative to longitudinal axis 238 to resist removal of the fastener once inserted.

In the embodiment shown, the leading edges of the first and second barbs are all disposed at a common angle 216. The angle or angles of the leading edges of the first and second barbs can be selected to manage the force required for insertion and minimize damage to bone tissue, while maintaining sufficient resistance to removal. For example, for a given barb length, as the angle of the leading edge increases, so does the distance the barb extends from the shaft and the resistance to removal. However, the farther the barb extends outward from the shaft, the greater the chances of tissue fibers being damaged by insertion of the fastener instead of simply permitting the barb to slip past those tissue fibers so they can be engaged by the barb's trailing edge. In some embodiments, angle 216 is between 7.5 and 17.5 degrees, for example, between any two of 7.5, 10, 12.5, 15, and/or 17.5 degrees (e.g., between 10 and 15 degrees). In the depicted embodiment, angle 216 is between 12 and 13 degrees. In other embodiments, the leading edges of the first barbs are disposed at a first angle, and the leading edges of the second barbs are disposed at a second angle that is different than the first angle. For example, the leading edges of first barbs 204a can be disposed at first angle that is between 7.5 and 17.5 degrees, for example, between any two of 7.5, 10, 12.5, 15, and/or 17.5 degrees (e.g., between 10 and 15 degrees, or between 12 and 13 degrees), and the leading edges of the second barbs 204b can be disposed at a second angle that is between 10 and 20 degrees, for example, between any two of 10, 12.5, 15, 17.5, and/or 20 degrees (e.g., between 12.5 and 17.5 degrees, or between 15 and 16 degrees).

Additionally, in the depicted embodiment, the trailing edges of the first barbs (204a) and the trailing edges of the second barbs (204b) are staggered along the length of shaft to increase the likelihood that at least one of the barbs will securely engage bone tissue. For example, when used to secure an implant over a portion of a bone (e.g., the humerus), the implant may have different thicknesses in different regions or may have not lay perfectly against the bone, such that barbs disposed at different distances from the head of the fastener make it more likely that at least one set of barbs will encounter and engage bone tissue underlying the implant to resist removal of the fastener. More specifically, in the depicted embodiment, first barbs 204a are disposed closer to shaft distal end 224 than to shaft proximal end 240, and second barbs 204b are disposed closer to the shaft proximal end 240 such that their trailing edges 250b are disposed between the trailing edges 250a of the first barbs and shaft and head 236. Stated another way, trailing edges 250b of second barbs 204b are closer to shaft proximal end 240 than are trailing edges 250a of first barbs 204a. As indicated in FIG. 2C, a proximal-most points or edges 212a of first barbs 204a are a distance 220 from shaft distal end 224, and proximal-most points or edges 212b of second barbs 204b are a greater distance 218 proximal of points or edges 212a.

In the embodiment shown, first barbs 204a are disposed at equiangular intervals around the shaft (i.e., around the cross-sectional perimeter of the shaft), and second barbs 204b are also disposed at equiangular intervals around the shaft. Specifically, four first barbs 204a are disposed at angular intervals of 90 degrees, and four second barbs 204b are disposed at angular intervals of 90 degrees. In other embodiments with a different number of barb and equiangular intervals, the equiangular intervals will necessarily also vary (e.g., three barbs would be disposed at angular intervals of 120 degrees. In other embodiments, barbs may be disposed at different angular intervals around the shaft; for example, three barbs could be disposed at 90 degree intervals, such that two of the three barbs would be spaced by 180 degrees.

To further facilitate insertion through an implant and into soft tissue, an outer surface 210 of the shaft defines a tapered section 222 adjacent shaft distal end 224. Tapered section 222 extends from a first outer transverse dimension 226 at shaft distal end 224 and increases in size in a proximal direction to a larger outer transverse dimension 228 that may, as in the depicted embodiment, be substantially equal to an outer transverse dimension along a remainder of the length of the shaft between the tapered section and the head (236). In the embodiment shown, tapered section 222 is tapered linearly at a taper angle 264 relative to axis 238, but in other embodiments may be tapered in a non-linear fashion (e.g., along a curved path). Taper angle 264 may in some embodiments be equal to angle 216, and/or may be between 7.5 and 17.5 degrees, for example, between any two of 7.5, 10, 12.5, 15, and/or 17.5 degrees (e.g., between 10 and 15 degrees, or between 12 and 13 degrees). For example, in the depicted embodiment, taper angle 264 is between 19 and 20 degrees and is equal to angle 216 such that tapered section 222 and leading edge 266 follow a continuous, linear path.

In the embodiment shown, the shaft (234) is also configured to receive a portion of a delivery tool (described below). In particular, an internal surface 254 of the shaft defines an internal passage 258 that extends through the length of the shaft along axis 238 and through both of proximal end 240 and distal end 224. Passage 258 is configured to receive a portion of a tool (e.g., a spike or trocar) such that that the tool extends through passage 258 and beyond distal end 224 to permit the tool to form a pilot hole in the tissue into which the tool (or a user of the tool) can simultaneously drive the fastener and, after the fastener is inserted into the tissue, the tool can thereafter be removed via the passage (258). In some embodiments, passage 258 has a first inner transverse dimension at the shaft proximal end (240) and a smaller second inner transverse dimension at the shaft distal end (224), such that the passage tapers (e.g., linearly) from smaller at the shaft distal end to larger as the passage approaches the shaft proximal end. For example, in the embodiment shown, passage 258 tapers linearly at an angle 260 of one degree. Such a taper can be advantageous in reducing the resistance to removal of a tool after insertion of the fastener. For example, during insertion of the fastener, soft tissue will be displaced and tend to exert an inward force around the perimeter of a tool in the passage. The provision of a taper, such that a proximal portion of the passage is larger than a distal portion of the passage, typically helps mitigate the compressive forces on a tool in the passage during insertion of the fastener and thereby typically reduces resistance to removal of the tool from the passage after such insertion. In some embodiments, such as the one shown, inner surface 254 and outer surface 210 meet at shaft distal end 224 to form an edge 256. Such an edge at the distal end of the shaft minimizes the cross-sectional area of fastener at the distal end to facilitate insertion of the fastener into tissue.

In the embodiment of FIG. 2A -FIG. 2E, shaft 234 has a circular cross-sectional shape, such that an outer transverse dimension 230 is the diameter of a circle that defines the outer perimeter of that shape. In some embodiments, transverse dimension 230 is between 1.75 mm and 2.5 mm, for example, between any two of: 1.75 mm, 1.85 mm, 1.95 mm, 2.05 mm, 2.15 mm, 2.25 mm, 2.35 mm, 2.45 mm, and/or 2.5 mm (e.g., between 2.05 mm and 2.25 mm). For example, in the embodiment shown, diameter 230 is between 2.1 and 2.2 mm. In other embodiments, the shaft can have other cross-sectional shapes, such as, for example, square, hexagonal, octagonal, or the like.

As described above, the barbs of the fastener extend outwardly from the shaft and therefore span a maximum transverse dimension that is larger than that of the shaft, such as, for example, the barbs extend radially to an imaginary circle (perpendicular to axis 156) having a maximum transverse dimension that is between any two of: 110%, 120%, 130%, 140%, 150%, 160%, 170%, 180%, 190%, and/or 200% of the maximum transverse dimension of the shaft (e.g., between 130% and 140% of the diameter of the shaft). For example, in the embodiment shown, barbs 204a, 204b each extend radially outward to a point between 1.4 and 1.5 mm from axis 238 (i.e., each of point/edge 212a, 212b is between 1.4 and 1.5 mm from axis 238).

As also described above, outriggers 244 extend outwardly from enlarged head 282 beyond the lateral extend of the barbs and therefore span a maximum transverse dimension 248 that is larger than that of the barbs, such as, for example, a maximum transverse dimension that is between any two of: 220%, 230%, 240%, 250%, 260%, 270%, 280%, 290%, 300%, 310%, and/or 320% of the maximum transverse dimension of the shaft (e.g., between 260% and 270% of the maximum transverse dimension of that shaft). For example, in the embodiment shown, outriggers 244 each extend radially outward to a point that is between 2.8 mm and 2.9 mm from axis 238.

In some embodiments, the overall length of the fastener is between 9 mm and 15 mm, such as, for example, between any two of 9 mm, 10 mm, 11 mm, 12 mm, 13 mm, 14 mm, and/or 15 mm (e.g., between 10 mm and 13 mm). For example, in the embodiment shown, length 232 is nominally 11.5 mm, and therefore between 11 mm and 12 mm. In other embodiments, length 232 can be selected for a particular use. For example, when using a bone fastener with an implant through which the bone fastener extends, it is generally desirable that the fastener have a shaft length sufficient to permit the fastener to extend through the implant and into the bone a sufficient distance for the trailing edges of all of the barbs to engage bone.

In at least some embodiments, the fastener (i.e., shaft 234, head 236, outriggers 244, and barbs 204a, 204b) are defined by a unitary piece of material. For example, it is sometimes desirable for bone fasteners to be durable and non-bioresorbable and, in such instances, the fastener can be molded of a non-bioresorbable polymer such as polyether ether ketone (PEEK).

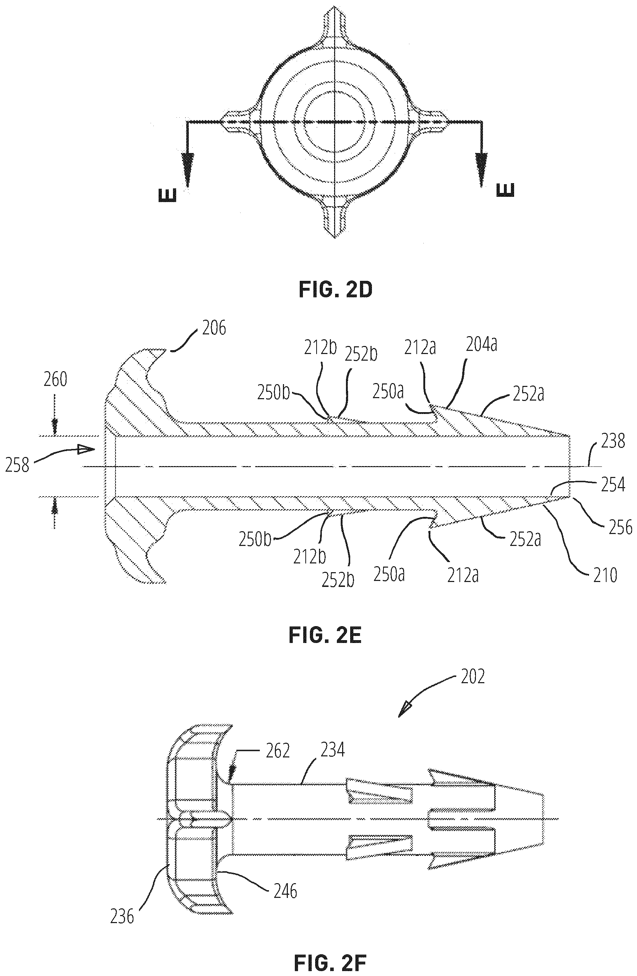

FIG. 2D depicts proximal end view of the fastener of FIG. 2A.

FIG. 2E depicts a side cross-sectional view along the plane D-D of FIG. 2C.

FIG. 2F depicts a side view of a variation of the fastener of FIG. 2A.

FIG. 2F depicts a side view of a variation of the fastener of FIG. 2A. The fastener depicted in FIG. 2F is substantially similar to fastener 802 with the primary difference being that distal side 1306 of head 1504 is entirely concave and forms a continuous concave surface with the distal side of outriggers 102, as shown. Specifically, in the depicted embodiment, a radius 1304 forms a concave surface 262 that meets the nominal diameter of shaft 234.

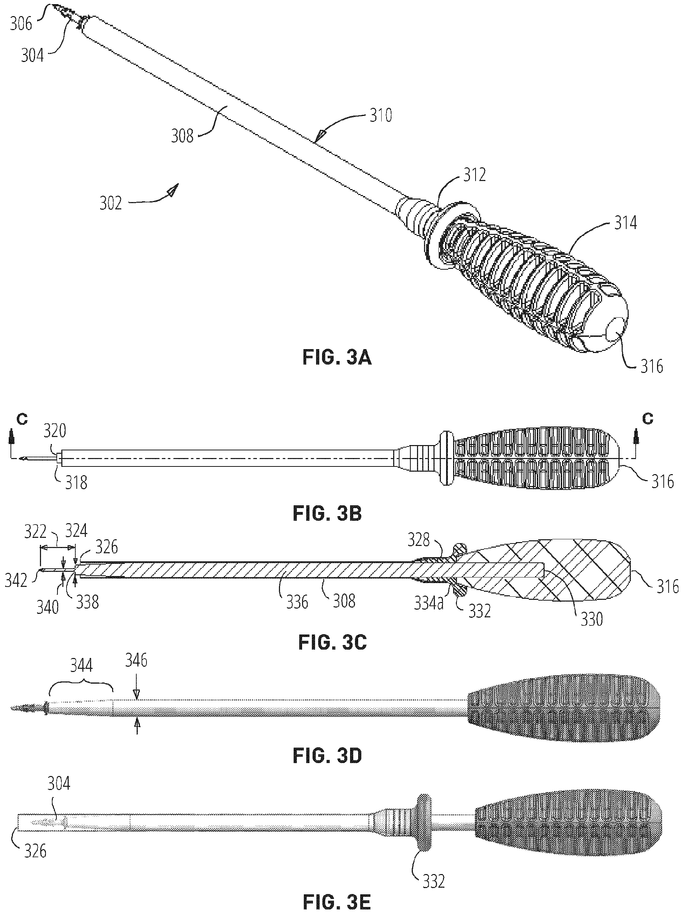

FIG. 3A depicts a perspective view of a first embodiment of an exemplary tool for delivery of a fastener of FIG. 1A or FIG. 2A, with a fastener of FIG. 2A coupled to a distal end of the tool.

Referring now to FIG. 3A -FIG. 3E, a first embodiment of a tool 302 for delivery of a fastener 108 (FIG. 1A - FIG. 1H) or fastener 202 (FIG. 2A -FIG. 2E), shown with a fastener 202 coupled to a distal end of the tool. In the embodiment shown, tool 302 comprises an elongated body 336, a spike 306, and a handle 314, and a movable a shield assembly 310. Body 336 has a proximal end 330 and a distal end 318. Spike 306 has a spike proximal end 338 coupled to body distal end 318, and a spike distal end 342 extending from the body distal end. In the embodiment shown, spike 306 is unitary (formed of a single piece of material) with body 336. For example, body 336 and spike 306 may be machined from a single piece of material (e.g., stainless steel). As shown, spike 306 is sized to extend through the central passage (e.g., 126, 258) of a fastener that the tool is configured to deliver. For example, for a fastener with a passage having a minimum internal diameter of 1 mm, the spike may have an outer diameter of 0.90 mm or 0.95 mm. Additionally, the interface between spike 306 and body 336 is configured to allow a user to push or drive the fastener into tissue (soft tissue or bone). For example, as shown in FIG. 3C, spike proximal end 338 has a transverse dimension 340 that is smaller than a transverse dimension 324 of the body distal end, such that a shoulder 320 is defined at the spike proximal end. Shoulder 320 configured to abut a head (e.g., 106, 236) of a fastener received over the spike, such as is illustrated for fastener 202.

Spike 306 has a length 322 that is greater than a length (e.g., 130, 232) of a fastener with which tool 302 is configured to be used, such that distal end 342 extends beyond the fastener distal end to allow spike distal end 342 to create a pilot hole in tissue (soft tissue or bone tissue) into which the fastener can follow. In some embodiments, spike length 322 is between 3 mm and 8 mm longer than the length (e.g., 130, 232) of a fastener intended for use with the tool, such as, for example, between any two of: 3 mm, 4 mm, 5 mm, 6 mm, 7 mm, 8 mm, and/or 9 mm (e.g., between 4 mm and 6 mm) longer than the corresponding fastener length. For example, in the depicted embodiment, tool 302 is configured to deliver bone fasteners 202 with an overall length of 11.5 mm, and spike 306 is configured to extend 5 mm beyond fastener distal end, such that spike 306 has a length 322 of 16.5 mm (e.g., between 16 mm and 17 mm). In other embodiments, such as those configured solely for delivery of soft-tissue fasteners (e.g., 108) having a length of 6, 7, and/or 8 mm, spike length 322 may be 5 mm longer than the a single intended fastener length (e.g., 11, 12, or 13 mm) for use with a single intended fastener configuration, or may be 5 mm longer than the longest intended fastener length (e.g., 13 mm) for use with any of several intended fastener lengths.

In the embodiment shown, which is configured to deliver a bone fastener, spike distal end 342 is configured as a conventional trocar tip, in which three or four planar surfaces converge at the spike distal end to form a point or chisel tip. In testing of the present fasteners, trocar tips have demonstrated particularly effective performance in forming an initial pilot hole into which the tool (via shoulder 320) can drive bone fastener 202 (e.g., without separately drilling a pilot hole prior to insertion of the spike distal end 342 and fastener 202 together). In other embodiments of tool 302, such as those configured solely for delivery of soft-tissue fasteners (e.g., 108), spike distal end 342 can be configured with a conical point.

In the depicted embodiment, handle 314 is coupled to body proximal end 330. In this configuration, handle 314 is generally symmetrical around a longitudinal axis so that use is not dependent on rotational position. Handle 314 is coupled to body 336 with sufficient rigidity to permit a user to strike (e.g., with a mallet) a proximal end 316 of handle 314 to drive a bone fastener (e.g., 202) into bone. Handle can be machined or molded from a material sufficiently rigid to receive such a strike and that is capable of sterilization, such as, for example, metals and metal alloys such as aluminum or stainless steel, or any of various polymers such as polyphenylsulfone (PPSU).

Shield assembly 310 comprises an elongated tubular shield 308 coupled in fixed relation to a hub 312. Shield 308 has a proximal end 328 coupled to hub 312 and a distal end 326 extending from hub 312. Hub 312 also defines a flange 332 configured to be engaged by a user to retract the shield assembly, wherein a distal portion of the flange 332 tapers distally 334a to a smaller cross-sectional size. More particularly, shield assembly 310 is disposed around body 336 and movable (e.g., slideable) between a retracted position (FIGs. 3A, 3B) in which shield distal end 326 is proximal to shoulder 320, and an extended position (FIG. 3E) in which shield distal end 326 extends past (distal of) spike distal end 342. In use, such as during arthroscopic surgery, the shield can be disposed in the extended position when the fastener is first passed through a cannula or port to a surgical site, such as to protect the fastener and prevent tissue from engaging or "catching" on the fastener before the fastener reaches a desired insertion position. Once the spike distal end 342 and fastener are disposed near a desired insertion position, a user can engage flange 332 to pull shield assembly 310 proximally relative to body 336 and handle 314 (e.g., from the extended position of FIG. 3A to the retracted position of FIG. 3E). A user can, simultaneously or subsequently, press or strike handle 306 in a distal direction to drive the spike distal end (342) and fastener (e.g., 202) into a desired location in soft tissue or bone tissue.

To facilitate insertion of a fastener and the ability of the shield to move relative to the body and fastener, the depicted configuration of body 336 includes a tapered section 344 at shoulder 320. Specifically, tapered section 344 tapers from a nominal transverse dimension (diameter) 346 to the relatively smaller transverse dimension (diameter) 324 at shoulder 320. In this configuration, dimension 324 is larger than the corresponding maximum transverse dimension of the fastener shaft (e.g., 134, 230) but smaller than the corresponding maximum transverse dimension of the outriggers (e.g., 148, 248), such that the outriggers are permitted a degree of flexibility in the longitudinal direction during insertion (e.g., to account for variations in underlying surfaces and facilitate full insertion of the fastener shaft and barbs). Of course, to facilitate the described movement of the shield relative to body 336 and a fastener disposed thereon, shield 308 has an inner transverse dimension (e.g., diameter) that is larger than corresponding maximum transverse dimensions of the fastener (e.g., dimension 148 or 248 of the outriggers) and of the body 336. To facilitate user visibility during use, shield 308 is transparent in at least some embodiments to enable a user to perceive the position of the fastener and spike distal end relative to the distal end of the shield. For example, shield 308 may be formed of polycarbonate or other suitably durable polymers that are capable of being sterilized and/or are transparent or sufficiently translucent to permit a user to perceive the position of a fastener within the shield.

FIG. 3B depicts a side view of the tool of FIG. 3A, shown without the fastener of FIG. 2A.

FIG. 3C depicts a side cross-sectional view of the tool of FIG. 3A, along the plane C-C of FIG. 3B.

FIG. 3D depicts a side view of the tool of FIG. 3A with a shield of the tool omitted.

FIG. 3E depicts the tool of FIG. 3A, with the shield included and disposed in an extended position covering the fastener.

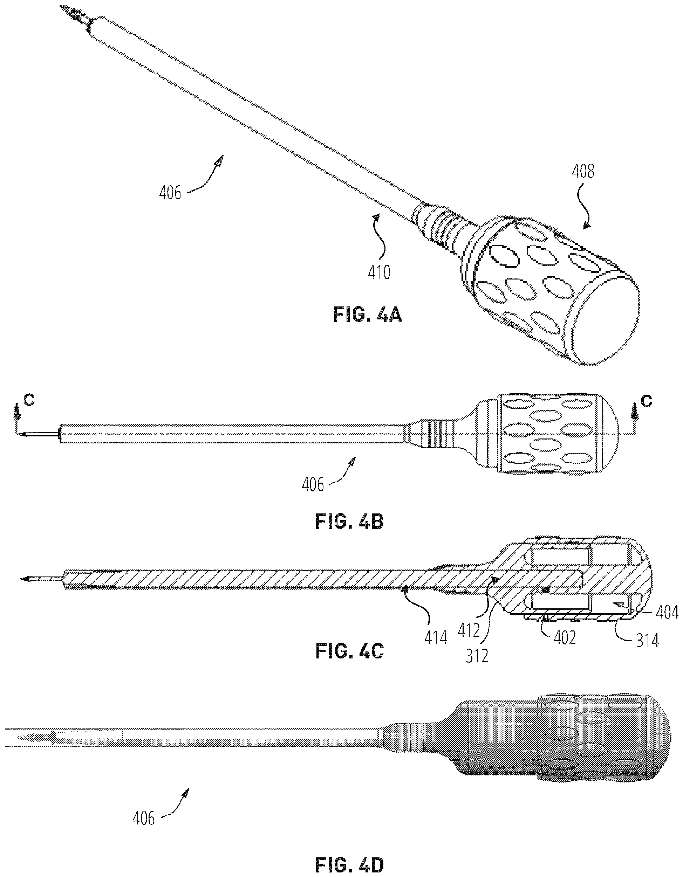

FIG. 4A depicts a perspective view of a second embodiment of a tool for delivery of a fastener of FIG. 1A or FIG. 2A, with a fastener of FIG. 2A coupled to a distal end of the tool.

FIG. 4B depicts a side view of the tool of FIG. 4A, shown without the fastener of FIG. 2A.

FIG. 4C depicts a side cross-sectional view of the tool of FIG. 4A, along the plane C-C of FIG. 4B.

FIG. 4A- FIG. 4D depict a second embodiment 406 of an exemplary tool for delivery of a fastener 108 (FIG. 1A -FIG. 1H) or fastener 202 (FIG. 2A -FIG. 2E), shown with a fastener 202 coupled to a distal end of the tool. Tool 406 is substantially similar to tool 302, with the primary difference being that tool 406 includes a handle of delivery tool 408 that is shaped to interact with an elongated shield hub 410. More particularly, handle of delivery tool 408 defines an annular recess 412 facing distally and configured to receive a proximal portion 404 of hub 410. In the depicted embodiment, tool 406 is particularly well-suited for implementations in which the tool is provided to a user with the tool pre-loaded with a corresponding fastener (e.g., 202). For example, the longitudinal overlap of hub 410 and handle of delivery tool 408 facilitates the inclusion of a frangible pin 402 (shown broken in FIG. 4C) that extends through the handle and into or proximal to the hub when the shield is in the extended position (FIG. 4D) to resist movement of the shield until a user applies sufficient longitudinal compressive force between the handle and the hub to break the pin, and thereby retract the shield and expose the fastener. In other embodiments, instead of the frangible pin, a detent or other structure can be used that resists inadvertent retraction of the shield but still permits a user to overcome that resistance by hand when ready to deploy the fastener. With tools in a pre-loaded state, the present kits can comprise a plurality of (e.g., three to five) bone tools (e.g., 406) pre-loaded with bone fasteners (e.g., 202) and having their respective shield assemblies in an extended position, and/or a plurality of (e.g., six to eight) soft-tissue tools (e.g., 406) pre-loaded with soft-tissue fasteners (e.g., 108) and having their respective shield assemblies in an extended position. Such a kit can be sterile and enclosed in a sealed package (e.g., sealed tray or a blister pack).

FIG. 4D depicts the tool of FIG. 4A, with the shield disposed in an extended position covering the fastener.

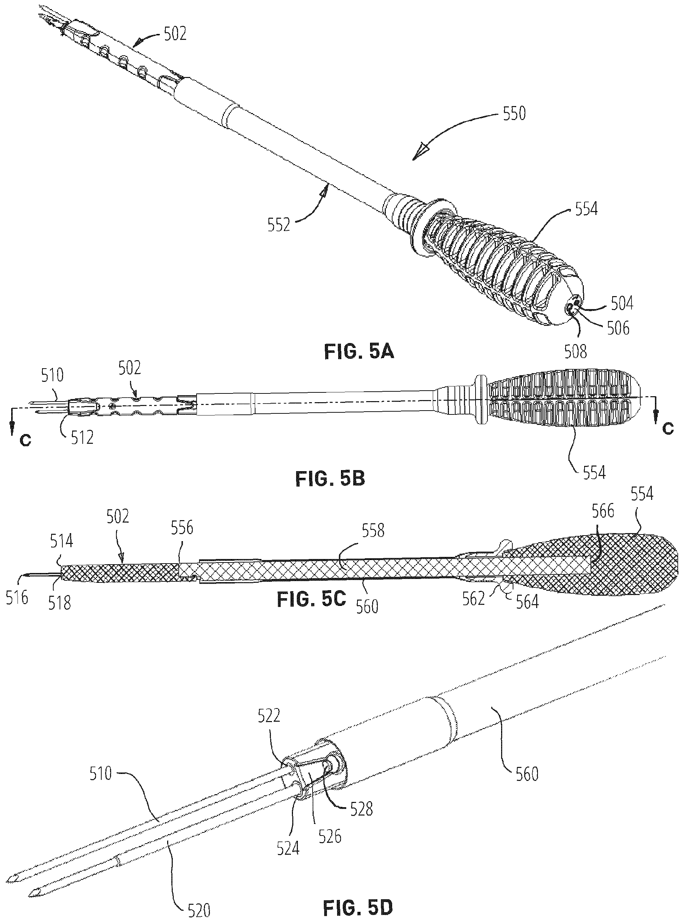

FIG. 5A depicts a perspective view of a third embodiment of a tool for delivery of a fastener of FIG. 1A or FIG. 2A via a cartridge, with a fastener of FIG. 2A coupled to a distal end of the cartridge and the cartridge coupled to a distal end of the tool.

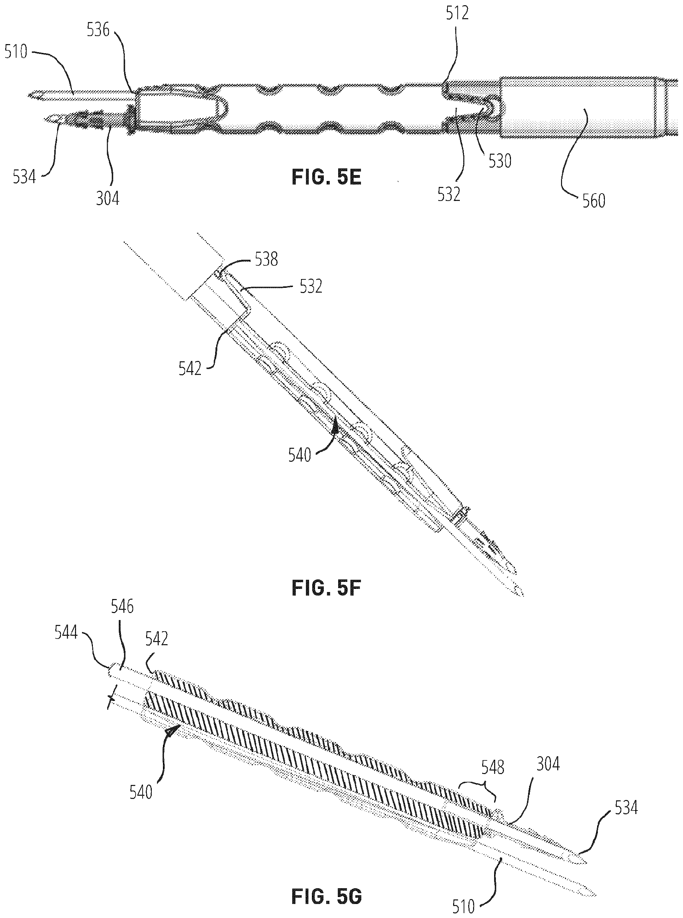

Referring now to FIGS. 5A-5F, FIGS. 5A-5C depict a third embodiment of an exemplary tool 550 including a cartridge 502 for delivery of a bone fastener of FIG. 2A with a bone fastener of FIG. 2A coupled to a distal end of the cartridge; FIG. 5D depicts tool 550 without the cartridge but with a guidewire and a trocar; and FIGS. 5E-5G show enlarged views of the cartridge. Tool 550 is similar in several respects to tool 302, with the primary differences being that tool 550 is configured to facilitate pre-drilling or pre-formation of pilot holes in bone with a spatial reference provided by a guidewire, after which a fastener cartridge 502 can be slipped laterally over the guidewire and engaged by a distal end of the tool to align the fastener with, and insert the fastener into, the pilot hole.

More particularly, body 558 of tool 550 extends from a body proximal end 566 to a body distal end 556. In this configuration, body 558 defines internally a first body passage 522 and a second body passage 524, both of which extend through and between body proximal end 330 and body distal end 318. First body passage 522 has a central, longitudinal first axis, and second body passage 524 has a central, longitudinal second axis separated from the first axis by a distance that remains constant along the body length (i.e., such that the first axis (of the first body passage) is parallel to the second axis (of the second body passage). In the embodiment shown, first body passage 522 is configured to receive a guidewire (e.g., 510), and second body passage 524 configured to receive a trocar (e.g., 520) or rod (e.g., 546). The trocar or rod may be larger than the guidewire, and therefore second body passage 524 may be larger than first body passage 522. Handle 554 is similar to handle 314, with the primary exception that handle 554 is configured to permit access to the first and second body passages 522, 524 through the body proximal end (i.e., through a portion of the handle). More particularly, in this configuration, the handle defines corresponding first and second passages 504, 508 that correspond to (and align with) ones of the first and second passages 522, 524 of the body such that first body passage 522 and first handle passage 504 cooperate to define an essentially continuous first passage between and through handle proximal side 316 and body distal end 318, and such that second body passage 524 and second handle passage 508 cooperate to define an essentially continuous second passage between and through handle proximal side 316 and body distal end 318. In some embodiments, the first and second passages 504, 508 originate at a distal tip 506 of the handle 554.

In the embodiment shown, body distal end 556 is configured to engage a proximal end (512) of the cartridge. More particularly, an outer surface of body 558 defines a recess 526 that extends longitudinally inward from body distal end 556 and radially inward from outermost portions of the cross-sectional perimeter of the body. Recess 526 narrows at it extends proximally to facilitate rotational alignment of the cartridge relative to body 558, and recess 526 further includes a radially deeper portion 528 at a proximal portion of the recess to receive a detent or portion of a projection (532) of the cartridge, as described below, to resist inadvertent longitudinal separation of the cartridge from body distal end 318.

In the depicted embodiment, cartridge 502 includes a proximal end 512 and a distal end 536. Proximal end 512 is configured to engage body distal end 318, and distal end 536 is configured to receive a bone fastener (e.g., 202). Proximal end 512 is configured to engage body distal end 318 via a projection 532 that extends longitudinally outward from a longitudinal engagement surface 542. As shown, projection 532 narrows to a proximal end 530 in a way that corresponds to that of recess 526 to ensure radial alignment of the cartridge relative to body 336 as the cartridge and tool body are pushed together. Projection 532 also includes a radial projection or detent 538 that is configured to extend into the radially deeper portion 528 to resist separation of the cartridge from body distal end 318.

Cartridge 502 defines a longitudinally extending cartridge groove 540 that extends through and between proximal end 512 and distal end 536 of the cartridge, and is open to a lateral external surface of the cartridge such that the cartridge can be laterally slipped over guidewire 510 (such that guidewire 510 is received in groove 540) while a first end of the guidewire is disposed in tissue and a second end of the guidewire extends into first body passage 522 of the tool. Additionally, cartridge 502 comprises a spike 534 coupled to and extending distally from distal end 536 of the cartridge, and a rod 546 coupled to and extending proximally from proximal end 512 of the cartridge. Spike 534 has a spike proximal end 518 coupled to the distal end 536 of the cartridge, and a spike distal end 516 extending from distal end 536 of the cartridge. As described above for spike 306 of tool 302, proximal end 518 of spike 534 has a transverse dimension that is smaller than a transverse dimension of a corresponding portion of distal end 536 of the cartridge, such that a shoulder 514 is defined at spike proximal end 518. And, as with shoulder 320 of tool 302, shoulder 514 is configured to abut a head of a fastener received over the spike (534). Rod 546 has a proximal end 544 extending from cartridge proximal end 536, as shown. In this configuration, the cartridge is configured such that when guidewire 510 is fully received in cartridge groove 540, rod 546 is coaxial with first body passage 524, and cartridge can therefore be moved toward body distal end 318 to insert rod 546 into first body passage 524 and seat cartridge projection 532 into recess 526 to engage tool body 302. Once seated in the respective structures of tool body 302, guidewire 510, rod 546, and projection 532 cooperate to resist movement of cartridge 502 relative to tool body 302. In some embodiments, such as the one shown, spike 534 and rod 546 are unitary (i.e., formed of a single piece of material, such as stainless steel); but in other embodiments may be distinct pieces of material. In some embodiments spike distal end 516 can be configured as a conventional trocar tip, in which three or four planar surfaces converge at the spike distal end to form a point or chisel tip. However, particularly when the cartridge embodiment is primarily configured for separately forming a pilot hole with a distinct trocar, spike distal end 516 can alternatively be configured with conical point.

As with spike 306 described above, spike 534 has a length that is greater than a length (e.g., 130, 232) of a fastener with which tool cartridge 502 is configured to be used, such that the distal end extends beyond the fastener distal end to allow spike distal end 516 to guide the fastener into a pilot hole in bone tissue. In some embodiments, the spike length (from shoulder 514 to spike distal end 516) is between 3 mm and 8 mm longer than the length (e.g., 130, 232) of a fastener intended for use with the tool, such as, for example, between any two of: 3 mm, 4 mm, 5 mm, 6 mm, 7 mm, 8 mm, and/or 9 mm (e.g., between 4 mm and 6 mm) longer than the corresponding fastener length. For example, in the depicted embodiment, cartridge 502 is configured to deliver bone fasteners 202 with an overall length of 11.5 mm, and spike 534 is configured to extend 5 mm beyond fastener distal end, such that spike 306 has a length 322 of 16.5 mm (e.g., between 16 mm and 17 mm).

As described above for body 336, to facilitate insertion of a fastener and the ability of the shield to move relative to the body and fastener, the depicted configuration of cartridge 502 includes a tapered section 548 at shoulder 514. Specifically, tapered section 548 tapers from a nominal transverse dimension of the cartridge to the relatively smaller transverse dimension 516 at shoulder 514. In this configuration, dimension 516 is larger than the corresponding maximum transverse dimension of the fastener shaft (e.g., 134, 230) but smaller than the corresponding maximum transverse dimension of the outriggers (e.g., 148, 248), such that the outriggers are permitted a degree of flexibility in the longitudinal direction during insertion (e.g., to account for variations in underlying surfaces and facilitate full insertion of the fastener shaft and barbs). Of course, to facilitate the described movement of the shield assembly 552 (including shield 560 and hub 562 having a hub flange 564) relative to body 558 and a fastener disposed thereon, shield 560 has an inner transverse dimension (e.g., diameter) that is larger than corresponding maximum transverse dimensions of the fastener (e.g., dimension 148 or 248 of the outriggers) and of the body 558. To facilitate user visibility during use, shield 560 is transparent in at least some embodiments to enable a user to perceive the position of the fastener and spike distal end relative to the distal end of the shield. For example, shield 560 may be formed of polycarbonate or other suitably durable polymers that are capable of being sterilized and/or are transparent or sufficiently translucent to permit a user to perceive the position of a fastener within the shield.

In use, distal end 556 of body 558 (without cartridge 502) is disposed at a position at which a user desires to insert a bone fastener (e.g., 202). A guidewire is then inserted through first handle passage 504 and first body passage 522 into bone tissue adjacent the targeted fastener position. The tool is then rotated around the guidewire, if needed, to align the second body passage 524 with the targeted fastener position, and a trocar 520 is inserted through second handle passage 508 and second body passage 504 pushed and/or rotated into the bone tissue to form a pilot hole in the bone tissue. In some embodiments, trocar 520 has a diameter (at its distal end) that is equal to or slightly smaller than the nominal diameter of the shaft (excluding barbs) of a fastener for which a pilot hole is drilled. For example, the trocar diameter may be between 85% and 100% (e.g., between 90% and 95%) of the fastener shaft diameter.

FIG. 5B depicts a side view of the tool of FIG. 5A, shown without the fastener of FIG. 2A.

FIG. 5C depicts a side cross-sectional view of the tool of FIG. 5A, along the plane C-C of FIG. 5B.

FIG. 5D depicts a perspective view of the distal end of the tool, without the cartridge but with a guidewire and a trocar.

FIG. 5D shows both the guidewire and trocar extending from body distal end 556. Next, trocar 520 is removed, and body 558 retracted sufficiently to permit cartridge 502 to be laterally positioned over guidewire 510 with rod 546 aligned with second body passage 524. Cartridge 502 and tool body 558 are then moved longitudinally together such that rod 546 is inserted into first body passage 524 and cartridge projection 532 is inserted into recess 526 to engage tool body 558. A user can then align spike 534 with the pilot hole in the underlying bone tissue, and advance the tool and cartridge toward the bone tissue, to drive the fastener (202) into the pilot hole and seat the fastener in the bone. If desired, shield assembly 552 can be moved to the extended position before positioning the fastener at or near the pilot hole (to protect the fastener and resist unintended engagement of the fastener with tissue during such positioning), and then shield assembly 552 can be retracted before or while the fastener is driven into the pilot hole.

With cartridges in a pre-loaded state (e.g., cartridge 502 with a fastener 202 pre-loaded on spike 534), the present kits can comprise a plurality of (e.g., three to five) cartridges 502 pre-loaded with bone fasteners (e.g., 202) and a single tool 550. Some such kits can further comprise at least one guidewire 510 and/or at least one trocar 520.. Such a kit can be sterile and enclosed in a sealed package (e.g., sealed tray or a blister pack).

FIG. 5E depicts an enlarged side view of a fastener cartridge of the tool of FIG. 5A with a shield of the tool omitted.

FIG. 5F depicts an enlarged lower perspective view of the fastener cartridge of FIG. 5E.

FIG. 5G depicts a cutaway perspective view of the fastener cartridge of FIG. 5E.

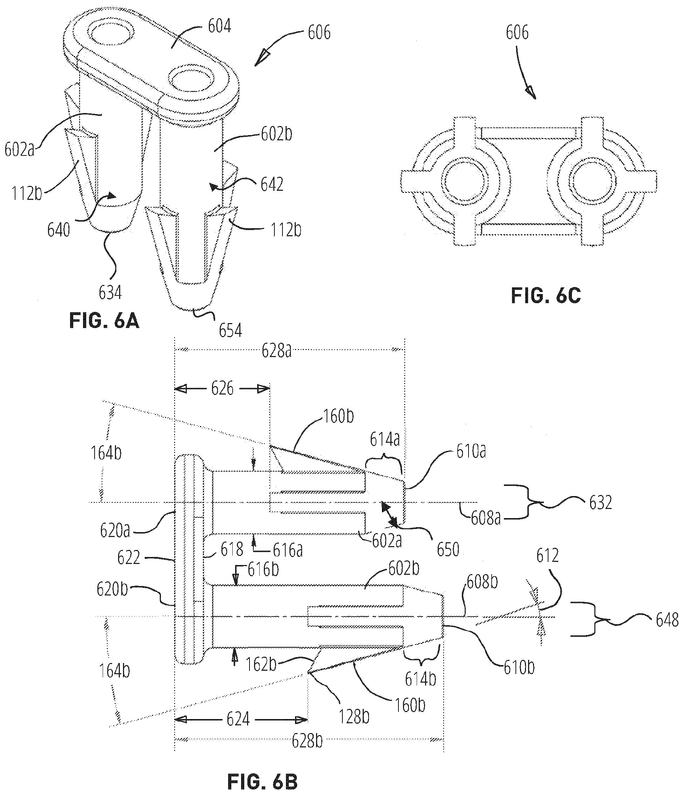

FIG. 6A depicts a perspective view of a second embodiment of the present soft-tissue fasteners.

Referring now to FIGs. 6A-6G, a second embodiment 606 of the exemplarary soft-tissue fasteners is shown. In the depicted example, fastener 606 is configured for coupling an implant to soft tissue, such as a tendon (e.g., the supraspinatus tendon or rotator cuff). As shown, fastener 606 comprises two elongated shafts 602a, 602b, an enlarged head 604, and a plurality of barbs 112b on each of the shafts 602a, 602b.

Each shaft 602a, 602b extends from a respective shaft proximal end 620a, 620b (and enlarged head 181) to a respective shaft distal end 610a, 610b. Shaft distal ends 610a, 610b are configured to be inserted (along with their respective barbs 112b) into soft tissue of a patient such that the barbs resist removal of the fastener from the soft tissue. For example, in at least some uses, the distal ends can first be inserted through an implant such that inserting the distal ends into soft tissue couples the implant to the soft tissue. To that end, enlarged head 604 extends between proximal ends 620a, 620b of the respective shafts to resist removal of such an implant over the proximal ends of the shafts. As shown, shaft 602b is longer than shaft 602a-i.e., length 628b of shaft 602b is larger than length 628a of shaft 602a. As a result, during insertion into soft tissue, distal end 610b of shaft 602b will enter the tissue before distal end 610a of shaft 602a, thereby reducing (relative to a similar structure with two similar shafts of the same length the cross-sectional area of fastener 606 being pushed into the tissue.

In the embodiment shown, each shaft (602a, 602b) is also configured to receive a portion of a delivery tool (described below). In particular, an internal surface 638a, 638b of the respective shaft defines an internal passage 630a, 630b that extends through the length of the shaft along axis 608a, 608b and through both of the respective proximal and distal ends. Each passage 630a, 630b is configured to receive a portion of a tool (e.g., a spike or trocar) such that that the tool extends through passages 630a, 630b and beyond distal ends 610a, 610b to permit the tool to form a pilot hole in the tissue into which the tool (or a user of the tool) can simultaneously drive the fastener and, after the fastener is inserted into the tissue, the tool can thereafter be removed via the passages (630a, 630b). In some embodiments, each passage 630a, 630b has a first inner transverse dimension at the respective shaft proximal end (620a, 620b) and a smaller second inner transverse dimension at the respective shaft distal end (610a, 610b), such that the passage tapers (e.g., linearly) from smaller at the shaft distal end to larger as the passage approaches the shaft proximal end. For example, in the embodiment shown, each passage 630a, 630b tapers linearly at an angle 636a, 636b, each of one degree. Such a taper can be advantageous in reducing the resistance to removal of a tool after insertion of the fastener. For example, during insertion of the fastener, soft tissue will be displaced and tend to exert an inward force around the perimeter of a tool in the passage. The provision of a taper, such that a proximal portion of the passage is larger than a distal portion of the passage, typically helps mitigate the compressive forces on a tool in the passage during insertion of the fastener and thereby typically reduces resistance to removal of the tool from the passage after such insertion. In some embodiments, such as the one shown, each inner surface 638a, 638b and respective 640, 641 meet at the respective shaft distal end 610a, 610b to form a respective first edge 654, and second edge 634. Such an edge at the distal end of the shaft minimizes the cross-sectional area of fastener at the distal end to facilitate insertion of the fastener into tissue.

In the embodiment of FIGs. 6A-6F, each shaft 602a, 602b has a circular cross-sectional shape, such that an outer transverse dimension 616a, 616b is the diameter of a circle that defines the outer perimeter of that shape. In some embodiments, transverse dimensions 616a, 616b are each between any two of: 1.25 mm, 1.35 mm, 1.45 mm, 1.55 mm, 1.65 mm, 1.75 mm, 1.85 mm, 1.95 mm, and/or 2.05 mm (e.g., between 1.55 mm and 1.75 mm). For example, in the embodiment shown, diameters 616a, 616b are each between 1.6 and 1.7 mm. In other embodiments, the shaft can have other cross-sectional shapes, such as, for example, square, hexagonal, octagonal, or the like.

As described above, the barbs of the fastener extend outwardly from the shaft and therefore span a maximum transverse dimension that is larger than that of the shaft, such as, for example, a maximum transverse dimension that is between any two of: 130%, 140%, 150%, 160%, 170%, 180%, 190%, 200%, 210%, 220%, and/or 230% of the maximum transverse dimension of the shaft (e.g., between 170% and 190% of the diameter of the corresponding shaft). For example, in the embodiment shown, barbs 112b each extend radially outward to a point between 1.4 and 1.6 mm from the respective axis 608a, 608b (i.e., each of point/edge 128b is between 1.4 and 1.6 mm from the respective axis 608a, 608b).

In some embodiments, the overall length of the fastener is between 5 mm and 9 mm, such as, for example, between any two of 5 mm, 5.5 mm, 6 mm, 6.5 mm, 7 mm, 7.5 mm, 8 mm, and/or 8.5 mm (e.g., between 5.5 mm and 6.5 mm, between 6.5 mm and 7.5 mm, or between 7.5 mm and 8.5 mm). For example, in the embodiment shown, length 628b second shaft 602b is nominally 7.1 mm, and therefore between 6.5 mm and 7.5 mm, and length 628a of first shaft 602a is nominally 6.1 mm. In other embodiments, lengths 628a, 628b can be selected for a particular use. For example, when using a soft-tissue fastener with a tendon that overlies and must move relative to other anatomical structure, it is generally desirable that the distal end of the fastener not extend into the underlying anatomical structures. As an example of one type of tendon, for use with a relatively smaller person with a relatively thinner supraspinatus tendon, a fastener with maximum nominal length 628b of 6 mm may be appropriate; while, for use with a relatively larger person with a relatively thicker supraspinatus tendon, a fastener with a maximum nominal length 628b of 8 mm may be appropriate.

In at least some embodiments, the fastener (i.e., shafts 602a and 602b, head 604, and barbs 112a) are defined by a unitary piece of material. For example, it is sometimes desirable for soft-tissue fasteners to be bioresorbable and, in such instances, the fastener can be molded of a bioresorbable polymer such as poly lactic-co-glycolic acid (PLGA) or polylactic acid (PLA or PLLA). In some uses, it is desirable for complete resorption to occur in less than 9 months (e.g., between 3 months and 9 months).

FIG. 6B depicts a side view of the fastener of FIG. 6A. As shown, head 604 includes a head proximal side 622, and a head distal side 618. In this configuration, at least a portion of the head distal side is longitudinally aligned with shaft proximal ends 620a, 620b, for example as shown in FIG. 6B. In the embodiment shown, head 604 is substantially planar in that proximal side 622 is defined by a planar surface spanning a majority of a region between the shafts, and distal side 618 is defined by a planar surface spanning a majority of a region between the shafts.

As shown, each of barbs 112b extends longitudinally along a portion of a length 628a, 628b of the respective shaft. In this configuration, each barb extends linearly along the shaft, but in other embodiments, the barbs may be helical or otherwise curved or angled along the shaft. Each of the barbs has a respective distal leading edge 160b and a respective proximal trailing edge 162b that is spaced from the respective shaft proximal end 620a, 620b. For each barb, the leading edge faces distally (toward the respective shaft distal end 610a, 610b) and is disposed between the shaft distal end and the respective trailing edge. As shown, each of the leading edges is disposed at an acute angle relative to longitudinal axis 608a, 608b to facilitate insertion into tissue, and each of the trailing edges is also disposed at an acute angle relative to longitudinal axis 608a, 608b to resist removal of the fastener once inserted. In the embodiment shown, the leading edges of the barbs are disposed at a common angle relative to the respective longitudinal axis. For example, leading edges 160b of barbs 112b are disposed at an angle 164b. In some embodiments, angle 164b is between 10 and 20 degrees, for example, between any two of 10, 12.5, 15, 17.5, and/or 20 degrees (e.g., between 12.5 and 17.5 degrees). In the depicted embodiment, angle 164b is between 14.5 and 15.5 degrees. In other embodiments, the leading edge angles can be varied for different barbs, such as to manage the force required for insertion and minimize local tissue damage, while maintaining sufficient resistance to removal. For example, for a given barb length, as the angle of the leading edge increases, so does the distance the barb extends from the shaft and the resistance to removal. However, the farther the barb extends outward from the shaft, the greater the chances of tissue fibers being damaged by insertion of the fastener instead of simply permitting the barb to slip past those tissue fibers so they can be engaged by the barb's trailing edge. For example, in some embodiment, each shaft 602a, 602b can include a set of three barbs with three different leading edge angles, such as those used for fastener 108 described above.

Additionally, in the depicted embodiment, the trailing edges of the barbs on shaft 602b are staggered along the length of shaft relative to those on shaft 602a to increase the likelihood that at least some of the barbs will securely engage soft tissue. For example, when used with certain tendons (e.g., the supraspinatus tendon), the tendon may have different thicknesses in different regions or may have partial-thickness tears, such that barbs disposed at different distances from the head of the fastener make it more likely that at least one barb will encounter and engage soft tissue of sufficient integrity to resist removal of the fastener. More specifically, in the depicted embodiment, the longer second shaft 602b places trailing edges 162b on the first shaft closer to head 604 than are the barbs on first shaft 602a. As indicated in FIG. 6B, a proximal-most point or edge 128a of each barb 112b on shaft 602a is a distance 626 distal of shaft proximal end 620a, and a proximal-most point or edge 128a of each barb 112b on shaft 602b is a larger distance 624 distal of shaft proximal end 620b.

In the embodiment shown, barbs 112b on first shaft 602a are disposed at equiangular intervals around one half of that shaft, and barbs 112b on second shaft 602b are disposed at equiangular intervals around an opposing half of that shaft (i.e., such that two of the barbs on a shaft are separated by 180 degrees, and the barbs on each shaft do not extend toward the other shaft). Specifically, on shaft 602a, three barbs 112b are disposed at angular intervals of 90 degrees and on shaft 602b, three barbs 112b are disposed at angular intervals of 90 degrees. In other embodiments with a different number of barbs and equiangular intervals, the equiangular intervals will necessarily also vary (e.g., four barbs in one half of a shaft perimeter would be disposed at angular intervals of 60 degrees. In other embodiments, barbs may be disposed at different angular intervals around the shaft.

To further facilitate insertion through an implant and into soft tissue, an outer surface 640, 642 of each shaft defines a tapered section 614a, 614b adjacent a respective one of shaft distal ends 610a, 610b. Each tapered section 614a, 614b extends from a first outer transverse dimension 648, 632 at the respective shaft distal end (610a, 610b) and increases in size in a proximal direction to a larger outer transverse dimension 616a, 616b that may, as in the depicted embodiment, be substantially equal to an outer transverse dimension along a remainder of the length of the shaft between the tapered section and the head (604). In the embodiment shown, each tapered section 614a, 614b is tapered linearly at a taper angle 612 relative to respective axis 608a, 608b, but in other embodiments may be tapered in a non-linear fashion (e.g., along a curved path). Angle 650 may in some embodiments be equal to angle 164b, and/or may be between 10 and 20 degrees, for example, between any two of 10, 12.5, 15, 17.5, and/or 20 degrees (e.g., between 12.5 and 17.5 degrees). For example, in the depicted embodiment, angle 612 is between 14.5 and 15.5 degrees, and is equal to angle 164b such that tapered section 144 and leading edge 160b follow a continuous, linear path.

FIG. 6C depicts a distal end view of the fastener of FIG. 6A.

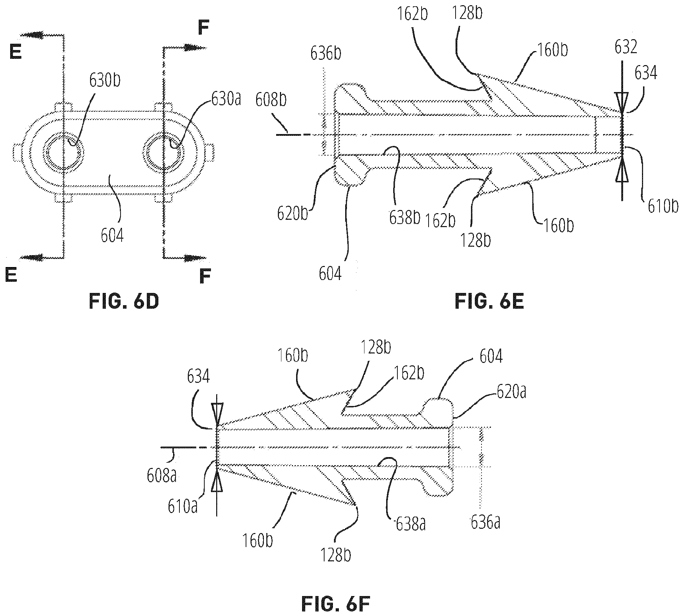

FIG. 6D depicts a proximal end view of the fastener of FIG. 6A.

FIG. 6E depicts side cross-sectional views of the fastener of FIG. 6A, along the planes E-E of FIG. 6D.

FIG. 6F depicts side cross-sectional views of the fastener of FIG. 6A, along the planes F-F of FIG. 6D.

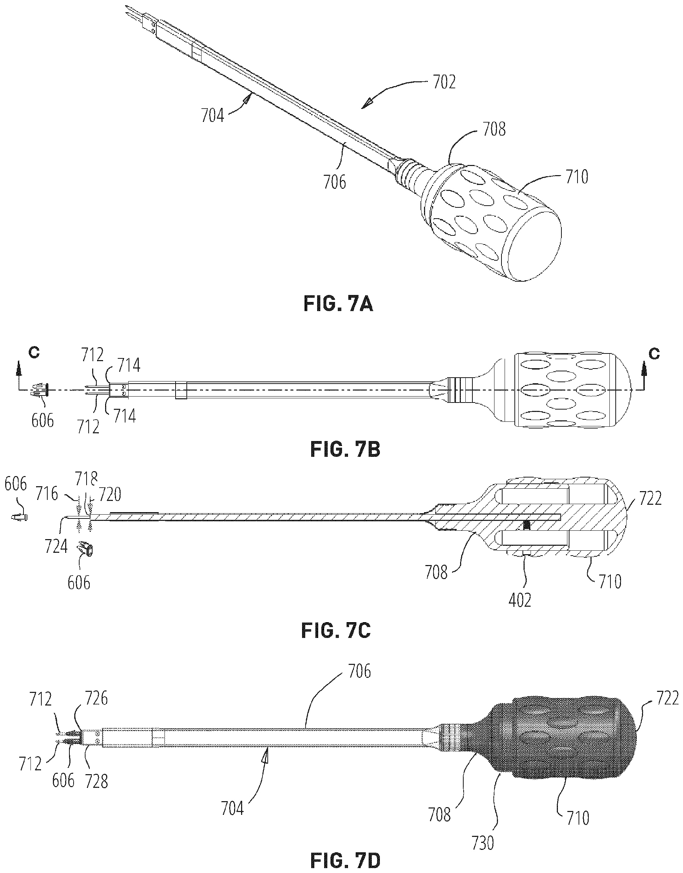

FIG. 7A depicts a perspective view of an embodiment of an exemplary tool for delivery of a fastener of FIG. 6A, with a fastener of FIG. 6A coupled to a distal end of the tool.

Referring now to FIG. 7A -FIG. 7D, an embodiment of a tool 702 for delivery of a fastener 606 of FIG. 6A is shown, with a fastener 606 shown adjacent a distal end of the tool (FIG. 7A -FIG. 7C) or coupled to a distal end of the tool (FIG. 7D). Tool 702 is substantially similar to tool 406, with the primary difference being that tool 702 comprises two spikes 712 that are spaced apart and parallel to each other to receive a fastener 606 with one of spikes 712 in each of passages 630a, 630b of respective fastener shafts 602a, 602b. Of course, to facilitate use of tool 702 to drive a fastener into soft tissue, each spike proximal end 718 has a transverse dimension 716 that is smaller than a corresponding transverse dimension 720 of the body distal end such that a shoulder 714 is defined at each spike proximal end 718. And, as described above for shoulder 320 of tool 406, the shoulder(s) (714) is/are configured to abut a head of a fastener received over the pair of spikes. In some embodiments, tool 702 comprises a shield assembly having shield 706 and hub 708 having a hub flange 730. In some embodiments tool 702 comprises a handle 710 having a proximal tip 722, similar to other tools described herein.

As described above for tool 406, tool 702 is also particularly well-suited for implementations in which the tool is provided to a user with the tool pre-loaded with a corresponding fastener (e.g., 606). For example, the longitudinal overlap of hub 708 and handle 710 facilitates the inclusion of a frangible pin 402 (shown broken in FIG. 7C) that extends through the handle 710 and into or proximal to the hub when the shield is in the extended position (in which distal end of shield 706 extends beyond distal ends 724 of spikes 712) to resist movement of the shield until a user applies sufficient longitudinal compressive force between the handle and the hub to break the pin, and thereby retract the shield and expose the fastener. In other embodiments, instead of the frangible pin, a detent or other structure can be used that resists inadvertent retraction of the shield but still permits a user to overcome that resistance by hand when ready to deploy the fastener. With tools 702 in a pre-loaded state, the present kits can comprise a plurality of (e.g., three to five) soft-tissue tools (e.g., 702) pre-loaded with soft-tissue fasteners (e.g., 606) and having their respective shield assemblies in an extended position, and/or a plurality of (e.g., six to eight) bone tools (e.g., 406) pre-loaded with bone fasteners (e.g., 802, described below) and having their respective shield assemblies in an extended position. Such a kit can be sterile and enclosed in a sealed package (e.g., sealed tray or a blister pack).

FIG. 7B depicts a side view of the tool of FIG. 7A.

FIG. 7C depicts a side cross-sectional view of the tool of FIG. 7A, along the plane C-C of FIG. 7B.

FIG. 7D depicts an additional side view of the tool of FIG. 3A.

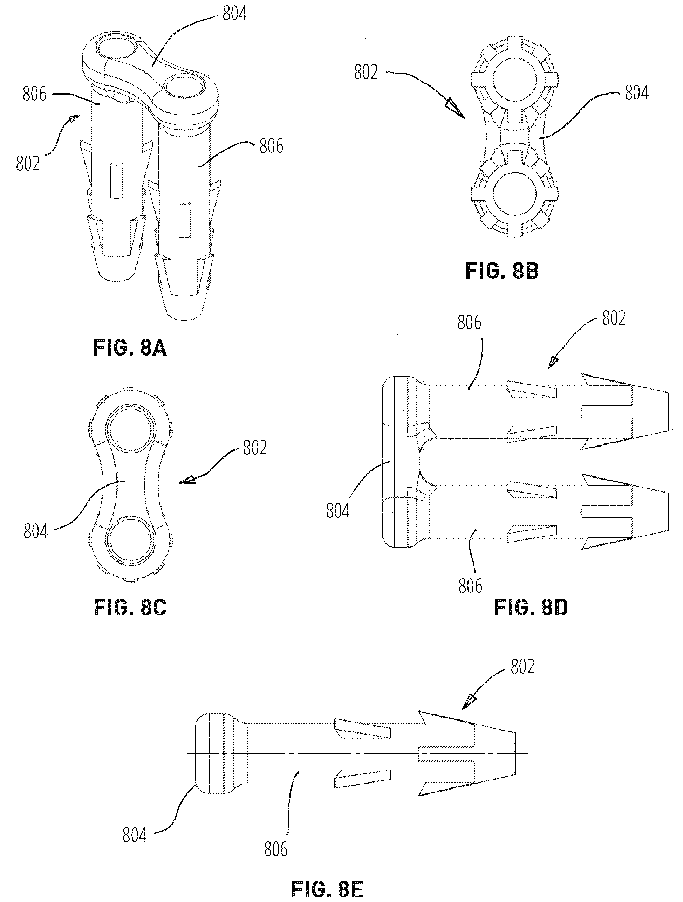

Referring now to FIG. 8A -FIG. 8C, a second embodiment 802 of exemplary fasteners is shown. FIG. 8A depicts a perspective view of the second embodiment of exemplary bone fasteners. Fastener 802 is substantially similar to fastener 202 with the primary difference being that fastener 802 includes two shafts 806 and an enlarged head 804 that spans both shafts 806, and that fastener 802 omits outriggers 102. Each of shafts 806 is substantially similar to shaft 234.

FIG. 8B depicts a distal end view of the fastener of FIG. 8A

FIG. 8C depicts a proximal end view of the fastener of FIG. 8A.

FIG. 8D depicts a first side view of the fastener of FIG. 8A.

FIG. 8E depicts a second side view of the fastener of FIG. 8A.

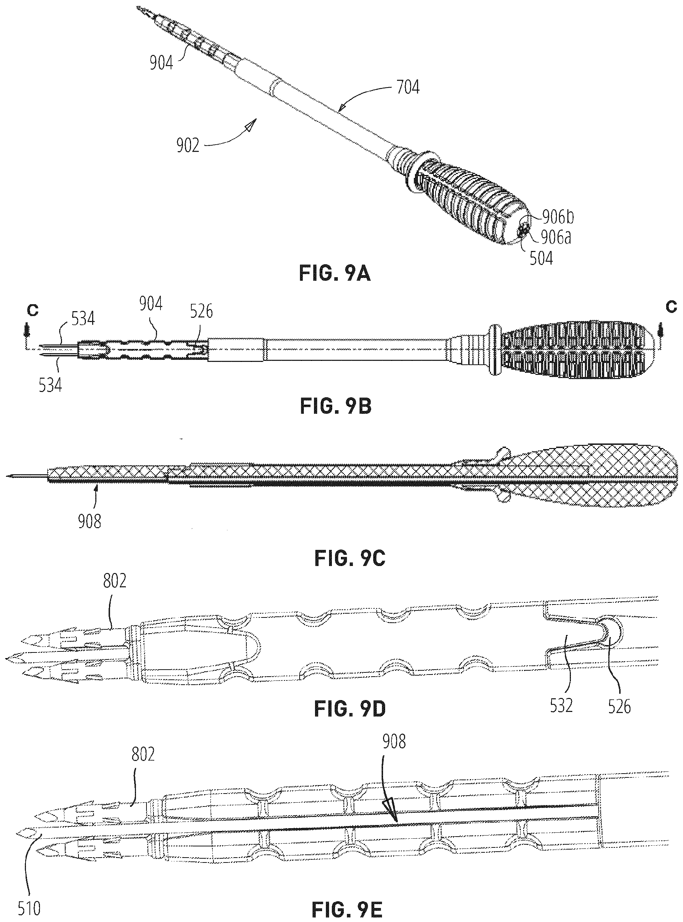

FIG. 9A depicts a perspective view of an embodiment of an exemplary tool for delivery of a fastener of FIG. 8A via a cartridge, with a fastener of FIG. 8A coupled to a distal end of the cartridge, and the cartridge coupled to a distal end of the tool.

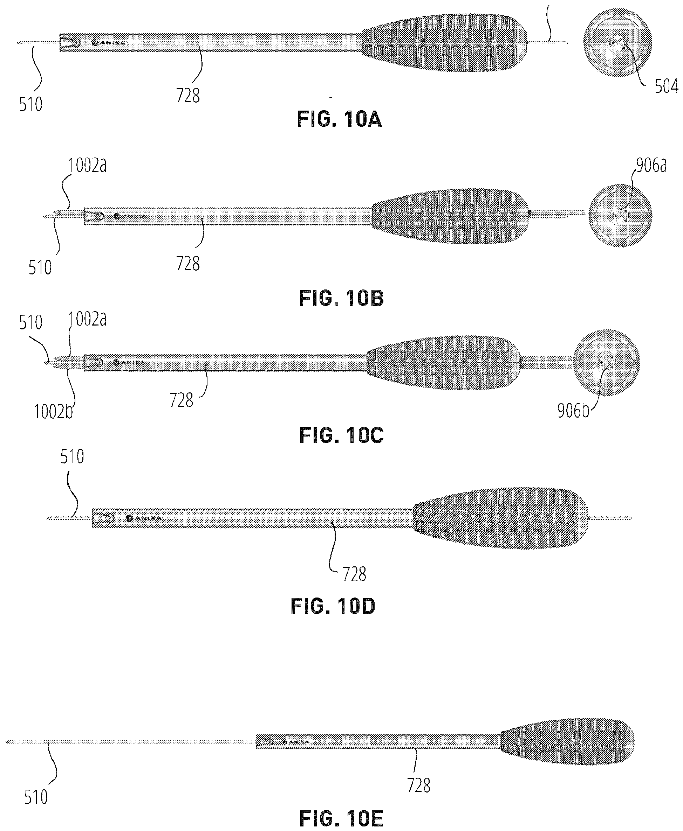

Referring now to FIGS. 9A-9G and FIGs. 10A-10K; FIGS. 9A-9G depict an embodiment of an exemplary tool 902 for delivery of a fastener 802 via a cartridge 904, with a fastener 802 coupled to a distal end of the cartridge (FIG. 9A and FIG. 9D- FIG. 9F), and the cartridge coupled to a distal end of the tool; and FIGs. 10A-10K depict an exemplary sequence of steps for deployment of a fastener 802 via tool 902.

Tool 902 is substantially similar to tool 550, with the primary exception being that tool 902 includes two trocar passages aligned with handle passages 906a, 906b in addition to guidewire aligned with handle passage 504.

FIG. 9B depicts a side view of the tool of FIG. 9A.

FIG. 9C depicts a side cross-sectional view of the tool of FIG. 9A, along the plane C-C of FIG. 9B.

Cartridge 904 is substantially similar to cartridge 502, with the primary exception being that cartridge 904 includes two spikes 534 on a first side 910, and two rods 546, and that groove 908 opens laterally in a direction that opposes projection 532 and is perpendicular to a plane passing through both of spikes 534 (see FIG. 9B). As shown, spikes 534 are spaced apart and parallel to each other to receive a fastener 802 with one of spikes 534 in each of passages 258 of respective fastener shafts 806, 214b. As described above for shoulder 514 of cartridge 502, shoulder(s) (648a) is/are configured to abut a head of a fastener received over the pair of spikes.

FIG. 9D depicts an enlarged first side view of a fastener cartridge of the tool of FIG. 9A.

FIG. 9E depicts an enlarged second side view of the fastener cartridge of the tool of FIG. 9A.

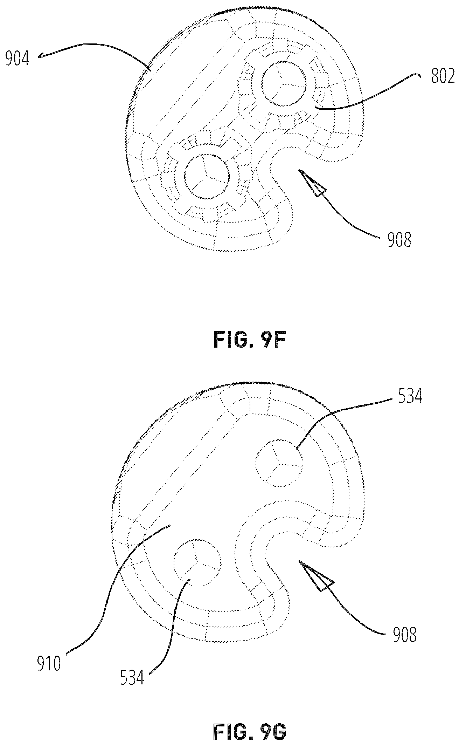

FIG. 9F depicts a distal end view of the tool of FIG. 9A, with the bone fastener coupled to the cartridge.

FIG. 9G depicts a distal end view of the tool of FIG. 9A, with the bone fastener omitted.

FIG. 10A depicts an exemplary step for a sequence of deployment of a fastener of FIG. 8A via the tool of FIG. 9A.

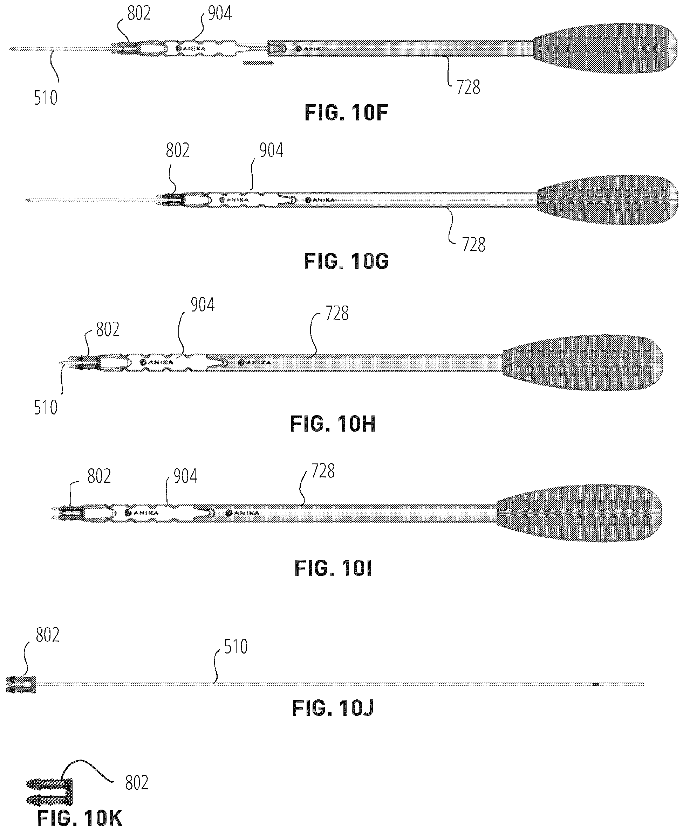

In use, distal end 726 of body 728 (without cartridge 904) is disposed at a position at which a user desires to insert a bone fastener (e.g., 802). A guidewire 510 is then inserted through first handle passage 504 and the corresponding guidewire passage in body 728 into bone tissue adjacent the targeted fastener position (FIG. 10A). The tool is then rotated around the guidewire, if needed, to align the trocar passages (and handle passages 906a, 906b) with the targeted fastener position, and a first trocar 1002a is inserted through handle passage 906a and the corresponding passage in body 728, and pushed and/or rotated into the bone tissue to form a first pilot hole in the bone tissue (FIG. 10B). A second trocar 1002b is inserted through handle passage 906b and the corresponding passage in 728, and pushed and/or rotated into the bone tissue to form a second pilot hole in the bone tissue (FIG. 10C). Next, trocars 1002a, 1002b are removed (FIG. 10D), and body 728 retracted in a proximal direction sufficiently to permit cartridge 904 to be laterally positioned over the guidewire with guidewire 510 in groove 908 with rods 546 aligned with the corresponding passages in body 728 (FIG. 10F). Cartridge 904 and tool body 558 are then moved longitudinally together such that rods 546 are inserted into the corresponding passages in body 728 and cartridge projection 532 is inserted into recess 526 to engage tool body 728 (FIG. 10G). A user can then align spikes 534 with the pilot holes in the underlying bone tissue, and advance the tool and cartridge toward the bone tissue (FIG. 10H), to drive the fastener (202) into the pilot hole and seat the fastener in the bone (FIG. 10I). If desired, shield assembly 704 (FIG. 9A- FIG. 9C) can be moved to the extended position before positioning the fastener at or near the pilot hole (to protect the fastener and resist unintended engagement of the fastener with tissue during such positioning), and then shield assembly 704 can be retracted before or while the fastener is driven into the pilot hole. The tool can then be retracted in a proximal direction to slide the tool 728 and cartridge 904 over and off of guidewire 510 (FIG. 10J), after which the guidewire can be removed, leaving fastener 202 in place (FIG. 10K).

With cartridges in a pre-loaded state (e.g., cartridge 904 with a fastener 802 pre-loaded on spikes 534), the present kits can comprise a plurality of (e.g., three to five) cartridges 904 pre-loaded with bone fasteners (e.g., 802) and a single tool 902. Some such kits can further comprise at least one guidewire 510 and/or at least two trocars 520. Such a kit can be sterile and enclosed in a sealed package (e.g., sealed tray or a blister pack).

FIG. 10B depicts an exemplary step for a sequence of deployment of a fastener of FIG. 8A via the tool of FIG. 9A.

FIG. 10C depicts an exemplary step for a sequence of deployment of a fastener of FIG. 8A via the tool of FIG. 9A.

FIG. 10D depicts an exemplary step for a sequence of deployment of a fastener of FIG. 8A via the tool of FIG. 9A.

FIG. 10E depicts an exemplary step for a sequence of deployment of a fastener of FIG. 8A via the tool of FIG. 9A.

FIG. 10F depicts an exemplary step for a sequence of deployment of a fastener of FIG. 8A via the tool of FIG. 9A. plary step for a sequence of deployment of a fastener of FIG. 8A via the tool of FIG. 9A.

FIG. 10G depicts an exemplary step for a sequence of deployment of a fastener of FIG. 8A via the tool of FIG. 9A.

FIG. 10H depicts an exemplary step for a sequence of deployment of a fastener of FIG. 8A via the tool of FIG. 9A.

FIG. 10I depict an exemplary step for a sequence of deployment of a fastener of FIG. 8A via the tool of FIG. 9A.

FIG. 10J depicts an exemplary step for a sequence of deployment of a fastener of FIG. 8A via the tool of FIG. 9A.

FIG. 10K depicts an exemplary step for a sequence of deployment of a fastener of FIG. 8A via the tool of FIG. 9A.

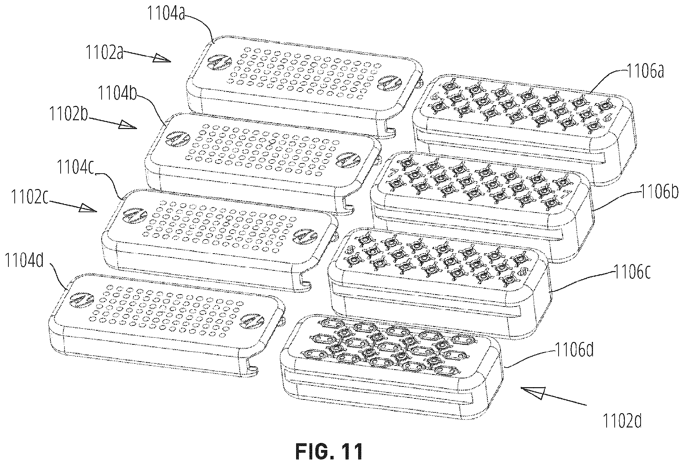

FIG. 11 depicts a perspective view of a number of examples of trays or caddies each configured to hold a plurality of the present fasteners.

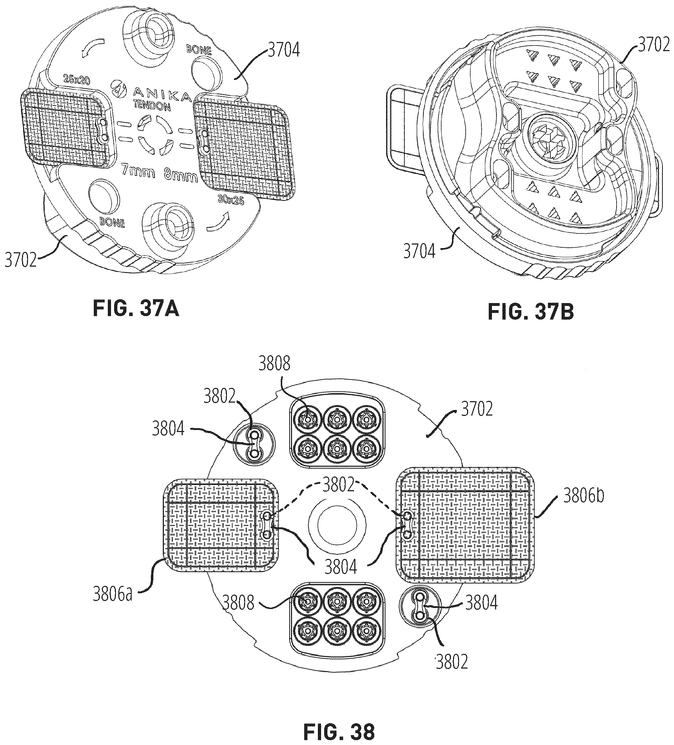

Referring now to FIG. 11, several examples of trays or caddies 1102a, 1102b, 1102c, 1102d are shown, each configured to hold a plurality of the present fasteners (e.g., 108, 606, 202, 802). Each of the trays or caddies includes a respective base 1106a, 1106b, 1106c, 1106d configured to receive the respective plurality of fasteners, and a respective lid or cover 1104a, 1104b, 1104c, 1104d to enclose the fasteners. Each of trays/caddies 1102a, 1102b, 1102c are configured to hold a plurality of single-shaft fasteners (e.g., 108 and/or 202), while tray/caddy 1102d is configured to hold a plurality of single-shaft fasteners (e.g., 108 and/or 202) and a plurality of dual-shaft fasteners (e.g., 606 and/or 802). As shown, the bases are configured to receive and support the fasteners with the respective fastener proximal ends (e.g., 150, 620a, 240) facing upward to facilitate loading each fastener onto a spike of a corresponding tool. FIG. 37A to FIG. 40B depict other embodiment of exemplary trays or caddies for delivering an implant, as described herein.

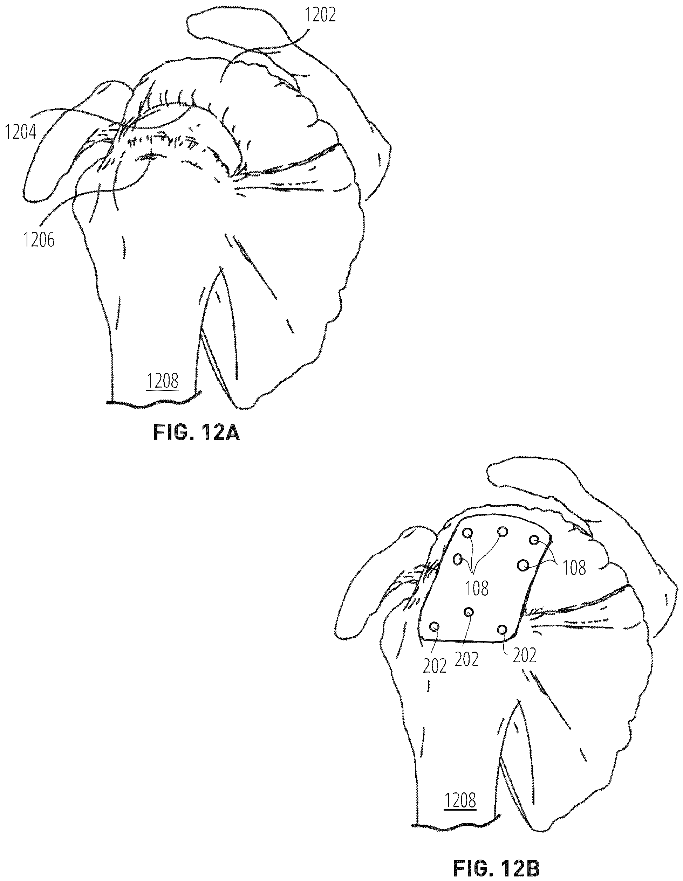

FIG. 12A depicts a perspective, cutaway view of the connection of a damaged supraspinatus tendon to the humerus in a human shoulder.

Referring now to FIG. 12A and FIG. 12B, FIG. 12A depicts a perspective, cutaway view of the connection of a damaged supraspinatus tendon to the humerus in a human shoulder; and

FIG. 12B depicts a perspective, cutaway view of the connection of a damaged supraspinatus tendon to the humerus in a human shoulder, showing a repair in accordance with an example of the present methods.

As shown in FIG. 12A, and described in United States Patent Application No. US 2008/0188936, the rotator cuff is the complex of four muscles that arise from the scapula and whose tendons blend in with the subjacent capsule as they attach to the tuberosities of the humerus. The subscapularis arises from the anterior aspect of the scapula and attaches over much of the lesser tuberosity. The supraspinatus muscle arises from the supraspinatus fossa of the posterior scapula, passes beneath the acromion and the acromioclavicular joint, and attaches to the superior aspect of the greater tuberosity. The infraspinatus muscle arises from the infraspinous fossa of the posterior scapula and attaches to the posterolateral aspect of the greater tuberosity. The teres minor arises from the lower lateral aspect of the scapula and attaches to the lower aspect of the greater tuberosity. Proper functioning of the rotator, 3 to 4 millimeters thick, depends on the fundamental centering and stabilizing role of the humeral head with respect to sliding action during anterior and lateral lifting and rotation movements of the arm.

In rotator cuff injuries, and shown in FIG. 12A, suprasinatus 1202 frequently tears away from the humerus 1208 due to high stress activity or traumatic injury. Supraspinatus 1202 has separated from humerus 1208 along its lateral edge 1204 away from its attachment surface or "footprint" in the greater tuberosity 1206.

FIG. 12B depicts a perspective, cutaway view of the connection of a damaged supraspinatus tendon to the humerus in a human shoulder, showing a repair in accordance with an example of the present methods.

As shown in FIG. 12B, the present methods of repairing a torn rotator cuff, and in some particular implementations, a torn supraspinatus tendon can comprise disposing an implant (e.g., as described herein, including for example, a fibrous implant) 2302 over a torn supraspinatus tendon 1202; securing a first portion of the implant 2302 to the supraspinatus tendon 1202 with a plurality of soft-tissue fasteners (e.g., 108, 606), for example using one of the present tools (e.g., 302, 400a, 550, 702, 902); and securing a second portion of implant 2302 to the humerus (1208) with a plurality of bone fasteners (e.g., 202, 802), for example using one of the present tools (e.g., 302, 406, 550, 702, 902). In some implementations of the present methods, the first portion of the implant 2302 (e.g., an edge of the first portion of implant 2302) is also sutured to the supraspinatus tendon, for example, to ensure more-continuous engagement between the implant and the tendon and thereby encourage tissue growth to and into the implant.

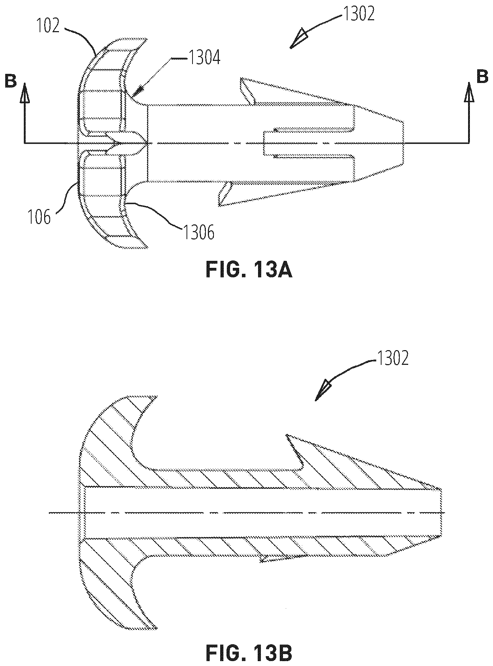

FIG. 13A depicts a side view of a third embodiment of exemplary soft-tissue fasteners.

Referring now to FIGs. 13A and 13B, FIG. 13A depicts a side view of a third embodiment 1302 of examplary soft-tissue fasteners, and FIG. 13B depicts a side cross-sectional view of fastener 1302, taken along the plane B-B of FIG. 13A. Fastener 1302 is substantially similar to fastener 108 of FIGs. 1A-1H, with the primary difference being that distal side 1306 of head 1504 is entirely concave and forms a continuous concave surface with the distal side of outriggers 102, as shown. Specifically, in the depicted embodiment, a radius 1304 forms a concave surface that meets the nominal diameter of shaft 110.

FIG. 13B depicts a side cross-sectional view of the fastener of FIG. 13A, taken along the plane B-B of FIG. 13A.

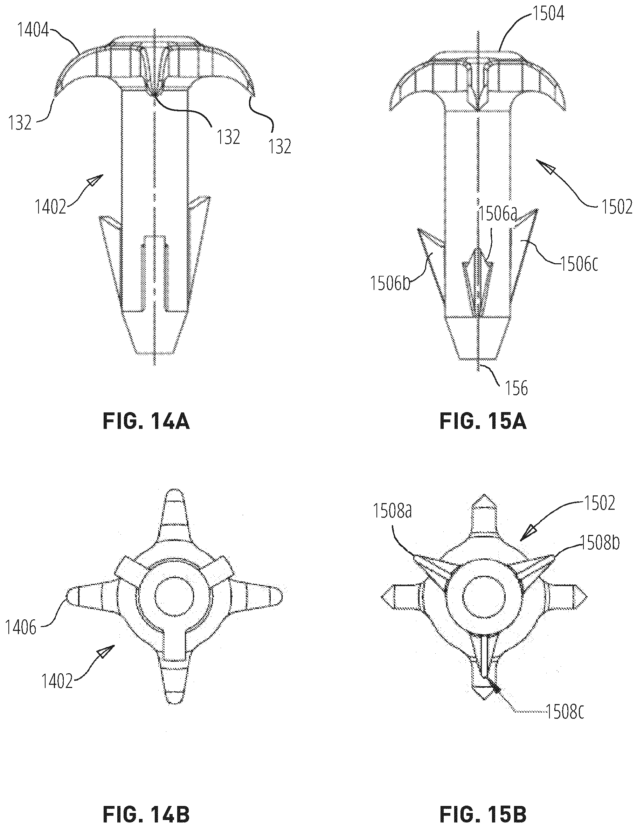

FIG. 14A depicts a side view of a fourth embodiment of exemplary soft-tissue fasteners. Referring now to FIG. 14A and FIG. 14B, FIG. 14A depicts a side view of a fourth embodiment 1402 of exemplary soft-tissue fasteners, and FIG.

14B depicts a distal end view of fastener 1402. Fastener 1402 is substantially similar to fastener 1302 of FIG. 13A -FIG. 13B , with the primary difference being that each outrigger 1404 of fastener 1402 taper to a distal end 132 that is not pointed (omits point 120) and instead curves to an edge 1406 that is oriented substantially radially rather than longitudinally. In this configuration of outriggers 1404, distal ends 132 are less likely to tear or cut into an implant or underlying tissue.

FIG. 14B depicts a distal end view of the fastener of FIG. 14A.

FIG. 15A depicts a side view of a fifth embodiment of exemplary soft-tissue fasteners.

Referring now to FIG. 15A and FIG. 15B, FIG. 15A depicts a side view of a fifth embodiment 1502 of exemplary soft-tissue fasteners, and FIG. 15B depicts a distal end view of fastener 1502. Fastener 1502 is substantially similar to fastener 1302 of FIG. 13A and FIG. 13B, with the primary differences being that barbs 1506a, 1506b, 1506c are shaped differently than barbs 112a, 112b, 112c, and that barbs 1506a, 1506b, 1506c extend farther radially outward from longitudinal axis 156 than do barbs 112a, 112b, 112c. More particularly, barbs 1506a, 1506b, 1506c each have a substantially triangular profile. Specifically, unlike barbs 112a, 112b, 112c that are each defined by a respective pair of planar sides that are parallel to each other (*see* FIG. 1B), barbs 1506a, 1506b, 1506c are each defined by a respective pair of planar sides that angle toward each other (both laterally and longitudinally, such that each barb narrows with increasing distance from axis 156 and with increasing distance from head 18) and are joined by a radius that provides a curved outer surface along the apex of the respective barb (*see* FIG. 15B) rather than an edge. This substantially triangular cross-sectional profile is configured to facilitate insertion of the fastener by reducing insertion force (relative to a rectangular profile with a similar radial dimension), and the absence of a sharp edge configured to reduce the likelihood that the barbs will cut a fabric like implant through which the fastener is inserted.

As described above for fastener 108, the barbs of fastener 1502 extend outwardly from the shaft and therefore extend to an imaginary circle (perpendicular to axis 156) having a maximum transverse dimension that is larger than that of the shaft. For fastener 1502, however, that maximum transverse dimension is between any two of: 160%, 170%, 180%, 190%, 200%, 210%, 220%, 230%, 240%, 250%, 260%, and/or 270% of the maximum transverse dimension of the shaft (e.g., between 210% and 220% of the diameter of the shaft). For example, in the embodiment shown, barbs 1506a, 1506b, 1506c each extend radially outward to a point between 1.7 and 1.8 mm from axis 156 (i.e., each of point/edge 1508a, 1508b, 1508c is between 1.7 and 1.8 mm from axis 156). This increased radial dimension is configured to engage more tissue and resist removal of the fastener from tissue. While the increased radial dimension also increases the force required for insertion (relative to a similar profile with smaller radial dimension), the substantially triangular profile discussed above offsets the increase due to the larger radial extent and ultimately makes for an insertion force similar to or slightly less than that of fastener 108.

FIG. 15B depicts a distal end view of the fastener of FIG. 15A.

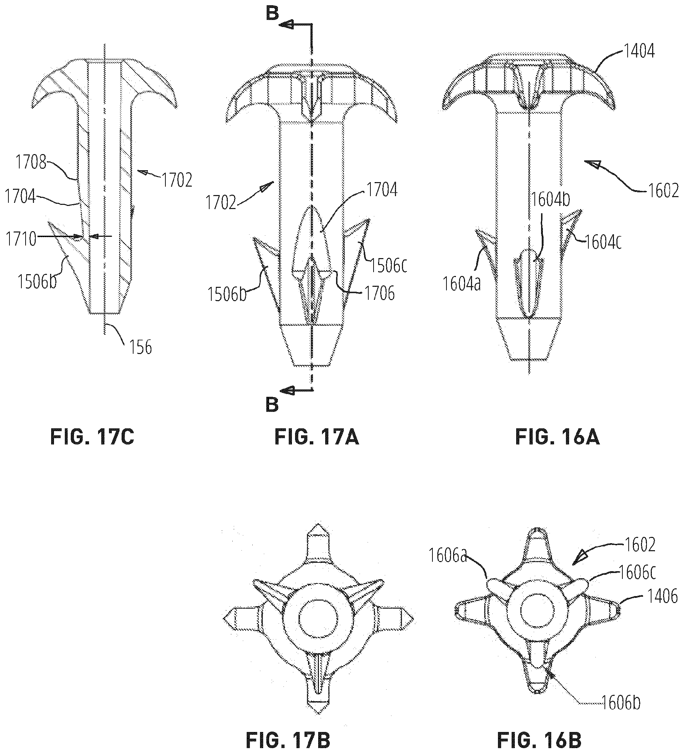

FIG. 16A depicts a side view of a sixth embodiment of exemplary soft-tissue fasteners.

Referring now to FIG. 16A and FIG. 16B, FIG. 16A depicts a side view of a sixth embodiment 1602 of exemplarary soft-tissue fasteners, and FIG. 16B depicts a distal end view of fastener 1602. Fastener 1602 is substantially similar to fastener 1302 of FIG. 13A and FIG. 13B, with the primary differences being that barbs 1604a, 1604b, 1604c are shaped differently than barbs 112a, 112b, 112c, that barbs 1506a, 1506b, 1506c extend farther radially outward from longitudinal axis 156 than do barbs 112a, 112b, 112c, and that the fastener includes outriggers 1404 like those of fastener 1402 of FIG. 14A and FIG. 14B . More particularly, barbs 1604a, 1604b, 1604c each have a substantially triangular profile like that of barbs 1506a, 1506b, 1506c, except barbs 1604a, 1604b, 1604c have a larger radius at their respective apexes. As with barbs 1506a, 1506b, 1506c, the cross-sectional profile of barbs 1604a, 1604b, 1604c is configured to facilitate insertion of the fastener by reducing insertion force relative to a rectangular profile with a similar radial dimension, and the absence of a sharp edge configured to reduce the likelihood that the barbs will cut a fabric like implant through which the fastener is inserted.

As described above for fastener 108, the barbs of fastener 1602 extend outwardly from the shaft and therefore extend to an imaginary circle (perpendicular to axis 156) having a maximum transverse dimension that is larger than that of the shaft. For fastener 1602, however, that maximum transverse dimension is between any two of: 140%, 150%, 160%, 170%, 180%, 190%, 200%, 210%, 220%, 230%, and/or 240% of the maximum transverse dimension of the shaft (e.g., between 180% and 190% of the diameter of the shaft). For example, in the embodiment shown, barbs 1604a, 1604b, 1604c each extend radially outward to a point between 1.5 and 1.6 mm from axis 156 (i.e., each of point/edge 1606a, 1606b, 1606c is between 1.5 and 1.6 mm from axis 156). This increased radial dimension is configured to engage more tissue and resist removal of the fastener from tissue. While the increased radial dimension also increases the force required for insertion (relative to a similar profile with smaller radial dimension), the profile discussed above offsets the increase due to the larger radial extent and ultimately makes for an insertion force similar to or slightly less than that of fastener 108.

FIG. 16B depicts a distal end view of the fastener of FIG. 16A.

FIG. 17A depicts a side view of a seventh embodiment of the present soft-tissue fasteners.

Referring now to FIG. 17A and FIG. 17B depicts a side view of a seventh embodiment 1702 of the present soft-tissue fasteners, FIG. 17B depicts a distal end view of fastener 1702, and FIG. 19C depicts a side cross-sectional view of fastener 1702, taken along the plane C-C of FIG. 17A. Fastener 1702 is substantially similar to fastener 1502 of FIG. 15A and FIG. 15B, with the primary difference being that fastener 1702 includes hollowed portions 1704 extending into shaft 14fat the base of each barb 1506a, 1506b, 1506c. More particularly, each hollowed portion 1704 is defined by a planar surface extending from a proximal end 1708 that is a first distance (equal to diameter 134) from axis 156, and a distal end 1706 that is a second distance 1710 from axis 156, with second distance 1706 being less than diameter 134 such that the planar surface tapers toward axis 156 with increasing distance from head 1504. In this configuration, the radial distance of the proximal side of each barb available to engage tissue is increased without increasing the maximum radial extent of the barb. In other embodiments, surface of a proximal side of a barb may be non-planar (e.g., curved). In the embodiment shown, distal end 1706 of each hollowed portion is joined to the proximal side of the respective barb via a curve or radius.

FIG. 17B depicts a distal end view of the fastener of FIG. 16A.

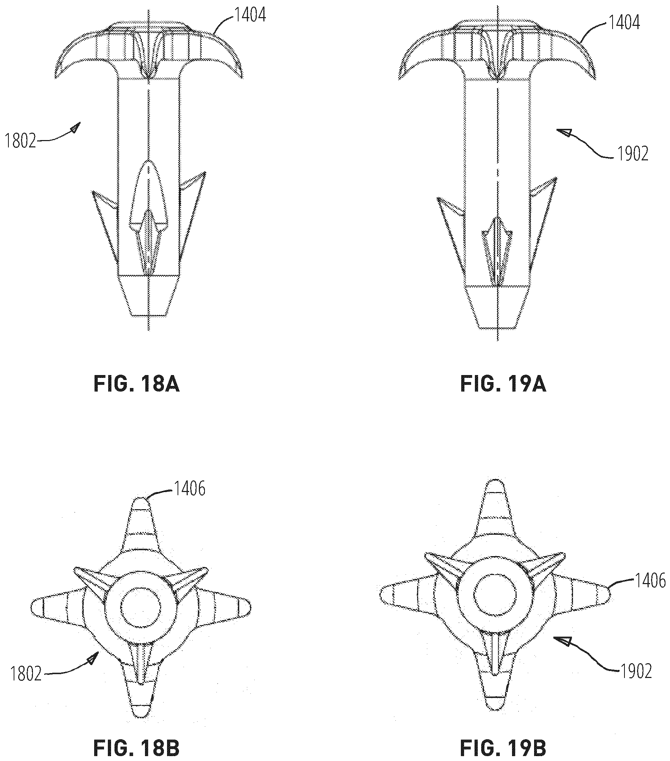

FIG. 17C depicts a side cross-sectional view of the fastener of FIG. 19A, taken along the plane C-C of FIG. 17A.

FIG. 18A depicts a side view of an eighth embodiment of the present soft-tissue fasteners.

Referring now to FIG. 18A and FIG. 18B, FIG. 18A depicts a side view of an eighth embodiment 1802 of exemplary soft-tissue fasteners, and FIG. 18B depicts a distal end view of fastener 1802. Fastener 1802 is substantially similar to fastener 1702 of FIG. 17A -FIG. 17C, with the primary differences being that fastener 1802 includes outriggers 1404 that like those of fastener 1402 of FIG. 14A and FIG. 14B.

FIG. 18B depicts a distal end view of the fastener of FIG. 18A.

FIG. 19A depicts a side view of a ninth embodiment of the present soft-tissue fasteners.

Referring now to FIG. 19A and 19B, FIG. 19A depicts a side view of a ninth embodiment 1902 of exemplary soft-tissue fasteners, and FIG. 19B depicts a distal end view of fastener 1902. Fastener 1902 is substantially similar to fastener 1502 of FIG. 15A and FIG. 15B , with the primary differences being that fastener 1902 includes outriggers 1404 that like those of fastener 1402 of FIG. 14A and FIG. 14B.

FIG. 19B depicts a distal end view of the fastener of FIG. 19A.

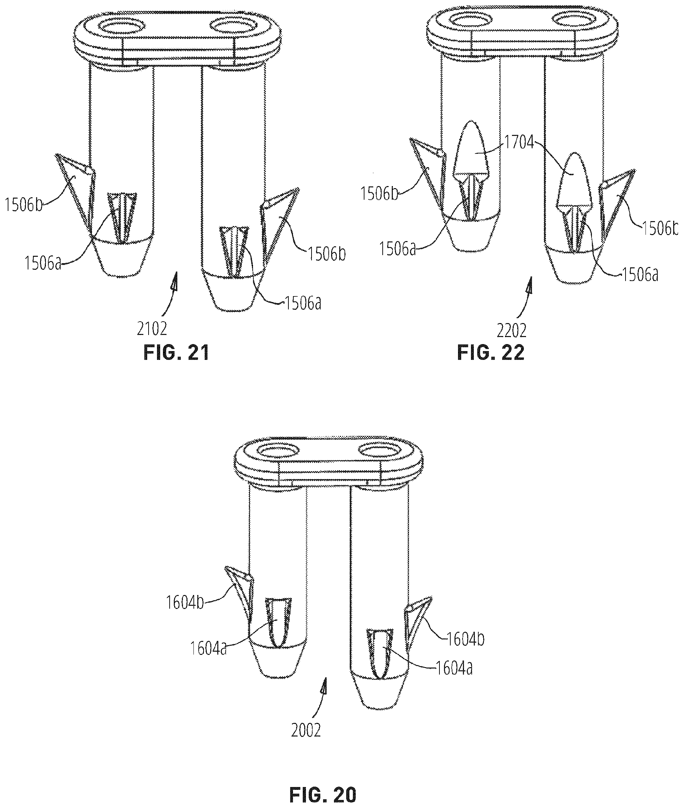

FIG. 20 depicts a side view of a tenth embodiment of exemplary soft-tissue fasteners.

Referring now to FIG. 20, shown therein is a tenth embodiment 2002 of the present soft-tissue fasteners. Fastener 2002 is substantially similar to 606 of FIG. 6A -FIG. 6F, with the primary difference being that each of shafts 602a, 602b includes barbs 1604a, 1604b, 1604c (not shown but 180° opposite barb 1604a) like those of fastener 1602 of FIG. 16A and FIG. 16B, instead of three barbs 112b.

FIG. 21 depicts a side view of an eleventh embodiment of exemplary soft-tissue fasteners.

Referring now to FIG. 21, shown therein is an eleventh embodiment 2102 of exemplary soft-tissue fasteners. Fastener 2102 is substantially similar to 606 of FIG. 6A -FIG. 6F, with the primary difference being that each of shafts 602a, 602b includes barbs 1506a, 1506b, 1506c (not shown but 180° opposite barb 1506a) like those of fastener 1502 of FIGs. 15A-15B), instead of three barbs 112b.

FIG. 22 depicts a side view of a twelfth embodiment of exemplary soft-tissue fasteners.

Referring now to FIG. 22, shown therein is a twelfth embodiment 2202 of the present soft-tissue fasteners. Fastener 2202 is substantially similar to 606 of FIG. 6A -FIG. 6F, with the primary difference being that each of shafts 602a, 602b includes barbs 1506a, 1506b, 1506c (not shown but 180° opposite barb 1506a) like those of fastener 1502 of FIG. 15A and FIG. 15B, and hollowed portions 1704 like those of fastener 1702 of FIG. 17A -FIG. 17C corresponding to barbs 1506a, instead of three barbs 112b. In other embodiments, fastener 2202 can include hollowed portions 1704 for each of barbs 1506b and/or 1506c (additional or alternative to the depicted hollowed portions for each of barbs 1506a).

As described herein, FIG. 23A depicts a perspective view of an exemplary implant as described herein. In some embodiments, the implant 2302 comprises a fibrous, fabric-like implant for use with a fastener described herein.

Referring now to FIG. 23A -FIG. 23C, FIG. 23A, FIG. 23B, and FIG. 23C depict perspective, end, and plan views, respectively, of a flexible, fibrous, fabric-like implant 2302 for use with the exemplary fasteners described herein. Any implant described herein, including comprising biocompatible textiles may be used with any fastener and/or delivery tool described herein. In the embodiment shown, implant 2302 has a first end edge 2306, a second end edge 2304, and a pair of lateral edges 2308a, 2308b that extend between the first and second end edges. As shown, implant 2302 is substantially planar when in a flattened state in which the first end edge is separated from the second end edge under a first tension of 5 Newtons (N) applied perpendicular to each of the end edges, and in which the lateral edges are separated from each other under a tension of 5 N applied perpendicular to each of the lateral edges. In this embodiment, implant 2302 comprises a woven layer 2310 and a nonwoven layer 2312 coupled to the woven layer. More particularly, in the embodiment shown, nonwoven layer 2312 is stitched to the woven layer 2310 with a (mono- or multi-filament) thread 2314 along a peripheral path 2316 that is offset inward from the peripheral edges (2306, 2304, 2308a, 2308b) of the implant, as well as internal cross paths 2318a, 2318b, 2318c, 2318d that are offset further inwardly of each of the peripheral edges of the implant. In the depicted embodiment, both of layers 2310 and 2312 comprise a biocompatible polymer.

FIG. 23B depicts an end view of a fibrous, fabric-like implant for use with the present fasteners.

FIG. 23C depicts a plan view of a fibrous, fabric-like implant for use with the present fasteners.

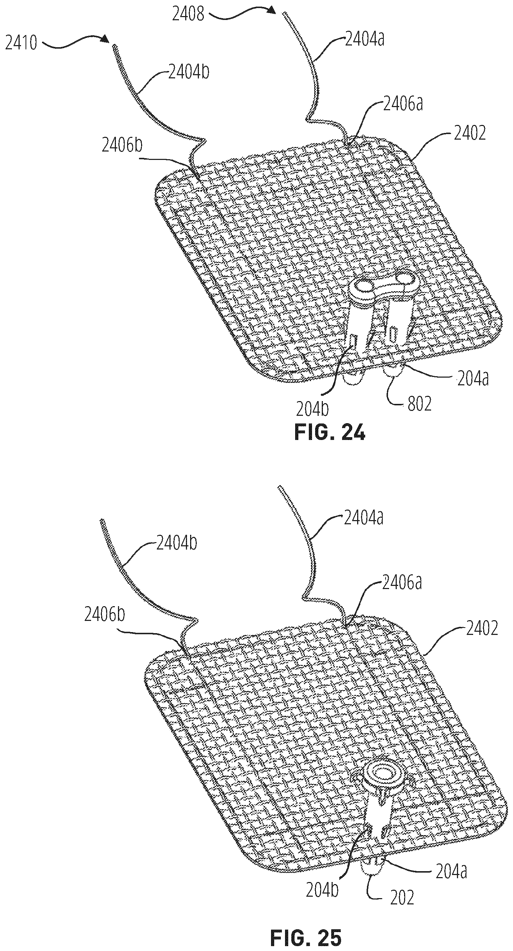

FIG. 24 depicts an exemplary implant (as described herein) in combination with a fastener of FIG. 8A-FIG. 8E.

Referring now to FIG. 24, an implant 2402 is shown in combination (e.g., preloaded) with a fastener 802 of FIGs. 8A-8E. Implant 2402 is substantially similar to implant 2302, with the exception that implant 2402 further comprises at least one suture leg coupled to the implant at a point that is closer to the second end edge than to the first end edge, the suture leg having a free portion that is configured to extend beyond the second end edge. For example, in the embodiment shown, implant 2402 comprises: a first suture leg 2404a coupled to the implant at a first point 2406a that is closer to second end edge 2304 than to first end edge 2306, with first point 2406a also being closer to first lateral edge 2308a than to second lateral edge 2308b; and a second suture leg 2404b coupled to the implant at a second point 2406b that is closer to second end edge 2304 than to first end edge 2306, with second point 2406b also being closer to the second lateral edge 2308b of the lateral edges than to first lateral edge 2308a. As shown, first suture leg 2404a has a first free portion 2408 that is configured to extend beyond second end edge 2304, and second suture leg 2404b has a second free portion 2410 that is configured to extend beyond second end edge 2304. In the embodiment shown, shaft 806 of fastener 802 extends through implant 2302 such that the trailing edge of each of barbs 204b are disposed on a first side of the implant, and at least a portion of the leading edges of each of barbs 204a are disposed on a second side of the implant. In other embodiments, all or a portion (e.g., the leading edges) of barbs 204b may also be disposed on the second side of the implant. In the embodiment shown, each suture leg 2404a, 2404b comprises a single strand extending from the implant and may, for example, be unitary with thread 2314. In other embodiments, each suture leg can comprise a loop of thread that passes through or is otherwise secured to the implant at the respective first or second point 2406a, 2406b such that each free portion 2408, 2410 is defined by the remaining portion of the respective loop.

FIG. 25 depicts another implant (as described herein) in combination with a fastener of FIG. 2A-FIG. 2E.

Referring now to **FIG. 25,** an implant 2402 is shown in combination (e.g., preloaded) with a fastener 202 of FIGs. 2A-2E. As with the embodiment of FIG. 24, in the embodiment of FIG. 25, shaft 234 of fastener 202 extends through implant 2302 such that the trailing edge of each of barbs 204b are disposed on a first side of the implant, and at least a portion of the leading edges of each of barbs 204a are disposed on a second side of the implant. In other embodiments, all or a portion (e.g., the leading edges) of barbs 204b may also be disposed on the second side of the implant.

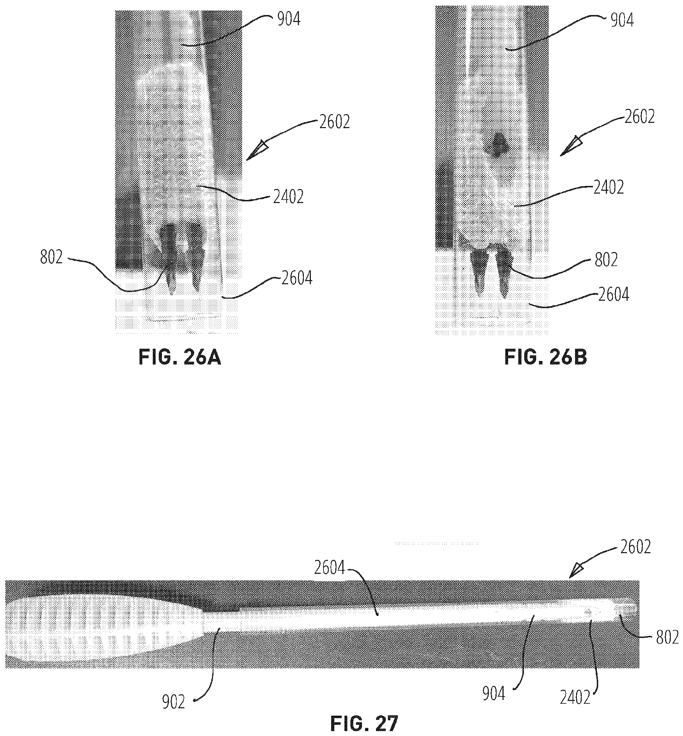

FIG. 26A depicts a first side view of one of the present assemblies with the fastener and implant of FIG. 24 preloaded onto a cartridge of FIG. 9D - FIG. 9G with a shield around the implant and cartridge.

Referring now to FIG. 26A and FIG. 26B, FIG. 26A and FIG. 26B depict first and second side views of one of the exemplary assemblies 2602 with fastener 802 and implant 2402 of FIG. 24 preloaded onto a cartridge 904 of FIG. 9D and FIG. 9G with a shield 2604 around the implant and cartridge. Shield 2604 is substantially similar to shield 704 with the primary exception that shield 2604 does not include a hub (464a). In the embodiment shown, fastener 202 is disposed through point 2406a that is closer to first end edge 2306 than to second end edge 2304, and the implant extends proximally (toward the proximal end of cartridge 904) such that second end edge 2304 is closer to the proximal end of the cartridge 904 with a first portion of the implant wrapped around a corresponding portion of the cartridge. As shown, the first portion of the implant is disposed between the cartridge and shield 2604 such that the shield contains the implant around the cartridge. In the embodiment shown, shield 2604 is transparent to permit a user to view the implant and fastener during use, for example, to facilitate placement and insertion of the fastener (and implant) to and at a desired position (e.g., at a portion of the humerus when used for repair of a rotator cuff tear). In other embodiments, the shield may be translucent or opaque. In the depicted configuration, the shield is in an extended state in which a distal end of the shield extends distally beyond the distal end of the fastener shafts and the corresponding spikes of the cartridge, for example, to prevent the spikes from snagging or catching on tissue before the fastener is disposed at a desired insertion/fastening point.

FIG. 26B depicts a second side view of one of the present assemblies with the fastener and implant of FIG. 24 preloaded onto a cartridge of FIG. 9D and FIG. 9G with a shield around the implant and cartridge.

FIG. 27 depicts the assembly of FIG. 26A and FIG. 26B coupled to an apparatus of FIG. 9A- FIG. 9C.

Referring now to FIG. 27 depicts assembly 2602 of FIG. 26A and FIG. 26B coupled to an apparatus or tool 902 of FIG. 9A - FIG. 9C. While assembly 2602 is shown with fastener 802 and cartridge 904, any of the present fasteners can be preloaded with an implant in similar fashion with a shield retaining the implant around a corresponding cartridge or tool. For example, fastener 802 can be preloaded with an implant as shown in FIG. 24 and the fastener and implant can be coupled to and around an apparatus 702 of FIG. 7A -FIG. 7D. By way of further example, a fastener 202 can be preloaded with an implant as shown in FIG. 25 and the fastener and implant can be coupled to and around cartridge 502 or an apparatus 302 or 400a with a corresponding shield retaining the implant around the cartridge or apparatus. Additionally, while shown with bone fasteners 202 and 802, other embodiments of the present assemblies can include soft-tissue fasteners (e.g., 108, 606-2202) and a corresponding cartridge or apparatus/tool.

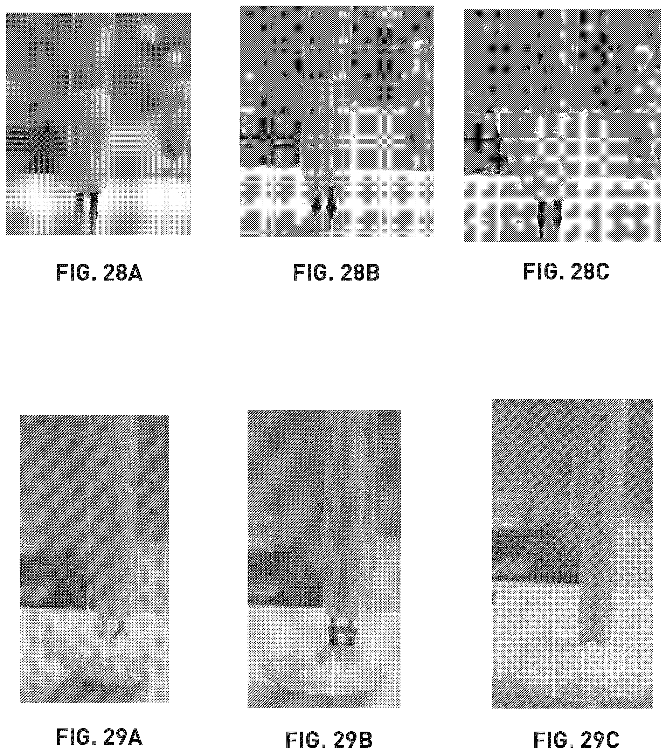

FIG. 28A depicts the deployment of a fastener from the assembly of FIG. 26A and FIG. 26B with the fastener not inserted into tissue for illustrative purposes.

Referring now to FIG. 28A -FIG. 28C and FIG. 29A -FIG. 29C, FIG. 28A-28C depicts an exemplary deployment of a fastener from the assembly of FIG. 26A and FIG. 26B, with the fastener not inserted into tissue for illustrative purposes, and FIG. 29A-29C depicts the deployment of a fastener from the assembly of FIG. 26A and FIG. 26B. In use, the assembly is disposed at a position in which the shafts of the fastener are to be inserted into a first portion of tissue (e.g., bone) of a patient; the shield (e.g., 2604) is retracted (as illustrated by the progression of FIG. 28A -FIG. 28C or of FIG. 29A -FIG. 29C) to a position that permits the second end edge of the implant to exit the shield (as shown in FIG. 28C and FIG. 29C); the implant is spread along a second portion of tissue (e.g., soft tissue) of the patient (e.g., as shown in FIG. 24). The implant is coupled to the first portion of tissue via fastener 202 (either before or after retraction of the shield and/or spreading of the implant), and the implant is coupled to the second portion of tissue via the suture legs and/or via one or more soft tissue fasteners (e.g., 108, 606-2202). Once fastener 202 is inserted into the first portion of tissue, the cartridge is decoupled from the fastener such that the spikes of the cartridge are removed from the internal passages of the shafts of the fastener. By way of example, when used to repair a rotator cuff tear (as illustrated in FIG. 12B), fastener 202 is inserted into a portion of the humerus to secure the implant to the humerus, and the suture legs and/or one or more soft-tissue fasteners secure the implant to the supraspinatus tendon.

FIG. 28B depicts the deployment of a fastener from the assembly of FIG. 26A and FIG. 26B with the fastener not inserted into tissue for illustrative purposes.

FIG. 28C depicts the deployment of a fastener from the assembly of FIG. 26A and FIG. 26B with the fastener not inserted into tissue for illustrative purposes.

FIG. 29A depicts the deployment of a fastener from the assembly of FIG. 26A and FIG. 26B.

FIG. 29B depicts the deployment of a fastener from the assembly of FIG. 26A and FIG. 26B.

FIG. 29C depicts the deployment of a fastener from the assembly of FIG. 26A and FIG. 26B.

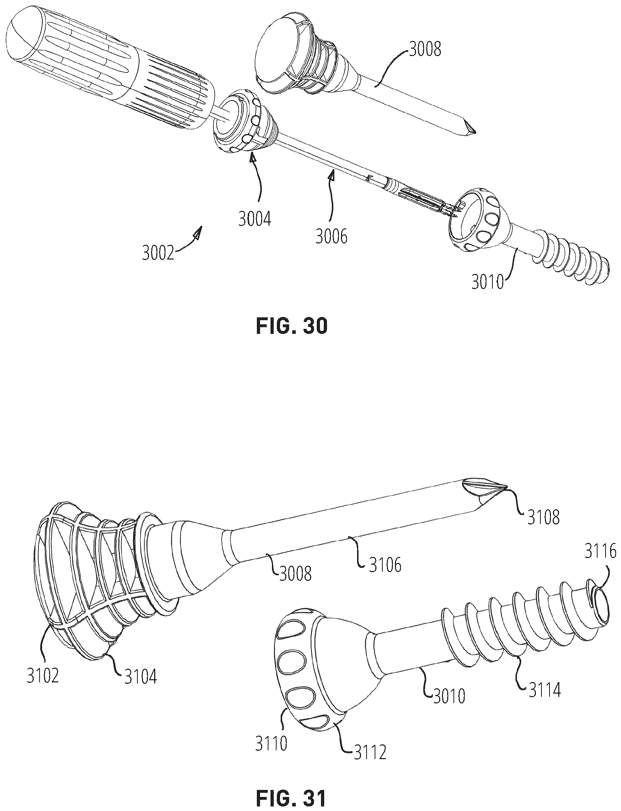

FIG. 30 - FIG. 52B depict other exemplary embodiments of a delivery system for delivering an implant as described herein.

FIG. 30 is a perspective view of an exemplary delivery system, including a cannula, an obturator, a seal assembly, and a delivery tool in a first configuration.

Referring now to the drawings, and more particularly to FIG. 30, shown there and designated by the reference numeral 3002 is an exemplary embodiment of a delivery system. System 3002 includes a cannula 3010, an obturator 3008, a seal assembly 3004, and a delivery tool 3006. As shown, seal 3004 is configured to receive a portion of tool 3006 such that the tool can be inserted into the cannula. As described in more detail below, seal assembly 3004 is configured to seal around the portion of the tool in the seal assembly, as well as to be coupled to a proximal end of cannula 3010 to form a seal between seal assembly 3004 and cannula 3010.

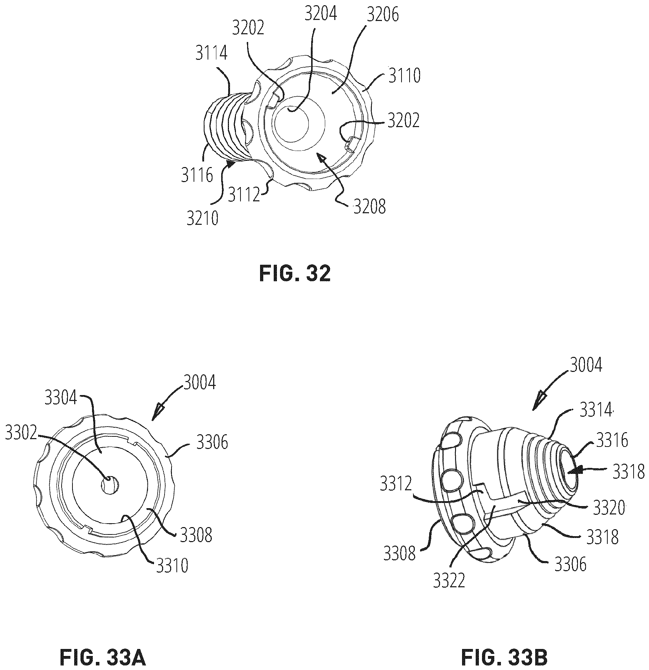

FIG. 31 is the cannula and the obturator of FIG. 30. Referring now to FIG. 31 and FIG. 32, FIG. 31 shows an enlarged perspective view of cannula 3010 and obturator 3008.

As shown, cannula 3010 comprises a cannula distal end 3116, a cannula proximal end 3110 configured to be coupled to the seal assembly (3004), and a cannula passage 3204 extending through and between the cannula proximal end and the cannula distal end. As shown in FIG. 32, cannula 3010 also defines a cannula proximal surface 3206 tapered distally (toward cannula distal end 3116) and inward toward the cannula passage (3204). Cannula proximal surface 3206 is configured to cooperate with a distal surface of seal assembly 3004 to provide a seal therebetween. In the embodiment shown, at cannula proximal end 3110 is a head or hub 3112 that is enlarged in the radial direction relative to at least longitudinally adjacent portions of the cannula. Head or hub 3112 is configured to be grasped by a user to vary the depth and/or direction of the cannula, such as when the cannula is inserted through soft tissue of a patient. In this configuration, cannula 3010 defines a recess 3208 that extends from cannula proximal end 3110 inward toward cannula distal end 3116, with cannula proximal surface 3206 disposed at the bottom or distal-most portion of recess 3208. Cannula 3010 can also be configured to engage seal assembly 3004, such as by threads, projections and corresponding grooves, an interference fit, or the like.

FIG. 32 is a proximal perspective view of the cannula of FIG. 30. FIG. 32 is a proximal perspective view of the cannula 3010 of FIG. 30. FIG. 32 shows a proximal perspective view of cannula 3010 alone.

For example, and as best seen in FIG. 32, cannula 3010 defines two projections 3202 that extend inward from the inner peripheral surface 3206 that defines recess 3208. As described in more detail below, projections 3202 are configured to engage seal assembly 3004 to resist separation of the cannula and the seal assembly. In the depicted embodiment, a cannula outer surface 3210 of the cannula includes one or more cannula threads 3114 that are configured to engage soft tissue of a patient as the cannula is rotated.

Obturator 3008 has an obturator elongated body 3106 with a obturator proximal end 3102 and an tapered obturator distal end 3108. As shown, obturator 3008 includes an enlarged head or handle region 3104 at obturator proximal end 3102. In use, obturator elongated body 3106 is configured to extend through cannula 3010 such that obturator distal end 3108 extends distally beyond cannula distal end 3116 to facilitate insertion of the cannula through soft tissue of a patient. For example, a user such as a physician can make an initial incision through the skin of a patient, and then the obturator and cannula can be together inserted through the incision. More specifically, distal end 3108 is first inserted through the incision such that the tapered shape expands the incision to enable the user to rotate cannula 3010 and thereby cause threads 3114 to engage and draw the cannula into the tissue (and/or draw the tissue outward over the threads).

FIG. 33A is a proximal perspective view of the seal assembly of FIG. 30. FIG. 33A is a proximal perspective view of the seal assembly of FIG. 30. FIG. 33A shows a proximal perspective view of seal assembly 3004.

As shown, seal assembly 3004 comprises: an annular seal body 3306 having a seal proximal end 3308 and a seal distal end 3316. Seal body defines a seal passage 3318 extending through and between the seal proximal end and the seal distal end, and a tapered distal surface 3318 disposed around the seal passage at the seal distal end. Seal body 3306 also defines one or more grooves or channels to receive projection(s) 3202 of cannula 3010.

FIG. 33B is a distal perspective view of the seal assembly 3004 of FIG. 30. FIG. 33B shows a distal perspective view of seal assembly 3004.

As shown in FIG. 33B, body 3306 defines distinct J-slots 3322 on a peripheral surface of the body, with each J-slot configured to receive one of projections 3202 of cannula 3010. In the depicted configuration, each J-slot 3322 includes a longitudinal portion 3320 extending generally in the longitudinal direction, and a circumferential portion 3312 extending in a generally circumferential direction around a peripheral portion of the seal body. In operation, seal distal end 3316 is inserted into recess 3208 of cannula 3010 with projections 3202 each aligned with corresponding one of longitudinal portions 3320 until the projections reach the circumferential portions 3312 of the J-slots 3322, at which point the seal body (3306) is rotated to position each projection 3202 in the end of the corresponding circumferential portion 3312 that is farthest from the corresponding longitudinal portion 3320. In the depicted embodiment, one or more resilient annular seal members 3314 are disposed on distal surface 3318 and sized such that the seal members (3314) are compressed to some degree when projections 3010 of the cannula are disposed in circumferential portions 3312 of the J-slots in seal body 3306. Once compressed, these seal members 3314 exert a force tending to urge the cannula proximal surface 3206 and seal distal surface 3318 apart, as well as ensure a degree of friction between the cannula proximal surface and the seal distal surface, to resist unintentional rotation of the seal body in a way that could otherwise unintentionally allow separation of the seal body from the cannula.

In the embodiment shown, seal assembly 3004 also includes a resilient membrane 3304 disposed at least around an interior periphery of the seal passage. Resilient membrane 3304 is configured to cooperate with an elongated body of delivery tool 3006 (as shown in FIG. 30) to substantially seal the seal passage (3318) when the body of the apparatus extends through the resilient membrane and the seal passage. In this configuration, membrane 3304 is attached directly to an interior surface 3310 of seal body 3306 that defines a proximal portion of seal passage 3318, and extends radially inward to define a central membrane opening 3302 that is aligned with a central longitudinal axis of the seal passage (3318). In this configuration, opening 3302 is circular to corresponding in shape to the circular cross-section of the elongated body of the delivery tool (3006), and is sized with an interior diameter that is smaller than the exterior diameter of the corresponding portion of the elongated body, such that the resilient membrane stretches somewhat when the elongated body is inserted through opening 3302 and the resilient membrane exerts a compressive force against the elongated body and provide a sealed interface therebetween.

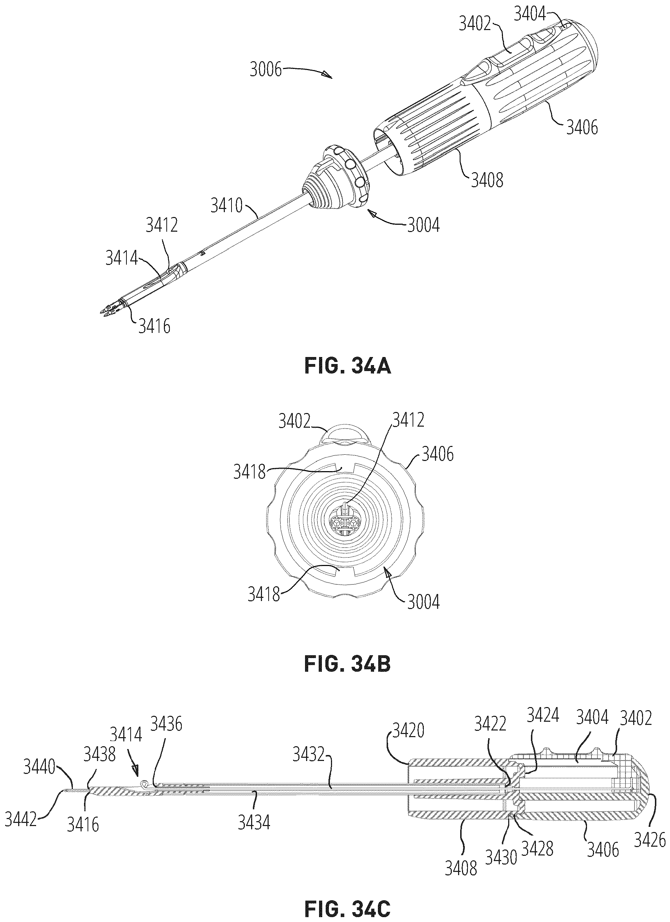

FIG. 34A is a perspective view of the delivery tool and seal assembly of FIG. 30, with the delivery tool in the a configuration.

As described in more detail below, the delivery tool is configured to deliver an implant (e.g., any implant described herein) and a fastener (e.g., any fastener described herein) at the same time, such that the fastener can secure the implant to a portion of a patient (e.g., a bone or soft tissue) in a single motion. As shown, delivery tool 3006 comprises an elongated body 3410 extending from a body proximal end 3422 to a body distal end 3416. Body 3410 defines a body proximal opening 3504 through the body proximal end, a body recess 3414 in a peripheral surface of the body at point that is offset from the body distal end (3416), and a body passage 3434 extending between the body proximal opening and a body distal opening 3436 within the body recess. Delivery tool 3006 also includes one or more spikes 3440 (e.g., two spikes 3440, in the depicted embodiment) each having a spike proximal end 3438 coupled to the body distal end 3416, and a spike distal end 3442 extending from the body distal end. As shown, the spike proximal end has a transverse dimension 3514 that is smaller than a transverse dimension 3512 of the body distal end such that a shoulder is defined at the spike proximal end (and such that the shoulder is configured to abut a head of a fastener 3804 received over the spike, as shown in FIG. 34A).

As shown, delivery tool 3006 also includes a hub 3408 and a handle 3406. Hub 3408 has a hub proximal end 3424 and a hub distal end 3420, and is configured to receive proximal end 3422 of body 3410 through the hub distal end. More particularly, hub 3408 includes a hub first portion 3502 defining a hub recess 3508 that is sized to receive proximal end 3422 of body 3410, which can be secured in the hub recess 3508 via a friction or interference fit, adhesive, and/or the like, such that the hub can be rotated to rotate the elongated body (3410). Additionally, hub proximal end 3424 defines a central opening 3506 in fluid communication with and aligned with a central longitudinal axis of body passage 3434. Handle 3406 has a handle proximal end 3426 and a handle distal end 3430. As shown, handle distal end 3430 is coupled to hub proximal end 3422, for example, via one or more interlocking tabs and grooves or notches. By way of example, in the depicted configuration, hub proximal end 3424 includes one or more tabs 3426 that project radially outward, and handle distal end 3430 defines an annular flange with one or more corresponding grooves that receive the tab(s) 3426 to resist separation of the handle (3406) from the hub (3408) while still permitting the handle to rotate relative to the hub, at least within a defined angular range (e.g., less than any one of or between any two of: 30 degrees, 45 degrees, and/or 90 degrees). In the depicted configuration, such rotation of the handle (3406) relative to the hub (3408) also causes the pusher to rotate, such that when or as the distal end of the pusher (e.g., 3432, 4504a, 4504b) is extended distally, the distal end of the pusher can move laterally to apply urge lateral portions of an implant (e.g., 4502) toward the bone or other tissue to which the implant is to be secured.

Delivery tool 3006 also includes an elongated pusher 3432 and a slider 3402. Pusher 3432 has a pusher proximal end 3520 and a pusher distal end 3412, and slider 3402 is coupled to pusher proximal end 3520. Pusher distal end 3412 includes a curved section 3522 that is wider in the radial direction than an adjacent portion of the pusher, to resist snagging when the pusher slides against an implant as described below. In the depicted embodiment, the elongated pusher 3432 is formed from an elongated wire, such as nitinol or stainless steel, with its distal end bent to define the curved section 3522. In other embodiments, the elongated pusher 3432 can be formed as a solid piece, in which the curved section is defined by machining or the like, or can be assembled from multiple pieces, such as a wire body and a paddle-shaped piece attached (e.g., welded) to the distal end of the wire to define the curved section.

FIG. 34B is a distal end view of the delivery tool and seal assembly of FIG. 34A.

FIG. 34C is a side cross-sectional view of the delivery tool and seal assembly of FIG. 34A.

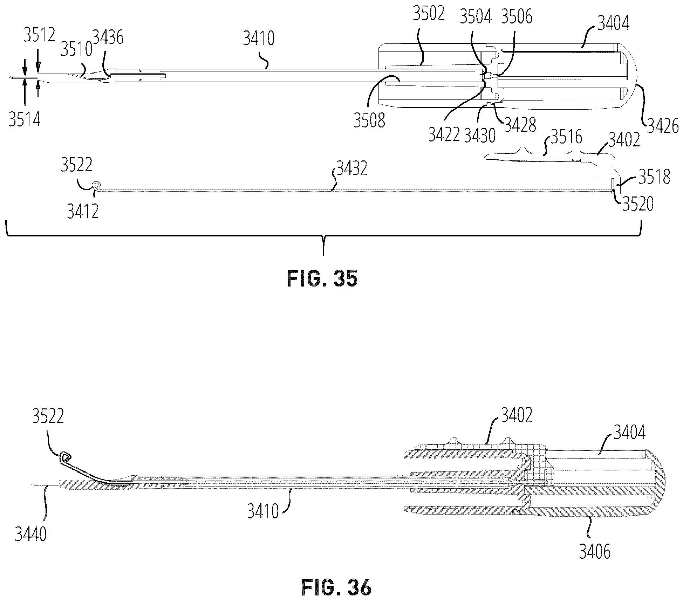

FIG. 35 is a partially exploded, cross-sectional side view of the delivery tool of FIG. 30. As shown in FIG. 34C, pusher 3432 is disposed in and extending through body passage 3434 such that pusher distal end 3412 is disposed beyond body distal opening 3436.

FIG. 36 is a side cross-sectional view of the delivery tool of FIG. 34A- FIG. 34C, shown in a second configuration.

Slider 3402 is coupled to pusher proximal end 3520, and is configured to move longitudinally relative to the handle from a retracted first position (FIG. 34C) to a deployed position (FIG. 36) to move pusher distal end 3412 longitudinally away from the body distal opening 3436 and laterally outward from the body, as shown in FIG. 36.

In the depicted configuration, slider 3402 includes a second hub portion 3518 and a radial projection 3516. The second hub portion 3518 aligns with the longitudinal axis of the body opening 3434, and radial projection 3516 extends radially outward through a slot 3404 in handle 3406 such that a user can push radial projection 3516, such as with the user's thumb, to move slider 3402 distally relative to handle 3406 and extend pusher distal end 3412 distally (from FIG. 34C vs. FIG. 36) and similarly shift radial portion 3516 proximally to retract pusher distal end 3412 (from FIG. 36 to FIG. 34C). As can be seen in FIG. 34C, FIG. 35, and FIG. 36, body recess 3414 defines a sloped surface 3510 spaced from body distal opening 3436 and positioned such that, when the slider is moved from the retracted position (FIG. 34C) to the deployed position (FIG. 36), first curved portion 3522, and then an additional portion of the pusher, slide along sloped surface 3510 to cause pusher distal end 3412 to extend laterally or radially outward from body 3410 as shown in FIG. 36.

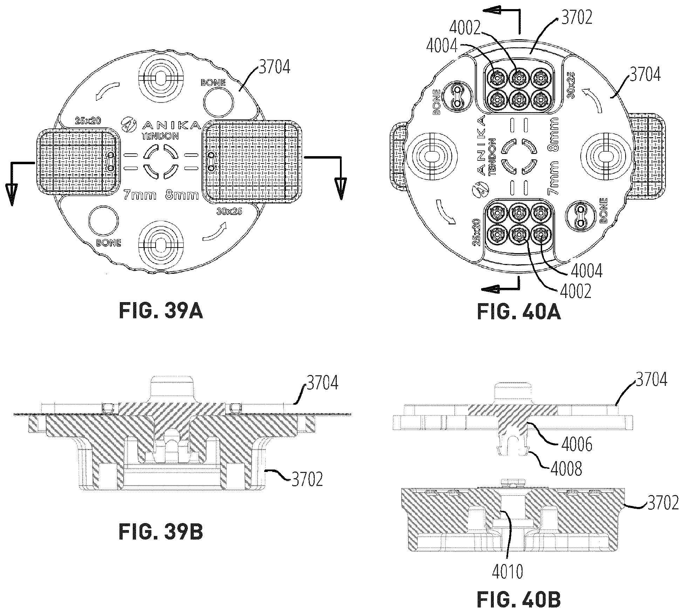

FIG. 37A is a upper perspective view of a first embodiment of the present caddy systems shown pre-loaded with implants and fasteners. Referring now to FIG. 37A and FIG. 40B; FIG. 37A and FIG. 37B depict upper and lower perspective views, respectively, of a first embodiment 4102 of the present caddy systems shown pre-loaded with implants 4502 and fasteners (e.g., tac fasteners 3808, bone fasteners 3804).

FIG. 37B is a lower perspective view of a first embodiment of the present caddy systems shown pre-loaded with implants and fasteners.

FIG. 38 is a top view of a base of the caddy system of FIG. 35.

FIG. 39A is a top view of the caddy system of FIGs. 37A-37B with a lid of the caddy system 4102 in a first configuration relative to the base. FIG. 39A depicts a top view of caddy system 4102 with a lid 3704 in a first configuration relative to base 3702.

FIG. 39B is a side cross-sectional view of the caddy system of FIG. 39A.

FIG. 40A is a top view of the caddy system of FIG. 37A and FIG. 37B with a lid of the caddy system 4102 in a second configuration relative to the base. FIG. 40A depicts a top view of caddy system 4102 with lid 3704 in a second configuration relative to base 3702

As shown generally, caddy system 4102 incudes a caddy base 3702 and a caddy lid 3704. The base generally defines one or more first fastener recesses configured to receive a first type of fastener. For example, in the depicted embodiment, caddy base 3702 defines a plurality of first fastener recesses 3802 configured to receive bone fasteners 3804 (staple-type fasteners with two substantially parallel projections). The caddy lid 3704 is configured to be rotatably coupled to the base such that the lid can be rotated to selectively cover or expose the first fastener recesses (e.g., between FIG. 39A and FIG. 40A). For example, in the depicted embodiment, caddy lid 3704 includes a central shaft 4006 extending from a lower side of the lid, and shaft 4006 includes resilient barbs 4008 such that the shaft can be inserted through a corresponding central opening 4010 in the base. During insertion, barbs 4008 are deflected radially inward and, once the shaft is fully inserted through opening 4010, barbs 4008 move radially outward to their relaxed state and thereby resist removal of the lid from the base (at least unless and until barbs 4010 are subsequently deflected radially inward again).

In the embodiment shown, a first implant 3806a (which can be any implant described herein) is disposed over a first one of the first fastener recesses 3802 and a bone fastener 3804 extends through the first implant and into the corresponding first fastener recess, both to preload the first implant with a bone fastener and to resist movement of the first implant relative to base 3702. In this configuration, spikes 3440 of delivery tool 3006 can be inserted into bone fastener 3804 in first implant 3806a to remove both of the bone fastener and the first implant and then simultaneously deliver that implant and fastener to a site in a patient and insert the bone fastener into a bone of a patient to secure the implant relative to that bone. In the depicted embodiment, a second implant 3806b is also disposed over a second one of the first fastener recesses 3802 and a second bone fastener 3804 similarly extends through the second implant and into the corresponding first fastener recess. As shown, each of first and second implants are flexible sheet-like (e.g., fibrous) implants, and second implant 3806b is larger than first implant 3806a (e.g., 30 mm x 25 mm versus 25 mm x 20 mm). In other embodiments, the first and second implants may be the same size. In some embodiments, the upper side of base 3702 also includes one or more implant recesses each overlapping at least one of the first fastener recesses 3802.

Once the implant is initially secured to the bone, caddy lid 3704 can be rotated relative to base 3702 (e.g., from FIG. 39A to FIG. 40A) to expose additional bone fasteners 3804 and soft tissue fasteners 4004. For example, base 3702 also includes additional first fastener recesses 3802 and corresponding bone fasteners 3804 that may be used to provide additional securement of one or both of the implants to the corresponding bone(s). For example, after the initial bone fastener 3804 is inserted to initially secure an implant to a bone, delivery tool 3006 may be withdrawn and inserted through one of the additional bone fasteners in base 3702, and the bone fastener can then be withdrawn from the base and inserted through the initially secured implant to further secure the implant to the bone. By way of further example, base 3702 also includes a plurality of second fastener recesses 4002 configured to receive soft-tissue fasteners 4004. As shown, soft tissue fasteners 4004 are dart-like fasteners (with a single projection) that can be inserted with a different delivery tool having a single spike at its distal end (in other embodiments, the soft-tissue fasteners may take a staple-like configuration similar to that of bone fasteners 3804, such that the bone fasteners and soft-tissue fasteners can both be delivered with the same delivery tool). As described above for bone fasteners, a single-spike tool can be inserted through one of soft-tissue fasteners 4004 and the fastener removed from base 3702 and inserted through a corresponding implant into soft tissue of a patient. This can then be repeated until the implant is sufficiently secured relative to the soft tissue. As one example, for repair of the supraspinatus tendon, a first portion of the implant can first be secured to the humeral head via one or more bone fasteners 3804, after which a second portion of the implant can be secured to the tendon via one or more soft-tissue fasteners 4004.

FIG. 40B is an exploded, side cross-sectional view of the caddy system of FIG. 40A.

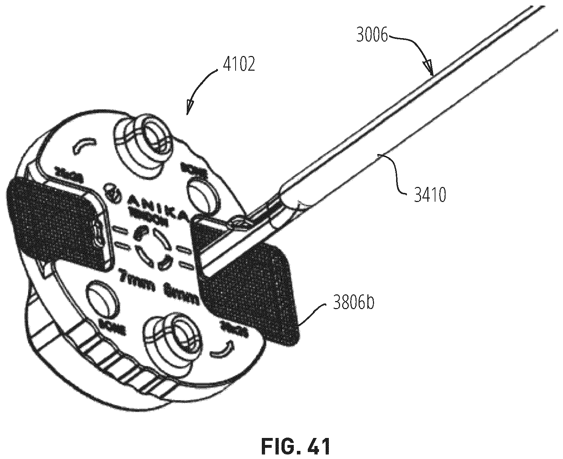

FIG. 41 is a perspective view of the delivery tool of FIG. 30 engaging a fastener of the caddy system of FIG. 37A and FIG. 37B. FIG. 41 - FIG. 43 depict an example of a method of deploying an implant and fastener from caddy system 4102 to a bone of a patient. As shown in FIG. 41, delivery tool 3006 (with seal assembly 3004 disposed around body 3410 as shown in FIG. 30) is aligned with bone fastener 3804 corresponding to implant 3806b, and spikes 3440 of the delivery tool inserted into the bone fastener.

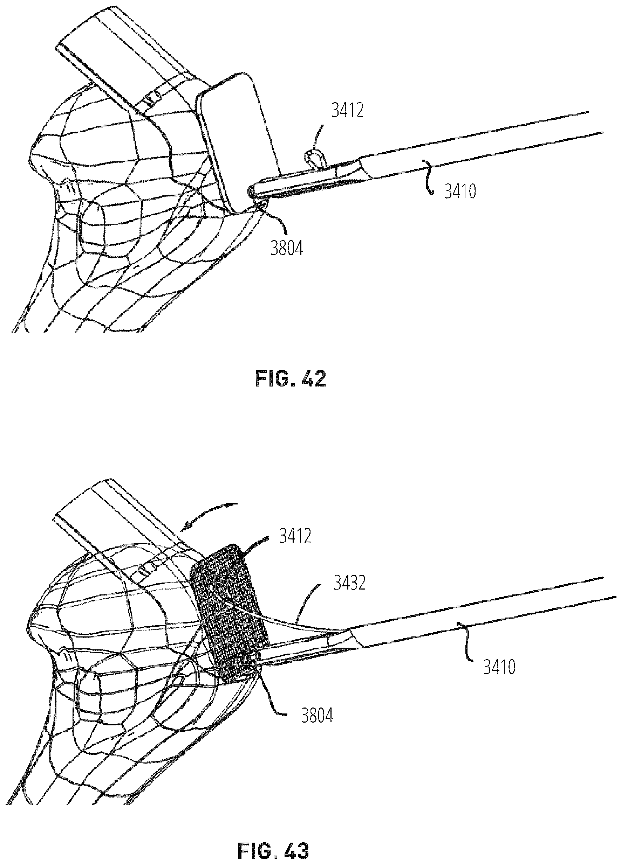

Implant 3806b and the corresponding bone fastener are then removed from caddy system 4102 and inserted through a cannula 3010 (not shown in FIG. 42 for clarity) to deliver the implant and bone fastener to a position over a patient's humeral head. Typically before insertion of the bone fastener into the humeral head, the distal end of seal assembly 3004 (not shown in FIG. 42 for clarity) is aligned with and inserted into recess 3208 on the proximal end of cannula 3010, and the seal assembly and cannula are secured together to seal the cannula so the region around the patient's humeral head can be filled with liquid such as saline to create space and improve visibility (typically via camera inserted through a separate port or incision). Once the seal assembly is secured to the cannula, and the region is optionally filled with liquid, the delivery tool can be manipulated to steer the implant and bone fastener to a desired location on the humeral head, and then to insert the bone fastener into the humeral head to secure a corresponding portion of the implant relative to the bone.

FIG. 42 is a perspective view of the delivery tool of FIG. 30 deploying a fastener to a bone of a patient to secure a first portion of an implant relative to the bone.

FIG. 43 is a perspective view of the delivery tool of FIG. 30, in the second configuration, deploying a second portion of the implant toward soft tissue.

Once the bone fastener is inserted, and as shown in FIG. 43, slider 3402 of the delivery tool can be moved from its retracted position (FIG. 34C) to its extended position (FIG. 36 and FIG. 42) to cause distal end 3412 of pusher 3432 to move distally and radially outward relative to body 3410. In doing so, curved portion 3522 of pusher 3432 slides along implant 3806b to urge the offset portion of the implant (which is offset in the longitudinal dimension of the implant from fastener 3804) toward the tendon to facilitate proper placement of the implant relative to the tendon. Additionally, handle 3406 of the delivery tool can be rotated relative to hub 3408 to cause distal end 3412 of pusher 3432 to sweep clockwise and/or counterclockwise (as indicated by arrow 328) to urge lateral extends of the offset portion of the implant toward the tendon. Thereafter, seal assembly 3004 can be disconnected from the cannula (e.g., after depressurizing the region around the humeral head) and delivery tool 3006 and seal assembly 3004 can be removed from the cannula, after which additional tools may be inserted (and additional seal assemblies attached) to deliver one or more soft-tissue fasteners (and/or to install sutures) to secure the offset portion of the implant to the tendon.

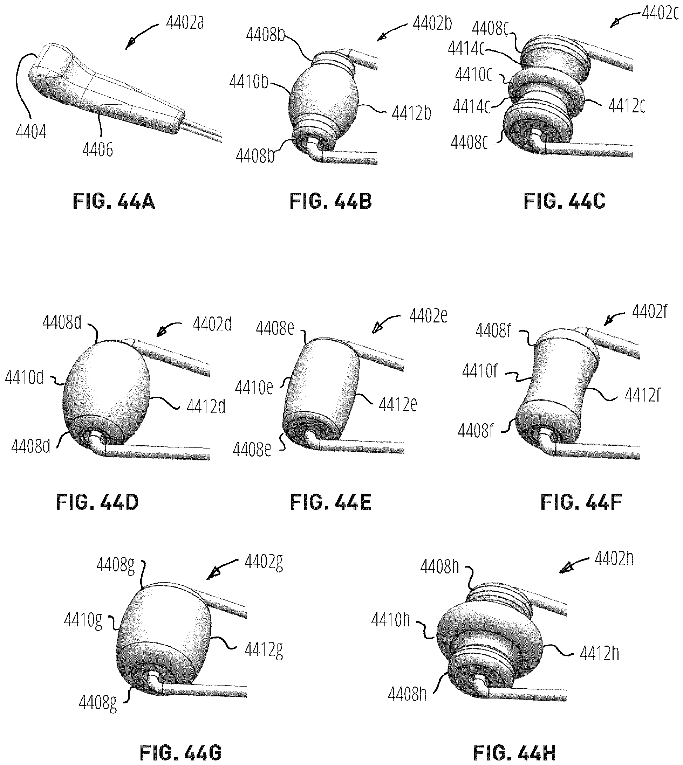

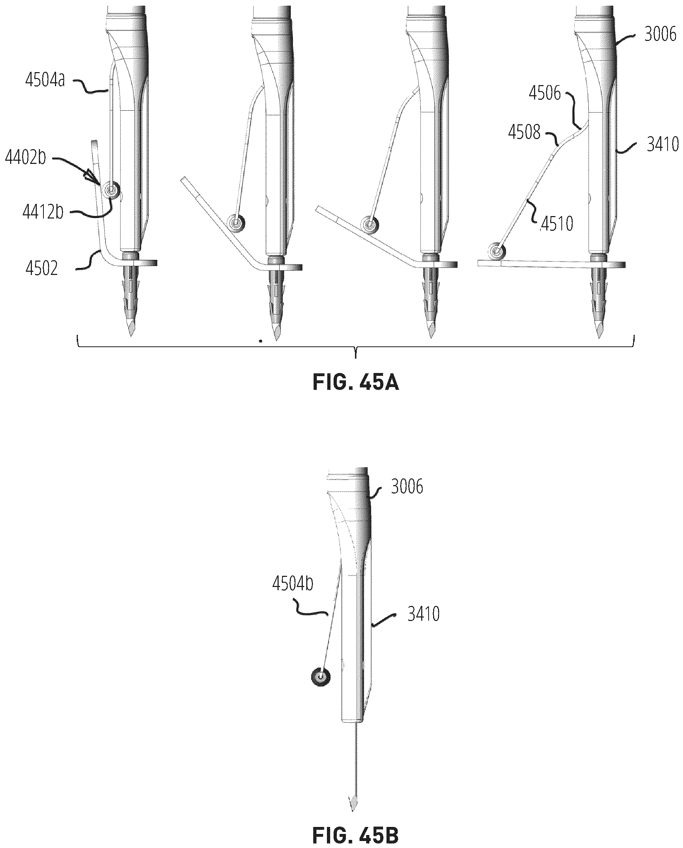

FIG. 44A depicts a perspective view of a configuration of a distal end of a pusher of the delivery tool. FIG. 44A illustrates an aspect of the subject matter in accordance with one embodiment. FIG. 44B -FIG. 44H depict variations of distal end 3522 that include rollers of various shapes configured to facilitate displacement of the distal end relative to an implant to deploy the implant (e.g., FIG. 45A). FIG. 44A -FIG. 44H depict perspective views of various configurations of a distal end of a pusher of the delivery tool 3432. FIG. 44A depicts a first variation 4402a of a distal end that includes an enlarged (relative to the wire or body of the pusher) club or foot 4406 that is overmolded or otherwise coupled to the body or wire of the pusher). Club or foot 4406 includes a curved distal surface 4404 configured to slide relative to the implant to deploy the implant (e.g., FIG. 42 to FIG. 43).

FIG. 44B depicts a perspective view of a configuration of a distal end of a pusher of the delivery tool. FIG. 44B illustrates an aspect of the subject matter in accordance with one embodiment. FIG. 44B depicts a first variation 4402b of a distal end that includes a roller 4412b, having medial section 4410b, and configured to rotate around a portion of the wire or body of the pusher that defines a rotational axis that is substantially perpendicular to the direction of movement of the pusher during deployment of an implant (e.g., FIG. 44A). In other embodiments, roller 4412b may be configured with an interference fit such that the roller may not rotate relative to the pusher during deployment of an implant; in such embodiments, however, the curved outer surface of the roller still facilitates movement of the pusher relative to the implant because of the curvature of that surface.

FIG. 44C depicts a perspective view of a configuration of a distal end of a pusher of the delivery tool. FIG. 44C illustrates an aspect of the subject matter in accordance with one embodiment. FIG. 44C depicts a further variation 4402c of a distal end that includes a roller 4412c. Roller 4412c is similar to roller 4412b, with the exception that roller 4412c includes a relatively narrow medial section 4410c that is separated by grooves 4414c from lateral sections 4408c, which have a common outer diameter that is the same as or slightly smaller (e.g., smaller by 10% or less) than the diameter of medial section 4410c.

FIG. 44D depicts a perspective view of a configuration of a distal end of a pusher of the delivery tool. FIG. 44D illustrates an aspect of the subject matter in accordance with one embodiment. FIG. 44D depicts a further variation 4402d of a distal end that includes a roller 4412d. Roller 4412d is similar to roller 4412b, with the exception that roller 4412d includes a convex, curved medial section 4410d that spans substantially all of the width of the roller, and the maximum outer diameter of medial section 4410d is between 20% and 80% (e.g., 40% to 50%) larger than the outer diameters of lateral sections or edges 4408d.

FIG. 44E depicts a perspective view of a configuration of a distal end of a pusher of the delivery tool. FIG. 44E illustrates an aspect of the subject matter in accordance with one embodiment. FIG. 44E depicts a further variation 4402e of a distal end that includes a roller 4412e. Roller 4412e is similar to roller 4412d, with the exception that the outer diameter of medial section 4410e is the same as or slightly larger (e.g., larger by 10% or less) than the diameter of lateral sections or edges 4408e.

FIG. 44F depicts a perspective view of a configuration of a distal end of a pusher of the delivery tool. FIG. 44F illustrates an aspect of the subject matter in accordance with one embodiment. FIG. 44F depicts a further variation 4402f of a distal end that includes a roller 4412f. Roller 4412f is similar to roller 4412e, with the exception that the outer diameter of medial section 4410f is slightly smaller (e.g., smaller by 10% or less) than the diameter of lateral sections or edges 4408f, such that roller 4402f defines a concave outer surface.

FIG. 44G depicts a perspective view of a configuration of a distal end of a pusher of the delivery tool. FIG. 44G illustrates an aspect of the subject matter in accordance with one embodiment. FIG. 44G depicts a further variation 4402g of a distal end that includes a roller 4412g having a medial section 4410g. Roller 4412g is similar to roller 4412e, with the exception that the lateral sections 4408g are each defined by a larger radius to define a more-rounded peripheral edge at either lateral end of the roller.

FIG. 44H depicts a perspective view of a configuration of a distal end of a pusher of the delivery tool. FIG. 44H illustrates an aspect of the subject matter in accordance with one embodiment. FIG. 44H depicts a further variation 4402h of a distal end that includes a roller 4412h. Roller 4412h is similar to roller 4412c, with the exception that the outer diameter of medial section 4410g is between 20% and 80% (e.g., 40% to 50%) larger than the outer diameters of lateral sections 4408h and medial section 4410g is proportionally wider than medial section 4410c.

FIG. 45A is a sequence of side views of a distal end of the delivery tool with a further embodiment of the pusher, illustrating the deployment of an implant. FIG. 45A is a sequence of side views of a distal end of the delivery tool 3006 with a further embodiment of the pusher 4504a, illustrating the deployment of an implant (from left to right in illustrated sequence). As slide 3402 (FIG. 34A) is advanced distally, distal end 4402b of pusher 3432 advances distally and radially away from elongated body 3410. In the depicted embodiment, pusher 4504a includes a wirelike body comprising a resilient material (e.g., stainless steel, nitinol or other shape-memory alloy, or the like) that is pre-shaped to assume a desired configuration upon extension from the interior of the elongated body (within which the shape of the pusher is constrained). As shown in the rightmost portion of FIG. 45A, pusher 4504a includes a first curve 4506 curving outward relative to the axis of the proximal portion of pusher 4504a, a second curve 4508 curving back inward relative to the axis of the proximal portion of pusher 4504a, and an axial distal portion 4510 extending from second curve 4508 to distal end 4402b. In this configuration, the tangent at the distal end of first curve 4506 is angled at an angle of between 40 and 60 degrees (e.g., 45 degrees) relative to the axis of the proximal portion of pusher 4504a, and second curve 4508 reverses direction and curves back to a distal end tangent that is disposed at an angle of between 20 and 40 degrees relative to the axis of the proximal portion of pusher 4504a. As a result, distal portion 4510 of pusher 4504a is disposed at an angle of between 20 and 40 degrees (e.g., 30 degrees) relative to the axis of the proximal portion of pusher 4504a, and roller 4412b is spaced from the body distal end 3416 by a distance that spans a majority of a corresponding dimension of implant 4502 to facilitate deployment of the implant, as shown. In the depicted examples, pusher 4504a includes a wire extending through a central passage of the roller and having two similar portions extending to wire ends 3520 within hub portion 3518 of slider 3402 (e.g., along a similar path as is shown in FIG. 35). In some examples, such as the one shown, pusher 4504a comprises a 316 LVM Stainless Steel wire, for example, with a diameter of 0.021 mm or smaller.

In the embodiments of FIG. 45A and FIG. 45B, seal body 4718 is also elongated (relative to seal body 3306 of FIG. 33A and FIG. 33B) such that a proximal portion 4706 extends from a knurled flange 4704 to proximal end 4720 by a greater length than that of seal body 3306. Proximal portion 4706 is sized to be received in hub distal end 3420 of hub 3408 (FIG. 34B-FIG. 34C), and proximal portion 4706 also defines a pair of grooves 4708 configured to receive ridges 3418 (FIG. 34B) of the hub to resist relative rotation between seal body 4718 and the hub when proximal portion 4706 of the seal body is received in distal end 3420 of the hub. In this configuration, the relatively greater length (and shallower angle) of first segment 4714 allows for a more-gradual compression of seal members 3314, reducing the amount of torque necessary to rotate seal assembly 3004 relative to cannula 3010; and the addition of second segment 4710, which is perpendicular to the longitudinal axis of seal body 3306, reduces the chance of counter-rotation of seal assembly 3004 relative to cannula 3010 during use (and potential loss of seal therebetween).

FIG. 45B is a side view of a distal end of the delivery tool with a further embodiment of the pusher. Other configurations of the pusher may include any number of curves or bends to facilitate deployment of an implant. For example, FIG. 45B depicts a side view of a distal end of the delivery tool 3006 with a further embodiment of the pusher 4504b, which is substantially similar to pusher 4504a with the exception that the wirelike body of pusher 4504b includes a relaxed shape that is substantially linear (e.g., similar to pusher 3432 in FIG. 35), as opposed to including pre-defined curves or angles. In this configuration, as pusher 4504b is moved longitudinally relative to body 3410 in a distal direction, sloped surface 3510 will urge the distal end of pusher 4504b laterally outward relative to body 3410 and, as the roller (e.g., 4412b) contacts tissue such as bone, the roller will roll against the tissue to facilitate the further lateral extension of pusher 4504b. In this way, the roller may in some configurations be extended laterally outward until the roller is spaced from the body distal end 3416 by a distance that spans a majority of a corresponding dimension of implant 4502 to facilitate deployment of the implant, as shown.

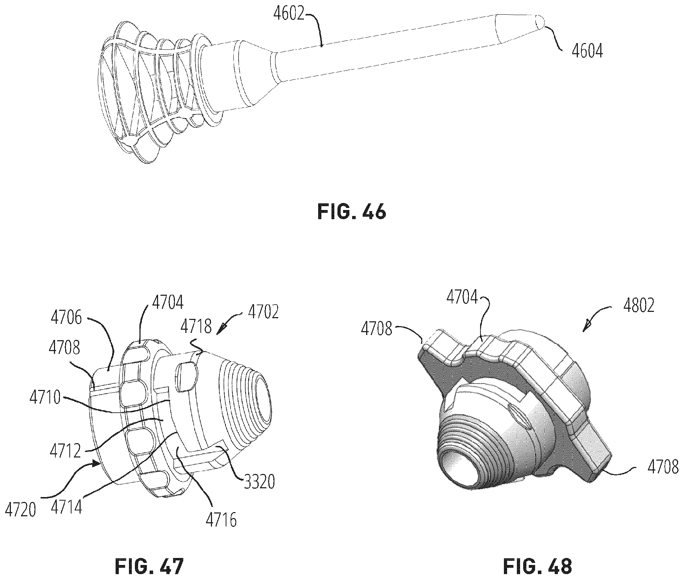

FIG. 46 is a perspective view of a further embodiment of the obturator of FIG. 30 and FIG. 31. FIG. 46 is a perspective view of a further embodiment 4602 of the obturator of FIG. 30 and FIG. 31. Obturator 4602 is substantially similar to obturator 3008 of FIG. 30 and FIG. 31, with the primary exception that obturator 4602 has an obturator distal end 4604 that conically tapers to a rounded end (as opposed to planar sides converging to a point).

FIG. 47 is a distal perspective view of a further embodiment 4702 of the seal assembly of FIGs. 30 and 33A-33B. Seal assembly 4702 is substantially similar to seal assembly 3004, with the primary exceptions that seal assembly 4702 includes modified J-slots 4716 and an elongated seal body 4718. As shown in FIG. 47, each of J-slots 4716 includes a longitudinal portion 3320 extending generally in the longitudinal direction, and a circumferential portion 4712 extending in a generally circumferential direction around a peripheral portion of the seal body. Circumferential portion 4712 includes a first segment 4714 and a second segment 4710. First segment 4714 includes a distal surface that extends helically around the perimeter of the seal body such that a line tangential to the curve of distal surface is angled relative to central, longitudinal axis of seal body 4718 by an angle of between 80 degrees and 90 degrees (e.g., between 83 degrees and 87 degrees). In this embodiment, first segment 4714 has a length in the circumferential direction that is at least 200% of a width (in the same direction) of longitudinal portion 3320. Second segment 4710 is configured such that its distal surface is substantially perpendicular to the central, longitudinal axis of seal body 4718.

FIG. 48 is a distal perspective view of a further embodiment 4802 of the seal assembly of FIG. 30, FIG. 33A, FIG. 33B, FIG. 45A, FIG. 45B. Seal 4802 is similar to seal 4702, with the primary exception that seal 4802 includes a pair of protruding handles 4708 extending outward from the circumferential surface of knurled flange 4704 to facilitate the application of a greater torque by a user (e.g., during single-handed operation).

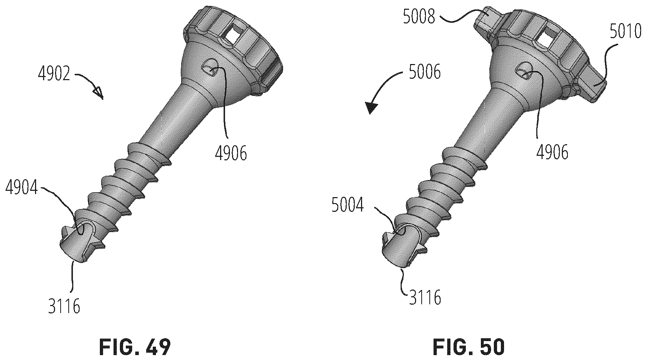

FIG. 49 is a distal perspective view of a further embodiment 4902 of the cannula of FIG. 30, FIG. 31, and FIG. 32. Cannula 4902 is similar to cannula 3010, with the primary exception that cannula 4902 defines a notch 4904 at distal end 3116. Notch 4904 is configured to be aligned with distal end 3522, 4402a, 4402b, etc. to create additional space for, and thereby facilitate deployment of, the implant via extension of the distal end of the pusher (e.g., as shown in FIG. 45). In this embodiment, cannula 4902 also includes a visual and/or physical indicator 4906 that is circumferentially aligned with notch 4904 to enable a user (e.g., surgeon) to align slider 3402, and thereby the distal end of the pusher, with notch 4904. In the depicted embodiment, indicator 4906 is a divot or impression that can be felt by a user without requiring visual confirmation; in other variations, indicator may be a protrusion, may be a painted indicator with minimal physical variation from the surrounding surface, or may be any other feature or combination of features by which a user can perceive the alignment of notch 4904 while distal end 3116 of the cannula is disposed in a patient.

FIG. 50 is a distal perspective view of a further embodiment 5006 of the cannula of FIG. 30 - FIG. 32 and FIG. 47.

Cannula 5006 is similar to cannula 4902, with the primary exception that cannula 5006 includes a pair of protruding handles (5008, 5010) extending outward from the knurled circumferential surface of head or hub 3112 to facilitate the application of a greater torque by a user (e.g., during single-handed operation). Cannula 5006 further includes a notch 5004.

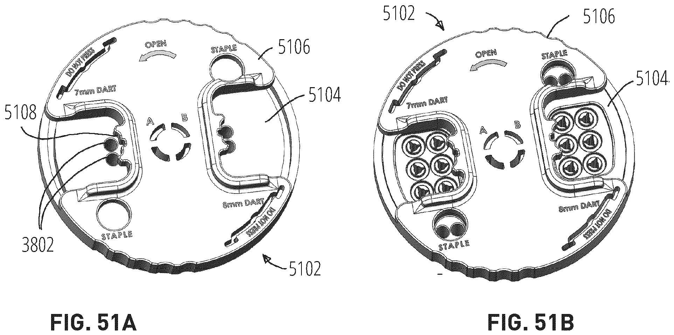

FIG. 51A is an upper perspective view of a further embodiment of the caddy system of FIG. 37A -FIG. 40B. FIG. 51A and FIG. 51B are upper perspective views of a further embodiment 5102 of the present caddy systems. In FIG. 51A, lid 5106 is in a first rotational position relative to base 5104 in which the implants regions are accessible.

Caddy system 5102 is substantially similar to caddy system 4102, with the primary exception that lid 5106 defines a tool guide having a lateral surface 5108 disposed above (and spaced apart from) first fastener openings 3802, with surface 5108 configured to contact a portion of an elongated body 3410 of tool 3006, as the tool is inserted through a bone fastener (e.g., fastener 3804) in or into first fastener recesses 3802, to encourage alignment of the fastener (e.g., fastener 3804) with first fastener recesses 3802 and/or alignment of spikes 3440 of the tool with the corresponding passages in the fastener. In the embodiment shown, surface 5108 is shaped to contour to an exterior shape of the elongated body 3410 of the tool 3006. In other embodiments, surface 5108 may include one or more bumps or peaks to facilitate centering or alignment of tools of different cross-sectional shapes.

FIG. 51B is an upper perspective view of a further embodiment of the caddy system of FIG. 37A - FIG. 40B. In FIG. 51B, lid 5106 is in a second rotational position relative to base 5104 (rotated 90 degrees relative to the first rotational position of FIG. 51A) in which the implant regions are not accessible but the tendon (soft tissue) fasteners and additional bone fasteners are accessible.

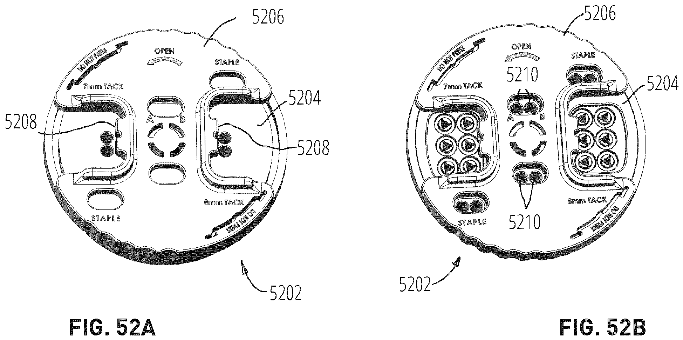

FIG. 52A is an upper perspective view of a further embodiment of the caddy system of FIG. 51A and FIG. 51B. FIG. 52A and FIG. 52B are upper perspective views of a further embodiment 5202 of the present caddy systems. In FIG. 52A, lid 5206 is in a first rotational position relative to base 5204 in which the implants regions are accessible. In FIG. 52B, lid 5206 is in a second rotational position relative to base 5204 (rotated 90 degrees relative to the first rotational position of FIG. 52A) in which the implant regions are not accessible but the tendon (soft tissue) fasteners and additional bone fasteners are accessible. Caddy system 5202 is substantially similar to caddy system 5102, with the primary exception caddy system 5202 includes storage for an additional pair of bone fasteners and tool guides with surfaces 5208 having a different shape.

FIG. 52B is an upper perspective view of a further embodiment of the caddy system of FIG. 51A and FIG. 51B. First, base 5206 defines two additional first fastener recesses 5210, and lid 5206 defines two additional corresponding openings to permit access to the bone fasteners in first fastener recesses 5210 when lid 5206 is in the second rotational position (FIG. 52B) relative to base 5206. Second, tool guide lateral surfaces 5208 are each flat with a medial alignment notch rather than being contoured to a curved outer surface of a tool (e.g., 3006). In this configuration, surfaces 5208 are offset radially from the center of lid 5206 to space the corresponding fasteners further from the edge of the corresponding implants; however, in other embodiments, surfaces 5108 and 5208 can be disposed at any position to achieve desired placement relative to the corresponding implants.

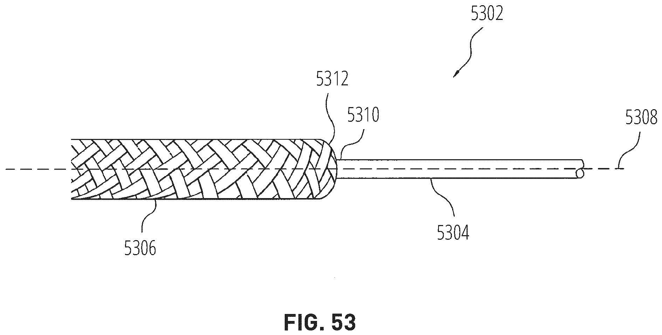

FIG. 53 shows, in schematic form, a portion of a multipart suture 5302 prepared according to principles of the invention. The exemplary suture 5302 includes a first monofilament portion 5304 and a second braided portion 5306. The first 5304 and second 5306 portions are generally elongate and uniform and share a common longitudinal axis 5308. It will be appreciated that although the illustrated portion of longitudinal axis 5308 is shown as generally linear, the first 5304 and second 5306 portions will typically be more or less flexible and, consequently, at any given moment, the longitudinal axis will be curved according to the instantaneous configuration of the suture portions 5304, 5306.

The first portion 5304 is coupled to the second portion 5306 at respective ends 5310, 5312. As will be further discussed below, this coupling will be achieved by any of a variety of coupling arrangements, according to various considerations associated with a particular anticipated application of the multipart suture 5302 and with considerations of manufacturing and manufacturability.

The first 5304 and second 5306 portions of the suture 5302 will, in certain embodiments, have a generally uniform cross-sectional profile, as taken along the longitudinal axis 5308. In suture 5302, as illustrated, both first 5304 and second 5306 portions have a generally circular cross-sectional profile. As will be further discussed below, however, a wide variety of alternative profiles will be employed in other aspects and embodiments of the invention, including embodiments in which the profile of a suture portion varies with respect to longitudinal axis 5308.

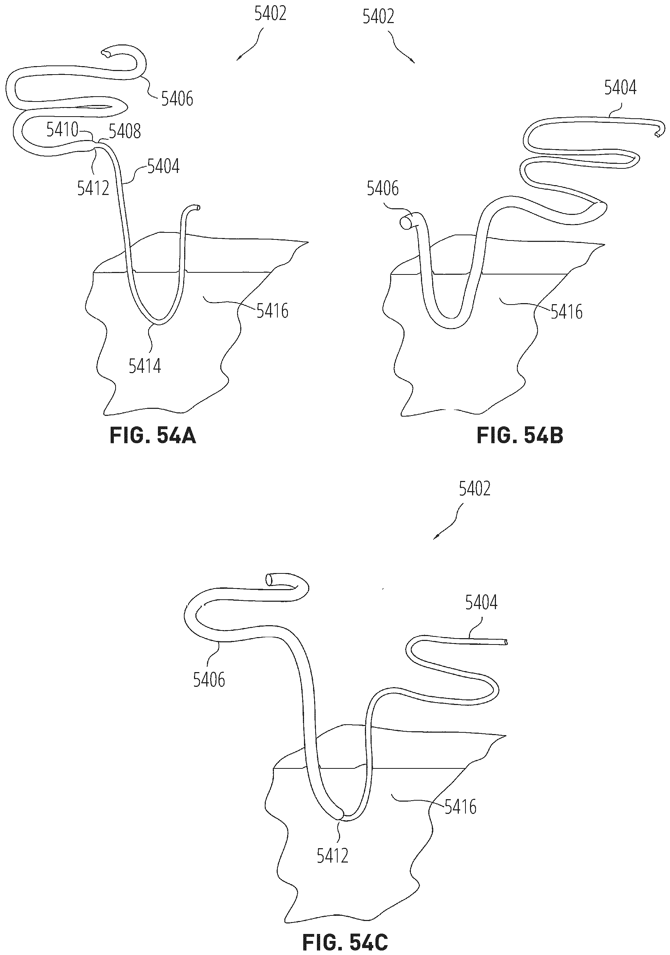

] Referring again to FIG. 53, and as previously noted, the suture 5302 includes a first portion 5304 shown as a monofilament suture portion and a second portion 5306 shown as a braided suture portion. It should be understood that these are merely illustrative of characteristics that will be incorporated in suture portions of respective embodiments of the invention. Thus, for example, in certain embodiments, the monofilament portion 5304 will include a bioabsorbable material and the braided portion 5306 will include a non-bioabsorbable material. Thus, consistent with the discussion above of suture 5402 in relation to FIG. 54A-FIG. 54C, a user can select for application in a particular circumstance, a bioabsorbable or a non-bioabsorbable suture simply by drawing the suture more or less completely through the tissue matrix.

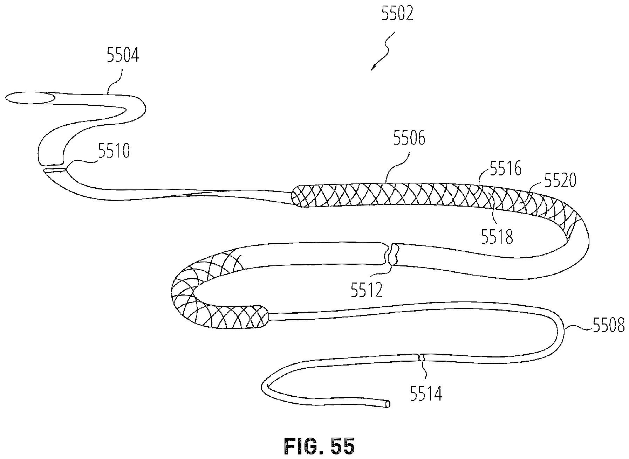

It will be further understood that, in certain embodiments (and conversely to the example above), a braided portion of the suture will include a bioabsorbable material while the monofilament portion of the suture will include a non-bioabsorbable material. Further, and as exemplified in FIG. 55, in certain embodiments a single suture will incorporate more than two portions, where different physical configurations and different levels of bioabsorbability will be exhibited by the respective portions.

] FIG. 54A, FIG. 54B, and FIG. 54C illustrate schematically certain attributes of a multicomponent suture 5402 prepared according to principles of the invention when in use. FIG. 54A shows a suture 5402 having first 5404 and second 5406 longitudinal portions. The first and second portions are coupled to one another at respective adjacent ends thereof 5408, 5410 at a coupling region 5412 of the suture. In the illustrated exemplary application, part 5414 of first longitudinal portion 5404 is disposed within a tissue matrix 5416.

It will be appreciated that the tissue matrix may be any of a wide variety of materials including, for example and without limitation, muscle, bone, tendon, ligament, and any organ including, again without limitation, skin, eye, kidney, heart, brain, lung, spleen, etc. it will also be appreciated that the tissue matrix may be human or otherwise, and that a suture according to principles of the invention will be used in medical applications, veterinary applications and other applications limited only by the imagination of the user. The part 5414 of first longitudinal portion 5404 will have been disposed within the tissue matrix 5416 by any appropriate method or apparatus including without limitation, by coupling a region of the longitudinal portion 5404 to a needle (not shown) and using the needle in a conventional manner to draw the first longitudinal portion 5404 into and through the tissue matrix 5416.

Having achieved the illustrated configuration, a user can elect to cut off and discard the second portion 5406 of the suture, proceeding thereafter to use the first portion 5404 of the suture in any conventional manner.

Alternately, the user can draw the suture 5402 through the tissue matrix 5416 so as to achieve the configuration illustrated in FIG. 54B. Thereafter, the user can elect to cut off and discard the first portion 5404 of the suture, proceeding thereafter to use the second portion of the suture 5406 in any conventional manner.

The attributes of the suture of the invention will exhibit particular advantages in circumstances such as, for example, use by a paramedic or a combat medic. For such a user, access to an inventory of different sutures having different characteristics, while desirable, can be highly problematic. Moreover, selection of suture characteristics may be rendered difficult by the exigent circumstances (e.g., combat, natural disaster, fire, wilderness first aid and rescue, etc.). In such circumstances, the ability to carry a single suture exhibiting multiple characteristics can be greatly beneficial.

It should be understood, however, that the same characteristics can be similarly beneficial in the operating theater. Often, the surgeon or other medical professional will be faced with circumstances that evolve during the course of an operation or other therapeutic procedure. The ability to select desired characteristics of a suture in real time simply by drawing the suture more or less completely through a tissue penetration can ease decision-making in an otherwise highly stressful environment. Moreover, in some circumstances, the ability to modify the characteristics of the suture in use as circumstances evolve will avoid the need to withdraw one suture after it has been inserted and replace it with another. This can reduce the time necessary for the procedure and, potentially, the number of personnel required to assist.

It will be appreciated that, in appropriate circumstances, all of these benefits of the present invention will tend to save time in the course of a procedure and facilitate a beneficial outcome. Notwithstanding that anesthesia has improved vastly in recent years, every surgical procedure retains some level of risk, and the ability to operate efficiently and quickly remains of great advantage to the likelihood of a successful outcome and, thus, to the ultimate interest of the patient.

As yet another alternative, the user can elect to draw suture 5402 through the tissue matrix 5416 until the coupling region 5412 is within the tissue matrix 5416, as illustrated in FIG. 54C. Thereafter, the user can use any part of the first 5404 and second 5406 portions of the suture 5402 in whatever manner is deemed by the user to be beneficial.

In regarding the suture 5402, and its method of use, as exemplified in FIG. 54A -FIG. 54C it will become evident to the creative practitioner of ordinary skill in the art that a suture according to principles of the invention has the great advantage of allowing the user to select for use, in particular circumstances, the portion of the suture having characteristics most suitable to those circumstances. Thus, for example, rather than having to identify, in advance of deployment, the suture characteristic that will be most beneficial to a particular circumstance, the user can identify those characteristics at the moment of deployment and adjust the suture accordingly.

FIG. 55 thus illustrates a suture 5502 including a first portion 5504, second portion 5506, and a third portion 5508. Each of portions 5504, 5506 and 5508 has a different physical and/or chemical characteristic and may be selected for use as described above. Thus, for example, in certain embodiments first portion 5504 will be a bioabsorbable monofilament having a first relatively short time constant for bioabsorption, second portion 5506 will be a substantially non-bioabsorbable woven suture and third portion 5508 will be a bioabsorbable monofilament having a second relatively long time constant for bioabsorption. Moreover, while the cross-sectional profile of the first, second and third portions will, in certain embodiments be similar to one another (as, for example, all substantially circular) in the illustrated suture 5502, illustrative cutaways show that the cross-sectional profile 5510 of first portion 5504 is flattened, or elliptical, as compared with the substantially circular cross-sectional profile 5512 of second portion 5506 and the likewise substantially circular cross-sectional profile 5514 of third portion 5508.

In certain embodiment of the invention, it will be beneficial to color code a particular portion of a suture so as to readily identified to a user the characteristics of that portion of the suture. Thus, for example, in certain embodiments a monofilament portion such as portion 5504 will include a dye within this formulation producing a uniform and identifiable color at its surface. In other embodiments, a colored coating will be applied to an external surface of the suture material. In still further embodiments, a colored material will be disposed internally within the suture (as, for example, by co-extrusion) and be visible through a transparent or translucent outer material of the suture. Still further, a woven suture such as that of portion 5506 will include, in respective embodiments, one or more fibers (e.g., 5516, 5518, 5520) exhibiting one or more respective colors, where the color is the result of an intrinsic component material, a coating, or a co-extruded internal portion, etc., as discussed above.

It will be appreciated by one of ordinary skill in the art that in addition to coloration, patterns and patterns of colors will be provided in respective embodiments to identify particular suture portions and characteristics of suture portions. Such patterns will be applied by any appropriate method, such as is known or becomes known, including, for example and without limitation, material dying, printing, painting, dipping, bleaching, laser marking including chemical activation, ablation, bleaching, and burning. In certain embodiments, the identifying pattern will consist of readable symbols including numbers, letters, other characters, and machine-readable markings including barcodes and other machine-readable codes.

In still further embodiments, surface markings and/or surface texture will be employed to help a user readily identify the characteristics of a particular portion of the suture. Thus, for example, the characteristics of a particular portion of a suture will be apparent to a user based on the sense of touch, even where the portion of the suture in question is obscured by its placement within a wound or a body cavity, by the absence of light in an emergency or first aid situation, or by other factors. A surface marking or surface texture will be included on a portion of a suture either as a result of an intrinsic characteristic of the suture portion (as, for example the difference between a woven, knitted or braided portion and extruded monofilament or multifilament portion) or as a result of an active manufacturing step. Thus, in certain embodiment, manufacturing of a portion of a suture will include embossing, grooving, cutting, laser marking, rolling, or any other process appropriate to make a portion of a suture identifiable by touch. It should be recognized that along with other benefits, a particular cross-sectional profile will aid in the identification of a particular portion of a suture to a user.

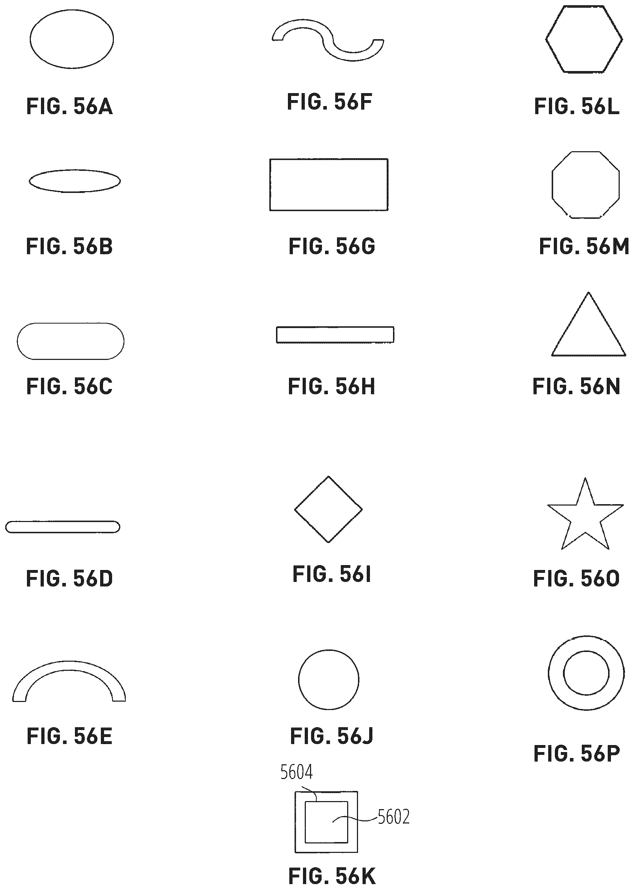

As noted above, different embodiments and aspects of the invention will include different cross-sectional profiles associated with one or more portions of a suture. FIG. 56A-FIG. 56P illustrate, in schematic form and purely for exemplary purposes, various cross-sectional profile that will be beneficially applied in respective embodiments of the invention. Accordingly, FIG. 56A shows a cross-section of a portion of a suture having a generally elliptical profile.

FIG. 56B shows a cross-section of a portion of a suture having a further elliptical profile with a larger differential between the horizontal and vertical axes (as compared with that of FIG. 56A) so as to present a flattened aspect like that of a ribbon or tape. It will be understood that the ratio between the horizontal and vertical axes of the cross-sectional profile are referred to as an aspect ratio of the cross-sectional profile. In various embodiments of the invention, a suture portion will include a cross-sectional profile having an aspect ratio of between, for example 0.001 (1/1000) and 1 (one or unity).

More commonly, a suture portion will include a cross-sectional profile have an aspect ratio of between 0.1 (1/100) and one. Typically, the aspect ratio of a suture portion will be substantially constant as a function of position along the longitudinal axis of the suture portion. In some embodiments, however, the aspect ratio will vary as a function of location along the suture portion.

FIG. 56C show the cross-sectional portion of the suture having a flat-oval profile with first and second substantially linear regions and, disposed therebetween, semicircular regions again, giving more or less the impression of a ribbon or tape.

In some embodiments, the cross-sectional portion of the suture has a tape-like characteristic. For example, the suture portion profile presented in FIG. 56D has a generally flat-oval characteristic.

FIG. 56E illustrates an example of a suture portion having an arcuate tape profile where, in various embodiments, the arc will reflect a circular arc, an elliptical arc, or any other arc appropriate to a particular application.

FIG. 56F illustrates an example of a generally tape-like cross-sectional profile, here incorporating a reverse curve. It will be appreciated that any complex curve including, for example, multiple reverse curve, will be employed in appropriate embodiments of the invention.

In some embodiments, the cross-sectional portion of the suture has a rectangular profile such as shown in FIG. 56G.

In some embodiments, the cross-sectional portion of the suture has a flattened rectangular profile (such as a tape or ribbon style profile) such as that shown, for example, in FIG. 56H.

In some embodiments, the cross-sectional portion of the suture has a a square or diamond-shaped profile. For example, the the cross sectional portion of the suture may have a diamond-shaped profile as shown in FIG. 56I.

In some embodiments, the cross-sectional portion of the suture has a substantially circular profile such as, for example, shown in FIG. 56J.

In some embodiments, the cross-sectional portion of the suture has a tubular profile as illustrated by the square tubular profile of FIG. 56K.

It will be appreciated that in the case of a tubular profile, an internal region of the suture portion may be evacuated, or may be open to the ambient atmosphere, or may contain a solid or liquid or gaseous material having characteristics that make the overall characteristics of the suture portion desirable. In like fashion, the internal region 5602 of the tubular suture portion may form the structural portion of the apparatus, while the exterior of the tubular suture portion 5604 may have other beneficial characteristics including desirable lubricity, desirable optical characteristics, desirable texture, desirable biocompatibility, or any other desirable characteristic.

In some embodiments, the cross-sectional portion of the suture has a hexagonal profile, as illustrated by the hexagonal profile of FIG. 56L.

In some embodiments, the cross-sectional portion of the suture has a octagonal profile, as illustrated by the octagonal profile of FIG. 56M.

In some embodiments, the cross-sectional portion of the suture has a triagonal profile, as illustrated by the triagonal profile of FIG. 56N.

In some embodiments, the cross-sectional portion of the suture has a stellate profile, as illustrated by the stellate profile of FIG. 56O

FIG. 56P shows a further cross-section of a suture portion, here substantially circular and tubular (i.e., circular cylindrical) where all of the various considerations discussed above in relation to the square tubular will likewise apply in respective embodiments and aspects of the invention. Of course it will be appreciated that in addition to square and circular tubular configurations, other geometrical configurations will be beneficially employed in respective applications of the invention.

Again, it should be emphasized that these various geometrical configurations disclosed here are not intended to form an extensive list, but are merely exemplary of the wide variety of cross-sectional profile that will be beneficially employed and readily apparent to one of ordinary skill in the art who considers the matter in light of the foregoing disclosure.

It will be appreciated, of course, that any other profile appropriate to a particular application of the invention will be beneficially applied to a respective portion of a suture, and that such application is considered to fall within the scope of the invention, and to be proprietary to the extent that it is ultimately claimed.

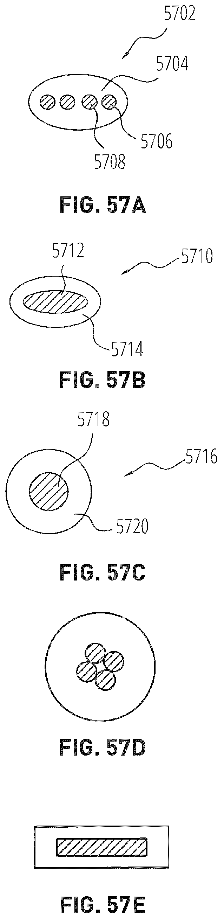

FIG. 57A-FIG. 57E illustrate further aspects of the invention, according to which reinforcing materials are disposed within one or more portions of a suture prepared according to principles of the invention. Thus, FIG. 57A shows, in cross-section, a portion of a suture 5702 according to principles of invention, the cross-section having a generally ribbon elliptical configuration where a first material 5704 surrounds and encapsulates a plurality of generally really flexible longitudinal members, e.g., 5706, 5708 of generally circular cross-section.

In one exemplary embodiment, the generally flexible longitudinal members 5706, 5708 will be selected for their tensile characteristics, while the surrounding material 5704 provides a desirable cushioning characteristic, a desirable degree of biochemical imperviousness, or other desirable characteristic. It should be understood that in other embodiments, the external material 5704 may be selected for its tensile properties while the internal material 5706, 5708, etc. will have other beneficial characteristics.

FIG. 57B shows, in cross-section, a further suture portion embodiment 5710 of similarly generally elliptical configuration, again with an internal portion 5712 and an external portion 5714. As with the embodiment described above, the characteristics of the internal portion 5712 and the external portion 5714 will differ and be selected to complement one another. Thus, for example, the internal portion 5712 may have desirable tensile characteristics while the external portion 5714 may have other desirable physical properties.

FIG. 57C shows a still further portion of an embodiment 5716 of generally circular configuration. Once again, the characteristics of the internal portion 5718 and the external portion 5720 will differ and be selected to complement one another. Thus, for example, the internal portion 5718 may have desirable tensile characteristics while the external portion 5720 may have other desirable physical properties.

it should be noted that, in certain embodiments and aspects of the invention, one or the other of the internal portion 5718 and the external portion 5720 will be bioabsorbable while the other will not be. Alternately, the two portions 5718, 5720 may both be bioabsorbable, but with different bioabsorption time constants such that one is absorbed more rapidly than the other. Thus, a particular application of the suture can be engineered to provide varying characteristics over the *in vivo* life of the suture.

Purely as an example, external material 5720 will be chosen, in certain embodiments, to be substantially less elastic than internal material 5718 and also external material 5720 will be chosen to have a substantially shorter bioabsorption time constant than internal material 5718. Consequently, after being deposited *in vivo,* during a first time period the suture will have a first relatively inelastic characteristic. Upon the more or less complete absorption of the external material 5720, however, the characteristics of the internal material will come to dominate the structure such that the overall elasticity of the suture become higher than it was at installation; i.e., the suture as a whole becomes more elastic with time.

Likewise, FIG. 57D illustrates an exemplary arrangement and embodiment with characteristics that are self-evident in light of the figures themselves and the foregoing explanation.

FIG. 57E also illustrates an exemplary arrangement and embodiment with characteristics that are self-evident in light of the figures themselves and the foregoing explanation.

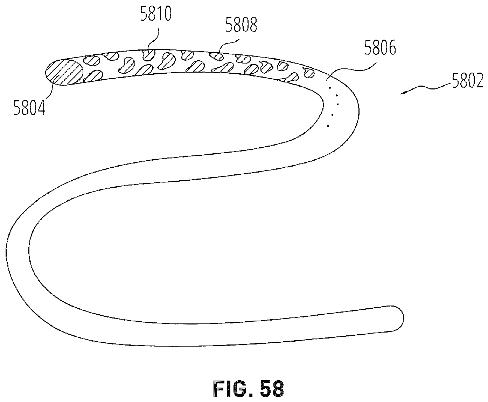

FIG. 58 shows a portion 5802 of a further suture embodiment according to principles of the invention. As discussed above, suture portion 5802 includes an internal material 5804 and an external material 5806 having different respective characteristics. In the illustrated embodiment, the external material includes a plurality of preparations, e.g., 5808, such that certain regions 5612 of the internal material 5804 are exposed.

As with the embodiments discussed above, the materials of suture portion 5802 will be selected, in certain embodiments, to have different time constants for bioabsorption. In certain embodiments, the internal material 5804 will have a shorter bioabsorption time constant then the external material 5806. Consequently, the suture portion 5802 can be arranged such that as the internal material 5804 is absorbed, the overall assembly of suture portion 5802 becomes more flexible and/or elastic.

The bioabsorption time constant of the external suture material 5806 will, in certain embodiments, be substantially indefinite, such that the extra material remains behind substantially permanently after the internal material dissolves. As such it will be understood that while the discussion of Figure 53 involves selecting one portion of the multi-part suture while removing and discarding another portion, it is also within the scope of the invention to prepare an employee multipart suture were more than one part of the suture is used, and where different parts of the suture may be co-located. In certain embodiments, the different parts will have different characteristics including different markings, different chemical and physical properties, and different levels of persistence when applied *in vivo.*

Again, referring to FIG. 53, where two portions 5304, 5306 of a suture are coupled together, various coupling means will be employed in respective embodiments of the invention. In some embodiments, where the configuration or structure of the two portions 5304, 5306 differ but the materials of the two portions 5304, are identical or substantially similar, the two portions may be formed integrally without any discrete coupling mechanism. In other embodiments, as discussed below, various coupling mechanisms appropriate to the particular circumstances and as known in the art or as will be discovered, are employed. Examples follow, on the basis of which the practitioner skilled in the art will understand further principles of the invention, and readily arrive at many additional and derivative arrangements and embodiments.

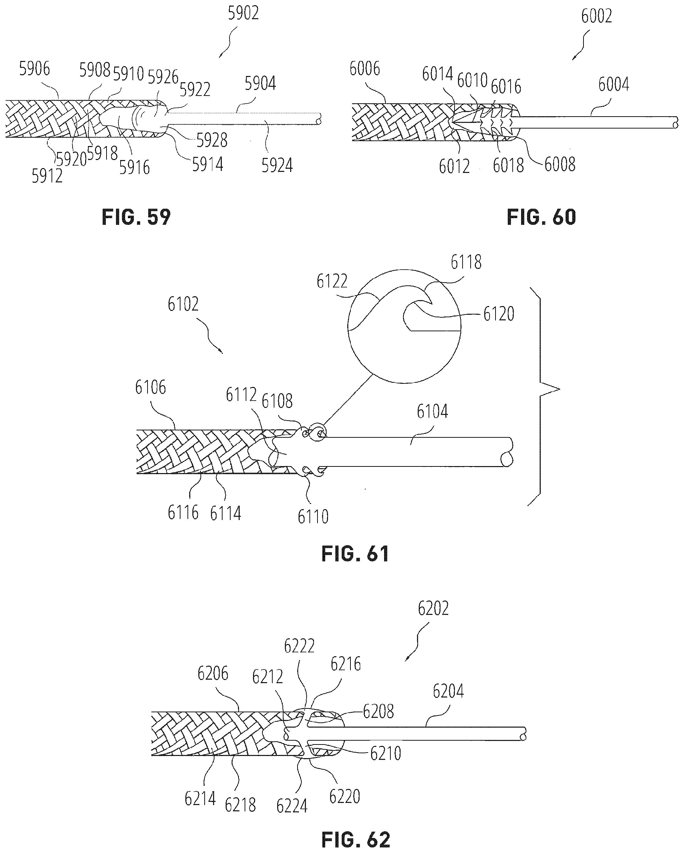

FIG. 59 shows, in cutaway schematic view, a portion of an exemplary suture 5604 prepared according to principles of the invention. The exemplary suture 5604 includes a first monofilament portion 5606 and a second braided portion 5608. The first 5606 and second 5608 portions are generally elongate and share a common longitudinal axis (not shown). In the illustrated example, braided portion 5608 includes a plurality of fibers, threads or yarns e.g., 5610, 5612 arranged and configured to form a generally tubular portion 5614 and a substantially closed-end referred to as a sock toe portion 5616.

Like the toe of a sock, the closed-end sock toe portion terminates the tubular portion and serves to close one end of an internal cavity 5618 defined within the tubular portion 5614. In the illustrated embodiment, the fibers, threads or yarns exemplified by 5610 and 5612 define apertures therebetween, e.g. 5620, 5622. Similar apertures are present in the sock toe portion 5616 and, in the illustrated embodiment, a region of the second monofilament portion 5606 is disposed within one such aperture, such that part 5922 of monofilament portion 5606 is disposed within cavity 5618 and another part 5924 of monofilament portion 5606 is disposed outside of cavity 5618.

As illustrated, the part 5922 of monofilament 5606 disposed within cavity 5618 includes an enlarged portion 5926. In the illustrated embodiment, this enlarged portion 5926 embodies a generally spherical aspect, having a peripheral surface region 5928 arranged and adapted to interfere mechanically with fibers of the sock toe region and effectively couple the first monofilament portion 5606 and second braided portion 5608 of the exemplary suture 5604 substantially coherently to one another.

It will be appreciated by one of skill in the art that the flexible nature of the braided fibers, threads or yarns, along with the arcuate spherical shape of the peripheral surface region 5928 will allow flexibility at the joint so that a longitudinal axis of the first monofilament portion 56106 can pivot somewhat freely with respect to a longitudinal axis of the second braided portion 5608.

It will be further understood that enlarged portion 5926 need not be spherical, but may include any of a wide variety of forms appropriate to particular circumstances. Moreover, enlarged portion 5926 may be integrally formed as part of the monofilament portion 5924 (e.g., be molded or extruded in place or formed by plastic deformation with or without reheating), or may be a separate element or component coupled to the balance of first monofilament portion 5924 by any appropriate method. Thus, in various embodiments, the enlarged portion 5926 and the balance of the monofilament portion 5924 will be coupled by the application of one or more of a combination of internal and external threads, respectively, thermal welding, ultrasonic welding, chemical adhesion, physical pressfit/friction fit, swaging, riveting, the use of a fastener such as a nail, a screw, a bolt, a roll pin, a cotter pin, or any other fastening means which is or becomes known to those of skill in the art.

FIG. 60 shows, in cutaway schematic view, a portion of a further exemplary suture 6002 prepared according to principles of the invention. The exemplary suture 6002 includes a first monofilament portion 6004 and a second braided portion 6006. The second braided portion 6006 includes a sock toe region 6010. A portion of monofilament portion 6004 is disposed through an aperture between fibers of the sock toe region 6010, and an enlarged portion 6008 of monofilament portion 6004 is disposed within a cavity 6012 defined within the braided portion 6006.

In the illustrated embodiment, the enlarged portion 6008 is configured to include a pointed end 6014 and a plurality of barbs, e.g., 6016, 6018. The pointed end 6014 facilitates insertion of the enlarged portion 6008 into and through the aperture between fibers of the sock toe region 6010 during manufacturing (or otherwise prior to application) of the suture 6002. The barbs, e.g. 6016, 6018 include their own respective points which tend to pass into fibers, threads or yarns of the braided portion 6006 and/or into other apertures between those fibers, thereby preventing this engagement of the first monofilament portion 6004 from the second braided portion 6006.

As with the generally spherical enlarged portion 5926 described above with respect to FIG. 59 the barbed enlarged portion 6008 of the present embodiment will be, in certain embodiments, integrally formed with the monofilament portion 6004, or maybe a separate element or component coupled to the balance of monofilament portion 6004 by any of the means and methods discussed above. Likewise, it may be formed of any appropriate material including, for example, nylon, stainless steel, titanium, ultrahigh molecular weight polyethylene, polytetrafluoroethylene, gold, or any other appropriate material, bioabsorbable or non-bioabsorbable according to the requirements of the particular application.

] FIG. 61 shows, in cutaway schematic view, a portion of a further exemplary suture 6102 prepared according to principles of the invention. The illustrated embodiment includes a monofilament portion 6104 and a braided portion 6106. A plurality of barbed features 6108, 6110 are disposed adjacent a proximal end 6112 of the monofilament portion 6104. Consistent with the foregoing description, the barbed features 6108, 6110 are adapted to pass through apertures between fibers, threads or yarns, e.g. 6114, 6116 of the braided portion 6106.

In certain embodiments, the barbed features include an arcuate distal end 6118 having a generally concave inner surface region 6120 well adapted to interfere with and couple to the fibers, threads or yarns of the braided portion 6106, and a generally convex outer surface region well adapted to minimize interference of the barbed portion with features of the environment including a destination tissue matrix.

In certain embodiments of the invention, the barbed features 6108, 6110 initially lack the arcuate surfaces 6120, 6122. During manufacturing of the suture 6102, physical forces are applied to deform the barbed features 6108, 6110 so that their surfaces assume the subject arcuate configuration and effectively locking the barbed features to the fibers, threads or yarns of the braided portion 6106.

FIG. 62 shows, in cutaway schematic view, a portion of a still further exemplary suture 6202 prepared according to principles of the invention. The illustrated embodiment includes a monofilament portion 6204 and a braided portion 6206. A plurality of barbed features 6208, 6210 are disposed adjacent a proximal end 6212 of the monofilament portion 6204. Consistent with the foregoing description, the barbed features 6208, 6210 are adapted to pass through apertures between fibers, threads or yarns, e.g. 6214, 6218 of the braided portion 6206. In the illustrated embodiment, the respective ends 6216, 6220 of the barbed features are deformed after passing through the apertures so as to more effectively couple the monofilament portion 6204 to the braided portion 6206.

In certain embodiments, this deformation of the ends 6214, 6218 is a plastic deformation achieved with or without thermal heating of the barbed features 6208, 6210. Thus in certain embodiments, the ends 6214, 6218 are deformed by convective thermal heating, by radiative thermal heating, by conductive thermal heating, by laser heating, by microwave heating, by frictional heating, by compressive heating, or without any heating at all. Again, in certain embodiments, the deformation process is arranged and adapted to produce on an external surface 6222, 6224 of each barb 6208, 6210 respectively, a substantially smooth surface region well adapted to avoid unwanted interference with a tissue matrix and any other environmental features during installation of the suture 6202.

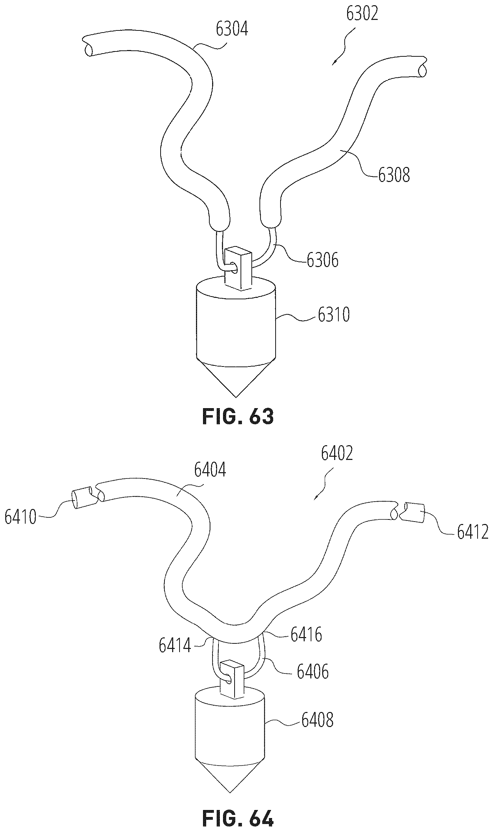

FIG. 63 shows, in schematic view, a further aspect of the invention in which a multipart suture assembly 6302 includes first 6304, second 6306 and third 6308 suture portions, as well as, for example, a suture anchor portion 6310. It should be appreciated that the suture anchor illustrated displays only general schematic features and may represent any of a wide variety of suture anchors.

The suture portion 6306 is adapted by physical configuration and selection of materials to be well adapted to provide a substantial and reliable interface to the suture anchor portion 6310. Likewise the suture portions 6304 and 6308 are similarly adapted by physical configuration and selection of materials to be well adapted to provide a substantial and reliable interface to, for example a soft tissue matrix. It will be appreciated that the suture coupling methods described above will be effectively employed in the preparation of a multipart suture assembly like that of suture assembly 6302.

FIG. 64 shows, in schematic view, a further aspect of the invention in which a multipart suture assembly 6402 includes first 6404 and second 6406 suture portions as well as a suture anchor portion 6408. As illustrated, the first suture portion 6404 is substantially continuous from a first end 6410 to a second end 6412. At intermediate points 6414, 6416 of the first suture portion 6404, respective ends of the second suture portion 6406 are coupled to the first suture portion, forming a loop including suture portion 6406. The loop of suture portion 6406 is coupled to the suture anchor portion 6408 as indicated. It will be appreciated by one of skill in the art that the coupling between the ends of the second suture portion 6406 and the first suture portion 6404 will be achieved, in certain embodiments, using barbed features or other apparatus similar to that described above.

In certain embodiments, the materials of suture portion 6404 and suture portion 6406 will be selected such that the bioabsorption time constant of the second suture portion 6406 will be substantially shorter than that of the first suture portion 6404 (and in some cases the first suture portion 6404 will be non-bioabsorbable). Consequently, after a period of time determined by the characteristics of the material of the second suture portion 6406, the suture 6402 will become detached from the suture anchor while retaining its relation to, for example, soft tissue in which the suture portion 6404 is disposed. As a result, the multipart suture assembly 6402 is well adapted to provide a temporary stabilization of soft tissue by attaching it to, for example, adjacent bone tissue matrix, during initial healing. Thereafter, when the same level of stabilization is no longer required, the soft tissue is released from the bone by bioabsorption of suture portion 6406, but remains supported by the suture portion 6404.

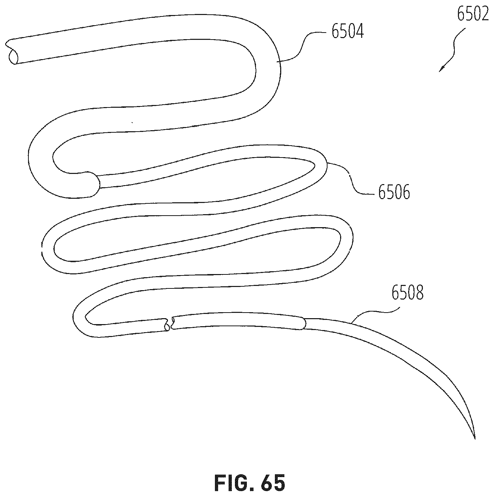

FIG. 65 shows a further aspect of the invention in which a multipart suture 6502, similar to that shown in FIG. 53, is provided as part of a kit or integrated unit including a first suture portion 6504, a second suture portion 6506 and an integrated needle 6508.

A variety of general considerations relating to any and all of the foregoing disclosure is now reviewed. It will be understood by one of skill in the art that the foregoing references to bioabsorbable and non-bioabsorbable materials are intended to cover any bioabsorbable and non-bioabsorbable materials respectively as known in the art, as well as additional material that may become known with the further development of the technology. Thus the term bioabsorbable materials intended to include, without limitation materials such as polydioxanone (PDS), polyglycolic acid (PGA), polylactic acid (PLA), copolymers including the (L/D) LA copolymers which are a mixture of D- and L-isomers of PLA, PLGA copolymers which include a combination of PLA and PGA and self-reinforcing materials including a competent structure made from partially crystalline/amorphous material of oriented fibers/fibrils and a binding matrix. In summary: SR PLLA; PLLA; P (D/L) LA 70/30; PLA/PGA (PLGA) 80/20; SR PGA; PDA: and PGA.

In certain embodiments, the multicomponent suture will include a braided ultrahigh molecular weight (UHMW) polyethylene or polyester suture coupled at one end to a corresponding end of a monofilament of absorbable or nylon suture. In other embodiments, a suture prepared according to principles of the invention will include a polyvinylidene fluoride (PVDF) material (known commercially as KynarTM), and in still other embodiments a suture prepared according to principles of the invention will include a polytetrafluoroethylene (PTFE) (known commercially as TeflonTM). In still other embodiments, a suture prepared according to principles of the invention will include a metallic material such as, for example, stainless steel, titanium and gold. It will be appreciated that a particular embodiments of the invention may include any combination of the foregoing, as well as any other material known in the art, or which becomes known in the art to be useful with respect to the present technology. In addition, perforated and expanded materials will find application with any or all of the above-described embodiments.

Beyond this, it will be understood that while several exemplary methods have been described above for the coupling of first and second suture portions, a wide variety of other coupling methods will be applicable to the present invention, and are intended to fall within the scope of the present disclosure. Thus, for example, chemical adhesives types and forms will be beneficially applied. For example, a cyanoacrylate based adhesive will be advantageously applied in certain embodiments. It should be noted that, in instances where one or the other portion of the suture is to be detached and removed (i.e. not left within the patient) it will, in certain embodiments, be desirable to use a non-biocompatible adhesive or other material in the portion that is intended to be removed.

It should be noted that the coupling between suture portions will be effected in certain embodiments with a thermally bonded joint; an ultrasonically welded joint; an integrally molded joint; preloaded (with the end of the braided suture) injected molded suture and followed by an extruded length of monofilament; a barbed coupling feature; and where the barbed coupling feature is a barbed fastener inserted longitudinally through a sock toe end of a braided suture and into the monofilament along the longitudinal axis thereof; a metallic titanium fastener; and metallic stainless steel fasteners. Moreover, as described above, where barbed coupling features are included, the barbed feature may be placed longitudinally or transversely into a receiving feature and, in certain embodiments, the barbs will be deformed to effect a permanent coupling.

In still other embodiments, a sleeve will be disposed circumferentially around a coupling that includes a barbed coupling where the sleeve provides a smooth external surface and prevents catching of the barbs on, for example, a surrounding tissue matrix. Moreover, combinations of the foregoing including, for example, a barbed coupling and a chemical adhesive, a barbed coupling and a laser welded bond, a barbed coupling and an ultrasonically welded bond, a barbed coupling at a thermally welded bond, a spherical coupling in any of the foregoing additional/supplemental bonds and a threaded coupling feature along with an ancillary or supplemental bond all will be advantageously employed in certain respective embodiments of the invention.

In certain embodiments, a suture according to principles of the invention will include a flat absorbable suture like a tape; absorbable and non-absorbable polymers; a fabric such as a polyester; and a coated suture having observable or absorbable coating. In certain embodiments, an internal reinforcing material will include a woven textile. In others a bioabsorbable material will be applied to a textile material by dipping or by coextrusion.

As noted above, certain kits and prepackaged assemblies will be made available within the scope of the invention including kits that contain a multipart suture and a separate or pre-connected needle; a multipart suture and a separate or pre-connected suture anchor; a multipart suture in any a variety of surgical instruments including forceps, scissors, an anchor insertion tool, or other surgical instruments. In still other embodiments, suture material will be provided including a large number of alternating suture portions of corresponding alternating characteristics all wound on a spool or other carrier device. Finally, in certain embodiments, a supply kit and apparatus will be provided that allow the preparation of a custom suture including a customized combination of suture portions of various length, physical properties and composition.

While the exemplary embodiments described above have been chosen primarily from the field of human surgical apparatus and techniques, one of skill in the art will appreciate that the principles of the invention are equally well applied, and that the benefits of the present invention are equally well realized in a wide variety of other areas of endeavor including, for example, veterinary medicine, horticulture, dentistry, etc. Further, while the invention has been described in detail in connection with the presently preferred embodiments, it should be readily understood that the invention is not limited to such disclosed embodiments. Rather, the invention can be modified to incorporate any number of variations, alterations, substitutions, or equivalent arrangements not heretofore described, but which are commensurate with the spirit and scope of the invention. Accordingly, the invention is not to be seen as limited by the foregoing description, but is only limited by the scope of the appended claims.

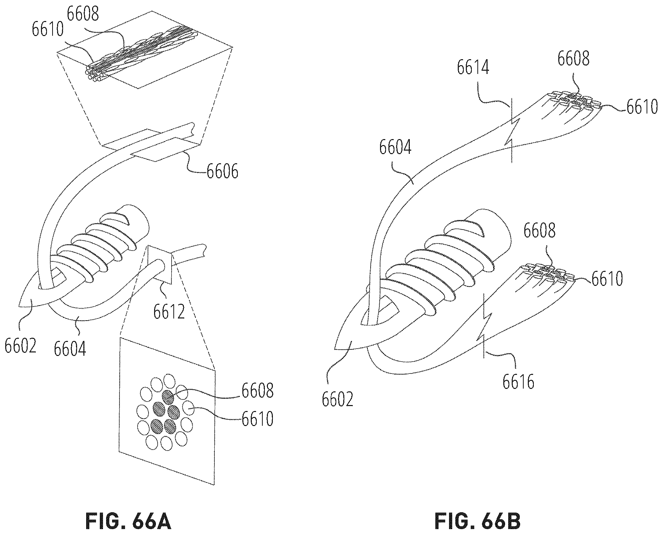

FIG. 66A - FIG. 86 describe further embodiments of biocompatible textiles, which may be incorporated as an implant as described herein. FIG. 66A is a perspective schematic diagram of suture anchor 6602 with a composite suture 6604 according to the invention. The composite suture 6604 according to the invention has, as shown in longitudinal cross section taken at 606 and lateral cross section taken at 6612, a hyaluronan-based fiber bundle 6608 and a non-resorbable fiber bundle 6610.

The suture can be off indefinite length as indicated by break-away lines 6616 and 6616 in FIG. 66B. The anchor 6602 can be biostable or bioresorbable.

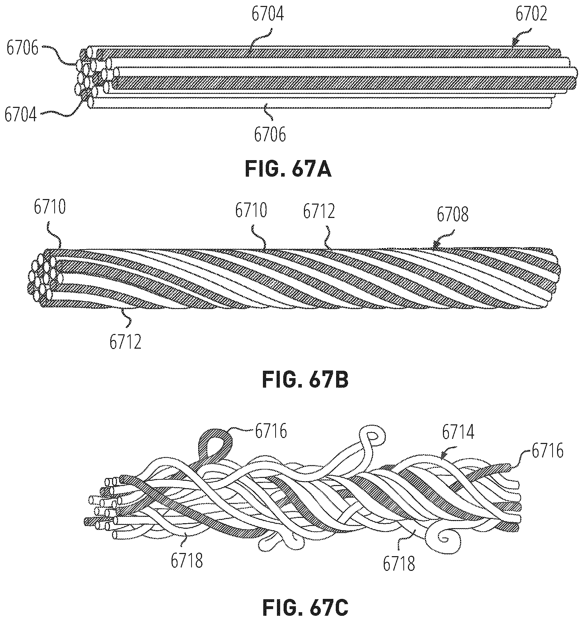

FIG. 67A is a schematic diagram of untwisted filament yarn 6702 having hyaluronan-based bioresorbable fibers 6704 and non-resorbable fibers 6706.

FIG. 67B is an illustration of twisted filament yarn 6708 having hyaluronan- based bioresorbable fibers 6710 and non-resorbable fibers 6712.

FIG. 67C is a schematic picture of a high bulk filament yarn 6714 having hyaluronan-based bioresorbable fibers 6716 and non-resorbable fibers 6718. Other constructions are possible.

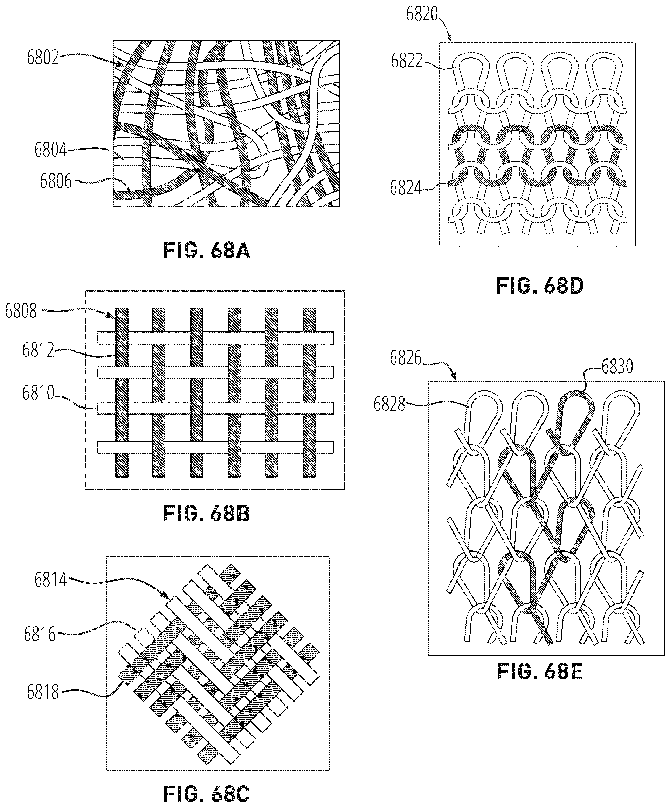

FIG. 68A is a schematic diagram illustrating a nonwoven medical textile 6802 according to the invention with bioresorbable hyaluronan-based fibers 6806 and non-resorbable fibers 6804.

FIG. 68B is a schematic diagram of a woven medical textile 6808 with hyaluronan-based bioresorbable fibers 6812 and non-resorbable fibers 6810.

FIG. 68C is a schematic diagram of a braided construction 3814 with hyaluronan-based bioresorbable fibers 6818 and non-resorbable fibers 6816.

FIG. 68D is a schematic representation of a weft-knit medical textile 6820 having hyaluronan-based bioresorbable fibers 6824 and non- resorbable fibers 6822.

FIG. 68E is a schematic diagram of a warp knit medical textile 6826 having hyaluronan-based bioresorbable fibers 6830 and non- resorbable fibers 6828.

FIG. 69A is a perspective view of a soft anchor assembly 6902 according to the invention. The soft anchor assembly 6902 has a suture 6904 which passes through a flexible tubular anchor 6906. As shown in FIG. 69A, the suture 6904 can be comprised of a plurality of hyaluronan-based bioresorbable fibers 6908 and non-resorbable fibers 6910.

As shown in FIG. 69B, the tubular anchor 6906 can be comprised of a braided construction of hyaluronan-based bioresorbable fibers 6912 and non-resorbable fibers 6914. The construct can be varied, for example by making one of the tubular anchor 6906 and the suture 6904 completely from non- resorbable fibers or from bioresorbable fibers such as the hyaluronan-based bioresorbable fibers.

FIG. 69C is an expanded view of area 69C in FIG. 69A.

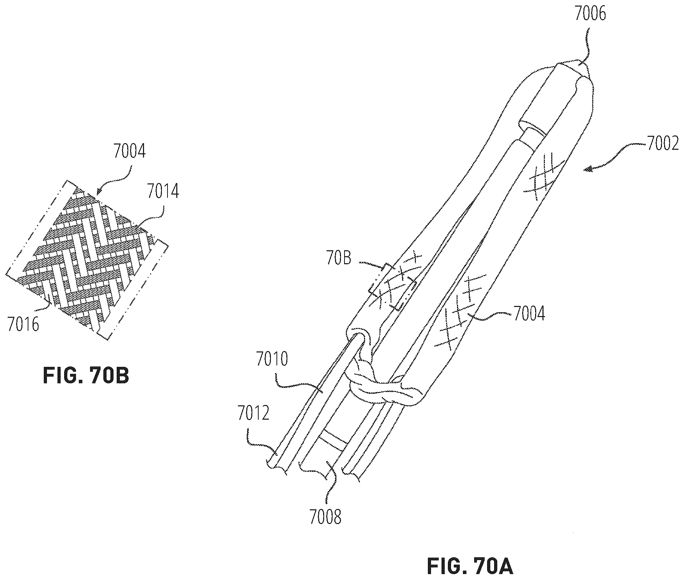

FIG. 70A - FIG. 74 depict exemplary anchors and/or anchor assemblies for composite sutures, as described herein.

Figure 70 is a perspective view of a rigid bone anchor assembly 7002 according to the invention, in a first mode of operation. The rigid bone anchor assembly 7002 includes a flexible tubular locking material 7004 communicating with an aperture in a rigid anchor 7006.

Anchor securing sutures 7010 and tissue fixation sutures 7012 can also be provided. A deployment tool 7008 can be used to deploy the anchor assembly 7002. As shown in FIG. 70A, the flexible tubular anchor 7004 can be comprised of a braided medical textile of hyaluronan-based bioresorbable fibers 7014 and non-resorbable fibers 7016.

FIG. 70B is an expanded view of area 70B in FIG. 70A.

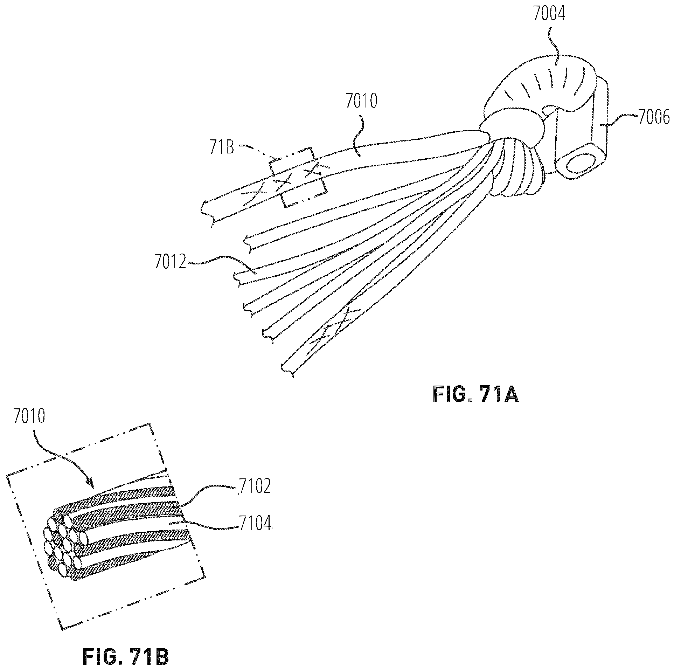

FIG. 71A is a perspective view the rigid bone anchor assembly 7002, in a deployed mode of operation. As shown in FIG. 71A, the anchor securing sutures 7010 can be comprised of a twisted assembly of hyaluronan-based bioresorbable fibers 7102 and non-resorbable fibers 7104. An assembly of hyaluronan-based bioresorbable fibers and non-resorbable fibers can also be used for the tissue fixation sutures 7012.

FIG. 71B is an expanded view of area 71B in FIG. 71A.

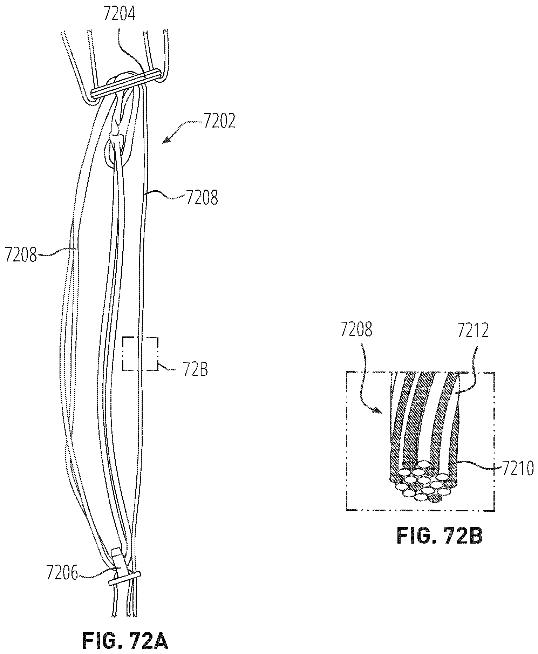

Figure 72 is a side elevation of a joint repair assembly 7202 according to the invention having surgical button 7204, fixation device 7206, and connecting suture 7208. The suture 7208 can include an assembly of hyaluronan-based bioresorbable fibers 7210 and non-resorbable fibers 7212, as shown in FIG. 72A.

FIG. 72B is an enlarged view of area 72B in FIG. 72A.

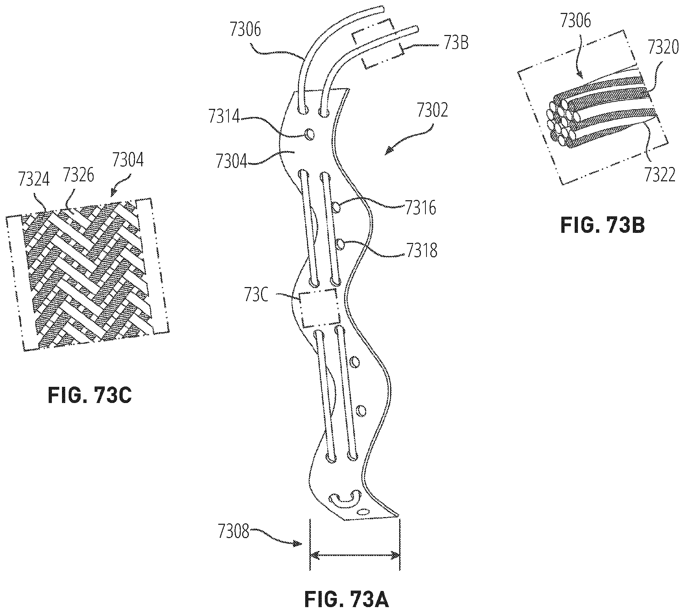

Figure 73 is a side elevation of a tissue anchor 7302 the general features of which are shown and described in US 10,918,372 "Suture Anchor" issued Feb. 16, 2021. The tissue anchor 7302 includes a webbing portion 7304 and a deployment suture 7306. In some embodiments the webbing portion 7304 will include a plurality of apertures 7314,7316, 7318 within which an insertion tool may be temporarily disposed. As shown in FIG. 73A, the deployment suture 7306 can be comprised of a plurality of hyaluronan-based bioresorbable fibers 7320 and non-resorbable fibers 7322.

The webbing portion 7304 can also be comprised of hyaluronan-based bioresorbable fibers 7324 and non-resorbable fibers 7326 as shown in FIG. 73B.

FIG. 73C is an enlarged view of area 73C in FIG. 73A.

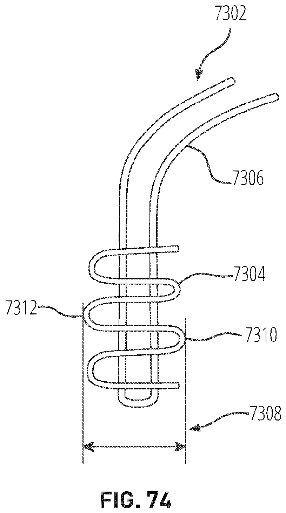

The dimension 7308 can expand as the webbing portion 7304 transitions from the un-constricted configuration to the constricted configuration shown in FIG. 74. Surface regions 7310 and 7312 will be urged radially outwardly to engage the surrounding matrix of bone, soft tissue or other media.

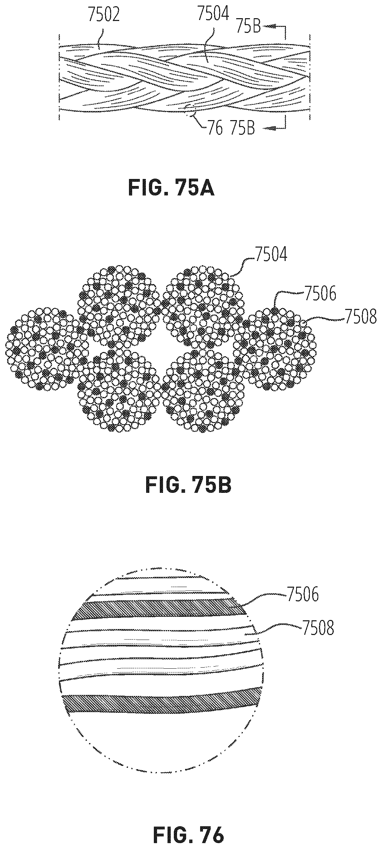

FIG. 75A and FIG. 75B illustrate a side elevation of a braided medical textile 7504 which is comprised of braided strands 7504. As shown in FIG. 75A, each of the strands 7504 is comprised of a plurality of hyaluronan-based bioresorbable fibers 7506 and non-resorbable fibers 7508.

FIG. 75B is a cross-section taken along line 75B-75B in FIG. 75A.

FIG. 76 is an enlarged view of area FIG. 76 in FIG. 75A-FIG. 75B. The hyaluronan-based bioresorbable fibers 7506 closely adjoin the non- resorbable fibers 7508 and significant inter-fiber friction is possible. Such inter- fiber friction is reduced by the hyaluronan-based fibers 7506, which as noted above are self-lubricating.

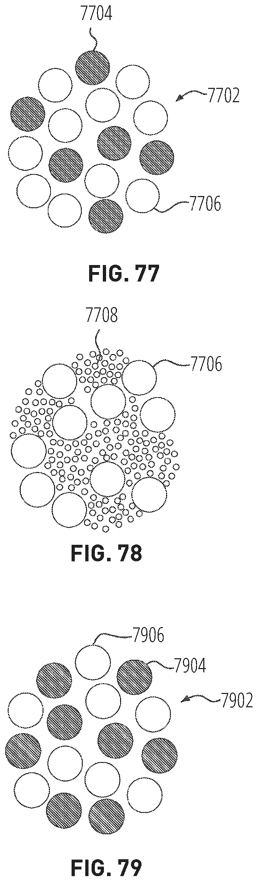

FIG. 77 is a schematic cross-section of a medical textile 7702 according to the invention. The medical textile 7702 includes a plurality of hyaluronan-based bioresorbable fibers 7704 and non-resorbable fibers 7706.

As shown in FIG. 78, in time the hyaluronan-based bioresorbable fibers 7704 will fully resorb and cells 7708 from the patient will propagate in the location of the resorbed hyaluronan-based bioresorbable fibers 7704. Also, stem cells can be positioned to propagate as the hyaluronan-based fibers resorb.

Many different configurations of hyaluronan-based bioresorbable fibers and non-resorbable fibers are possible in medical textiles according to the invention. FIG. 79 is a schematic cross-section of a medical textile 7902 with a mixed plurality of hyaluronan-based bioresorbable fibers 7904 and non- resorbable fibers 7906.

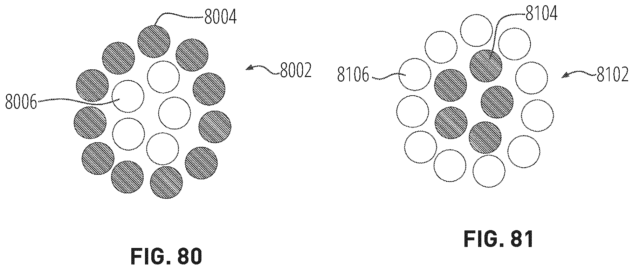

FIG. 80 is a schematic cross-section of the medical textile 8002 with a plurality of hyaluronan-based bioresorbable fibers 8004 surrounding non- resorbable fibers 8006.

FIG. 81 is a schematic cross-section of a medical textile 8102 with an array of hyaluronan-based bioresorbable fibers 8104 surrounded by non- resorbable fibers 8106.

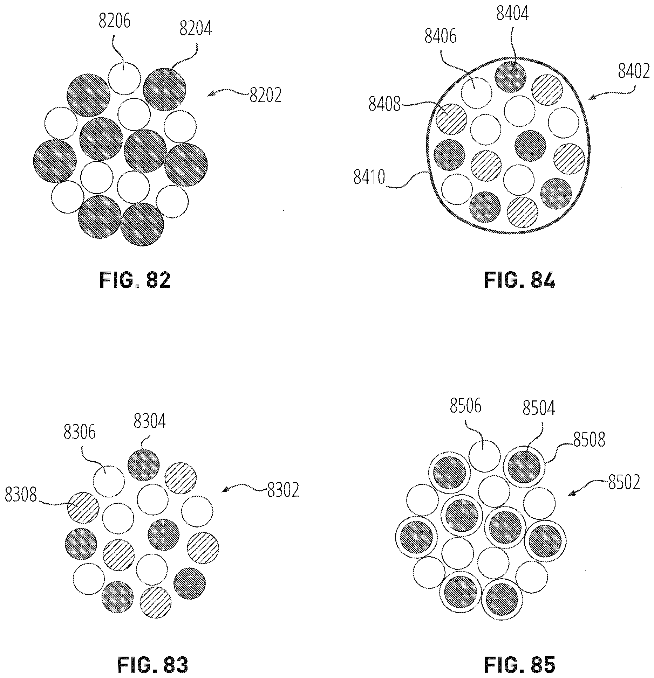

FIG. 82 is a schematic cross-section of a medical textile 8202 with a plurality of hyaluronan-based bioresorbable fibers 8204 with non-resorbable fibers 8206. The hyaluronan-based bioresorbable fibers 8204 have a larger diameter than the diameter of the bioresorbable fibers 8206. It is also possible that the hyaluronan-based bioresorbable fibers could have a smaller diameter than the diameter of the non-resorbable fibers.

FIG. 83 is a schematic cross-section of a medical textile 8302 in which hyaluronan-based bioresorbable fibers 8304 and non-resorbable fibers 8306 are provided with another fiber 8308, which can be bioresorbable or non- resorbable. Any number of different kinds of hyaluronan-based bioresorbable fibers, and other fibers both hyaluronan-based and non-resorbable, are possible.

FIG. 84 is a schematic cross-section of the medical textile 8402 which is comprised of hyaluronan-based bioresorbable fibers 8404, and non-resorbable fibers 8406, and possibly other fibers 8408 which can be hyaluronan-based bioresorbable fibers or non-resorbable fibers. The fiber bundle is surrounded by a coating of an active material 8410.

FIG. 85 is a schematic cross-section of a medical textile 8502 in which hyaluronan-based bioresorbable fibers 8504 and non-resorbable fibers 8506 are provided. The hyaluronan-based bioresorbable fibers 6704 are coated with an active material 8508. The active material 8508 could additionally or alternatively be provided on the non-resorbable fibers 8506, or on other bioresorbable fibers.

Exemplary Characteristics and Properties for Composite Medical Textiles

FIG. 87 - FIG. 104C provide exemplary characteristics and properties for composite medical textiles, which may be incorporate with any implant described herein. For example, as described herein, a method for repairing a portion of a mammalian body can include the step of providing a composite medical textile comprising a plurality of non- resorbable fibers and a plurality of bioresorbable hyaluronan-based fibers. The composite medical textile is manipulated to connect two tissue locations of a patient body, or possibly to connect a medical device to a portion of the patient body. In one embodiment, a composite suture according to the invention is provided and a suturing process is used to suture portions of the mammalian body. The medical textiles of the invention can be used in many different processes, such as connecting and repairing soft tissue and bony tissue, soft tissue augmentation, and stabilizing tissue of the joint. This method would allow for sufficient mechanical fixation and load carrying capability to allow the patient to begin nearly immediate post-operative rehabilitation activities to provide for an accelerated return to activity and minimize the potential for loss of motion or scarring following the surgical repair.

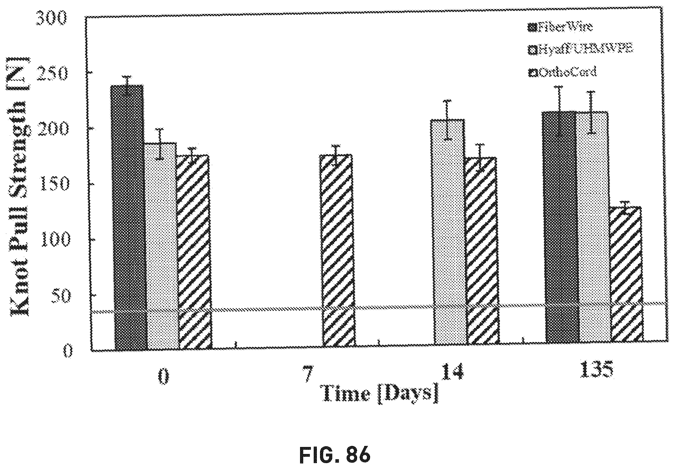

The tensile strength of composite Hyaff/UHMWPE suture and competitor products was evaluated by performing Knot-Pull testing according to USP <881> Tensile Strength. Suture samples were each cut to ~20 cm in length, then placed in 50 ml 1X Phosphate Buffered Saline (PBS) at 37 C for: 1 hour (t=0), t days, 14 days, and 135 days to simulate physiological conditions. Peak knot pull strength was evaluated on a Mark Xuniaxial tensile tester with a 5-cm gauge length tested at 100 mm/min crosshead speed. N=3 samples were tested for each group and the average $\pm$ standard deviation is shown in FIG. 86. The Orthocord (DePuySynthes/Johnson&Johnson, Raynham MA) suture decreases in strength slightly out to 135 days, while the FiberWire (Arthrex, Naples FL) and Hyaff/UHMWPE samples exhibit no statistically significant decrease in Knot Pull strength between t=0 and t=135 days as the load is primarily carried by the UHMWPE portion of the composite suture. The horizontal line indicates the USP limit on average knot-pull for a Class I non-absorbable suture (34.5 N). The sutures of the invention demonstrate very good initial (to) knot pull strength and maintain knot pull strength at t=135 days.

FIG. 87 shows a typical tensile stress/strain relation for tendon/ligament.

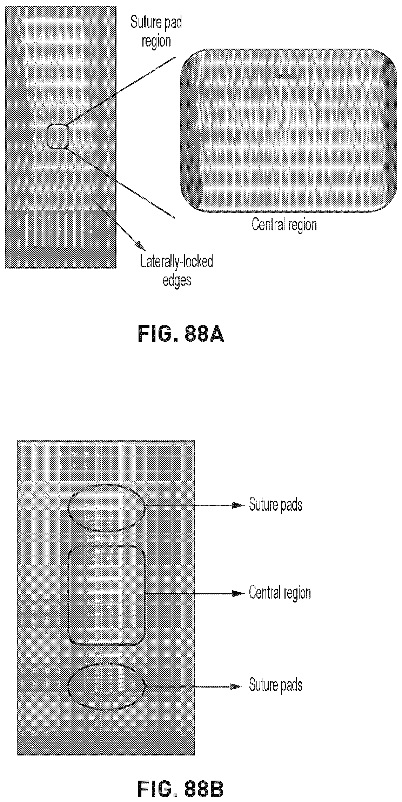

FIG. 88A shows an embodiment of the bioabsorbable textile of the invention (leno-woven bioabsorbable textile), wherein the weft yarns equally outdistanced throughout the textile length with the pore length. The bioabsorbable textiles can be prepared starting from the extrusion of the yarn using any technique familiar to those skilled in the art selected from the group consisting of melt spinning, ring spinning, air-jet spinning, open-end spinning, mule spinning, wet spinning, dry spinning, electrospinning, or extrusion. In a preferred embodiment, yarn composing the bioabsorbable textiles is extruded using a wet spinning technique.

In a preferred embodiment, yarn composing the bioabsorbable textile may be obtained starting by the dissolution of polymeric material powder in dimethyl sulfoxide (DMSO) at a concentration ranging from about 130 mg/ml to about 160 mg/ml prepared in a chemical reactor at a temperature of about 30 °C in order to obtain a viscosity ranging from about 70 to about 85 Pa*s. This dissolution is performed in two subsequent steps, namely about 75% of the DMSO calculated to obtain the target concentration and viscosity is initially used and mixed for about 60 minutes using a mixing speed ranging from about 50 to about 60 rpm. Afterwards, the remaining 25% of DMSO is added to the solution and mixed for a minimum of 3 hours at about 60 rpm and then overnight at about 20 to 30 rpm. Once the dissolution phase is completed, the polymeric solution is filtered for about 2 to about 3 hours using a nitrogen pressure of about 30 psi and a temperature of about 86 °F. After the filtration phase, the polymeric solution is then degassed for about 18 hours using a degas pressure ranging from about 20 to 30 inHg and a temperature of about 86 °F. The polymeric solution is then fed by a peristaltic pump at a speed of about 1.6 rpm into a spinneret for wet spinning composed of 150 holes each measuring 65 microns in diameter. The extruded multi-filament yarn is passed into a coagulation bath containing special denatured alcohol (SDA) comprising 80% absolute ethanol and 20% acetone and is then moved over transporting rollers into three successive rinsing baths, also containing SDA. The speeds of the single rollers are set at about 3.8 rpm. Once the multi-filament yarn has been passed through the rinsing baths, it is dried with warm air at a temperature ranging from about of 25 °C to about 45 °C and collected onto rotating cylindrical bobbin cores mounted onto a winding frame. These bobbin cores are switched every 2 to 3 hours.

In some embodiments, the multifilament yarn comprising the bioabsorbable textile may comprise a plurality of polymeric materials selected from the group consisting in polyethers, polyesters, polyols, poloxamers, proteins, polysaccharides (e.g. HA and its derivatives) or a combination thereof. In some embodiments, the extruded yarn will be lubricated by using a spin finish to allow downstream processability. In a preferred embodiment, the addition of a spin finish oil is applied at the end of the extrusion phase and, preferably, before any other manufacturing steps. Spin finish oils can have an origin selected from the group consisting of mineral oils, polymeric oils, or a combination thereof. In some embodiments, the spin finish content ranges between 0.1 mg per meter of yarn and 20 mg per meter of yarn. In a preferred embodiment, the spin finish content ranges between 1 mg per meter of yarn and 5 mg per meter of yarn.

The extruded yarn comprising the bioabsorbable textiles can be composed of mono- or multi-filaments. In some embodiments, yarns are composed of multi-filaments ranging from about 50 to about 10.000. In preferred embodiments, the number of filaments composing a single multi-filament yarn range from about 100 to about 150. In other preferred embodiments, the number of filaments composing a single multi-filament yarn range from about 5.000 to about 7.000.

In some embodiments, each filament composing a single multi-filament yarn has a mean diameter ranging from about 1 $\mu$m to about 100 $\mu$m. In preferred embodiments, the mean diameter of a single filament composing a single multi-filament yarn range from about 10 $\mu$m to about 40 $\mu$m. In a preferred embodiment, these filaments comprising the multi-filament yarn are separated to each other. In some embodiments, the extruded yarn comprising the bioabsorbable textile can display a diameter ranging from about 100 μm to about 3000 μm. In a preferred embodiment, the extruded yarn comprising the bioabsorbable textile can display a diameter ranging from about 200 to about 800 μm. In some embodiments, the extruded yarn comprising the bioabsorbable textiles can display a wet linear mass density ranging from about 10 tex to about 1.500 tex (where tex is a direct measure of linear density - *i.e.,* number of grams per one kilometer of yarn). In a preferred embodiment, the extruded yarn displays a linear mass density ranging from about 30 tex to about 100 tex. In other preferred embodiments, the extruded yarn displays a linear mass density ranging from about 1.000 tex to about 1.300 tex.

In some embodiments, the extruded yarn comprising the bioabsorbable textiles can display a breaking strength ranging from about 1 N to about 100 N and an extension at break from about 1% to about 30%. In a preferred embodiment, extruded yarn displays a breaking strength ranging from about 4 N to about 12 N and an extension at break from about 1% to about 12%.

In a preferred embodiment the bioabsorbable textile according to the invention comprises the at least polymeric yarn which shows: linear mass density from about 30 tex to about 100 tex; a breaking strength in the range from about 1 N to about 100 N, preferably from about 4 N to about 12 N, and an extension at break from about 1% to about 30%, preferably from about 1% to about 12%.

In some embodiments, the extruded yarn comprising the bioabsorbable textiles can be twisted. In some embodiments, a yarn is produced by co-twisting together from one to about sixteen yarns in the opposite direction to make a thicker yarn. In a preferred embodiment, a yarn is produced by twisting one single yarn.

In some embodiments, the twisted yarn can have a number of twists per meter ranging from about 10 to about 1.000. In a preferred embodiment, the twisted yarn can have a number of twists per meter ranging from about 50 to about 500.

In some embodiments, the twisted yarn can have S twists (up-left direction) and in some other embodiments, the twisted yarn can have Z twists (up-right direction).

The bioabsorbable textiles can be prepared by interconnecting the extruded, and preferentially twisted yarn, using any technique familiar to those skilled in the art selected from the group consisting of weaving, leno weaving, knitting, spacer knitting fabric, braiding, crocheting, embroidery, ropemaking, and sewing.

In a preferred embodiment, the bioabsorbable textiles are prepared by weaving or leno weaving or by weft knitting or warp knitting. The bioabsorbable textile could be a weave where the ends of transversal (weft) direction yarns are structurally locked while the ends of longitudinal (warp) direction yarns remain open or a knitted structure.

The bioabsorbable textile could be a leno-weave in which a multiple amount of warp yarns is twisted around a single amount the weft yarns. In preferred embodiments, the leno-weave is composed by two to six warp yarns twisted around one to about two weft yarns.

In some embodiments, the bioabsorbable textiles are isotropic being woven with the same amount of yarns spanning along the longitudinal (warp) direction and the lateral (weft) direction. In a preferred embodiment, the bioabsorbable textiles are anisotropic with the amount of yarns in the warp direction being higher than the amount of yarns in the weft direction, where the warp direction corresponds to the direction of the tensile load in the target joint tissue. In a more preferred embodiment, the number of warp yarns per textile width ranges from about 10 yarns/cm to about 50 yarns/cm and the number of weft yarns per textile length ranges from about 4 yarns/cm to about 18 yarns/cm.

In preferred embodiments, the warp yarns are equally outdistanced throughout the textile width with the pore width (i.e. the distance between two adjacent warp yarns) ranging from about 1 to about 100 μm.

FIG. 88A shows an embodiment of the bioabsorbable textile of the invention (leno-woven bioabsorbable textile), wherein the weft yarns equally outdistanced throughout the textile length with the pore length. In some preferred embodiments, the weft yarns are equally outdistanced throughout the textile length with the pore length (i.e. the distance between two adjacent weft yarns) ranging from about 100 to about 2500 μm (FIG. 88A).

FIG. 88B shows another embodiment of the bioabsorbable textile of the invention (leno-woven bioabsorbable textile), wherein the weft yarns are equally distanced in the central region of the bioabsorbable textile. In some other preferred embodiments, the weft yarns are equally distanced in the central region of the bioabsorbable textile, whereas the central region represents about 50 percent to about 80 percent with respect to the total textile length (FIG. 88B,Central region). In these preferred embodiments, the weft yarns are equally distanced within the central region of the textile with a pore length (i.e. the distance between two adjacent weft yarns within the central region of the textile) ranging from about 1300 μm to about 2300 μm. In these preferred embodiments, the number of weft yarns per textile length ranges from about 4 yarns/cm to about 10 yarns/cm.

In these preferred embodiments, the weft yarns are more densely packed at the external portions (top and bottom ends with respect to the central region) of the bioabsorbable textile in comparison with the central region of the textile, whereas the external portions represent about 25 percent to about 10 percent each with respect to the total textile length (FIG. 88B, Suture pads). In these preferred embodiments, the weft yarns are equally distanced within the external portions of the textile with a pore length (i.e. the distance between two adjacent weft yarns within the external portions of the textile) ranging from about 200 μm to about 700 μm. In these preferred embodiments, the number of weft yarns per textile length ranges from about 6 yarns/cm to about 18 yarns/cm.

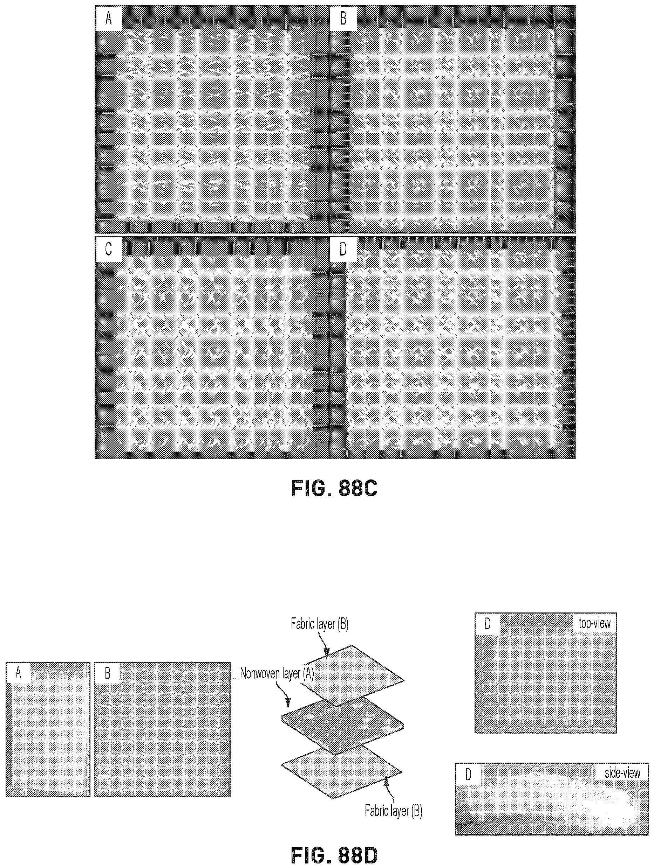

As shown in FIG. 88A, FIG. 88B, FIG. 88C, and FIG. 88D, the denser, external regions are also referred as suture pads since their scope within the bioabsorbable textiles is to provide a reinforced area for suturing the textile to the target joint tissue to reduce the likelihood of sutures tearing the textile when strain is applied. The open ends of the warp yarns at the suture pads can be locked using any edge bonding technique familiar to those skilled in the art selected from the group consisting of solvent welding, embroidery, sewing, radiofrequency welding, ultrasonic welding, laser welding, and hot temperature welding. In some preferred embodiments, the top and bottom edges of the bioabsorbable textile are solvent-welded by laying down a thin layer of polymeric solution at the edges (FIG. 88B). In some other preferred embodiments, the top and bottom edges of the bioabsorbable textile are mechanically sealed via embroidery or sewing, where the initial reinforcement created by inserting straight stitches in a plurality of sets of a plurality of rows each (FIG. 88A).In preferred embodiments the reinforcement stitches are inserted as 2 sets of 3 rows each. In these preferred embodiments, reinforcement stitches are grouped by the addition of an overstitched stitch row. In some preferred embodiments, the overstitched stitch row displays a zig- zag pattern. In some embodiments, the reinforcement stitches and the overstitched stitch row comprising the suture pads of the bioabsorbable textile may comprise a plurality of polymeric materials selected from the group consisting in polyethers, polyesters, polyols, poloxamers, proteins, polysaccharides (e.g. HA and its derivatives) or a combination thereof. In a preferred embodiment, the reinforcement stitches and the overstitched stitch row comprising the suture pads of the bioabsorbable textile comprises a benzyl ester of hyaluronic acid modified with an esterification degree ranging from about 80% to about 100%. In some embodiments, the suture pads are folded on themselves and this step is followed by edge bonding. In preferred embodiments, the suture pads are suture-closed as a single, unfolded layer.

FIG. 88C contains four schematic representations of embodiments of the textile of the of warp-knitted bioabsorbable textile. The bioabsorbable textile could be a knitted structure. 4 different design variants (FIG. 88C) were conceived by varying knit structure and two input parameters, namely the mesh areal density and the inter-yarn porosity.

FIG. 88D is a schematic representation of preferred embodiments of the bioabsorbable textiles with single or a plurality of layers superimposed on each other, where each layer comprises textiles as described in FIG. 88A and in FIG. 88B or nonwoven pad. In some embodiments, the bioabsorbable textiles may comprise single or a plurality of layers superimposed on each other, where each layer comprises textiles manufactured as described above or nonwoven pad (FIG. 88D).

In some embodiments, the bioabsorbable textiles may comprise single or a plurality of layers superimposed on each other, where each layer comprises textiles manufactured as described above or nonwoven pad (FIG. 88D).

In some embodiments, the bioabsorbable textiles may show a length ranging from about 5 mm to about 150 mm, a width ranging from about 5 to about 150 mm, and a thickness ranging from about 0.1 mm to about 5 mm. In a preferred embodiment, the bioabsorbable textiles may show a length ranging from about 15 mm to about 105 mm, a width ranging from about 5 to about 55 mm, and a thickness ranging from about 0.3 mm to about 3 mm.

Noteworthy, the above-mentioned values refer to the dry state of the bioabsorbable textiles. When hydrated in physiological conditions (i.e. pH of about 7.4 and temperature of about 37 °C), in some embodiments the bioabsorbable textiles, show a volumetric as well as a thickness swelling ratio ranging from about 0.1% to about 20%. In a preferred embodiment, the bioabsorbable textiles, may have a volumetric as well as thickness swelling ratio ranging from about 0.1% to about 5%.

When hydrated in physiological conditions (i.e. pH of about 7.4 and temperature of about 37 °C), in some embodiments the bioabsorbable textiles, show a weight swelling ratio ranging from about 0.1% to about 150%. In a preferred embodiment, the bioabsorbable textiles, may have a volumetric as well as thickness swelling ratio ranging from about 35% to about 80%.

Related to the above-mentioned physical sizes in dry conditions, in a preferred embodiment of the bioabsorbable textiles the areal density ranges from about 100 g/m$^2$ to about 1000 g/m$^2$. In preferred embodiments, the bioabsorbable textiles showed an accessible porosity ranging from about 20% to about 85%.

The bioabsorbable textiles can be prepared using yarns comprising a raw material selected from the group consisting in polyethers, polyesters (e.g. poly lactic acid, poly caprolactone, etc.), polyols (e.g., polyvinyl alcohol, polyethylene glycol, polypropylene glycol), poloxamers, proteins or natural polymers (e.g. chitosan, collagen, gelatin, alginate, etc.), polysaccharides (e.g. hyaluronic acid and its derivatives), or a combination thereof. In some embodiments, the bioabsorbable textile can be prepared using multiple yarns comprising different raw materials selected from the group consisting in polyethers, polyesters, polyols, poloxamers, proteins, polysaccharides (e.g. hyaluronic acid and its derivatives) or a combination thereof. In some other embodiments, the yarn comprising the bioabsorbable textile can consist of multi-filaments comprising one or multiple raw materials. In a preferred embodiment, the bioabsorbable textile can be prepared using yarns comprising only one raw material for each filament comprising the multi-filament yarn.

In preferred embodiments, the bioabsorbable textiles are prepared using multi-filament yarns comprising hyaluronic acid derivatives as raw material selected from the group consisting of hyaluronic acid esters wherein a part or all of the carboxy functions are esterified with alcohols of the aliphatic, aromatic, arylaliphatic, cycloaliphatic, heterocyclic series, especially with benzyl alcohol; cross-linked esters of hyaluronic acid wherein part or all of the carboxy groups are esterified with the alcoholic functions of the same polysaccharide chain or of other chains; cross-linked compounds of hyaluronic acid wherein part or all of the carboxy groups are esterified with polyalcohols of the aliphatic, aromatic, arylaliphatic, cycloaliphatic, heterocyclic series, generating cross-linking by means of spacer chains; hemiesters of succinic acid or heavy metal salts of the hemiester of succinic acid with hyaluronic acid or with partial or total esters of hyaluronic acid; 0-sulphated derivatives or N-sulphated derivatives; quaternary ammonium salts, such as salts with tetrabutylammonium and phenyltrimethylammonium, of hyaluronic acid or a hyaluronic acid derivative selected from the group consisting of N-sulphated hyaluronic acid, 0-sulphated hyaluronic acid, the hemiesters of succinic acid with hyaluronic acid and optionally partially salified with heavy metals. In more preferred embodiments, the bioabsorbable textiles are prepared using multi-filament yarns comprising benzyl esters of hyaluronic acid. In the most preferred embodiment, the bioabsorbable textiles are prepared using multi-filament yarns comprising a benzyl ester of hyaluronic acid modified with an esterification degree ranging from about 80% to about 100%.

The inventors found that, in order to favor and accelerate osteointegration, in some embodiments the bioabsorbable textile can be optionally coated by using any technique familiar to those skilled in the art selected from the group consisting of vapor deposition, chemical and electrochemical techniques, spraying, roll-to-roll coating, physical coating, or a combination thereof. In preferred embodiments, the bioabsorbable textile is coated by plasma spraying. In some embodiments, the coating material is an osteo-inductive and/or osteo-conductive material selected from the group consisting of hydroxyapatite, calcium phosphates, bone cements, demineralized bone matrix, titanium, magnesium, strontium, bioglasses, other resorbable glasses, or a combination thereof.

In another aspect the invention hence relates to a bioabsorbable textile coated with an osteo-inductive and/or osteo-conductive material selected from the group consisting of hydroxyapatite, calcium phosphates, bone cements, demineralized bone matrix, titanium, magnesium, strontium, bioglasses, other resorbable glasses, and a combination thereof.

In some embodiments, the coating is uniformly applied onto the whole textile surface. In preferred embodiments, the coating is applied only at one side of the textile that will be the side in direct contact with the bone or with the tendon-to-bone interface. In some embodiments, the coating thickness may range between 1 nm to 100 $\mu$m. Always in order to favor and accelerate osteointegration, in some other embodiments the bioabsorbable textile may be applied in combination with an injectable bone cement formulation. In preferred embodiments, the injectable bone formulation is applied onto either the tendon-to-bone interface or onto the textile implanted at the interface or in-between the interface and the textile. In some embodiments, the injectable bone formulation is applied before the textile is implanted; in some other embodiments, the injectable bone formulation is applied after the textile is implanted. In preferred embodiments, the injectable bone formulation comprises a solid phase comprising a material selected from the calcium phosphate cement family known to those skilled in the art. In preferred embodiments, the injectable bone formulation comprises a liquid phase comprising hyaluronic acid or its derivatives.

The Applicant have unexpectedly and surprisingly found that, in order to favor and accelerate tissue regeneration and healing, in some embodiments before implantation the bioabsorbable textile can optionally be mixed directly in the operating room with a biological component familiar to those skilled in the art selected from the group consisting of bone marrow aspirate, bone marrow aspirate concentrate, mesenchymal stem cells, bone marrow stem cells, adipose-derived stem cells, amniotic cells, micronized amniotic membrane, partially- or fully-digested tissue biopsies, aspirated adipose tissue, platelet rich plasma, autologous conditioned plasma, growth factors, or a combination thereof. In another aspect the invention hence relates to a bioabsorbable textile added with a biological component selected from the group consisting of bone marrow aspirate, bone marrow aspirate concentrate, mesenchymal stem cells, bone marrow stem cells, adipose-derived stem cells, amniotic cells, micronized amniotic membrane, partially- or fully-digested tissue biopsies, aspirated adipose tissue, platelet rich plasma, autologous conditioned plasma, growth factors, and a combination thereof. The Applicant also found that the combination of a given textile design with a given raw material in some embodiments, unexpectedly and surprisingly may result in tensile properties showing a viscoelastoplastic behavior matching the target human joint tissue. In preferred embodiments, the chosen textile design is a leno-weave or a knitted structure and the chosen raw material is a benzyl ester of hyaluronic acid. In the same preferred embodiments, the toe region may range between about 0% and about 4% of the tensile strain and an elastic region ranging from about 0% to about 8% of the tensile strain, unexpectedly and surprisingly matching the target human tendon tissue properties.

In some embodiments, the combination of a given textile design with a given raw material can show a maximum load ranging from 40 to 800 N, in preferred embodiments the bioabsorbable textiles can show a maximum load ranging from 50 to 250 N. In some embodiments, the bioabsorbable textiles can show a Young's modulus ranging from 0.1 to 800 MPa, in preferred embodiments the bioabsorbable textiles can show a Young's modulus ranging from 1 to 300 MPa. In some embodiments, the bioabsorbable textiles can show a failure stress ranging from 3 to 20 MPa, in preferred embodiments the bioabsorbable textiles can show a failure stress ranging from 4 to 15 MPa. In some embodiments, the bioabsorbable textiles can show a failure strain ranging from 10 to 70 %, in preferred embodiments the bioabsorbable textiles can show a failure strain ranging from 15 to 45 %. In some embodiments, the bioabsorbable textiles can show a yield stress ranging from 2 to 50 MPa, in preferred embodiments the bioabsorbable textiles can show a yield stress ranging from 2.5 to 15 MPa. In some embodiments, the bioabsorbable textiles can show a yield strain ranging from 0.5 to 8 %, in preferred embodiments the bioabsorbable textiles can show a yield strain ranging from 1.5 to 6.5 %. In some embodiments, the bioabsorbable textiles can show a yield load ranging from 25 to 300 N, in preferred embodiments the bioabsorbable textiles can show a yield load ranging from 30 to 150 N. In some embodiments, the bioabsorbable textiles can show a stiffness ranging from 10000 to 200000 N/m, in preferred embodiments the bioabsorbable textiles can show a stiffness ranging from 12000 to 80000 N/m. In some embodiments, the bioabsorbable textiles can show an apparent failure strain ranging from 30 to 95 %, in preferred embodiments the bioabsorbable textiles can show an apparent failure strain ranging from 40 to 85 %. In some embodiments, the bioabsorbable textiles can show an apparent yield strain ranging from 3 to 12 %, in preferred embodiments the bioabsorbable textiles can show an apparent yield strain ranging from 5 to 11 %. In some embodiments, the bioabsorbable textiles can show an apparent Young's modulus ranging from 1 to 500 MPa, in preferred embodiments the bioabsorbable textiles can show an apparent Young's modulus ranging from 1 to 120 MPa. In some embodiments, the bioabsorbable textiles can show a load at 5 mm ranging from 30 to 160 N, in preferred embodiments the bioabsorbable textiles can show a load at 5 mm ranging from 35 to 140 N. In some embodiments, the bioabsorbable textiles can show an elongation at 50 N ranging from 0.5 to 15 mm, in preferred embodiments the bioabsorbable textiles can show an elongation at 50 N ranging from 1 to 12 mm.

Taken all these data together, the leno-weave or knitted configurations combined together with the use of a benzyl ester of hyaluronic acid, make the bioabsorbable textile suitable for mechanical augmentation of the target joint.

In some embodiments, the combination of a given textile design with a given raw material, for example for lenowave or knitted designs made of a derivative of hyaluronic acid, resulted in mechanically- robust bioabsorbable textiles able to withstand at least up to 2500 loading cycles at physiological stress and frequency values. In preferred embodiments, the cyclic elongation of the bioabsorbable textiles may range from about 2 mm at the first cycle and about 12 mm at 2500^. In the same preferred embodiments the bioabsorbable textiles show load to failure values after 2500 cycles ranging between 50 N to 250 N.

The combination of a given textile design with a given raw material in some embodiments, for example for lenowave or knitted designs made of a derivative of hyaluronic acid, results in mechanically-robust bioabsorbable textiles able to show a suture pull-out strength ranging from about 8 N to about 800 N. In preferred embodiments, the suture pull-out strength of the bioabsorbable textiles ranges from about 20 N to about 100 N.

The combination of a given textile design with a given raw material in some embodiments, for example for lenowave or knitted designs made of a derivative of hyaluronic acid, results in bioabsorbable textiles showing the following analytical features: loss on drying ranging from about 1% to about 20%; a percent of esterification of the carboxylic group of hyaluronic acid ranging from about 60% to about 100%; and a percent of a free esterifying group detached from the hyaluronic acid derivative ranging from about 0% to about 0.4%. In preferred embodiments, the bioabsorbable textiles show a loss on drying ranging from about 2% to about 15%; a percent of esterification of the carboxylic group of hyaluronic acid ranging from about 85.0% to about 99.9%; and a percent of a free esterifying group detached from the hyaluronic acid derivative ranging from about 0.01% to about 0.2%.

The combination of a given textile design with a given raw material in preferred embodiments, for example for lenowave or knitted designs made of a derivative of hyaluronic acid, may result in bioabsorbable textiles showing excellent biological properties, such as a cell viability ranging from about 70% to about 100% and an *in vitro* collagen induction ranging from about 50 to about 150 ug/mg protein whereas the solid, mature collagen portion ranges from about 5 to about 50 ug/mg protein. The bioabsorbable textiles can be prepared as multi-layered or gradient structures incorporating differential mechanical and/or morphological properties in one or more directions.

In some embodiments, a bioabsorbable textile as described herein can be bonded (physically and/or chemically) to one or more additional or same bioabsorbable textiles.

In another aspect the invention relates to a multilayer structure for joint function restoration comprising at least one bioabsorbable textile of the invention.

The multi-layered structure can be tuned to incorporate, for example, a mechanically robust layer in one section to support a morphologically favorable (e.g., for cellular infiltration and proliferation) layer in another section. The multi-layered structure could further be used to incorporate a therapeutic agent in one section (*e.g.,* the lesion side), while omitting the therapeutic agent in another section. Bonding of one bioabsorbable textile can be achieved by numerous methods including mechanical (suturing, tacking, hook and loop, etc.), thermal (conductive heat, convective heat, radiative heat, RF welding, US welding, etc.), chemical (solvent, adhesive, etc.), or a combination of the above methods. In preferred embodiments, a two-layered composite textile is prepared by using solvent (e.g, DMSO, HFIP, DMF, etc.), heat (100-250 °C), and pressure (1-100 MPa) to bond a warp knitted bioabsorbable textile (*e.g,* HD1) to a nonwoven pad bioabsorbable textile (e.g. non-woven fabric based on HA benzyl ester ). The edges of the multi-layered structure can be sealed by means of cauterization (e.g, electrocautery, chemical cautery, hot knife, laser cutting, US welding, RF welding, induction, etc.). In preferred embodiments, the multi-layered composite textile structure is cut and cauterized with a $CO_2$

laser. The multi-layered structure can also be fabricated with one or more through-holes to facilitate passing of medical instruments or devices through the structure *(e.g,* needles, sutures, suture tapes, anchors, etc.). In some embodiments, the hole or holes can be manufactured mechanically *(e.g,* blade or punch), chemically *(e.g.,* solvent), or thermally *(e.g.,* hot die, hot blade, laser, US welding, RF welding, etc.). In some embodiments the hole or holes are approximately 0.1-10 mm in diameter. In preferred embodiments, the hole or holes are 0.5-2 mm in diameter. In some embodiments the holes comprise a non-circular profile *(e.g,* oblong, oval, rectangular, trapezoidal, etc.).

In another aspect the invention relates to the bioabsorbable textile for use in repairing the joint function. Specifically, the invention relates to the bioabsorbable textile of the invention for use in restoring joint function affected by partial thickness tears, small to medium full- thickness tears, large to massive full-thickness tears, acute and chronic/degenerative tears.

In another aspect the invention relates to the bioabsorbable textile of the invention for use in surgery in combination with fixation tools during open, mini-open or arthroscopic repair/augmentation procedures of joint tissue tears.

The textile can hence be used in a method of treatment for restoring the joint function.

The method of the invention comprises the implantation of the bioabsorbable textile in combination with fastening means (such as sutures, and/or pins, and/or tags, and/or suture anchors, and/or bone anchors, and/or staples) during surgical repair/augmentation procedures of joint tissue tears. Specifically, in some embodiments the textile may be delivered *in situ* after or at the same time the soft tissue tear has been repaired using one or a combination of the fixation tools above mentioned.

The method of the invention refers to open, mini-open or arthroscopic procedures. In some embodiments, the bioabsorbable textile may comprise specific features that improve its surgical deliverability in open, mini-open or arthroscopic settings. It is in the scope of this invention the treatment of partial thickness tears, small to medium full-thickness joint tissue tears; large to massive full-thickness tears acute and chronic/degenerative tears; and interstitial delamination of the fibers comprising the extracellular matrix of the joint tissues.

In some embodiments, the arthroscopic method to treat joint tears in order to restore their function starts by creating an access, which in some preferred embodiments may be posterior, between the glenoid and the humerus that will then be used to insert an arthroscopic guide having a diameter ranging from 5 to 10 mm. From this access, in preferred embodiments an arthroscope is inserted in order to approach the sub-acromial space. In some embodiments, the arthroscopic method may continue by creating one or a plurality of other accesses. In preferred embodiments, these accesses are created in lateral and antero-lateral positions. In some embodiments, the arthroscopic method may continue with the insertion in the joint of a plurality of shuttle wires implantation that are positioned considering the size of the bioabsorbable textile with the aid of a surgical ruler. In preferred embodiments, four to six shuttle wires are used, one for each corner of the bioabsorbable textile and two optional wires positioned at the middle of both edges. In other preferred embodiments, the shuttle wires are delivered and installed only at the musculotendinous junction side while a plurality of fasteners are used at the tendon-to-bone interface selected from a group consisting in suture anchors, staples, screws, and tacks. In preferred embodiments, the bone fasteners are selected from the group consisting in screw-in, interference-fit, tack-in, knotted or knotless fixation devices whose main body is made of a material selected from a group including but not limited to absorbable materials (PLGA, PLA, PCL, etc.) PEEK, titanium, stainless steel, or a combination thereof.

In some embodiments, the arthroscopic method to treat joint tears may continue by marking the bioabsorbable textile to help the end-user to visualize the upper and lower side. In some other embodiments, the bioabsorbable textile may be already pre-marked. In some embodiments, the arthroscopic method to treat joint tears may continue by stitching suture threads at the edges of the bioabsorbable textile by means of a plurality of knots. In preferred embodiments, suture threads with a size of at least zero or higher are used. In preferred embodiments, knots are made in the warp direction. In other preferred embodiments, the bioabsorbable textile may be already pre-sutured with one or with a plurality of suture threads placed at both edges (warp direction) to minimize surgeon's effort and time in the operating room. In some embodiments, suture tapes are used. In some embodiments the arthroscopic method may continue by inserting the textile into the joint through either an incision or by using an arthroscopic cannula from one of the pre-made accesses. In some embodiments, the arthroscopic cannula may have an internal diameter ranging from 6 to 15 mm.

In preferred embodiments, the arthroscopic method to treat joint tears may continue by fixing the bioabsorbable textile to the musculotendinous junction of the target joint, optionally taking advantage of the pre-implanted shuttle wires. In other embodiments, the bioabsorbable textile is fixed at the enthesis side first and then attached to the musculotendinous junction of the target joint. Once the textile is fixed at one side, the arthroscopic method to treat joint tears may end by fixing the other side of the textile to the tendon or to the tendon-to-bone interface of the target joint, optionally by leveraging either the pre-implanted shuttle wires or the pre-implanted bone fasteners. In more preferred embodiments, the bioabsorbable textile delivered *in situ* with a surgical inserter can be fixed at both ends by using resorbable or non-resorbable fasteners.

In some embodiments, the bone at the interface with the tendon may be optionally decorticated prior to the implantation of the textile. In some embodiments, the bone at the interface may be optionally treated with injectable bone cements. In some embodiments, the sutures fixing the textile to the tendon or to the tendon-to-bone interface may be passed through one or a plurality of pre-drilled bone tunnels. In some other embodiments, the open method to treat joint tears in order to restore their function may start by creating an access by means of a knife with a width ranging from about 1 to about 10 cm. In some embodiments, the open method may continue with the insertion in the joint of a plurality of fasteners that are positioned considering the size of the bioabsorbable textile with the aid of a surgical ruler. In preferred embodiments, four to six fasteners are used, one for each corner of the bioabsorbable textile and two optional wires positioned at the middle of both edges. In other preferred embodiments, resorbable fasteners are positioned and installed only at the musculotendinous junction side while a plurality of non-resorbable fasteners are used at the tendon-to-bone interface selected from a group consisting in suture anchors, staples, screws, and tacks. In preferred embodiments, the bone fasteners are selected from the group consisting in screw-in, interference-fit, tack-in, knotted or knotless fixation devices whose main body is made of a material selected from a group including but not limited to PEEK, titanium, stainless steel, or a combination thereof.

In some embodiments, the open method may continue by stitching suture threads at the edges of the bioabsorbable textile by means of a plurality of knots. In preferred embodiments, suture threads with a size of at least size 0 or higher are used. In preferred embodiments, knots are made in the warp direction. In more preferred embodiments, the bioabsorbable textile may be already pre-sutured with one or with a plurality of suture threads at the two edges of one border (warp direction) to minimize surgeon's effort and time in the operating room. Once suture threads have been stitched onto the bioabsorbable textile, in some embodiments the open method may continue by inserting the textile into the joint through the wide access. In preferred embodiments, the textile is inserted with the pre-stitched sutures facing upwards. In preferred embodiments, a surgical inserter may be used to deploy the bioabsorbable textile in situ.

In preferred embodiments, the open method to treat joint tears may continue by fixing the bioabsorbable textile to the musculotendinous junction of the target joint taking advantage of the pre-implanted shuttle wires. In other embodiments, the bioabsorbable textile is fixed at the enthesis side first and then attached to the musculotendinous junction of the target joint. Once the textile is fixed at one side, the open method to treat joint tears may end by fixing the other side of the textile to the tendon or to the tendon-to-bone interface of the target joint by leveraging either the pre-implanted shuttle wires or the pre-implanted bone fasteners. In more preferred embodiments, the bioabsorbable textile delivered in situ with a surgical inserter can be fixed at both ends by using resorbable or non- resorbable fasteners.

In some embodiments, the bone at the interface with the tendon may be optionally decorticated prior to the implantation of the textile. In some embodiments, the sutures fixing the textile to the tendon or to the tendon-to-bone interface may be passed through one or a plurality of pre-drilled bone tunnels.

EXPERIMENTAL PART

HYALONECT® is a resorbable knitted fabric surgical mesh made of benzyl ester of hyaluronic acid. It is prepared starting from a benzyl ester of hyaluronic acid multifilament 30 tex that is weaved using a circular knitting machine to obtain tubular mesh. The tubular mesh obtained is cut into pieces of the required size. HYALONECT® is intended for use in orthopedic and trauma reconstructive procedures to maintain the relative position of engrafted bone tissue (autograft, allograft, and bone graft substitutes) or bone fragments from comminuted fractures. The mesh is resorbable and suturable and may be fixed to the surgical site of application by means of sutures, or internal bone fixation devices. HYALOFAST® is a biodegradable support for the entrapment of mesenchymal stem cells for the repair of chondral and osteochondral lesions. It is a three-dimensional, nonwoven pad entirely composed of benzyl ester of hyaluronic acid. HYALOFAST® is initially filling the joint tissue defect and temporarily substituting the chondral and osteochondral tissue in the lesion until it is absorbed and replaced by the joint tissue.

GRAFTJACKET™ 5 x 10 cm, Thickness 0.89-1.40 mm Non-Meshed Standard, Lot # AH101078-085. GraftJacket is an allograft, in particular a non-cross-linked human dermis. Currently, GraftJacket (Wright Medical Technology) is the gold standard in augmentation procedures, for this reason in the following examples is used as a comparison scaffold.

REGENETEN 2.5 x 3 cm, Thickness 1.0-1.30 mm high porosity reconstituted type I collagen mesh of bovine origin.

**Examples**

The following examples further describe and demonstrate four preferred embodiments of the leno-weave bioabsorbable textile (later referred as Design 1, Design 2, Design 3 and Design 4, see Table 1) and five preferred embodiments of the warp-knitted bioabsorbable textile (later referred as High Density Double Bar HD2, Low Density Double Bar LD2, High Density Single Bar HD1, Low Density Single Bar LD1 and High Density Single Bar* HD1*, see Table 1) within the aim of the present disclosure. The examples are given solely for the purpose of illustration and are not be construed as limitations of the present disclosure, as many variations thereof are possible without departing from the spirit and scope of the disclosure.

**Table 1.** Bioabsorbable textile variants

| Bioabsorbable textile | Configuration | Nomenclature | Areal density | Thickness |
|---|---|---|---|---|
| Leno-weave | 17.5 dents per cm | Design 1 | 331 gsm | 0.755 mm |
| | 14 dents per cm | Design 2 | 270 gsm | 0.775 mm |
| | 4 warp yarns per dent | Design 3 | 531 gsm | 0.986 mm |
| | 6 warp yarns per dent | Design 4 | 665 gsm | 1.295 mm |
| Warp-knitted | High Density Double Bar | HD2 | 426 gsm | 0.761 mm |
| | Low Density Double Bar | LD2 | 274 gsm | 0.650 mm |
| | High Density Single Bar | HD1 | 248 gsm | 0.529 mm |
| | Low Density Single Bar | LD1 | 166 gsm | 0.464 mm |
| | High Density Single Bar* | HD1* | 325 gsm | 0.540 mm |

All the bioabsorbable textiles indicated in Table 1 were prepared as indicated in the detailed description for the configurations by starting from a yarn of benzyl ester of hyaluronic acid.

Example 1: Yarn characteristics

Described herein is the evaluation of yarn characteristics of one preferred embodiment of the yarn comprising the leno-weave bioabsorbable textiles according to the present disclosure.

One preferred embodiment of the yarn comprising the bioabsorbable textiles showed a linear mass density of $65.70 \pm 2.84$ tex, a breaking strength of $8.32 \pm 0.58$ N, an extension at break of $5.0 \pm 0.9\%$, an amount of spin finish of $3.58 \pm 0.01\%$ w/w, a percentage of esterification of $95.34 \pm 0.60\%$, an amount of free benzyl alcohol lower than the limit of quantification, a loss on drying of $6.55 \pm 0.57\%$, a residual amount of dimethyl sulfoxide of $0.13 \pm 0.02\%$, a residual amount of acetone lower than the limit of quantification, and a residual amount of ethyl alcohol of $0.09 \pm 0.04\%$.

Example 2: Mesh weave characteristics

Described herein is the evaluation of mesh weave characteristics of four preferred embodiments of the leno-weave bioabsorbable textiles according to the present disclosure.

One picture of each region (lower, middle, upper) of the specimen in both direction (warp and weft), with a ruler orthogonal aligned respect the side of the sample, was taken with a ZetaLine microscope. The number of yarns per unit length was counted. Three specimens of bioabsorbable textile were analyzed for Design 1 and Design 2, and nine specimens of bioabsorbable textile were analyzed for Design 3 and Design 4.

Warp direction: one preferred embodiment of the bioabsorbable textile showed $18.67 \pm 0.87$ number of yarns per cm (Design 1), one other preferred embodiment of the bioabsorbable textile showed $15.89 \pm 1.45$ number of yarns per cm (Design 2), one other preferred embodiment of the bioabsorbable textile showed $29.63 \pm 0.63$ number of yarns per cm (Design 3) and one other preferred embodiment of the bioabsorbable textile showed $35.11 \pm 1.93$ number of yarns per cm (Design 4).

Weft direction: one preferred embodiment of the bioabsorbable textile showed $11.33 \pm 2.65$ number of yarns for cm (Design 1), one other preferred embodiment of the bioabsorbable textile showed $8.44 \pm 1.94$ number of yarns for cm (Design 2), one other preferred embodiment of the bioabsorbable textile showed $7.79 \pm 0.41$ number of yarns for cm (Design 3) and one other preferred embodiment of the bioabsorbable textile showed $8.00 \pm 0.00$ number of yarns for cm (Design 4).

The results show a high value of standard deviation, in particular for weft direction for Design 1 and Design 2, due to the structure of suture pads. In these regions, for Design 1 and Design 2, the bioabsorbable textiles have a more compact structure with a major number of yarns per unit length.

Example 3: Thickness measurement

Described herein is the evaluation of thickness of four preferred embodiments of the leno-weave bioabsorbable textiles and four preferred embodiments of the knitted bioabsorbable textiles according to the present disclosure.

The thickness was measured following ASTM D1777 - 96(2015). The thickness of each leno-weave bioabsorbable textile was measured in three different points (lower, middle, upper) with a Wisamic digital Thickness gauge. Three specimens of bioabsorbable textile were analyzed for Design 1 and Design 2, nine specimens of bioabsorbable textile were analyzed for Design 3 and Design 4.

One preferred embodiment of the leno-weave bioabsorbable textiles showed $0.755 \pm 0.009$ mm (Design 1), one other preferred embodiment of the leno-weave bioabsorbable textiles showed 0.775 ± 0.015 mm (Design 2), one other preferred embodiment of the leno-weave bioabsorbable textiles showed 0.986 ± 0.032 mm (Design 3), one other preferred embodiment of the leno-weave bioabsorbable textiles showed 1.295 ± 0.035 mm (Design 4).

The thickness of each knitted bioabsorbable textile was measured in one point (middle) with a Wisamic digital Thickness gauge.

One preferred embodiment of the knitted bioabsorbable textiles showed about 0.761 mm (HD2), one other preferred embodiment of the knitted bioabsorbable textiles showed about 0.65 mm (LD2), one other preferred embodiment of the knitted bioabsorbable textiles showed about 0.529 mm (HD1), one other preferred embodiment of the knitted bioabsorbable textiles showed about 0.464 mm (LD1), one other preferred embodiment of the knitted bioabsorbable textiles showed about 0.540 mm (HD1*).

Example 4: Areal density measurement

Described herein is the evaluation of areal density of four preferred embodiments of the leno-weave bioabsorbable textiles and four preferred embodiments of the knitted bioabsorbable textiles according to the present disclosure.

The length and width of each prototypes was measured in three different regions (lower, middle, upper) with a Maurer digital caliper. Each specimen was weighted by an analytical balance. Three specimens of leno-weave bioabsorbable textile were analyzed for Design 1 and Design 2, nine specimens of leno-weave bioabsorbable textile were analyzed for Design 3 and Design 4.

One preferred embodiment of the leno-weave bioabsorbable textiles showed a mesh density of 330.8 ± 0.9 gsm (Design 1), one other preferred embodiment of the leno-weave bioabsorbable textiles 270.3 ± 1.3 gsm (Design 2), one other preferred embodiment of the leno-weave bioabsorbable textiles 531.2 ± 8.7 gsm (Design 3), one other preferred embodiment of the leno-weave bioabsorbable textiles 665.4 ± 7.8 gsm (Design 4).

The length and width of each prototypes was measured on one side (middle) with a Maurer digital caliper. Each specimen was weighted by an analytical balance.

One preferred embodiment of the knitted bioabsorbable textiles showed a mesh density of about 426 gsm (HD2), one other preferred embodiment of the knitted bioabsorbable textiles about 274 gsm (LD2), one other preferred embodiment of the knitted bioabsorbable textiles about 248 gsm (HD1), one other preferred embodiment of the knitted bioabsorbable textiles 166 gsm (LD1), one other preferred embodiment of the knitted bioabsorbable textiles 325 gsm (HD1*).

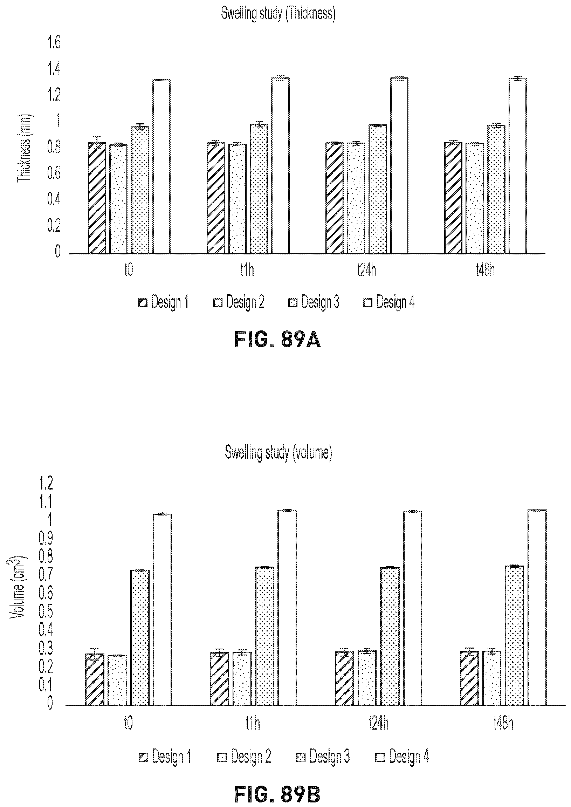

FIG. 89A shows the swelling behavior of four preferred embodiments of the textile of the invention, with reference to thickness as explained in Example 5.

Example 5: Swelling measurement

Described herein is the evaluation of swelling of four preferred embodiments (referred as Design 1, Design 2, Design 3 and Design 4) of the leno-weave bioabsorbable textiles according to the present disclosure. The samples were immersed with 15 ml of PBS in petri dishes at 37 °C to simulate the physiological environment. At different time points, 1h, 24h and 48h, the samples were blotted on paper to eliminate the excess PBS, and then weight and dimensions were measured. In particular, the length and width of each embodiment of the bioabsorbable textile were measured in three different regions (lower, middle, upper) with a Maurer digital caliper. The thickness was measured following ASTM D1777 - 96(2015). The thickness of each embodiment of the bioabsorbable textile was measured in three different points (lower, middle, upper) with a Wisamic digital Thickness gauge. Each sample was weighted by an analytical balance. Three specimens of each sample were analyzed. The swelling % was calculated following equation:

$$ Sw\% = \frac{Ws - Wi}{Wi} \times 100 $$

Ws is the weight of swelled specimen and Wi is the initial weight of dry specimen.

The preferred embodiments of the bioabsorbable textiles did not show an increase or decrease in thickness between swelled and dry conditions, moreover the dimensions did not change during the time of the experiment (FIG. 89A).

FIG. 89B shows the swelling behavior of four preferred embodiments of the textile of the invention, with reference to volume as explained in Example 5. The preferred embodiments of the bioabsorbable textiles did not show an increase or decrease in volume between swelled and dry conditions, moreover the dimensions did not change during the time of the experiment (FIG. 89B).

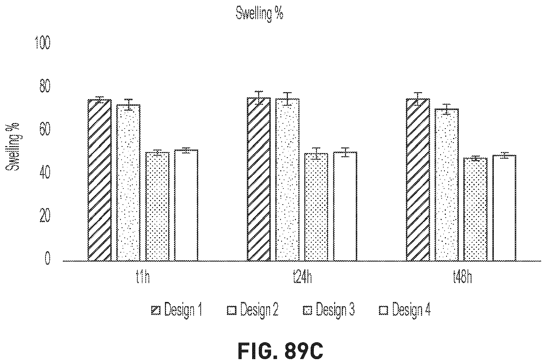

FIG. 89C shows the swelling behavior of four preferred embodiments of the textile of the invention, with reference to percentage as explained in Example 5. Instead, Design 1 and Design 2 showed a swelling % of about 75% after 1h in PBS and this value did not change during the experiment (FIG. 89C), Design 3 and Design 4 showed a swelling % of about 50% after 1h in PBS and this value did not change during the experiment (FIG. 89C).

Example 6: Porosity and pore size determination

Described herein is the evaluation of pore size of two preferred embodiment of the leno-weave bioabsorbable textiles according to the present disclosure.

The pore dimensions (macroporosity) were designed before production step. To analyze also the presence of micropores (inter and intra yarn porosity) a mercury intrusion porosimetry test was performed. Pore size measurement was performed by a Thermo Finningan (Waltman, USA) Pascal 140 e 240. The specimens were dried at 105 °C for 24 hours. Three specimens of each sample were analyzed. The results are related to the pores with a diameter <116 $\mu$m (higher limit of the mercury intrusion porosimeter used).

One preferred embodiment of the bioabsorbable textiles showed an accessible porosity of 27.67 $\pm$ 1.58 %, a total pore volume of 463.65 $\pm$ 29.55 mm$^3$/g, an average pore diameter of 3000 nm, and a median pore diameter of 39100 nm (Design 1) instead one other preferred embodiment of the bioabsorbable textiles showed an accessible porosity of 25.70 $\pm$ 2.94%, a total pore volume of 461.03$\pm$ 61.71 mm$^3$/g, an average pore diameter of about 5700 nm, and a median pore diameter of 45800 nm (Design 2).

This porous structure of the bioabsorbable textiles is suitable for allowing cell migration and proliferation, so ultimately providing biological augmentation to the target joint tissue.

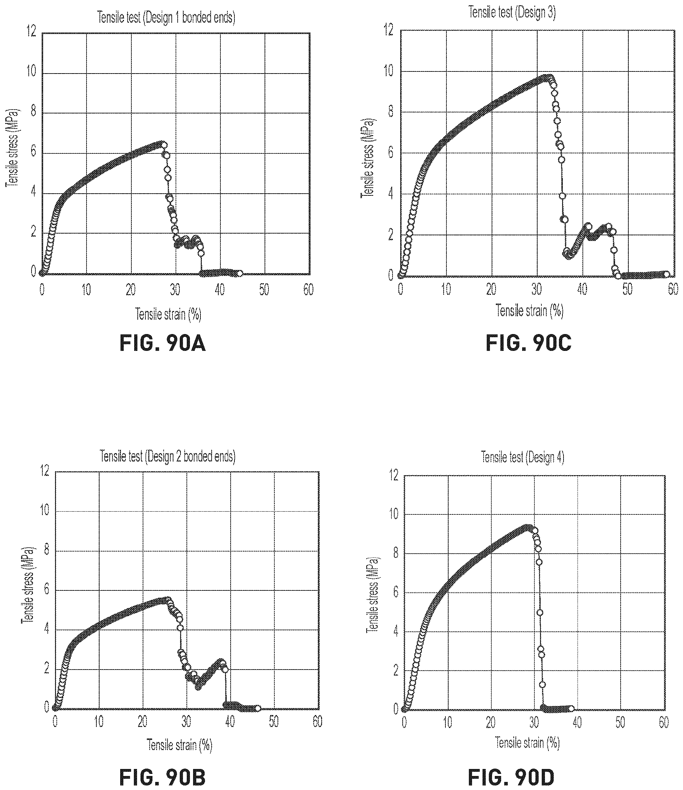

FIG. 90A shows the tensile behavior comparison between four preferred embodiments of the textile of the invention as explained in Example 7. FIG. 90A illustrates for example , the tensile testing for Design 1 with ILLUSTRATES FOR EXAMPLE, THE TENSILE TESTING FOR DESIGN 1 WITH bonded ends.

Example 7: Tensile strength and device stiffness

Described herein is the evaluation of tensile strength and device stiffness of four preferred embodiment of the leno-weave bioabsorbable textiles according to the present disclosure.

Tensile strength and device stiffness were calculated following ASTM D5034-09. The specimens (6 for Design 1 and Design 2; 9 for Design 3 and Design 4) were immersed 1 hour in PBS at 37 °C to simulate the physiological environment. The test was performed by an Instron 3345 Tensile testing apparatus with a load cell of 5 kN. The extension test was performed at 60 mm/min after a pre-load (until 0.4 N) at 10 mm/min. A gauge length of 3.5 cm for Design 1 and Design 2 and a gauge length of 3 cm for Design 3 and Design 4 was settled.

Tensile test was performed on Design 1 and Design 2 with and without bonded ends.

Preferred embodiments of the bioabsorbable textiles showed a mechanical behavior comparable to infraspinatus tendon (FIG. 90A, FIG. 90B, FIG. 90C, FIG. 90D). Prototypes showed a very short toe region and an elastic region in the first part of Stress vs Strain curve.

The mechanical properties of the four different bioabsorbable textiles are summarized in the following tables (Table 2 A, 1B and 1C)

Table 2. Mechanical properties of four different embodiments of the bioabsorbable textiles with: A) Design 1 and Design 2 open ends; B) Design 1 and Design 2 bonded ends; C) Design 3 and Design 4.

TABLE 2

| | Young's modulus [MPa] | Failure stress [MPa] | Failure strain [ε%] | Maximum Load [N] | Yield stress [Mpa] | Yield strain [%] | Yield Load [N] | Stiffness [N/m] | Apparent Young's modulus [Mpa] | Apparent Failure strain [ε%] | Apparent yield strain [%] |
|---|---|---|---|---|---|---|---|---|---|---|---|
| | | | | | | | | | | | |
| Design 2 | 187.43 ±18.05 | 6.12 ±0.40 | 25.23 2.11 | 59.54 ±0.62 | 3.73 ±0.24 | 3.43 ±0.29 | 36.25 ±1.25 | 22491.83 ±2719.21 | 85.47 ±5.95 | 53.54 ±4.13 | 7.14±0.68 |
| Design 1 | 189.39 ±12.08 | 6.86 ±0.33 | 26.74 ±0.89 | 71.58 ±0.84 | 4.07 ±0.09 | 3.65 ±0.37 | 42.57 ±1.42 | 25002.25±891.30 | 91.71 ±4.99 | 52.68 ±4.70 | 7.19±0.74 |
| | p>0.05 | p<0.05 | p>0.05 | p<0.05 | p<0.05 | p>0.05 | p<0.05 | p>0.05 | p>0.05 | p>0.05 | p>0.05 |

B)

| | Young's modulus [MPa] | Failure stress [MPa] | Failure strain [ε%] | Maximum Load [N] | Yield stress [Mpa] | Yield strain [%] | Yield Load [N] | Stiffness [N/m] | Apparent Young's modulus [Mpa] | Apparent Failure strain [ε%] | Apparent yield strain [%] |
|---|---|---|---|---|---|---|---|---|---|---|---|
| Design 2 | 132.37±3.58 | 5.89 ±0.33 | 27.27 ±2.55 | 57.22 ±1.22 | 3.31 ±0.19 | 3.88 ±0.30 | 31.75 ±0.84 | 16206.98 ±1263.73 | 62.89±4.38 | 55.71 ±2.72 | 7.81±0.92 |
| Design 1 | 116.13±26.03 | 5.96 ±0.43 | 25.99 ±2.87 | 65.64 ±1.93 | 3.24 ±0.26 | 4.29 ±0.50 | 34.67 ±3.21 | 16540.67 ±3133.94 | 56.35 ±12.30 | 51.05 ±6.27 | 8.28±1.02 |
| | p>0.05 | p>0.05 | p>0.05 | p<0.05 | p>0.05 | p>0.05 | p>0.05 | p>0.05 | p>0.05 | p>0.05 | p>0.05 |

C)

| | Young's modulus [MPa] | Failure stress [MPa] | Failure strain [ε%] | Maximum Load [N] | Yield stress [Mpa] | Yield strain [%] | Yield Load [N] | Stiffness [N/m] | Apparent Young's modulus [Mpa] | Apparent Failure strain [ε%] | Apparent yield strain [%] |
|---|---|---|---|---|---|---|---|---|---|---|---|
| | | | | | | | | | | | |
| Design 3 | 149.32 ±10.50 | 9.44 ±0.27 | 30.98 ±1.74 | 141.31 ±1.90 | 4.32 ±0.21 | 4.32 ±0.21 | 71.77 ±1.88 | 42992.99± 1680.82 | 87.57 ±4.95 | 53.40 ±2.85 | 7.44±0.33 |
| Design 4 | 119.43±4.32 | 9.24 ±0.36 | 29.10 ±1.56 | 184.74 ±4.05 | 4.73 ±0.19 | 5.43 ±0.26 | 94.63 ±2.84 | 45480.90 ±1758.78 | 68.62 ±2.57 | 50.74 2.97 | 9.46±0.40 |
| | p<0.05 | p>0.05 | p<0.05 | p<0.05 | p<0.05 | p<0.05 | p<0.05 | p<0.05 | p<0.05 | p>0.05 | p<0.05 |

FIG. 90B shows the tensile behavior comparison between four preferred embodiments of the textile of the invention as explained in Example 7.

FIG. 90C. shows the tensile behavior comparison between four preferred embodiments of the textile of the invention as explained in Example 7.

FIG. 90D shows the tensile behavior comparison between four preferred embodiments of the textile of the invention as explained in Example 7.

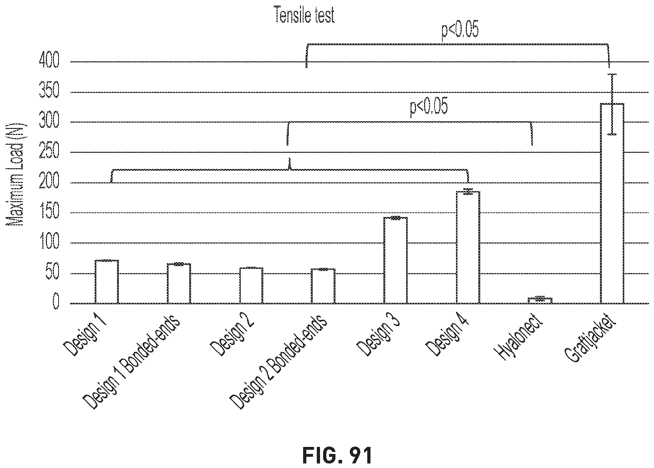

FIG. 91 shows the Maximum Load comparison among different textile configurations of the invention and two comparative devices as explained in Example 7. Leno-weave bioabsorbable textiles showed higher Maximum load (or failure load) than Hyalonect but lower than GraftJacket (FIG. 91).

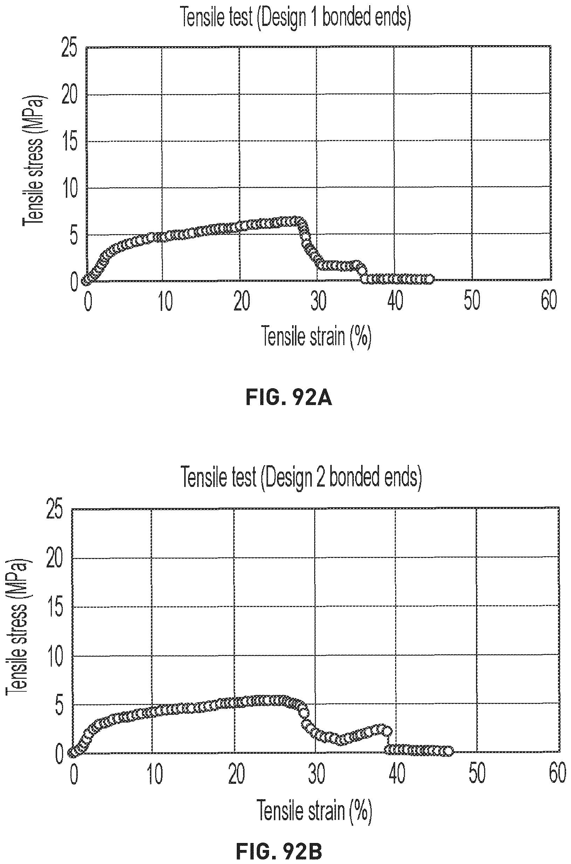

However, GraftJacket showed a long toe region (FIG. 92A - FIG. 92F), as also reported in literature. GraftJacket indeed reached the peak value of load after long extension, conversely to what observed in the native supraspinatus tendon making the properties not relevant for the application.

From the tensile test, elongation at 50 N and load at 5 mm were calculated. These are physiologically- important parameters, as elongation at 50 N is the physiologic load to which an augmentation device is exposed to (Aurora, 2010), while load at 5 mm elongation is the estimated maximum permissible retraction of tendon at the repair site before to lose repair continuity (Hughes, 1996; Burkhart, 1997). An ideal device for rotator cuff repair should show short elongation at 50 N (within 5-10 mm) (Burkhart, 1997) and high load at 5 mm elongation (>50 N).

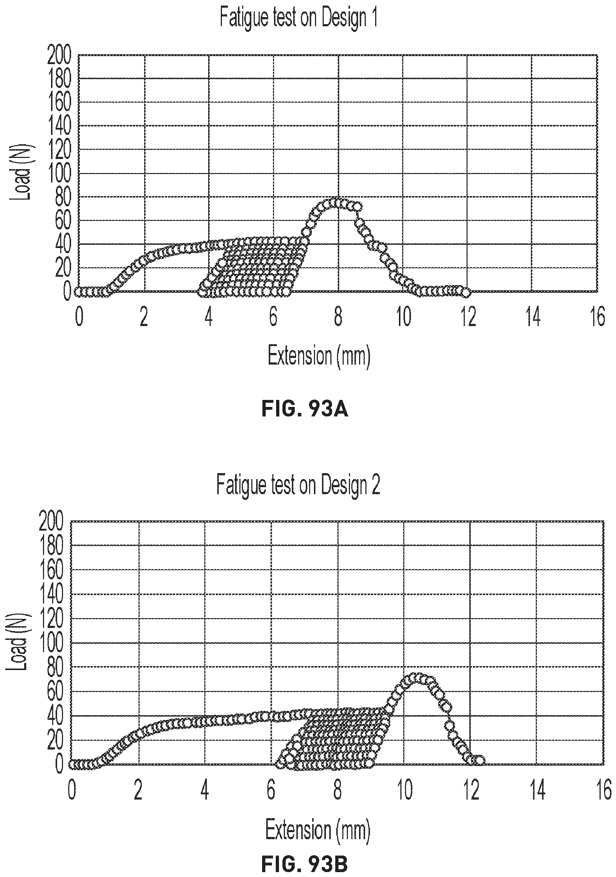

As shown in FIG. 93A - FIG. 93D GraftJacket showed low load values at short extensions. For Hyalonect, it is impossible to calculate the elongation at 50 N as it could not bear such mechanically-demanding loads. Conversely, the bioabsorbable textiles in all conditions (with and without bonded ends) showed values of elongation at 50 N and load at 5 mm typical of an ideal device for augmentation (Aurora, 2010; Hughes, 1996; Burkhart, 1997).

**Table 3.** Values of load at 5 mm and elongation at 50N

|  | Load at 5mm [N] | Elongation at 50N [mm] |
|---|---|---|
| Design 1 | 50.00 $\pm$ 0.67 | 5.08 $\pm$ 0.29 |
| Design 2 | 42.85 $\pm$ 1.48 | 9.27 $\pm$ 0.94 |
| Design 1 Bonded ends | 43.85 $\pm$ 2.58 | 7.60 $\pm$ 1.22 |
| Design 2 Bonded ends | 38.98 $\pm$ 0.51 | 11.73 $\pm$ 0.57 |
| Design 3 | 97.84 $\pm$ 1.05 | 1.40 $\pm$ 0.07 |
| Design 4 | 123.63 $\pm$ 1.78 | 1.44 $\pm$ 0.09 |
| Graft Jacket | 5.87 $\pm$ 0.37 | 12.70 $\pm$ 0.14 |
| Hyalonect | 3.02 $\pm$ 1.98 |  |

Example 8: Fatigue test

Described herein is the fatigue test of four preferred embodiments of the leno-weave bioabsorbable textiles according to the present disclosure.

A fatigue test was performed to simulate physiological conditions according to ASTM D3479 / D3479M - 12 and Amit Aurora et al., 2011 paper (Aurora, 2011). The specimens (3 for each prototype) were immersed 1 hour in PBS at 37 °C to simulate the physiological environment. The test was performed by an Instron 3345 Tensile testing apparatus with a load cell of 100 N for Design 1 and Design 2, with a load cell of 5 kN for Design 3 and Design 4. Fatigue loading was applied for 2500 cycles at 15 mm/min ranged from 1.2 N (2% of lower Maximum load obtained by tensile test) to 43 N (60% of higher Maximum load obtained by tensile test) after an initial ramp at 10 mm/min until 1.2 N for Design 1 and Design 2 and for 2500 cycles at 15 mm/min ranged from 2.8 N (2% of lower Maximum load obtained by tensile test) to 110.8 N (60% of higher Maximum load obtained by tensile test) after an initial ramp at 10 mm/min until 2.8 N for Design 3 and Design 4. The compressed air was set to around 5.5 bar and the gauge length to 3.5 cm for Design 1 and Design 2 and to 3 cm for Design 3 and Design 4. After the cyclic step, an absolute ramp at 60 mm/min was performed until 98 N or when the sample is broken for Design 1 and Design 2 and until 250 N or when the sample is broken for Design 3 and Design 4. The time of the entire test was about 3-4 hours and during the analysis, the specimens were kept wet with PBS solution at 37 °C.

FIG. 92B shows the tensile behavior comparison among four preferred embodiments of the textile of the invention and two comparative devices as explained in Example 7.

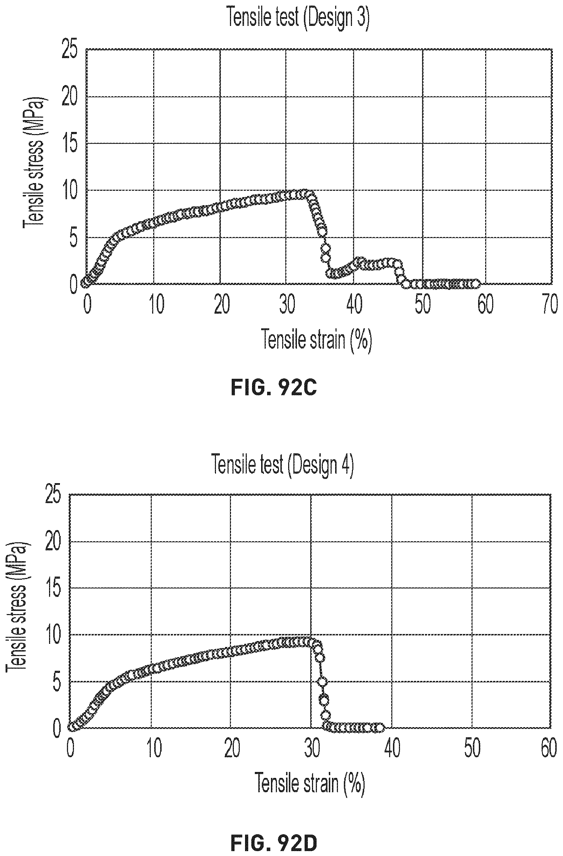

FIG. 92C shows the tensile behavior comparison among four preferred embodiments of the textile of the invention and two comparative devices as explained in Example 7.

FIG. 92D shows the tensile behavior comparison among four preferred embodiments of the textile of the invention and two comparative devices as explained in Example 7.

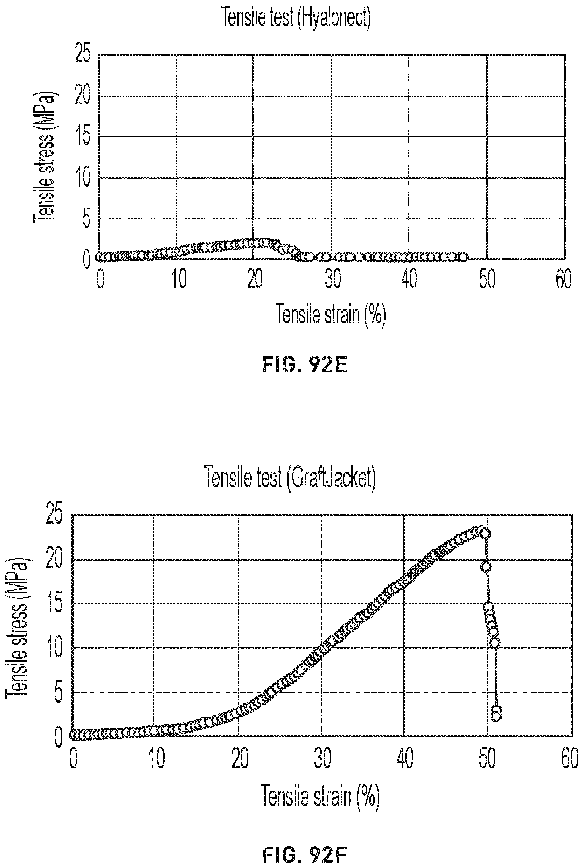

FIG. 92E shows the tensile behavior comparison among four preferred embodiments of the textile of the invention and two comparative devices as explained in Example 7.

FIG. 92F shows the tensile behavior comparison among four preferred embodiments of the textile of the invention and two comparative devices as explained in Example 7.

Examples of load vs extension curve obtained after fatigue test were reported in FIG. 93A-FIG. 93D.

The scaffolds were able to withstand the 2500 loading cycles projected for the early post-operative period (Aurora, 2011).

The four different embodiments of the bioabsorbable textiles show load to failure values after 2500 cycles of 75.29 ± 0.16 N (Design 1), 70.86 ± 0.80 N (Design 2), 189.56 ± 17.33 N (Design 3) and 190.05 ± 3.35 N (Design 4).

Cyclic elongation of the first and 2500th cycle, defined as the displacement between the valley at the end of the nth cycle and the valley at the beginning of the first cycle (Aurora, 2011) were calculated. One preferred embodiment of the bioabsorbable textiles showed a cyclic elongation at the first cycle of 2.77 ± 0.33 mm and at 2500th cycle of 5.52 ± 0.01 mm (Design 1), one other preferred embodiment of the bioabsorbable textiles showed a cyclic elongation at the first cycle of 5.07 ± 0.45 mm and at 2500th cycle of 7.49 ± 0.64 mm (Design 2), one other preferred embodiment of the bioabsorbable textiles showed a cyclic elongation at the first cycle of 7.36 ± 0.08 mm and at 2500th cycle of 9.50 ± 0.05 mm (Design 3), one other preferred embodiment of the bioabsorbable textiles showed a cyclic elongation at the first cycle of 2.81 ± 0.23 mm and at 2500th cycle of 4.91 ± 0.25 mm (Design 4). A device for rotator cuff augmentation should limit retraction of the tendon from the bone at the repair site in the range between 5-10 mm (maximum permissible retraction of the tendon from bone without lose repair continuity) (Burkhart, 1997). Considering the obtained data, at least one of the preferred embodiments of the bioabsorbable textiles, even during fatigue test, showed a mechanical behavior that matched the requirements for an ideal device for rotator cuff repair.

FIG. 93B shows the fatigue behavior comparison between four preferred embodiments of the textile of the invention as explained in Example 8.

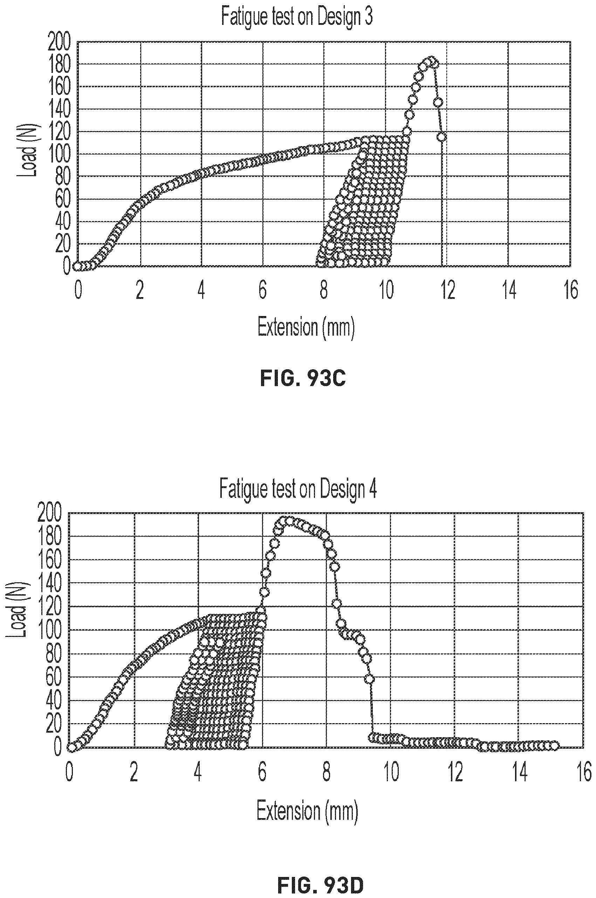

FIG. 93C shows the fatigue behavior comparison between four preferred embodiments of the textile of the invention as explained in Example 8.

FIG. 93D shows the fatigue behavior comparison between four preferred embodiments of the textile of the invention as explained in Example 8.

Example 9: Suture pull-out strength

Described herein is the evaluation of suture pull-out strength of four preferred embodiment of the leno-weave bioabsorbable textiles and of one preferred embodiment of the knitted bioabsorbable textiles according to the present disclosure.

Suture retention was calculated following a procedure slightly modified from the one described in Barber et al, 2006.

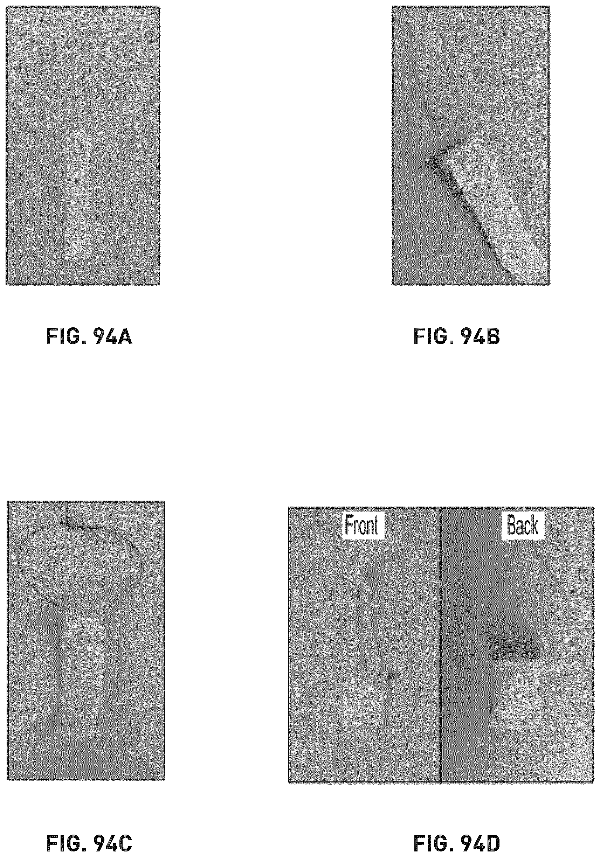

A single 2-0 sterile FiberWire® suture was passed through the textile in a horizontal mattress pattern. Each point of suture was set at one third of sample width apart and at 1/5 of sample width from the other suture point, and suture was carried out to a length of 6 cm from the textile for Design 1, Design 2, Hyalonect and GraftJacket (FIG. 94A and FIG. 94B).

The test was performed with the suture pads folded (3 specimens for prototypes) and not folded (3 specimens for each embodiment of the bioabsorbable textile) for Design 1 and Design 2 (FIG. 94A and FIG. 94B). For Design 3 and Design 4, the test was performed on 8 specimens. The HD1* was tested in both directions, machine and transverse (N=5 for each direction), to study if the material exhibits anisotropic or isotropic behavior.

FIG. 94B shows in suture technique on Design 1 and Design 2 (folded) as explained in Example 9.

1 Ethilon black monofilament nylon from Ethicon® suture was passed through the textile in a horizontal mattress pattern under the suture pad. The suture was placed 5 mm from the edge, and suture was carried out to a length of 6 cm from the textile for Design 3 and Design 4 according to Barber et al., 2006 (FIG. 94C).

FiberWire 2/5 metric Braided Polyblend Suture Blue with 2 Tapered Needles from Arthrex® suture was passed through the textile in a horizontal mattress pattern 5 mm from the edge, and suture was carried out to a length of 6 cm from the textile for HD1* according to Barber et al., 2006 **(FIG. 94D).**

The specimens were put 1 hour in PBS at 37 °C to mimic surgical scenario. The test was performed by an Instron 3345 Tensile testing apparatus with a load cell of 100 N for Design 1, Design 2 and HD1*, with a load cell of 5 kN for Design 3 and Design 4. The suture fiber was clamped in the upper clamp. The extension test was performed at 60 mm/min after a pre-load at 10 mm/min until 0.2 N for Design 1 and Design 2. The extension test was performed at 12.5 mm/min after a pre-load at 10 mm/min until 0.2 N for Design 3, Design 4 and HD1*.

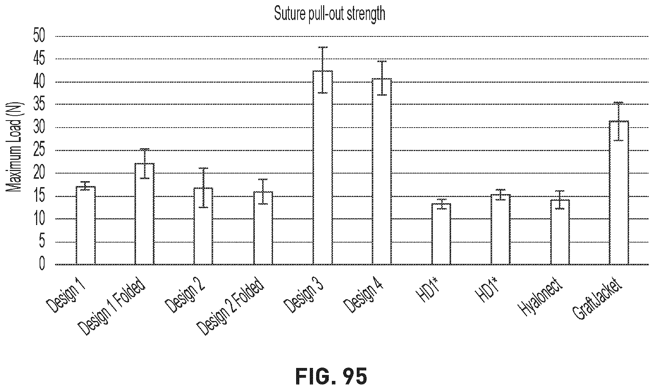

FIG. 95 shows a suture pull-out strength comparison among the preferred embodiments of the textile of the invention in different configurations as explained in Example 9.

The data were summarized in the FIG. 95 and Table 4.

**Table 4.** Suture pull-out strength (N)

| Desi gn 1 | Desi gn 1 Fold ed | Desi gn 2 | Desi gn 2 Fold ed | Desi gn 3 | Desi gn 4 | HD1* (one directi on) | HDi* (other directi on) | Hyalo nect | GraftJa cket Thin |
|---|---|---|---|---|---|---|---|---|---|
| 17.1 7 ± 0.72 | 22.0 0± 3.23 | 16.8 4 ± 4.30 | 15.8 8± 2.64 | 42.4 7 ± 5.01 | 40.7 9 ± 3.76 | 13.29 ± 1.02 | 15.26 ± 1.01 | 14.22 ± 1.84 | 31.33 ± 4.09 |

Example 10: Loss on drying evaluation

Described herein is the evaluation of loss on drying of four preferred embodiment of the leno-weave bioabsorbable textiles and one preferred embodiment of knitted bioabsorbable textile according to the present disclosure. The tested textiles of this example were those reported in Table 1.

The loss on drying was calculated measuring the weight of the specimen at the beginning and after drying the specimen (n=1) for 2 hours in oven at 105 °C.

One preferred embodiment of the bioabsorbable textiles showed a loss on drying of 9.11% (Design 1), one other preferred embodiment of the bioabsorbable textiles showed a loss on drying of 8.86% (Design 2), one other preferred embodiment of the bioabsorbable textiles showed a loss on drying of 11.77% (Design 3), one other preferred embodiment of the bioabsorbable textiles showed a loss on drying of 10.55% (Design 4), one preferred embodiment of the bioabsorbable textiles showed a loss on drying of about 10.23% (HD1*).

Example 11: Determination of percentage of esterification

Described herein is the evaluation of percentage of esterification of four preferred embodiments of the leno-weave bioabsorbable textiles and one preferred embodiment of knitted bioabsorbable textile according to the present disclosure. The tested textiles of this example were those reported in Table 1. The percentage of esterification was determined using HPLC 1260 Infinity Binary Pump equipped with an Infinity Poroshell 120 EC-C18 4.6x100 mm column. The mobile phase was a mixture of highly purified water/acetonitrile 50/50, a flow rate of 1 ml/min, an injection volume of 20 $\mu$l, a column temperature of 30 °C and a detection wavelength of 254 nm were chosen.

A calibration curve with benzyl alcohol was prepared.

Around 100 mg of sample (n=1) were treated with 10 mL of 0.1M NaOH solution at 60 °C for about 60 minutes for Design 1 and design 2 and for about 90 minutes for Design 3 and Design 4 to complete hydrolysis reaction. After the solution cooled to room temperature, 1 mL of 1 N HCl was added and the solution was diluted until 50 ml with mobile phase. The obtained solution was filtered through a

0.45 $\mu$m PTFE filter before HPLC injection.

One preferred embodiment of the bioabsorbable textiles showed a percentage of esterification of about 94.58% (Design 1), one other preferred embodiment of the bioabsorbable textiles showed a percentage of esterification of about 91.03% (Design 2), one other preferred embodiment of the bioabsorbable textiles showed a percentage of esterification of about 91.32% (Design 3), one other preferred embodiment of the bioabsorbable textiles showed a percentage of esterification of about 92.79% (Design 4), one other preferred embodiment of the bioabsorbable textiles showed a percentage of esterification of about 93.90% (HD1*).

Example 12: Determination of free Benzyl alcohol

Described herein the evaluation of free benzyl alcohol in four preferred embodiments of the leno-weave bioabsorbable textiles according to the present disclosure. The tested textiles of this example were those reported in Table 1.

The free benzyl alcohol was determined using HPLC 1260 Infinity Binary Pump equipped with an Infinity Poroshell 120 EC-C18 4.6x100 mm column. The mobile phase was a mixture of high purified water/acetonitrile 80/20, a flow rate of 1 ml/min, an injection volume of 20 $\mu$l, a column temperature of 30 °C and a detection wavelength of 254 nm were chosen.

A calibration curve with benzyl alcohol was prepared.

Around 100 mg of sample (n=1) were treated with 3.33 ml of acetonitrile to extract the free benzyl alcohol. The sample was filtered through a 0.45 $\mu$m PTFE filter. 0.5 ml of filtered solution was added to 0.5 ml of mobile phase before HPLC injection.

One preferred embodiment of the bioabsorbable textiles showed 0.023% (w/w) of free benzyl alcohol (Design 1), one other preferred embodiment of the bioabsorbable textiles 0.035% (w/w) of free benzyl alcohol (Design 2), one other preferred embodiment of the bioabsorbable textiles showed a peak lower than the limit of quantification (LOQ) (Design 3), one other preferred embodiment of the bioabsorbable textiles showed a peak lower than LOQ (Design 4).

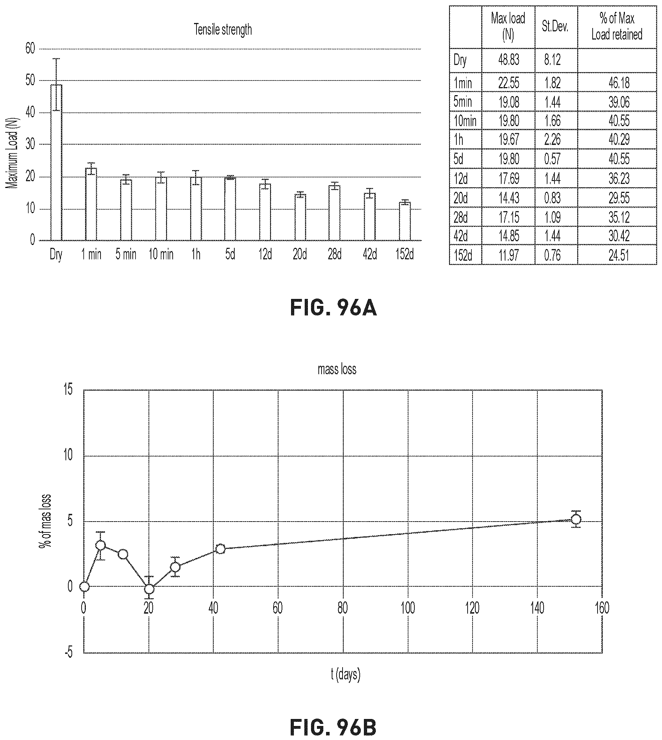

FIG. 96A shows the *in vitro* degradation results, mechanical results as explained in Example 13.

Example 13: *In vitro* degradation study

Described herein the evaluation of *in vitro* degradation in simulated physiological conditions of one preferred embodiment of the knitted bioabsorbable textile according to the present disclosure. The tested textiles of this example were those reported in Table 1.

Considering the composition of synovial fluid of the joints, the *in vitro* degradation study was performed in simulated synovial fluid (SSF). SSF will be prepared following Marques MRC et al., 2011, by solubilizing 3 g of HA in 1 L of PBS under magnetic stirring. The amount of synovial fluid present in a joint is about 0.5-4 ml within large joints such as the knee (Kraus, 2007). Therefore, in order to simulate clinical condition, HD1* was immersed in 3 ml of SSF (minimum volume to fully submerge the mesh) in 15 ml conical plastic tube and put in the oven at 37 °C.

Six (N=6) specimens, 20x25 mm HD1* were tested for each time point: N=3 for mass loss and % of esterification tests and N=3 for mechanical properties.

After 1 minute in SSF, HD1* lost about 50% of its mechanical strength, after 5 minutes until 5 days around 60% of its mechanical strength was lost, and after 152 days HD1* retained around 25% of initial maximum load (Fig. 96A).

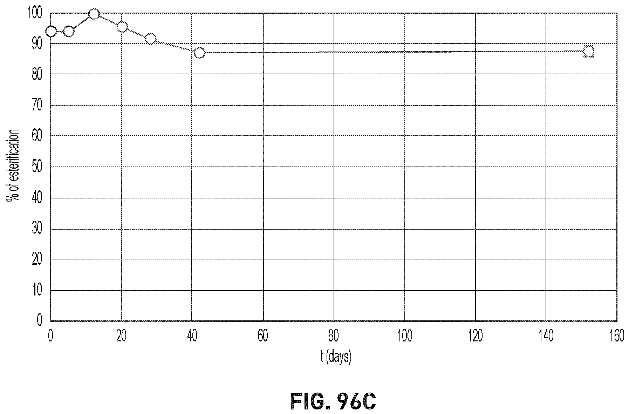

FIG. 96B shows the *in vitro* degradation results, % of mass loss during the time as explained in Example 13. After 152 days, HD1* lost about 5% of its initial mass and showed a % of esterification of about 87% (FIG. 96B and FIG. 96C).

Example 14: *In vitro* cytotoxicity

Described herein is the assessment of in vitro cytotoxicity of some preferred embodiments of the bioabsorbable textiles (hereafter referred as "test samples"). The tested textiles of this example were those reported in Table 1.

The test was conducted by following the ISO 10993-5:2009 with slight adaptations, using liquid extracts of the test samples. The samples were washed with isopropanol and dried before the test. Extraction method is particularly indicated for biodegradable and absorbable materials, for which not only leachable chemicals, but also degradation products must be tested for possible toxicity. The extraction of test samples was performed for $24 \pm 2h$ at $37 \pm 1$ °C using cell culture medium with serum as vehicle. The volume of extraction vehicle was calculated following what suggested in the ISO 10993-12:2012. The extraction vehicle not containing the test sample, retained in a vessel identical to the one used for the test samples, was incubated for $24 \pm 2h$ at $37 \pm 1$ °C and employed as Control. The Hyalonect extract was produced following the same conditions and used as an additional control. The cell line on which liquid extracts were tested was NIH 3T3 mouse fibroblasts, sub-cultured at least once before use. The extracts were incubated with cells for $24 \pm 2h$. Then the quantitative determination of cytotoxicity was done by MTS assay. The metabolic activity of cells put in contact with extracts was compared to that of the Control, which was assumed as 100% viability; then, cell viability (%) of each test condition was extrapolated thereby. Four preferred embodiments of the bioabsorbable textiles resulted in a relative cell viability of $83.19 \pm 6.72\%$

(Design 1), $79.75 \pm 3.28\%$ (Design 2), $98.44 \pm 3.60\%$ (Design 3), $97.29 \pm 3.42\%$ (Design 4), and $94.38 \pm 6.10\%$ (LD2) respectively. The effect of the textiles on cells was statistically equivalent or even better than that of the Hyalonect, producing a relative cell viability of $83.65 \pm 8.15$ % compared to Control. Overall, since all embodiments passed the cell viability threshold of 70% of Control, the bioabsorbable textiles can be considered not cytotoxic.

FIG. 96C shows the *in vitro* degradation results % of Hyaff 11 esterification during the time as explained in Example 13.

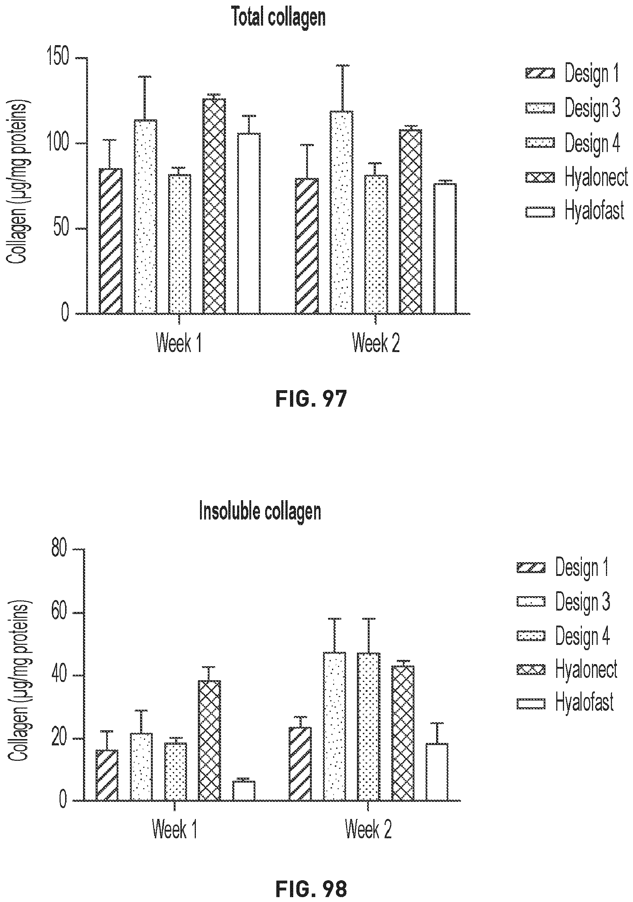

FIG. 97 shows the results of the *in vitro* quantitative assessment of collagen production of Example 15, specifically the quantity of total collagen after 1 and 2 weeks of cell culture. Error bars represent standard deviation.

Example 15: *In vitro* quantitative assessment of collagen production

Suitable scaffolds for cell culture were generated by cutting each textile sample with sterile scissors. The dimensions of the scaffolds were $6.8 \pm 0.2$ mm x 13 mm for Design 1, $7.5 \pm 0.3$ mm x 13 mm for Design 2, and $5.0 \pm 0.1$ mm x 15 mm for Design 3 and 4. HYALOFAST® of similar dimensions was used as control, proven to be a suitable cell scaffold and not to inhibit new collagen deposition. NIH 3T3 culture was performed with an initial cell number of 2 million seeded on each scaffold, in low adhesion 24-well plates. After overnight incubation in standard culture medium, cell-loaded textile samples were transferred to new wells and cultured in 1.5 mL of standard culture medium for 4 days to allow cell expansion; the culture medium was then substituted with the differentiation medium (DMEM High Glucose + 2 mM L-Glutamine + 1% PenStrep + 1% HEPES + 1% ITS, supplemented with 300 $\mu$g/mL of Ascorbic Acid 2-phosphate and 2 ng/mL of TGF$\beta$1), in which cell- loaded textile samples were cultured for 1 week. The medium was changed twice a week and collagen production was measured at 7 and 14 days. Newly-formed soluble collagen and insoluble collagen fibers were quantified with dedicated kits, the S1000 Sircol Collagen Assay and the S2000 Sircol Insoluble Collagen Assay, respectively (Biocolor). Appropriate calibration curves with collagen standards, provided by the kit, were built at each assay run. Soluble collagen was quantified, according to the specific kit protocol, from the spent culture medium retrieved and stocked at - 80 °C at each medium change. As far as concerned insoluble collagen determination at the established time points, the S2000 Sircol Insoluble Collagen Assay protocol required some adaptation for these textiles. The manufacturer's instructions recommended to start the protocol with tissue samples of 20-30 mg of wet weight, but the measured wet weight of entire scaffolds was $94.65 \pm 24.26$ mg. Thus, depending on its wet weight, each textile sample was cut into three to five similar portions, which were treated as 3 different samples for S2000 Sircol Insoluble Collagen Assay. The insoluble collagen quantity per textile sample (experimental sample) was reconstructed by summing the measured collagen quantities of the three to five derived samples. Finally, insoluble and soluble collagen quantities for each experimental sample were summed to gain total collagen, whose value was normalized on the total amount of proteins in the same sample, assessed by Bradford method. Results indicated that a considerable amount of total collagen was present in all textile samples, with only some embodiments (Design 1 and Design 3) equaling the total amount of collagen produced on HYALOFAST® at day 7. However, at day 14, all embodiments were comparable or even better than HYALOFAST® in terms of total collagen production. Moreover, looking just at the mature, insoluble (cross-linked) collagen, the most relevant collagen fraction in a tendon healing context, all embodiments surprisingly displayed 35 higher performances compared to HYALOFAST® at both timepoints, supporting the deposition of higher insoluble collagen quantities. This unexpected and exceptional finding has been attributed to the potential of the textile longitudinally-oriented fibers to support fast and mature collagen deposition. Data were summarized in FIG. 97 and FIG. 98.

FIG. 98 shows the results of the *in vitro* quantitative assessment of collagen production of Example 15, specifically the quantity of insoluble, mature collagen after 1 and 2 weeks of cell culture. Error bars represent standard deviation.

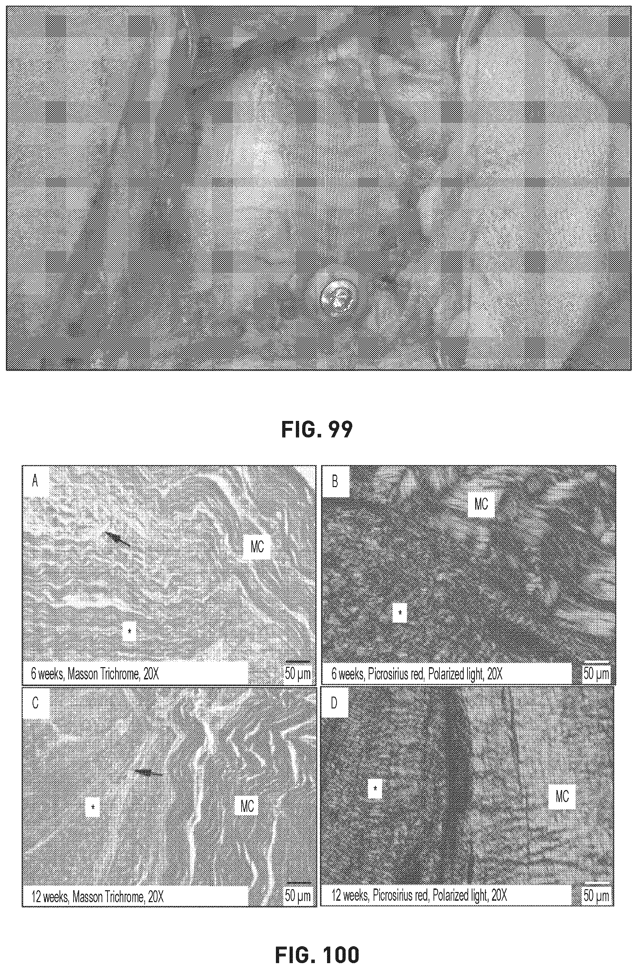

FIG. 99 shows the results of the preliminary evaluation of the efficacy and local tissue response in a sheep model, specifically an intraoperative picture of the bioabsorbable textile (Design 1) implanted in an ovine infraspinatus tendon model.

Example 16: Preliminary evaluation of the efficacy and local tissue response in a sheep model

Sheep were selected for this pilot study because of the similarities to humans in terms of healing rate and shoulder anatomy. Ovine models are well established and accepted in rotator cuff research (Coleman at al., 2003). Two animal studies were performed, one with leno-weave bioabsorbable textiles and one with knitted textiles.

The study with leno-weave bioabsorbable textile of Table 1 has been conducted based on ISO 10993-6. In 8 sheep, the infraspinatus tendon of the right leg was surgically released by two third of its width and with a full- thickness lesion. Two modified Mason-Allen sutures (at a minimum) were preplaced in the tendon at transection using 2 Fiber Wire. The bone at the detached insertion was decorticated using a nitrogen powered 4 mm burr. A minimum of 3 holes were drilled into the proximal humerus (using a 2 mm bit). Sterile saline was used for irrigation during drilling. The ends of the Fiber Wire were tunneled through these holes. At this point, 4 animals were randomly assigned to the Repair-only group (Group 1) and the other 4 animals to the Augmentation group (Group 2). For animals assigned to Group 1, no further treatment of the repair site was conducted; the tendon repair was completed. For animals assigned to Group 2, the bioabsorbable textile (Design 1) was affixed to the repaired tendon and anchored appropriately to the humerus. Two modified Mason-Allen sutures (0 Fiber Wire) were preplaced through the augmentation device and the tendon. An additional hole (burr size 2 mm) was drilled into the proximal humerus and a cortical bone screw and spiked washer was used to affix the distal end of the augmentation device to the bone. The preplaced Mason-Allen sutures were tied to allow for appropriate tension of the augmentation device over the repaired tendon (FIG. 99). Recovery from the anesthesia took place in the animal housing. Each animal was placed in a suspension system prior to recovery from anesthesia. Each animal was placed in the suspension system so that the animal was slightly elevated above the ground or was able to toe touch but was unable to bear full body weight on any limb for 14 days post-op.

At 6 and 12 weeks ($\pm$ 3 days) post-surgery, two animals per each group, arbitrarily selected, were euthanized to evaluate healing of repair site, local tissue response and degradation of the textile. The tendons and repair site were macroscopically observed to determine if they were intact and whether obvious relative movements had occurred. Absorption and degradation of the textile was scored according to defined criteria; efficacy of healing was addressed using pathologist defined criteria. For microscopic evaluation, tendon and repair site, as well as right and left axillary lymph nodes were collected and adequately treated for sectioning and histological analysis. The tissue characteristics observed were neovascularization, fibrosis, fatty infiltrate and necrosis, while the monitored cell types included polymorphonuclear cells, lymphocytes, plasma cells, macrophages and giant cells. Tissue and cellular alterations were graded according to severity (0-4) based on the scoring scheme in Annex E of ISO 10993-6. Moreover, tissue sections from the regional draining lymph nodes were evaluated for textile particles.

All animals survived to scheduled necropsy. At necropsy, all repair sites were macroscopically normal. Microscopically, the local reaction to the bioabsorbable textile at both 6- and 12-weeks post- surgery was consisted of minimal to mild numbers of macrophages and multinucleated giant cells, minimal to mild neovascularization, severe fibrosis/fibroplasia, and minimal fatty infiltrate. Inflammatory cells were almost exclusively found adjacent to suture and/or textile material. There was abundant extracellular textile material in all implant sites at 6 weeks post-surgery and significantly decreased until no extracellular textile material could be observed at 12 weeks post- surgery. Microscopic findings in the left and right axillary lymph nodes in test and control animals at either 6- or 12-weeks post-surgery were similar, and no adverse reactions were observed. There was no evidence of textile material within the microscopically examined axillary lymph node sections at either 6- or 12-weeks post-surgery.

FIG. 100 shows the results of the preliminary evaluation of the efficacy and local tissue response in a sheep model, specifically histological pictures of Group 2 at 6 weeks (A. Masson Trichrome and B. Picrosirius red under polarized light) and at 12 weeks (C. Masson Trichrome and D. Picrosirius red under polarized light). 20X Magnification. Scale bar: 50 μm.

Cellular homing as well as newly-formed extracellular collagenous matrix could be observed in the textile-augmented animals (Group 2) already at 6 weeks (Fig. 100A - Fig. 100B) with an increasing trend visible at 12 weeks (Fig. 100C - Fig. 100D). The final phase of healing, when tissue remodeling takes place and immature collagen is replaced with mature collagen, was still underway and could only be appreciated at longer timepoints.

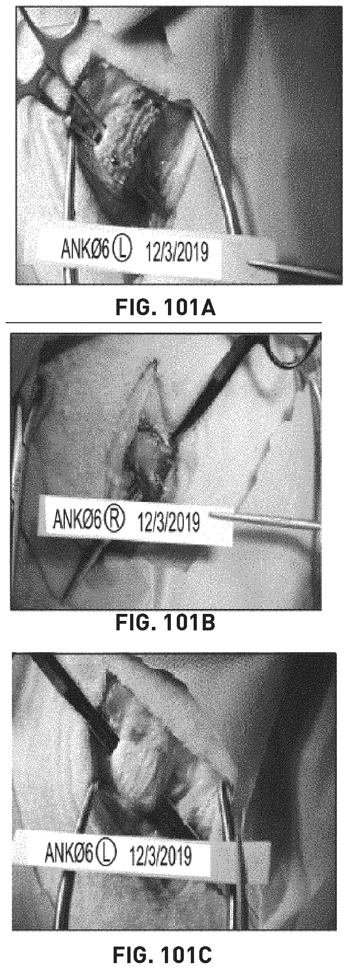

FIG. 101A shows the results of the preliminary evaluation of the efficacy and local tissue response in a sheep model, specifically an intraoperative picture of the bioabsorbable textile implanted in an ovine infraspinatus tendon disruption model.

The study with knitted bioabsorbable textiles of Table 1 has been conducted using LD2 in double layer configuration (Treatment 1) and LD2 in double layer configuration with a nonwoven pad (Treatment 2) based on HA benzyl ester between the two layers. Eight (8) animals were prepared for surgery and a bilateral combed fenestration (tendon disruption) of the infraspinatus tendons will be performed, the test articles described above were implanted in an overlay fashion on top of the disrupted tendon according to van Kampen et al, Muscles, Ligaments and Tendons Journal 2013. The fenestrations were made parallel to the infraspinatus tendon fibers. The tendon was not released from the humeral footprint. This "combing" procedure occurred only one time resulting in 4 fenestrations along the long axis of the tendon. Two pilot holes were drilled into the humerus along the distal edge of the infraspinatus tendon. Two ConMed CrossFT PEEK anchors loaded with Hi-Fi tape were deployed into the holes. The bioabsorbable textile was placed on top of the infraspinatus tendon and humeral footprint and sutured along the distal, lateral, medial and proximal edges using the Hi-Fi tape from each of the suture anchors. After the implantation, the animals were survived either until 6-weeks (+/- 4 days) or 12-weeks (+/- 4 days). Regeneten was used as a positive control group, and no augmentation was used as a negative control group (FIG. 101A and FIG. 101B).

FIG. 101B shows the results of the preliminary evaluation of the efficacy and local tissue response in a sheep model, specifically an intraoperative picture of the Regeneten implanted in an ovine infraspinatus tendon disruption model.

FIG. 101C shows the results of the preliminary evaluation of the efficacy and local tissue response in a sheep model, specifically an intraoperative picture of the no augmentation (Control) in an ovine infraspinatus tendon disruption model.

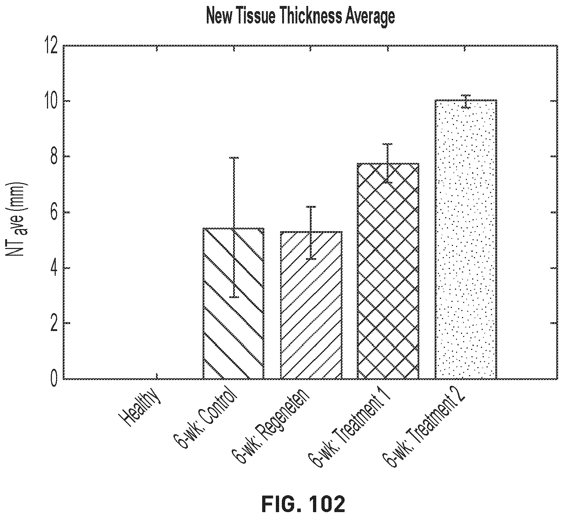

FIG. 102 shows the results of the preliminary evaluation of the efficacy and local tissue response in a sheep model, specifically the tendon thickness analysis at 6 weeks.

Results of overlay model: All explanted samples exhibited new tissue growth at 6-weeks, while the knitted textile treated tendons (Treatment 1 and 2) exhibited significantly more new tissue thickness at 6-weeks as compared to the negative control and Regeneten (FIG. 102).

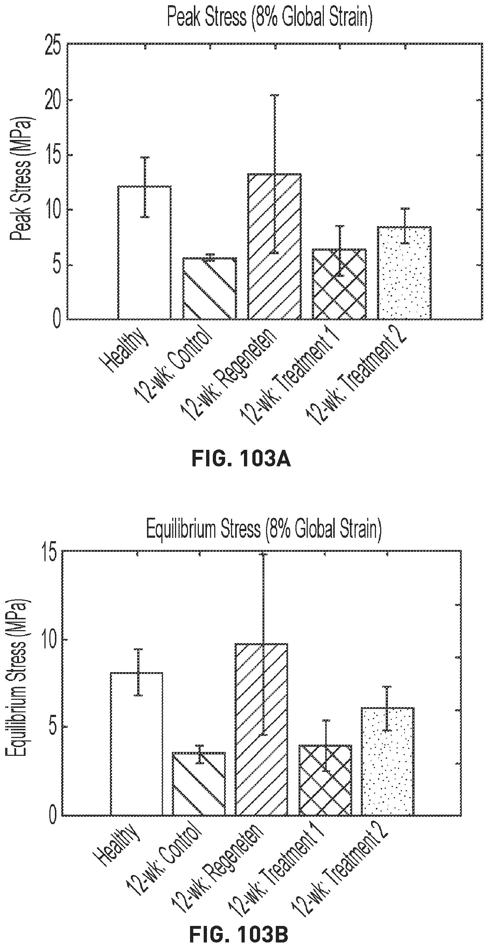

FIG. 103A shows the results of the preliminary evaluation of the efficacy and local tissue response in a sheep model, specifically the Peak Stress (8% Global Strain) analysis at 12 weeks.

At 12-weeks, knitted textile test articles (Treatment 1 and 2) exhibited improved Peak Stress (8% global strain) and Equilibrium Stress (8% global strain) as compared the control samples, and comparable values to Regeneten (Fig. 103).

FIG. 103B shows the results of the preliminary evaluation of the efficacy and local tissue response in a sheep model, specifically the Equilibrium Stress (8% Global Strain) analysis at 12 weeks.

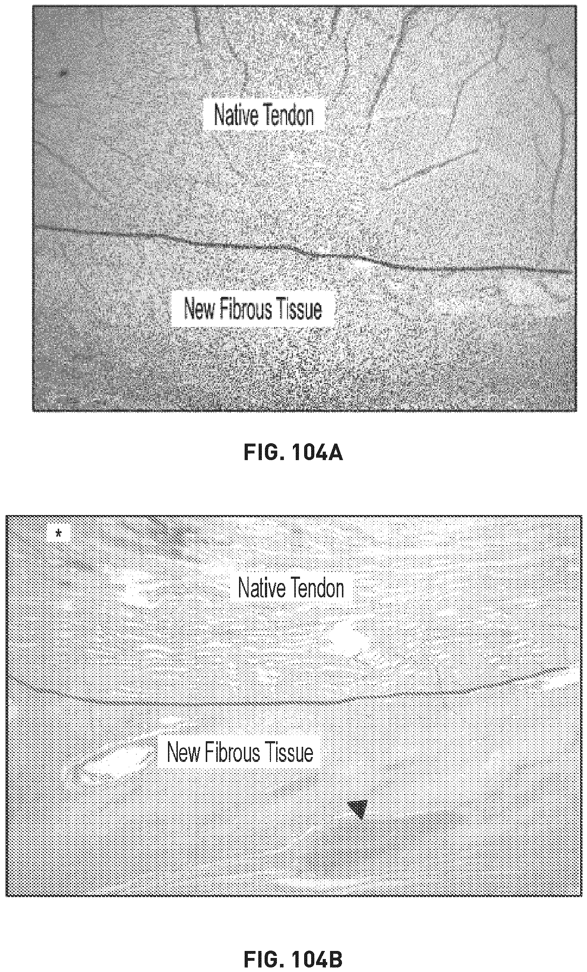

FIG. 104A shows the results of the preliminary evaluation of the efficacy and local tissue response in a sheep model, specifically the histological Overview after Regeneten in Ovine Tendon Disruption Model after 12 weeks.

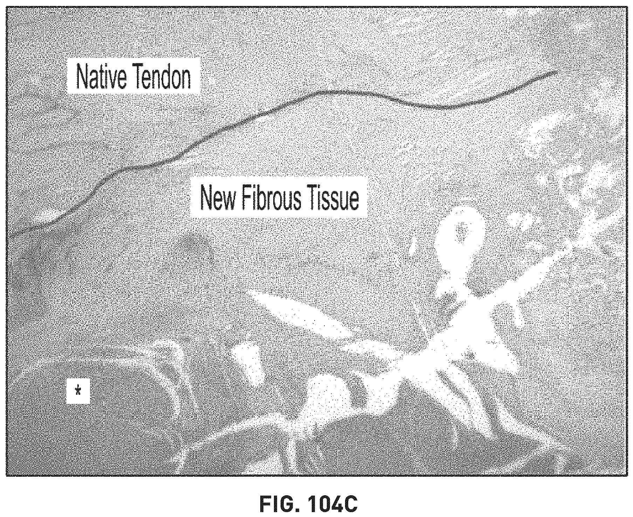

Histologically, the knitted textile test articles (Treatment 1 and 2) and Regeneten samples elicited similar biological responses, and both displayed a mild trend towards increased neo-vascularization and decreased inflammation, as compared to control animals at 6 weeks. There was an overall increase in collagen fiber organization in the knitted textile treatments from the 6-week timepoint to the 12-week timepoint. By 12-weeks, the performance of the knitted textile test articles (Treatment 1 and 2) were similar to Regeneten characterized primarily by degree of collagen organization, cellularity and the presence of Sharpey fibers. (FIG. 104A, FIG. 104B, FIG. 104C).

Example 17: Multi-layered Structure

Described herein is a method to incorporate differential mechanical and morphological properties into a single composite textile. In this exemplary embodiment, a 2.5 cm x 2.5 cm sheet of High Density Single Bar (HD1) warp-knitted textile and a 2.5 cm x 2.5 cm sheet of non-woven pad based on HA benzyl ester are bonded together to form a single composite multi-layered bioabsorbable textile. The HD1 textile is placed on a foil release layer, then dabbed with a lint-free laboratory wipe soaked with dimethyl sulfoxide (DMSO). The nonwoven pad based on HA benzyl ester is then placed on top of the HD1 textile and covered with a second foil release layer. The entire multi-layered structure is then subjected to heat and pressure by placing in a FS-205 Impulse Sealer and activating with approximately 50 N of force for 5-10 seconds. The composite textiles are then removed and dried overnight in a vacuum oven to remove any residual DMSO.

FIG. 104B shows the results of the preliminary evaluation of the efficacy and local tissue response in a sheep model, specifically the histological Overview after Treatment -1 in Ovine Tendon Disruption Model after 12 weeks.

FIG. 104C shows the results of the preliminary evaluation of the efficacy and local tissue response in a sheep model, specifically the histological Overview after Treatment -2 in Ovine Tendon Disruption Model after 12 weeks.

Exemplarily Embodiments

Exemplarily Embodiments

Embodiment 1: An apparatus for simultaneous delivery of a fastener and an implant, the apparatus comprising: an elongated body extending from a body proximal end to a body distal end, the body defining a body proximal opening through the body proximal end, a body recess in a peripheral surface of the body at point that is offset from the body distal end, and a body passage extending between the body proximal opening and a body distal opening within the body recess; one or more spikes each having a spike proximal end coupled to the body distal end, and a spike distal end extending from the body distal end, the spike proximal end having a transverse dimension that is smaller than a transverse dimension of the body distal end such that a shoulder is defined at the spike proximal end, the shoulder configured to abut a head of a fastener received over the spike; a hub having a hub proximal end and a hub distal end, the hub configured to receive the proximal end of the body through the hub distal end; a handle coupled to the hub proximal end; an elongated pusher having a pusher proximal end and a pusher distal end, the pusher disposed in the body passage such that the pusher distal end is disposed beyond the body distal opening; a slider coupled to the pusher proximal end, the slider configured to move longitudinally relative to the handle from a retracted position to a deployed position to move the pusher distal end longitudinally away from the body distal opening and laterally outward from the body.

Embodiment 2: The apparatus of embodiment 1, where the one or more spikes comprise a pair of spikes, where each of the pair of spikes are parallel to and laterally spaced from the other of the pair of spikes.

Embodiment 3: The apparatus of any of embodiments 1-2, where the hub is coupled in fixed relation to the body, and the handle is rotatable relative to the hub to cause the pusher distal end to rotate relative to the body.

Embodiment 4: The apparatus of embodiment 3, where the apparatus is configured such that moving the slider longitudinally relative to the handle, or rotating the handle relative to the hub, will move the distal end of the pusher independent of the body distal end.

Embodiment 5: The apparatus of any of embodiments 1-4, where the body recess defines a sloped surface spaced from the body distal opening, and where the apparatus is configured such that, when the slider is moved from the retracted position to the deployed position, a flexible portion of the pusher contacts the sloped surface to cause the pusher distal end to extend laterally outward from the body.

Embodiment 6: The apparatus of any of embodiments 1-5, where the pusher comprises a roller coupled to the pusher distal end.

Embodiment 7: The apparatus of any of embodiments 1-6, further comprising: a seal assembly comprising: an annular seal body having a seal proximal end and a seal distal end, the seal body defining a seal passage extending through and between the seal proximal end and the seal distal end, and a tapered distal surface disposed around the seal passage at the seal distal end; a resilient membrane disposed at least around an interior periphery of the seal passage, the resilient membrane configured to cooperate with the body of the apparatus to substantially seal the seal passage when the body of the apparatus extends through the resilient membrane and the seal passage.

Embodiment 8: The apparatus of embodiment 7, where the elongated body of the apparatus is disposed through the resilient membrane of the seal and the seal passage with the seal distal end facing the body distal end.

Embodiment 9: A system comprising: an apparatus of embodiment 7; a cannula comprising a cannula distal end, a cannula proximal end configured to be coupled to the seal body, and a cannula passage extending through and between the cannula proximal end and the cannula distal end, the cannula further defining a cannula proximal surface tapered distally and inward toward the cannula passage, the cannula proximal surface configured to cooperate with the seal distal surface to provide a seal between the cannula proximal surface and the seal distal surface.

Embodiment 10: The system of embodiment 9, where an outer surface of the cannula includes one or more threads that are configured to engage soft tissue as the cannula is rotated.

Embodiment 11: The system of any of embodiments 9-10, where a proximal end of the cannula includes an enlarged portion that is configured to be engaged by a user to vary the depth and/or direction of the cannula.

Embodiment 12: The system of any of embodiments 9-11, where the cannula distal end defines a notch configured to permit a portion of the pusher to deflect radially outward through the notch.

Embodiment 13: The system of any of embodiments 9-12, further comprising: an obturator having an elongated body with a obturator proximal end and a tapered obturator distal end, the obturator body configured to extend through the cannula such that the obturator distal end extends distally beyond the cannula distal end to facilitate insertion of the cannula through soft tissue of a patient.

Embodiment 14: A caddy system comprising: a base defining one or more first fastener recesses; a lid coupled to the base and configured to be rotated relative to the base to selectively cover or expose the first fastener recess(es).

Embodiment 15: The caddy system of embodiment 14, where the base further defines one or more implant recesses each overlapping at least one of the one or more first fastener recesses, where the lid is configured to be rotated relative to the base to selectively cover or expose the implant.

Embodiment 16: The caddy system of embodiment 15, where the one or more implant recesses includes a substantially planar lower surface offset below a substantially planar upper surface of the base.

Embodiment 17: The caddy system of any of embodiments 14-16, further comprising: a flexible, fibrous implant disposed over at least one of the first fastener recesses; and a first fastener extending through the implant and into the at least one of the first fastener recesses; where the lid is rotatable, relative to the base, over the first fastener and at least a portion of the implant.

Embodiment 18: The caddy system of any of embodiments 14-17, where the body further defines one or more second fastener recesses, where the lid is configured to be rotated relative to the base to selectively cover or expose the second fastener recess(es).

Embodiment 19: The caddy system of embodiment 18, further comprising: a second fastener extending into at least one of the second fastener recesses; where the lid is rotatable, relative to the base, over the second fastener.

Embodiment 20: A method comprising: disposing a shield distal end of an apparatus of any of embodiments 1-9 adjacent a bone of a patient, where the slider is in the retracted position, where a fastener is disposed over the one or more spikes, and the fastener extends through a flexible, fibrous implant; inserting the fastener into the bone to secure a first portion of the implant to the bone; and moving the slider from the retracted position toward the deployed position to urge a second portion of the implant away from the body of the apparatus and toward a soft tissue structure of the patient.

Embodiment 21: A method comprising: inserting the elongated body of an apparatus of any of embodiments 1-4 through a cannula that is disposed through soft tissue of a patient, the cannula comprising a cannula distal end, a cannula proximal end configured to be coupled to the seal body, and a cannula passage extending through and between the cannula proximal end and the cannula distal end, the cannula further defining a cannula proximal surface tapered distally and inward toward the cannula passage, the cannula proximal surface configured to cooperate with the seal distal surface to provide a seal between the cannula proximal surface and the seal distal surface; where, during insertion, a seal assembly is disposed around the elongated body of the apparatus, the seal assembly comprising: an annular seal body having a seal proximal end and a seal distal end, the seal body defining a seal passage extending through and between the seal proximal end and the seal distal end, and a tapered distal surface disposed around the seal passage at the seal distal end; a resilient membrane disposed at least around an interior periphery of the seal passage, the resilient membrane configured to cooperate with the body of the apparatus to substantially seal the seal passage when the body of the apparatus extends through the resilient membrane and the seal passage.

Embodiment 22: The method of embodiment 21, further comprising: coupling the seal body to the proximal end of the cannula such that the seal distal surface cooperates with the cannula proximal surface to provide a seal between the cannula proximal surface and the seal distal surface.

Embodiment 23: The method of embodiment 20, further comprising: after moving the slider from the retracted position toward the deployed position, rotating the handle relative to the hub to urge one or both of two lateral edges of the second portion of the implant toward the soft tissue structure.

Embodiment 24: The method of any of embodiments 20-23, further comprising: inserting a fastener through the second portion of the implant and into the soft tissue structure to couple the second portion of the implant to the soft tissue structure.

Embodiment 25: A method comprising: inserting the elongated body of the apparatus of any of embodiments 1-6 through the resilient membrane and the seal passage of the seal of embodiment 7 such that the seal distal end faces the body distal end.

Embodiment 26: The method of embodiment 25, further comprising: inserting, while the elongated body is disposed through the resilient membrane and seal passage, a fastener disposed over the one or more spikes through a flexible, fibrous implant.

Embodiment 27: The method of embodiment 25, further comprising: inserting the spikes of the apparatus through a fastener that is disposed through a flexible, fibrous implant.

Embodiment 28: A kit comprising: an apparatus of any of embodiments 1-8 or a system of any of embodiments 9-13; and a caddy system of any of embodiments 14-19.

Embodiment 29: A multicomponent suture comprising: fasteners for coupling an implant to tissue (e.g., soft-tissue and/or bone), fabric-like implants, and assemblies with fasteners pre-loaded with implants. The present implants generally comprise at least one flexible, fibrous layer that is substantially planar in a flattened state. In some embodiments of the present assemblies for delivery of a fastener, the assembly comprises fastener cartridge, a fibrous implant wrapped around a portion of the cartridge, a fastener extending through the implant, and an elongated shield disposed around the implant and the cartridge such that the implant is retained between the cartridge and the shield. Kits comprise a plurality of fasteners pre-loaded with implants. Some of the present kits also include one or more of the present fastener-delivery apparatuses or tools; for example, a plurality of pre-loaded fasteners with a single, reloadable tool; a plurality of tools each pre-loaded with a fastener that is pre-loaded with an implant; and/or a plurality of cartridges each pre-loaded with a fastener that is pre-loaded with an implant, and a common tool for use with the cartridges. A first substantially elongate suture portion having a first cross-sectional profile, said first substantially elongate suture portion including a first substantially bioabsorbable material; and an implant delivery tool comprising a shaft a channel therein, the channel configured to receive the BFT coupled with the bone fastener and implant therethrough, such that the BFT is configured to secure the implant about the targeted area via passing through a distal opening of the implant delivery tool to insert the bone fastener to a bone with the implant located therebetween. A second substantially elongate suture portion having a second cross-sectional profile, said second substantially elongate suture portion including a second substantially non-bioabsorbable material, said first and second substantially elongate suture portions being coupled to one another at respective mutually adjacent ends thereof so as to form an integrated suture having a first region that is substantially bioabsorbable and a second region that is substantially non-bioabsorbable.

Embodiment 30: A multicomponent suture as defined in embodiment 29 wherein said first substantially elongate suture portion has a first cross-sectional profile having a first aspect ratio, said first aspect ratio being substantially constant with respect to a longitudinal axis of said first substantially elongate suture portion, and wherein said second cross-sectional suture portion has a second cross-sectional profile having a second aspect ratio, said second aspect ratio being substantially constant with respect to a longitudinal axis of said second substantially elongate suture portion.

Embodiment 31: A multicomponent suture as defined in embodiment 30 wherein said first and second aspect ratios are related to one another by multiplicative factor of at least about 2.

Embodiment 32: A multicomponent suture as defined in embodiment 30 wherein said first and second aspect ratios are related to one another by multiplicative factor of at least about 5.

Embodiment 33: A multicomponent suture as defined in embodiment 30 wherein said first and second aspect ratios are related to one another by multiplicative factor of at least about 10.

Embodiment 34: A multicomponent suture as defined in embodiment 30 where at least one of said first and second cross-sectional profiles is substantially circular.

Embodiment 35: A multicomponent suture as defined in embodiment 30 where at least one of said first and second cross-sectional profiles is substantially elliptical.

Embodiment 36: A multicomponent suture as defined in embodiment 30 where at least one of said first and second cross-sectional profiles is substantially rectangular.

Embodiment 37: A multicomponent suture as defined in embodiment 29 wherein at least one of said first and second suture portions comprises a monofilament material.

Embodiment 38: A multicomponent suture as defined in embodiment 29 wherein at least one of said first and second suture portions comprises a braided textile material.

Embodiment 39: A multicomponent suture as defined in embodiment 29 wherein said first substantially elongate portion comprises a further substantially non-bioabsorbable material.

Embodiment 40: A multicomponent suture as defined in embodiment 29 wherein said first suture portion exhibits a color that is substantially different from a color of said second suture portion.

Embodiment 41: A multicomponent suture as defined in embodiment 29 wherein said first suture portion embodies a surface texture that is substantially different from a surface texture of said second suture portion.

Embodiment 42: A method of providing a medical professional with a suture comprising dispatching a suture assembly to a medical professional, said suture assembly being configured and adapted for piercing a first region of a patient's soft tissue with a needle, said needle being coupled to a first end of a suture, said suture including a first suture portion relatively proximal to said needle and a second suture portion relatively distal to said needle, said first and second suture portions being coupled to one another at a mutual coupling location thereof piercing a second region of said patient's soft tissue with said needle; drawing said suture through said first and second regions of said patient's soft tissue until said mutual coupling point enters and subsequently exits said first and second soft tissue portions; severing said suture at a point within said second suture portion; discarding said first suture portion including said needle, said coupling region and a portion of said second suture portion; and restraining said first and second soft tissue regions with said second suture portion.

Embodiment 43: A method of providing a medical professional with a suture as defined in embodiment 29 wherein said second suture portion exhibits a cross-sectional profile having an aspect ratio of at least 0.1.

Embodiment 44: A method of providing a medical professional with a suture as defined in embodiment 29 wherein said second suture portion exhibits a cross-sectional profile having an aspect ratio of at least 0.5.

Embodiment 45: A multicomponent suture comprising: a first portion disposed adjacent one end of said multicomponent suture, said first portion being substantially bioabsorbable; a second portion disposed adjacent another end of said multicomponent suture, said second portion being substantially non-bioabsorbable; a coupling device, said coupling device being disposed between said first and second suture portions, said coupling device being substantially permanently coupled between said first and second portions so as to maintain said first and second portions in proximity to one another until at least one of said first and second portions is severed.

Embodiment 46: A multicomponent suture as defined in embodiment 45 wherein said coupling device comprises a barbed coupling device.

Embodiment 47: A multicomponent suture as defined in embodiment 46 wherein said barbed coupling device comprises a plurality of barbs, at least one of said plurality of barbs being mechanically deformed to effect said substantially permanent coupling between said first and second portions.

Embodiment 48: A multicomponent suture as defined in embodiment 46 wherein said barbed coupling device comprises a plurality of barbs, at least one of said plurality of barbs being thermally deformed to effect said substantially permanent coupling between said first and second portions.

Embodiment 49: An assembly for delivery of a fastener, the assembly comprising: a fastener cartridge having a body with a proximal end, and a distal end, the proximal end configured to be removably coupled to a distal end of an apparatus to deliver a fastener coupled to the cartridge, the cartridge comprising: a spike having a spike proximal end coupled to the distal end of the cartridge, and a spike distal end extending from the distal end of the cartridge, the spike proximal end having a transverse dimension that is smaller than a transverse dimension of the distal end of the cartridge such that a shoulder is defined at the spike proximal end, the shoulder configured to abut a head of a fastener received over the spike; a fastener received over the spike, the fastener comprising: an elongated shaft having a shaft length extending from a shaft proximal end to a shaft distal end, and defining an internal passage extending along the shaft length; an enlarged head coupled to the proximal end of the shaft and abutting the shoulder of the cartridge; a plurality of outriggers extending from the enlarged head in a direction away from the internal passage; and a plurality of first barbs each extending along a portion of the shaft length, each first barb having a leading edge and trailing edge spaced from the proximal end of the shaft, the leading edge of each first barb being disposed between the trailing edge of that first barb and the distal end of the shaft; and where the shaft, head, outriggers, and first and second barbs are defined by a unitary piece of polymer; and a flexible, fibrous implant having a first end edge, a second end edge, and a pair of lateral edges extending between the first and second end edges, where the implant is substantially planar when in a flattened state in which the first end edge is separated from the second end edge under a first tension of 5 Newtons (N) applied perpendicular to each of the end edges, and in which the lateral edges are separated from each other under a tension of 5 N applied perpendicular to each of the lateral edges; an elongated, tubular shield disposed over the implant, at least a portion of the fastener, and at least a portion of the cartridge; where the shaft extends through the implant at a point that is closer to the first end edge than to the second end edge, a first portion of the implant extends proximally around at least a portion of the cartridge, and the first portion of the implant is disposed between the cartridge and the shield.

Embodiment 50: The assembly of embodiment 49, where the fastener further comprises: a plurality of second barbs that are unitary with the shaft, each of the second barbs extending along a portion of the shaft length, each of the second barbs having a leading edge and a trailing edge disposed between the head and the trailing edges of the first barbs, the leading edge of each second barb being disposed between the trailing edge of that second barb and the distal end of the shaft.

Embodiment 51: The assembly of embodiment 50, where the shaft of the fastener extends through the implant such that the trailing edge of each of the second barbs are disposed on a first side of the implant, and at least a portion of the leading edges of each of the first barbs are disposed on a second side of the implant.

Embodiment 52: The assembly of any of embodiments 49-51, where the implant comprises a woven layer.

Embodiment 53: The assembly of embodiment 52, where the implant further comprises a nonwoven layer coupled to the woven layer.

Embodiment 54: The assembly of embodiment 53, where the nonwoven layer is stitched to the woven layer.

Embodiment 55: The assembly of any of embodiments 49-54, where the implant further comprises at least one suture leg coupled to the implant at a point that is closer to the second end edge than to the first end edge, the suture leg having a free portion that is configured to extend beyond the second end edge.

Embodiment 56: The assembly of embodiment 55, where the implant comprises: a first suture leg coupled to the implant at a first point that is closer to the second edge than to the first end edge, the first point being closer to a first one of the lateral edges than to a second one of the lateral edges, the first suture leg having a first free portion that is configured to extend beyond the second end edge.

Embodiment 57: The assembly of embodiment 56, where the implant comprises: a second suture leg coupled to the implant at a second point that is closer to the second edge than to the first end edge, the second point being closer to the second one of the lateral edges than to the first one of the lateral edges, the second suture leg having a second free portion that is configured to extend beyond the second end edge.

Embodiment 58: The assembly of any of embodiments 49-56, where the polymer of the fastener is not bioresorbable.

Embodiment 59: The assembly of any of embodiments 49-58, where the plurality of first barbs of the fastener are disposed at equiangular intervals around the cross-sectional perimeter of the shaft.

Embodiment 60: The assembly of any of embodiments 49-59, where the plurality of first barbs of the fastener consists of four barbs.

Embodiment 61: The assembly of any of embodiments 2-60, where the plurality of second barbs of the fastener are disposed at equiangular intervals around the cross-sectional perimeter of the shaft.

Embodiment 62: The assembly of any of embodiments 2-61, where the plurality of second barbs of the fastener consists of four barbs.

Embodiment 63: The assembly of any of embodiments 1-62, where at least one of the first barbs of the fastener is not radially aligned with any of the outriggers.

Embodiment 64: The assembly of embodiment 63, where at least two of the first barbs are not radially aligned with any of the outriggers.

Embodiment 65: The assembly of embodiment 64, where each of the first barbs is not radially aligned with any of the outriggers.

Embodiment 66: The assembly of any of embodiments 50-65, where at least one of the second barbs of the fastener is not radially aligned with any of the outriggers.

Embodiment 67: The assembly of embodiment 66, where at least two of the second barbs are not radially aligned with any of the outriggers.

Embodiment 68: The assembly of embodiment 67, where each of the second barbs is not radially aligned with any of the outriggers.

Embodiment 69: The assembly of any of embodiments 49-62 and 66-68, where each of the first barbs of the fastener is radially aligned with one of the outriggers.

Embodiment 70: The assembly of any of embodiments 49-69, where at least a portion of the leading edge of each of the first barbs of the fastener is disposed at first barb angle relative to the central, longitudinal axis of the shaft.

Embodiment 71: The assembly of any of embodiments 49-70, where an outer surface of the shaft of the fastener defines a tapered section having an first outer transverse dimension at the shaft distal end and tapering toward the shaft proximal end to a second outer transverse dimension that is larger than the first outer transverse dimension.

Embodiment 72: The assembly of embodiment 71, where the tapered section of the shaft tapers at a first taper angle relative to a central, longitudinal axis of the shaft, and the first taper angle is substantially equal to the first barb angle.

Embodiment 73: The assembly of any of embodiments 50-72, where at least a portion of the leading edge of each of the second barbs of the fastener is disposed at second barb angle relative to the central, longitudinal axis of the shaft, and the second barb angle is substantially equal to the first barb angle.

Embodiment 74: The assembly of any of embodiments 71-72, where the inner surface and outer surface of the fastener define an edge at the shaft distal end.

Embodiment 75: The assembly of any of embodiments 49-74, where each of the outrigger distal ends is, when measured parallel to a central, longitudinal axis of the shaft, closer to the shaft distal end and is at least a portion of the distal side of the head of the shaft.

Embodiment 76: The assembly of any of embodiments 49-75, where the plurality of outriggers are disposed at equiangular intervals around the head.

Embodiment 77: The assembly of embodiment 76, where the plurality of outriggers consists of four outriggers.

Embodiment 78: The assembly of any of embodiments 49-77, where each outrigger of the fastener tapers to an edge, a point, or both at its outrigger distal end.

Embodiment 79: The assembly of any of embodiments 49-78, where the internal passage of the fastener has a first inner transverse dimension at the shaft proximal end, a second inner transverse dimension at the shaft distal end, and the second inner transverse dimension is smaller than the first inner transverse dimension.

Embodiment 80: The assembly of embodiment 79, where the internal passage of the fastener tapers linearly from the shaft distal end toward the shaft proximal end.

Embodiment 81: The assembly of any of embodiments 49-80, where the shaft length of the fastener is from 9 mm to 15 mm.

Embodiment 82: The assembly of any of embodiments 49-81, where at least a portion of the shaft of the fastener has a transverse dimension of from 1.75 mm to 2.5 mm.

Embodiment 83: An assembly for delivery of a fastener, the assembly comprising: a fastener cartridge having a body with a proximal end, and a distal end, the proximal end configured to be removably coupled to a distal end of an apparatus to deliver a fastener coupled to the cartridge, the cartridge comprising: a pair of spikes each having a spike proximal end coupled to the distal end of the cartridge, and a spike distal end extending from the distal end of the cartridge, the spike proximal end having a transverse dimension that is smaller than a corresponding transverse dimension of the distal end of the cartridge body such that a shoulder is defined at the spike proximal end, the shoulder configured to abut a head of a fastener received over the spikes; a fastener received over the spikes, the fastener comprising: a first elongated shaft having a first shaft length extending from a first shaft proximal end to a first shaft distal end, and defining an internal first passage extending along the first shaft length; a second elongated shaft having a second shaft length extending from a second shaft proximal end to a second shaft distal end, and defining an internal second passage extending along the second shaft length; an enlarged head coupled to the proximal ends of the first shaft and the second shaft, the had abutting the shoulder of the cartridge; a plurality of first barbs each extending along a portion of the first shaft length, each first barb having a leading edge and trailing edge spaced from the proximal end of the first shaft, the leading edge of each first barb being disposed between the trailing edge of that first barb and the first shaft distal end a plurality of second barbs each extending along a portion of the second shaft length, each second barb having a leading edge and trailing edge spaced from the proximal end of the second shaft, the leading edge of each second barb being disposed between the trailing edge of that second barb and the second shaft distal end; and where the shafts, head, and barbs are defined by a unitary piece of polymer. a flexible, fibrous implant having a first end edge, a second end edge, and a pair of lateral edges extending between the first and second end edges, where the implant is substantially planar when in a flattened state in which the first end edge is separated from the second end edge under a first tension of 5 Newtons (N) applied perpendicular to each of the end edges, and in which the lateral edges are separated from each other under a tension of 5 N applied perpendicular to each of the lateral edges; an elongated, tubular shield disposed over the implant, at least a portion of the fastener, and at least a portion of the cartridge; where the at least one of the first and second shafts of the fastener extends through the implant at a point that is closer to the first end edge than to the second end edge, a first portion of the implant extends proximally around at least a portion of the cartridge, and the first portion of the implant is disposed between the cartridge and the shield.

Embodiment 84: The assembly of embodiment 83, where each of the first and second shafts of the fastener extends through the implant at a point that is closer to the first end edge than to the second end edge, a first portion of the implant extends proximally around at least a portion of the cartridge, and the first portion of the implant is disposed between the cartridge and the shield.

Embodiment 85: The assembly of any of embodiments 83-84 where the fastener further comprises: a plurality of third barbs each extending along a portion of the first shaft length, each of the third barbs having a leading edge and a trailing edge disposed between the head and the trailing edges of the first barbs, the leading edge of each third barb being disposed between the trailing edge of that third barb and the first shaft distal end; and a plurality of fourth barbs each extending along a portion of the second shaft length, each of the fourth barbs having a leading edge and a trailing edge disposed between the head and the trailing edges of the second barbs, the leading edge of each fourth barb being disposed between the trailing edge of that fourth barb and the second shaft distal end.

Embodiment 86: The assembly of embodiment 85, where the first shaft of the fastener extends through the implant such that the trailing edge of each of the third barbs are disposed on a first side of the implant, and at least a portion of the leading edges of each of the first barbs are disposed on a second side of the implant.

Embodiment 87: The assembly of embodiment 86, where the second shaft of the fastener extends through the implant such that the trailing edge of each of the fourth barbs are disposed on a first side of the implant, and at least a portion of the leading edges of each of the second barbs are disposed on a second side of the implant.

Embodiment 88: The assembly of any of embodiments 83-87, where the implant comprises a woven layer.

Embodiment 89: The assembly of embodiment 88, where the implant further comprises a nonwoven layer coupled to the woven layer.

Embodiment 90: The assembly of embodiment 89, where the nonwoven layer is stitched to the woven layer.

Embodiment 91: The assembly of any of embodiments 83-90, where the implant further comprises at least one suture leg coupled to the implant at a point that is closer to the second end edge than to the first end edge, the suture leg having a free portion that is configured to extend beyond the second end edge.

Embodiment 92: The assembly of embodiment 91, where the implant comprises: a first suture leg coupled to the implant at a first point that is closer to the second edge than to the first end edge, the first point being closer to a first one of the lateral edges than to a second one of the lateral edges, the first suture leg having a first free portion that is configured to extend beyond the second end edge.

Embodiment 93: The assembly of embodiment 92, where the implant further comprises: a second suture leg coupled to the implant at a second point that is closer to the second edge than to the first end edge, the second point being closer to the second one of the lateral edges than to the first one of the lateral edges, the second suture leg having a second free portion that is configured to extend beyond the second end edge.

Embodiment 94: The assembly of any of embodiments 83-92, where the polymer of the fastener is not bioresorbable.

Embodiment 95: The assembly of any of embodiments 83-94, where the plurality of first barbs of the fastener are disposed at equiangular intervals around the cross-sectional perimeter of the first shaft.

Embodiment 96: The assembly of any of embodiments 83-95, where the plurality of first barbs of the fastener consists of four barbs.

Embodiment 97: The assembly of any of embodiments 83-96, where the plurality of second barbs of the fastener are disposed at equiangular intervals around the cross-sectional perimeter of the second shaft.

Embodiment 98: The assembly of any of embodiments 83-97, where the plurality of second barbs of the fastener consists of four barbs.

Embodiment 99: The assembly of any of embodiments 83-98, where at least one of the third barbs of the fastener is not radially aligned with any of the first barbs.

Embodiment 100: The assembly of embodiment 99, where each of the third barbs of the fastener is not radially aligned with any of the first barbs.

Embodiment 101: The assembly of any of embodiments 83-100, where the plurality of third barbs of the fastener are disposed at equiangular intervals around the cross-sectional perimeter of the first shaft.

Embodiment 102: The assembly of any of embodiments 83-101, where the plurality of third barbs consists of four barbs.

Embodiment 103: The assembly of any of embodiments 83-102, where the plurality of fourth barbs are disposed at equiangular intervals around the cross-sectional perimeter of the second shaft.

Embodiment 104: The assembly of any of embodiments 83-103, where the plurality of fourth barbs consists of four barbs.

Embodiment 105: The assembly of any of embodiments 83-104, where at least one of the fourth barbs of the fastener is not radially aligned with any of the second barbs.

Embodiment 106: The assembly of embodiment 105, where each of the fourth barbs is not radially aligned with any of the second barbs.

Embodiment 107: The assembly of any of embodiments 83-106, where at least a portion of the leading edge of at least one of the first barbs of the fastener is disposed at first barb angle relative to the central, longitudinal axis of the first shaft.

Embodiment 108: The assembly of any of embodiments 83-107, where at least a portion of the leading edge of at least one of the third barbs of the fastener is disposed at a second barb angle relative to the central, longitudinal axis of the first shaft.

Embodiment 109: The assembly of embodiment 108, where the second barb angle is substantially equal to the first barb angle.

Embodiment 110: The assembly of any of embodiments 83-109, where an outer surface of the first shaft of the fastener defines a tapered section having an first outer transverse dimension at the first shaft distal end and tapering toward the first shaft proximal end to a second outer transverse dimension that is larger than the first outer transverse dimension.

Embodiment 111: The assembly of embodiment 110, where the tapered section of the first shaft of the fastener tapers at a first taper angle relative to a central, longitudinal axis of the first shaft, and the first taper angle is substantially equal to the first barb angle.

Embodiment 112: The assembly of any of embodiments 110-111, where the inner surface and outer surface of the first shaft of the fastener define an edge at the first shaft distal end.

Embodiment 113: The assembly of any of embodiments 83-112, where at least a portion of the leading edge of at least one of the second barbs of the fastener is disposed at third barb angle relative to the central, longitudinal axis of the second shaft.

Embodiment 114: The assembly of any of embodiments 83-113, where at least a portion of the leading edge of at least one of the fourth barbs of the fastener is disposed at a fourth barb angle relative to the central, longitudinal axis of the second shaft.

Embodiment 115: The assembly of embodiment 114, where the fourth barb angle is substantially equal to the third barb angle.

Embodiment 116: The assembly of any of embodiments 83-115, where an outer surface of the second shaft of the fastener defines a tapered section having an first outer transverse dimension at the second shaft distal end and tapering toward the second shaft proximal end to a second outer transverse dimension that is larger than the first outer transverse dimension.

Embodiment 117: The assembly of embodiment 116, where the tapered section of the second shaft of the fastener tapers at a second taper angle relative to a central, longitudinal axis of the second shaft, and the second taper angle is substantially equal to the third barb angle.

Embodiment 118: The assembly of any of embodiments 116-117, where the inner surface and outer surface of the second shaft define an edge at the second shaft distal end.

Embodiment 119: The assembly of any of embodiments 83-118, where the first and second internal passages of the fastener each has a first inner transverse dimension at the respective shaft proximal end, a second inner transverse dimension at the respective shaft distal end, and each second inner transverse dimension is smaller than the corresponding first inner transverse dimension.

Embodiment 120: The assembly of embodiment 119, where each of the first and second internal passages tapers linearly from the respective shaft distal end toward the respective shaft proximal end.

Embodiment 121: The assembly of any of embodiments 83-120, where the first shaft length of the fastener and the second shaft length of the fastener are each between 9 mm and 15 mm.

Embodiment 122: The assembly of embodiment 121, where the second shaft length is substantially equal to the first shaft length.

Embodiment 123: The assembly of any of embodiments 83-122, where the at least a portion of the first shaft of the fastener and at least a portion of the second shaft of the fastener each has an outer transverse dimension of from 1.75 mm to 2.5 mm.

Embodiment 124: A method utilizing an assembly of any of embodiments 49-82 and an apparatus having a distal end coupled to a proximal end of the fastener body, the method comprising: inserting the shaft of the fastener into a first portion of tissue of a patient; retracting the shield to a position that permits the second end edge of the implant to exit the shield; spreading the implant along a second portion of tissue of the patient; coupling the implant to the second portion of tissue; decoupling the cartridge from the fastener such that the spike of the cartridge is removed from the internal passage of the shaft of the fastener.

Embodiment 125: The method embodiment 124, where the first portion of tissue comprises bone.

Embodiment 126: The method of any of embodiments 76-125, where the second portion of tissue comprises soft tissue.

Embodiment 127: The method of embodiment 126, where the soft tissue comprises tendon.

Embodiment 128: The method of any of embodiments 76-127, as depending from embodiment 56 or embodiment 57, where spreading the implant comprises pulling the first suture leg laterally away from the fastener.

Embodiment 129: The method of embodiment 129, as depending from embodiment 57, where spreading the implant further comprises pulling the second suture leg laterally away from the fastener.

Embodiment 130: The method of any of embodiments 128-129, where coupling the implant to the second portion of tissue comprises securing at least one of the suture legs to soft tissue.

Embodiment 131: A method utilizing an assembly of any of embodiments 83-123 and an apparatus having a distal end coupled to a proximal end of the fastener body, the method comprising: inserting the shafts of the fastener into a first portion of tissue of a patient; retracting the shield to a position that permits the second end edge of the implant to exit the shield; spreading the implant along a second portion of tissue of the patient; coupling the implant to the second portion of tissue; decoupling the cartridge from the fastener such that the spikes of the cartridge are removed from the internal passages of the shafts of the fastener.

Embodiment 132: The method embodiment 131, where the first portion of tissue comprises bone.

Embodiment 133: The method of any of embodiments 131-132, where the second portion of tissue comprises soft tissue.

Embodiment 134: The method of embodiment 133, where the soft tissue comprises tendon.

Embodiment 135: The method of any of embodiments 131-135, as depending from embodiment 92 or embodiment 93, where spreading the implant comprises pulling the first suture leg laterally away from the fastener.

Embodiment 136: The method of embodiment 135, as depending from embodiment 93, where spreading the implant further comprises pulling the second suture leg laterally away from the fastener.

Embodiment 137: The method of any of embodiments 135-136, where coupling the implant to the second portion of tissue comprises securing at least one of the suture legs to soft tissue.

Embodiment 138: A kit comprising: a plurality of assemblies of any of embodiments 49-123.

Embodiment 139: The kit of embodiment 138, further comprising: a plurality of any of the described soft-tissue fasteners.

Embodiment 140: The kit of any of embodiments 138-139, where the assemblies are sterile and sealed in a package.

Embodiment 141: The kit of embodiment 140, where the package comprises a tray.

Embodiment 142: A bioabsorbable textile for joint function restoration comprising polymeric yarns, wherein at least one of said polymeric yarns comprises a hyaluronic acid derivative.

Embodiment 143: The bioabsorbable textile according to embodiment 142, wherein said hyaluronic acid derivative is an ester.

Embodiment 144: The bioabsorbable textile according to embodiment 143, wherein said hyaluronic acid derivative is a benzyl ester.

Embodiment 145: The bioabsorbable textile according to embodiment 144, wherein the esterification degree of said hyaluronic acid derivative ranges between 80% to 100%.

Embodiment 146: The bioabsorbable textile according to embodiment 142, wherein the bioabsorbable textile is a knitted structure.

Embodiment 147: The bioabsorbable textile according to embodiment 142, wherein the bioabsorbable textile is a leno-weave structure.

Embodiment 148: The bioabsorbable textile according to embodiment 142, wherein said bioabsorbable textile is coated with an osteo-inductive and/or osteo-conductive material selected from the group consisting of hydroxyapatite, calcium phosphates, bone cements, demineralized bone matrix, titanium, magnesium, strontium, bioglasses, other resorbable glasses, and a combination thereof.

Embodiment 149: The bioabsorbable textile according to embodiment 142, wherein the bioabsorbable textile is added to a biological component selected from the group consisting of bone marrow aspirate, bone marrow aspirate concentrate, mesenchymal stem cells, bone marrow stem cells, adipose-derived stem cells, amniotic cells, micronized amniotic membrane, partially- or fully-digested tissue biopsies, aspirated adipose tissue, platelet rich plasma, autologous conditioned plasma, growth factors, and a combination thereof.

Embodiment 150: The bioabsorbable textile according to embodiment 142, wherein the at least one polymeric yam is characterized by: Linear mass density from about 30 tex to about 100 tex; a breaking strength in the range from about 1 N to about 100 N, and an extension at break from about 1% to about 30%.

Embodiment 151: The bioabsorbable textile according to embodiment 142, wherein the bioabsorbable textile is characterized by a volumetric and by a thickness swelling ratio in the range from about 0.1% to about 150%.

Embodiment 152: A multilayer structure for joint function restoration comprising at least one bioabsorbable textile according to embodiment 142.

Embodiment 153: The multilayer structure according to embodiment 152, comprising a knitted textile and a non-woven pad.

Embodiment 154: The multilayer structure according to embodiment 153, wherein the knitted textile is solvent-bonded to the non-woven pad.

Embodiment 155: The multilayer structure according to embodiment 153, wherein the knitted textile is thermally bonded to the non-woven pad.

Embodiment 156: The multilayer structure according to embodiment 153, wherein the knitted textile is mechanically attached to the non-woven pad.

Embodiment 157: The multilayer structure according to embodiment 152, comprising edges sealed by cauterization.

Embodiment 158: The multilayer structure according to embodiment 152, comprising at least one through-hole with a diameter of at least 100 $\mu$m.

Embodiment 159: A method of restoring joint function affected by partial thickness tears, small to medium full-thickness tears, acute and chronic/degenerative tears in patients in need thereof with the bioabsorbable textile according to embodiment 142 or with the multilayer structure according to embodiment 152, said method comprising: implanting in said patients said bioabsorbable textile or said multilayer structure with fastening means during surgical repair/augmentation procedures.

Embodiment 160: The method according to embodiment 159, wherein said surgical repair/augmentation procedures are carried out in combination with fixation tools during open, mini-open or arthroscopic procedures of joint tissue tears.

Embodiment 161: A composite medical textile, comprising a plurality of bioresorbable hyaluronan-based fibers and a plurality of non-resorbable fibers.

Embodiment 162: The composite medical textile of embodiment 161, wherein the bioresorbable hyaluronan-based fibers and the non-resorbable fibers are joined together by at least one selected from the group consisting of braided, knitted, adhered, intermeshed, weaved, interlocked, twisted, and heat set.

Embodiment 163: The composite medical textile of embodiment 161, wherein the hyaluronan-based fibers comprise at least one selected from the group consisting of hyaluronic acid, sodium hyaluronate, and esters of hyaluronic acid.

Embodiment 164: The composite medical textile of embodiment 163, wherein the esters of hyaluronic acid comprise benzyl esters of hyaluronic acid.

Embodiment 165: The composite medical textile of embodiment 161, wherein the medical textile comprises from 10 % to 98 % non-resorbable fibers, based on the weight of the non-resorbable fibers to the total weight of the non-resorbable suture fibers and bioresorbable hyaluronan-based polymer fibers.

Embodiment 166: The composite medical textile of embodiment 161, wherein the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

Embodiment 167: The composite medical textile of embodiment 161, further comprising bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxybutyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

Embodiment 168: The composite medical textile of embodiment 161, wherein the hyaluronan-based bioresorbable polymer fibers are prepared by at least one selected from the group consisting of ring spinning, air-jet spinning, open-end spinning, mule spinning, wet spinning, dry spinning, electrospinning, pneumatospinning, pultrusion, and extrusion.

Embodiment 169: The composite medical textile of embodiment 168, wherein the extrusion comprises at least one selected from the group consisting of solvent exchange extrusion, precipitation extrusion, phase exchange extrusion, and phase change extrusion.

Embodiment 170: The composite medical textile of embodiment 161, wherein bioresorbable hyaluronan-based fiber comprises at least one selected from the group consisting of mono- and multifilaments.

Embodiment 171: The composite medical textile of embodiment 161, wherein the composite medical textile has a diameter of from 25 $\mu$m to about 5000 $\mu$m.

Embodiment 172: The composite medical textile of embodiment 161, further comprising at least one active agent.

Embodiment 173: The composite medical textile of embodiment 172, wherein the active agent is an antimicrobial agent.

Embodiment 174: The composite medical textile of embodiment 173, wherein the antimicrobial agent comprises at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

Embodiment 175: The composite medical textile of embodiment 172, wherein the active agent is an analgesic.

Embodiment 176: The composite medical textile of embodiment 175, wherein the analgesic comprises at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine.

Embodiment 177: The composite medical textile of embodiment 172, wherein the active agent comprises a vasoconstrictive agent.

Embodiment 178: The composite medical textile of embodiment 177, wherein the vasoconstrictive agent comprises at least one selected from the group consisting of epinephrine, alpha-adrenoreceptor antagonists, vasopressin analogues, norepinephrine, phenylephrine, dopamine, dobutamine, serotonin agonists, and triptans.

Embodiment 179: The composite medical textile of embodiment 172, wherein at least one selected from the group consisting of the non-resorbable fibers and the bioresorbable hyaluronan-based fibers are wetted with the active agent.

Embodiment 180: The composite medical textile of embodiment 172, wherein at least one selected from the group consisting of the non-resorbable fibers and the bioresorbable hyaluronan-based fibers are coated with the active agent.

Embodiment 181: The composite medical textile of embodiment 172, wherein at least one selected from the group consisting of the non-resorbable fibers and the bioresorbable hyaluronan-based fibers have the active agent embedded within the fiber.

Embodiment 182: The composite medical textile of embodiment 161, where the composite medical textile is circular in cross section.

Embodiment 183: The composite medical textile of embodiment 161, wherein the composite medical textile is a tape and has a cross section comprising at least one selected from the group consisting of rounded rectangular, marquise, oblong and oval.

Embodiment 184: A method of making a medical textile, comprising the steps of: providing a plurality of non-resorbable fibers; providing a plurality of bioresorbable hyaluronan-based fibers; and, joining the non-resorbable fibers and the bioresorbable hyaluronan-based fibers into a composite medical textile.

Embodiment 185: The method of embodiment 184, wherein the medical textile comprises from 20 % to 80 % bioresorbable hyaluronan-based fibers, based on the total number of non-resorbable fibers and bioresorbable hyaluronan-based fibers.

Embodiment 186: The method of embodiment 184, wherein the composite medical textile further comprises an active agent, the active agent comprising at least one selected from the group consisting of antimicrobial agents, analgesic agents, and vasoconstrictive agents.

Embodiment 187: The method of embodiment 184, wherein the active agent is coated onto at least one selected from the group consisting of the bioresorbable hyaluronan-based fibers and the non-resorbable fibers.

Embodiment 188: The method of embodiment 187, wherein the coating step comprises at least one selected from the group consisting of a dip, brush, spray, or curtain coating process.

Embodiment 189: The method of embodiment 184, wherein the active agent is impregnated into at least one selected from the group consisting of the bioresorbable hyaluronan-based fibers and the non-resorbable fibers by passing the extruded fiber through a solution containing the active agent.

Embodiment 190: The method of embodiment 184, wherein the active agent is impregnated into at least one selected from the group consisting of the bioresorbable hyaluronan-based fibers and the non-resorbable fibers by co-extrusion, wherein a fiber precursor and the active agent are combined into a homogeneous mixture and co-extruded into a fiber.

Embodiment 191: The method of embodiment 184, wherein at least one selected from the group consisting of the bioresorbable hyaluronan-based fibers and the non-resorbable fibers are wetted with the active agent.

Embodiment 192: A method for repairing a portion of a mammalian body, comprising the steps of: providing a composite medical textile, comprising a plurality of non-resorbable fibers and a plurality of bioresorbable hyaluronan-based fibers; and, connecting the textile between two tissue portions of the mammalian body.

Embodiment 193: The method of embodiment 192, wherein the tissue portions comprise at least one selected from the group consisting of bony tissue and soft tissue.

Embodiment 194: The method of embodiment 192, wherein the textile is a suture, and further comprising the step of manipulating the suture in a suturing process to suture the tissue portions of the mammalian body.

Embodiment 195: The method of embodiment 192, further comprising the step of seeding the medical textile with stem cells.

Embodiment 196: A medical device comprising a plurality of bioresorbable hyaluronan-based fibers and a plurality of non-resorbable fibers.

Embodiment 197: The medical device of embodiment 196, wherein the medical device is an orthopedic attachment system which comprises at least one flexible connector comprising bioresorbable hyaluronan-based fibers joined with a plurality of non-resorbable fibers, and at least one orthopedic attachment device.

Embodiment 198: The medical device of embodiment 196, wherein the medical device comprises at least one selected from the group consisting of tubes, membranes, non-woven fabrics, gauzes, sponges, and sutures.

Embodiment 199: The medical device of embodiment 196, wherein the medical device comprises a tissue scaffold.

Embodiment 200: A soft-tissue fastener for coupling an implant to soft tissue, the soft-tissue fastener comprising: an elongated shaft having a shaft length extending from a shaft proximal end to a shaft distal end, and an internal surface defining an internal passage extending along the shaft length; an enlarged head coupled to the proximal end of the shaft, the enlarged head having a proximal side and a distal side; a plurality of outriggers each extending from the enlarged head in a direction away from the internal passage to an outrigger distal end; and a plurality of barbs each extending along a portion of the shaft length, each of the barbs having a leading edge and trailing edge spaced from the shaft proximal end, the leading edge of each barb being disposed between the trailing edge of that barb and the shaft distal end, where the trailing edge of a second one of the barbs is closer to the shaft proximal end than is the trailing edge of a first one of the barbs; where the shaft, head, outriggers, and barbs are defined by a unitary piece of polymer.

Embodiment 201: The soft-tissue fastener of embodiment 200, where the polymer is bioresorbable.

Embodiment 202: The soft-tissue fastener of any of embodiments 200-201, where the trailing edge of a third one of the barbs is closer to the shaft proximal end than is the trailing edge of the second one of the barbs.

Embodiment 203: The soft-tissue fastener of any of embodiments 200-202, where the plurality of barbs are disposed at equiangular intervals around the cross-sectional perimeter of the shaft.

Embodiment 204: The soft-tissue fastener of any of embodiments 200-203, where the plurality of barbs consists of three barbs.

Embodiment 205: The soft-tissue fastener of any of embodiments 200-204, where at least one of the barbs is not radially aligned with any of the outriggers.

Embodiment 206: The soft-tissue fastener of embodiment 205, where at least two of the barbs are not radially aligned with any of the outriggers.

Embodiment 207: The soft-tissue fastener of any of embodiments 200-206, where at least a portion of the leading edge of the first one of the barbs is disposed at first barb angle relative to the central, longitudinal axis of the shaft.

Embodiment 208: The soft-tissue fastener of embodiment 207, where at least a portion of the leading edge of the second one of the barbs is disposed at a second barb angle relative to the central, longitudinal axis of the shaft, and the second barb angle is smaller than the first barb angle.

Embodiment 209: The soft-tissue fastener of embodiment 208, as depending from embodiment 3, where at least a portion of the leading edge of the third one of the barbs is disposed at a third barb angle relative to the central, longitudinal axis of the shaft, and the third barb angle is smaller than the second barb angle.

Embodiment 210: The soft-tissue fastener of any of embodiments 200-209, where an outer surface of the shaft defines a tapered section having an first outer transverse dimension at the shaft distal end and tapering toward the shaft proximal end to a second outer transverse dimension that is larger than the first outer transverse dimension.

Embodiment 211: The soft-tissue fastener of embodiment 210, where the tapered section of the shaft tapers at a first taper angle relative to a central, longitudinal axis of the shaft, and the first taper angle is substantially equal to the first barb angle.

Embodiment 212: The soft-tissue fastener of any of embodiments 210-211, where the inner surface and outer surface define an edge at the shaft distal end.

Embodiment 213: The soft-tissue fastener of any of embodiments 200-212, where each of the outrigger distal ends is, when measured parallel to a central, longitudinal axis of the shaft, closer to the shaft distal end and is at least a portion of the distal side of the head of the shaft.

Embodiment 214: The soft-tissue fastener of any of embodiments 200-213, where the plurality of outriggers are disposed at equiangular intervals around the head.

Embodiment 215: The soft-tissue fastener of embodiment 214, where the plurality of outriggers consists of four outriggers.

Embodiment 216: The soft-tissue fastener of any of embodiments 200-215, where each outrigger tapers to an edge, a point, or both at its outrigger distal end.

Embodiment 217: The soft-tissue fastener of any of embodiments 200-216, where the internal passage has a first inner transverse dimension at the shaft proximal end, a second inner transverse dimension at the shaft distal end, and the second inner transverse dimension is smaller than the first inner transverse dimension.

Embodiment 218: The soft-tissue fastener of embodiment 217, where the internal passage tapers linearly from the shaft distal end toward the shaft proximal end.

Embodiment 219: The soft-tissue fastener of any of embodiments 200-218, where the shaft length is between 5 mm and 9 mm.

Embodiment 220: The soft-tissue fastener of any of embodiments 200-219, where the at least a portion of the shaft has an outer transverse dimension of from 1.25 mm to 2.0 mm.

Embodiment 221: A bone fastener for coupling an implant to bone, the bone fastener comprising: an elongated shaft having a shaft length extending from a shaft proximal end to a shaft distal end, and defining an internal passage extending along the shaft length; an enlarged head coupled to the proximal end of the shaft; a plurality of outriggers extending from the enlarged head in a direction away from the internal passage; and a plurality of first barbs each extending along a portion of the shaft length, each first barb having a leading edge and trailing edge spaced from the proximal end of the shaft, the leading edge of each first barb being disposed between the trailing edge of that first barb and the distal end of the shaft; and a plurality of second barbs each extending along a portion of the shaft length, each of the second barbs having a leading edge and a trailing edge disposed between the head and the trailing edges of the first barbs, the leading edge of each second barb being disposed between the trailing edge of that second barb and the distal end of the shaft; where the shaft, head, outriggers, and first and second barbs are defined by a unitary piece of polymer.

Embodiment 222: The bone fastener of embodiment 221, where the polymer is not bioresorbable.

Embodiment 223: The bone fastener of any of embodiments 221-222, where the plurality of first barbs are disposed at equiangular intervals around the cross-sectional perimeter of the shaft.

Embodiment 224: The bone fastener of any of embodiments 221-223, where the plurality of first barbs consists of four barbs.

Embodiment 225: The bone fastener of any of embodiments 221-224, where the plurality of second barbs are disposed at equiangular intervals around the cross-sectional perimeter of the shaft.

Embodiment 226: The bone fastener of any of embodiments 221-225, where the plurality of second barbs consists of four barbs.

Embodiment 227: The bone fastener of any of embodiments 221-226, where at least one of the first barbs is not radially aligned with any of the outriggers.

Embodiment 228: The bone fastener of embodiment 227, where at least two of the first barbs are not radially aligned with any of the outriggers.

Embodiment 229: The bone fastener of embodiment 228, where each of the first barbs is not radially aligned with any of the outriggers.

Embodiment 230: The bone fastener of any of embodiments 221-229, where at least one of the second barbs is not radially aligned with any of the outriggers.

Embodiment 231: The bone fastener of embodiment 230, where at least two of the second barbs are not radially aligned with any of the outriggers.

Embodiment 232: The bone fastener of embodiment 231, where each of the second barbs is not radially aligned with any of the outriggers.

Embodiment 233: The bone fastener of any of embodiments 221-226 and 31-33, where each of the first barbs is radially aligned with one of the outriggers.

Embodiment 234: The bone fastener of any of embodiments 221-233, where at least a portion of the leading edge of each of the first barbs is disposed at first barb angle relative to the central, longitudinal axis of the shaft.

Embodiment 235: The bone fastener of any of embodiments 221-234, where an outer surface of the shaft defines a tapered section having an first outer transverse dimension at the shaft distal end and tapering toward the shaft proximal end to a second outer transverse dimension that is larger than the first outer transverse dimension.

Embodiment 236: The bone fastener of embodiment 235, where the tapered section of the shaft tapers at a first taper angle relative to a central, longitudinal axis of the shaft, and the first taper angle is substantially equal to the first barb angle.

Embodiment 237: The bone fastener of any of embodiments 221-236, where at least a portion of the leading edge of each of the second barbs is disposed at second barb angle relative to the central, longitudinal axis of the shaft, and the second barb angle is substantially equal to the first barb angle.

Embodiment 238: The bone fastener of any of embodiments 235-236, where the inner surface and outer surface define an edge at the shaft distal end.

Embodiment 239: The bone fastener of any of embodiments 221-238, where each of the outrigger distal ends is, when measured parallel to a central, longitudinal axis of the shaft, closer to the shaft distal end and is at least a portion of the distal side of the head of the shaft.

Embodiment 240: The bone fastener of any of embodiments 221-239, where the plurality of outriggers are disposed at equiangular intervals around the head.

Embodiment 241: The bone fastener of embodiment 240, where the plurality of outriggers consists of four outriggers.

Embodiment 242: The bone fastener of any of embodiments 221-241, where each outrigger tapers to an edge, a point, or both at its outrigger distal end.

Embodiment 243: The bone fastener of any of embodiments 221-242, where the internal passage has a first inner transverse dimension at the shaft proximal end, a second inner transverse dimension at the shaft distal end, and the second inner transverse dimension is smaller than the first inner transverse dimension.

Embodiment 244: The bone fastener of embodiment 243, where the internal passage tapers linearly from the shaft distal end toward the shaft proximal end.

Embodiment 245: The bone fastener of any of embodiments 221-244, where the shaft length is from 9 mm to 15 mm.

Embodiment 246: The bone fastener of any of embodiments 221-245, where at least a portion of the shaft has a transverse dimension of from 1.75 mm to 2.5 mm.

Embodiment 247: An apparatus for delivery of a fastener, the apparatus comprising: an elongated body having a body length extending from a body proximal end to a body distal end; a spike having a spike proximal end coupled to the body distal end, and a spike distal end extending from the body distal end, the spike proximal end having a transverse dimension that is smaller than a transverse dimension of the body distal end such that a shoulder is defined at the spike proximal end, the shoulder configured to abut a head of a fastener received over the spike; a handle coupled to the proximal end of the body; and an elongated tubular shield having a shield proximal end and a shield distal end, the shield disposed around the body and movable between a retracted position in which the shield distal end is proximal to the shoulder, and an extended position in which the shield distal end extends past the spike distal end.

Embodiment 248: An apparatus for delivery of a fastener, the apparatus comprising: an elongated body having a body length extending from a body proximal end to a body distal end, the elongated body defining internal first and second body passages extending through and between the body proximal end and the body distal end, the first body passage having a central, longitudinal first axis, and the second body passage having a central, longitudinal second axis separated by the first axis by a distance that remains constant along the body length, the first body passage configured to receive a guidewire, and the second body passage configured to receive a trocar or rod; and a handle coupled to the proximal end of the body, the handle configured to permit access to the first and second body passages through the body proximal end.

Embodiment 249: The apparatus of embodiment 248, where the distal end of the body is configured to be coupled to a proximal end of a fastener cartridge that defines a longitudinal cartridge groove extending through and between the proximal end of the cartridge and a distal end of the cartridge and open to a lateral external surface of the cartridge, where the cartridge further comprises: a spike having a spike proximal end coupled to the distal end of the cartridge, and a spike distal end extending from the distal end of the cartridge, the spike proximal end having a transverse dimension that is smaller than a transverse dimension of the distal end of the cartridge such that a shoulder is defined at the spike proximal end, the shoulder configured to abut a head of a fastener received over the spike. where the proximal end of the cartridge is configured to be removably coupled to the distal end of the body such that the spike is coaxial with the cartridge passage, and the first axis of the first body passage extends through the cartridge groove.

Embodiment 250: The apparatus of embodiment 249, where the cartridge further comprises: a rod having a rod proximal end extending proximally from the proximal end of the cartridge, the proximal end of rod configured to be disposed in the second body passage of the body such that the cartridge is movable between a retracted position in which the rod proximal end is disposed in the second body passage, and an extended position in which the rod proximal ends do not extends into the body of the tool; where the rod is coaxial with the spike.

Embodiment 251: The apparatus of embodiment 250, where the spike is unitary with the rod.

Embodiment 252: The apparatus of any of embodiments 248-251, further comprising: a guidewire configured to be disposed in the first body passage.

Embodiment 253: The apparatus of embodiment 249, further comprising a fastener of any of embodiments 200-246 disposed on the spike such that the fastener proximal end abuts the shoulder.

Embodiment 254: The apparatus of any of embodiments 248-253, further comprising: an elongated tubular shield having a shield proximal end and a shield distal end, the shield disposed around the body and movable between a retracted position in which the shield distal end is proximal to the shoulder, and an extended position in which the shield distal end extends past the spike distal end.

Embodiment 255: A fastener cartridge for use with the apparatus of embodiment 248, the fastener cartridge having a proximal end, a distal end, and a lateral external surface between the proximal and distal ends, the fastener cartridge defining a longitudinal cartridge groove extending through and between the proximal and distal ends and open to the lateral external surface, the cartridge further comprising: a spike having a spike proximal end coupled to the distal end of the cartridge, and a spike distal end extending from the distal end of the cartridge, the spike proximal end having a transverse dimension that is smaller than a transverse dimension of the distal end of the cartridge such that a shoulder is defined at the spike proximal end, the shoulder configured to abut a head of a fastener received over the spike. Where the proximal end of the cartridge is configured to be removably coupled to the distal end of the body such that the spike is coaxial with the cartridge passage, and the first axis of the first body passage extends through the cartridge groove.

Embodiment 256: The fastener cartridge of embodiment 255, further comprising a fastener of any of embodiments 200-246 disposed on the spike such that the fastener proximal end abuts the shoulder.

Embodiment 257: A soft-tissue fastener for coupling an implant to soft tissue, the soft-tissue fastener comprising: an elongated first shaft having a first shaft length extending from a first shaft proximal end to a first shaft distal end, and an internal surface defining a first internal passage extending along the first shaft length; an elongated second shaft having a second shaft length extending from a second shaft proximal end to a second shaft distal end, and an internal surface defining a second internal passage extending along the second shaft length, where the second shaft length is larger than the first shaft length; an enlarged head coupled to the proximal ends of the first and second shafts, the enlarged head having a proximal side and a distal side; a plurality of first barbs each extending along a portion of the first shaft length, each of the first barbs having a leading edge and trailing edge spaced from the first shaft proximal end, the leading edge of each first barb being disposed between the trailing edge of that first barb and the first shaft distal end; and a plurality of second barbs each extending along a portion of the second shaft length, each of the second barbs having a leading edge and trailing edge spaced from the second shaft proximal end, the leading edge of each second barb being disposed between the trailing edge of that second barb and the second shaft distal end, where the trailing edge of at least one of the first barbs is closer to the distal side of the head than is the trailing edge of at least one of the second barbs; where the shafts, head, and barbs are defined by a unitary piece of polymer.

Embodiment 258: The soft-tissue fastener of embodiment 257, where the polymer is bioresorbable.

Embodiment 259: The soft-tissue fastener of any of embodiments 257-258, where: the trailing edge of a second one of the first barbs is closer to the shaft proximal end than is the trailing edge of a first one of the first barbs; and the trailing edge of a second one of the second barbs is closer to the shaft proximal end than is the trailing edge of a first one of the second barbs; and

Embodiment 260: The soft-tissue fastener of embodiment 259, where: the trailing edge of a third one of the first barbs is closer to the shaft proximal end than is the trailing edge of the second one of the first barbs; and the trailing edge of a third one of the second barbs is closer to the shaft proximal end than is the trailing edge of the second one of the second barbs.

Embodiment 261: The soft-tissue fastener of any of embodiments 259-260, where: at least a portion of the leading edge of the first one of the first barbs is disposed at first barb angle relative to the central, longitudinal axis of the shaft; and at least a portion of the leading edge of the first one of the second barbs is disposed at the first barb angle relative to the central, longitudinal axis of the shaft.

Embodiment 262: The soft-tissue fastener of embodiment 261, where: at least a portion of the leading edge of the second one of the first barbs is disposed at a second barb angle relative to the central, longitudinal axis of the shaft; at least a portion of the leading edge of the second one of the second barbs is disposed at the second barb angle relative to the central, longitudinal axis of the shaft; and the second barb angle is smaller than the first barb angle.

Embodiment 263: The soft-tissue fastener of embodiment 262, where: at least a portion of the leading edge of the third one of the first barbs is disposed at a third barb angle relative to the central, longitudinal axis of the shaft; at least a portion of the leading edge of the third one of the second barbs is disposed at the third barb angle relative to the central, longitudinal axis of the shaft; and the third barb angle is smaller than the second barb angle.

Embodiment 264: The soft-tissue fastener of any of embodiments 257-263, where the plurality of first barbs consists of three barbs.

Embodiment 265: The soft-tissue fastener of any of embodiments 257-264, where the plurality of second barbs consists of three barbs.

Embodiment 266: The soft-tissue fastener of any of embodiments 257-264, where at least a portion of the leading edge of at least one of the first barbs is disposed at first barb angle relative to the central, longitudinal axis of the first shaft.

Embodiment 267: The soft-tissue fastener of any of embodiments 257-266, where at least a portion of the leading edge of at least one of the second barbs is disposed at a second barb angle relative to the central, longitudinal axis of the second shaft.

Embodiment 268: The soft-tissue fastener of any of embodiments 257-267, where an outer surface of the first shaft defines a tapered section having an first outer transverse dimension at the first shaft distal end and tapering toward the first shaft proximal end to a second outer transverse dimension that is larger than the first outer transverse dimension.

Embodiment 269: The soft-tissue fastener of embodiment 268, where the tapered section of the first shaft tapers at a first taper angle relative to a central, longitudinal axis of the first shaft, and the first taper angle is substantially equal to the first barb angle.

Embodiment 270: The soft-tissue fastener of any of embodiments 268-269, where the inner surface and outer surface of the first shaft define an edge at the first shaft distal end.

Embodiment 271: The soft-tissue fastener of any of embodiments 257-270, where an outer surface of the second shaft defines a tapered section having an first outer transverse dimension at the first shaft distal end and tapering toward the first shaft proximal end to a second outer transverse dimension that is larger than the first outer transverse dimension.

Embodiment 272: The soft-tissue fastener of embodiment 271, where the tapered section of the second shaft tapers at a second taper angle relative to a central, longitudinal axis of the second shaft, and the second taper angle is substantially equal to the second barb angle.

Embodiment 273: The soft-tissue fastener of embodiment 272, as depending from embodiment 70, where the second taper angle is substantially equal to the first taper angle.

Embodiment 274: The soft-tissue fastener of any of embodiments 271-273, where the inner surface and outer surface of the second shaft define an edge at the second shaft distal end.

Embodiment 275: The soft-tissue fastener of any of embodiments 257-274, where the first and second internal passages each has a first inner transverse dimension at the respective shaft proximal end, a second inner transverse dimension at the respective shaft distal end, and each second inner transverse dimension is smaller than the corresponding first inner transverse dimension.

Embodiment 276: The soft-tissue fastener of embodiment 275, where each of the first and second internal passages tapers linearly from the respective shaft distal end toward the respective shaft proximal end.

Embodiment 277: The soft-tissue fastener of any of embodiments 257-276, where the first shaft length and the second shaft length are each between 5 mm and 9 mm.

Embodiment 278: The soft-tissue fastener of embodiment 277, where the second shaft length is from 0.5 mm to 1.5 mm longer than the first shaft length.

Embodiment 279: The soft-tissue fastener of any of embodiments 257-278, where the at least a portion of the first shaft and at least a portion of the second shaft each has an outer transverse dimension of from 1.25 mm to 2.0 mm.

Embodiment 280: A bone fastener for coupling an implant to bone, the bone fastener comprising: a first elongated shaft having a first shaft length extending from a first shaft proximal end to a first shaft distal end, and defining an internal first passage extending along the first shaft length; a second elongated shaft having a second shaft length extending from a second shaft proximal end to a second shaft distal end, and defining an internal second passage extending along the second shaft length; an enlarged head coupled to the proximal ends of the first shaft and the second shaft; a plurality of first barbs each extending along a portion of the first shaft length, each first barb having a leading edge and trailing edge spaced from the proximal end of the first shaft, the leading edge of each first barb being disposed between the trailing edge of that first barb and the first shaft distal end; a plurality of second barbs each extending along a portion of the first shaft length, each of the second barbs having a leading edge and a trailing edge disposed between the head and the trailing edges of the first barbs, the leading edge of each second barb being disposed between the trailing edge of that second barb and the first shaft distal end; a plurality of third barbs each extending along a portion of the second shaft length, each third barb having a leading edge and trailing edge spaced from the proximal end of the second shaft, the leading edge of each third barb being disposed between the trailing edge of that third barb and the second shaft distal end; and a plurality of fourth barbs each extending along a portion of the second shaft length, each of the fourth barbs having a leading edge and a trailing edge disposed between the head and the trailing edges of the third barbs, the leading edge of each fourth barb being disposed between the trailing edge of that fourth barb and the second shaft distal end; where the shafts, head, and barbs are defined by a unitary piece of polymer.

Embodiment 281: The bone fastener of embodiment 280, where the polymer is not bioresorbable.

Embodiment 282: The bone fastener of any of embodiments 280-281, where the plurality of first barbs are disposed at equiangular intervals around the cross-sectional perimeter of the first shaft.

Embodiment 283: The bone fastener of any of embodiments 280-282, where the plurality of first barbs consists of four barbs.

Embodiment 284: The bone fastener of any of embodiments 280-283, where the plurality of second barbs are disposed at equiangular intervals around the cross-sectional perimeter of the first shaft.

Embodiment 285: The bone fastener of any of embodiments 280-284, where the plurality of second barbs consists of four barbs.

Embodiment 286: The bone fastener of any of embodiments 280-285, where at least one of the second barbs is not radially aligned with any of the first barbs.

Embodiment 287: The bone fastener of embodiment 286, where each of the second barbs is not radially aligned with any of the first barbs.

Embodiment 288: The bone fastener of any of embodiments 280-287, where the plurality of third barbs are disposed at equiangular intervals around the cross-sectional perimeter of the second shaft.

Embodiment 289: The bone fastener of any of embodiments 280-288, where the plurality of third barbs consists of four barbs.

Embodiment 290: The bone fastener of any of embodiments 280-289, where the plurality of fourth barbs are disposed at equiangular intervals around the cross-sectional perimeter of the second shaft.

Embodiment 291: The bone fastener of any of embodiments 280-290, where the plurality of fourth barbs consists of four barbs.

Embodiment 292: The bone fastener of any of embodiments 280-291, where at least one of the fourth barbs is not radially aligned with any of the third barbs.

Embodiment 293: The bone fastener of embodiment 292, where each of the fourth barbs is not radially aligned with any of the third barbs.

Embodiment 294: The bone fastener of any of embodiments 280-293 where at least a portion of the leading edge of at least one of the first barbs is disposed at first barb angle relative to the central, longitudinal axis of the first shaft.

Embodiment 295: The bone fastener of any of embodiments 280-294, where at least a portion of the leading edge of at least one of the second barbs is disposed at a second barb angle relative to the central, longitudinal axis of the first shaft.

Embodiment 296: The bone fastener of embodiment 295, where the second barb angle is substantially equal to the first barb angle.

Embodiment 297: The bone fastener of any of embodiments 280-296, where an outer surface of the first shaft defines a tapered section having an first outer transverse dimension at the first shaft distal end and tapering toward the first shaft proximal end to a second outer transverse dimension that is larger than the first outer transverse dimension.

Embodiment 298: The bone fastener of embodiment 297, where the tapered section of the first shaft tapers at a first taper angle relative to a central, longitudinal axis of the first shaft, and the first taper angle is substantially equal to the first barb angle.

Embodiment 299: The bone fastener of any of embodiments 297-298, where the inner surface and outer surface of the first shaft define an edge at the first shaft distal end.

Embodiment 300: The bone fastener of any of embodiments 280-299, where at least a portion of the leading edge of at least one of the third barbs is disposed at third barb angle relative to the central, longitudinal axis of the second shaft.

Embodiment 301: The bone fastener of any of embodiments 280-300, where at least a portion of the leading edge of at least one of the fourth barbs is disposed at a fourth barb angle relative to the central, longitudinal axis of the second shaft.

Embodiment 302: The bone fastener of embodiment 301, where the fourth barb angle is substantially equal to the third barb angle.

Embodiment 303: The bone fastener of any of embodiments 280-302, where an outer surface of the second shaft defines a tapered section having an first outer transverse dimension at the second shaft distal end and tapering toward the second shaft proximal end to a second outer transverse dimension that is larger than the first outer transverse dimension.

Embodiment 304: The bone fastener of embodiment 303, where the tapered section of the second shaft tapers at a second taper angle relative to a central, longitudinal axis of the second shaft, and the second taper angle is substantially equal to the third barb angle.

Embodiment 305: The bone fastener of any of embodiments 303-304, where the inner surface and outer surface of the second shaft define an edge at the second shaft distal end.

Embodiment 306: The bone fastener of any of embodiments 280-305, where the first and second internal passages each has a first inner transverse dimension at the respective shaft proximal end, a second inner transverse dimension at the respective shaft distal end, and each second inner transverse dimension is smaller than the corresponding first inner transverse dimension.

Embodiment 307: The bone fastener of embodiment 306, where each of the first and second internal passages tapers linearly from the respective shaft distal end toward the respective shaft proximal end.

Embodiment 308: The bone fastener of any of embodiments 280-307, where the first shaft length and the second shaft length are each between 9 mm and 15 mm.

Embodiment 309: The bone fastener of embodiment 308, where the second shaft length is substantially equal to the first shaft length.

Embodiment 310: The bone fastener of any of embodiments 280-309, where the at least a portion of the first shaft and at least a portion of the second shaft each has an outer transverse dimension of from 1.75 mm to 2.5 mm.

Embodiment 311: An apparatus for delivery of a fastener, the apparatus comprising: an elongated body having a body length extending from a body proximal end to a body distal end; a pair of spikes each having a spike proximal end coupled to the body distal end, and a spike distal end extending from the body distal end, the spike proximal end having a transverse dimension that is smaller than a transverse dimension of the body distal end such that a shoulder is defined at the spike proximal end, the shoulder configured to abut a head of a fastener received over the spike, where each of the pair of spikes are parallel to and laterally spaced from the other of the pair of spikes; a handle coupled to the proximal end of the body; and an elongated tubular shield having a shield proximal end and a shield distal end, the shield disposed around the body and movable between a retracted position in which the shield distal end is proximal to the shoulder, and an extended position in which the shield distal end extends past the distal ends of the pair of spikes.

Embodiment 312: An apparatus for delivery of a fastener, the apparatus comprising: an elongated body having a body length extending from a body proximal end to a body distal end, the elongated body defining internal first, second, and third body passages extending through and between the body proximal end and the body distal end, the first body passage having a central, longitudinal first axis, the second body passage having a central, longitudinal second axis separated from the first axis by a distance that remains constant along the body length, the third body passage having a central, longitudinal third axis separated from the second axis by a distance that remains constant along the body length, the first body passage configured to receive a guidewire, the second body passage configured to receive a trocar or rod, and the third body passage configured to receive a trocar or rod; and a handle coupled to the proximal end of the body, the handle configured to permit access to the first, second, and third body passages through the body proximal end.

Embodiment 313: The apparatus of embodiment 312, where the distal end of the body is configured to be coupled to a proximal end of a fastener cartridge that defines a longitudinal cartridge groove extending through and between the proximal end of the cartridge and a distal end of the cartridge and open to a lateral external surface of the cartridge, where the cartridge further comprises: a pair of spikes each having a spike proximal end coupled to the distal end of the cartridge, and a spike distal end extending from the distal end of the cartridge, the spike proximal end having a transverse dimension that is smaller than a transverse dimension of the distal end of the cartridge such that a shoulder is defined at the spike proximal end, the shoulder configured to abut a head of a fastener received over the spike, where the proximal end of the cartridge is configured to be removably coupled to the distal end of the body such that the spike is coaxial with the cartridge passage, and the first axis of the first body passage extends through the cartridge groove.

Embodiment 314: The apparatus of embodiment 313, where the cartridge further comprises: a pair of rods each having a rod proximal end extending proximally from the cartridge proximal end, each rod configured to be disposed in one of the second and third second body passage of the body such that the cartridge is movable between a retracted position in which the rod proximal ends are disposed in respective ones of the second and third body passages, and an extended position in which the rod proximal ends do not extend into the body of the tool; where the pair of rods are each coaxial with one of the pair of spikes.

Embodiment 315: The apparatus of embodiment 314, where each spike is unitary with one of the rods.

Embodiment 316: The apparatus of any of embodiments 312-315, further comprising: a guidewire configured to be disposed in the first body passage.

Embodiment 317: The apparatus of embodiment 314, further comprising a fastener of any of embodiments 58-111 disposed on the spikes such that the fastener proximal end abuts the shoulder.

Embodiment 318: The apparatus of any of embodiments 312-317, further comprising: an elongated tubular shield having a shield proximal end and a shield distal end, the shield disposed around the body and movable between a retracted position in which the shield distal end is proximal to the shoulder, and an extended position in which the shield distal end extends past the spike distal end.

Embodiment 319: A fastener cartridge for use with the apparatus of embodiment 113, the fastener cartridge having a proximal end, a distal end, and a lateral external surface between the proximal and distal ends, the fastener cartridge defining a longitudinal cartridge groove extending through and between the proximal and distal ends and open to the lateral external surface, the cartridge further comprising: a pair of spike having a spike proximal end coupled to the distal end of the cartridge, and a spike distal end extending from the distal end of the cartridge, the spike proximal end having a transverse dimension that is smaller than a corresponding transverse dimension of the distal end of the cartridge such that a shoulder is defined at the spike proximal end, the shoulder configured to abut a head of a fastener received over the spikes; where the proximal end of the cartridge is configured to be removably coupled to the distal end of the body such that the spike is coaxial with the cartridge passage, and the first axis of the first body passage extends through the cartridge groove.

Embodiment 320: The fastener cartridge of embodiment 319, further comprising a fastener of any of embodiments 257-310 disposed on the spike such that the fastener proximal end abuts the shoulder.

Embodiment 321: A kit comprising: a plurality of soft-tissue fasteners of any of embodiments 200-220; and at least one apparatus of any of embodiments 247-256.

Embodiment 322: The kit of embodiment 321, further comprising: a plurality of bone fasteners of any of embodiment 221-246.

Embodiment 323: The kit embodiment 321, further comprising: a plurality of bone fasteners of any of embodiments 280-310; and at least one apparatus of any of embodiments 311-318.

Embodiment 324: The kit of any of embodiments 321-323, where the fasteners and apparatus(es) are sterile and sealed in a package.

Embodiment 325: The kit of embodiment 324, where the package comprises a tray.

Embodiment 325: A kit comprising: a plurality of soft-tissue fasteners of any of embodiments 257-279; and at least one apparatus of any of embodiments 311-318.

Embodiment 327: The kit of embodiment 325, further comprising: a plurality of bone fasteners of any of embodiment 221-246; and at least one apparatus of any of embodiments 48-57.

Embodiment 328: The kit of embodiment 325, further comprising: a plurality of bone fasteners of any of embodiments 280-310; and at least one fastener-delivery tool of any of embodiments 311-318.

Embodiment 329: The kit of any of embodiments 325-328, where the fasteners and apparatus(es) are sterile and sealed in a package.

Embodiment 330: The kit of embodiment 329, where the package comprises a tray.

The invention as shown in the drawings and described in detail herein disclose arrangements of elements of particular construction and configuration for illustrating preferred embodiments of structure and method of operation of the present invention. It is to be understood however, that elements of different construction and configuration and other arrangements thereof, other than those illustrated and described may be employed in accordance with the spirit of the invention, and such changes, alternations and modifications as would occur to those skilled in the art are considered to be within the scope of this invention as broadly defined in the appended claims. In addition, it is to be understood that the phraseology and terminology employed herein are for the purpose of description and should not be regarded as limiting. All references cited herein are hereby fully incorporated by reference.

The subject matter encompassed by the following clauses also forms part of the present invention, optionally in combination with the subject matter described above and/or defined in the claims that follow.

CLAUSE 1. A system for delivery of an implant to a targeted area of a subject, the system comprising:

the implant comprising a plurality of bioresorbable hyaluronan-based fibers and a plurality of non-resorbable fibers;

a bone fastener configured to couple the implant to bone, the bone fastener having a first elongated shaft extending from a first enlarged head, and one or more first barbs at least partially extending radially from the first elongated shaft;

a soft-tissue fastener configured to couple the implant to soft tissue, the soft-tissue fastener having a second elongated shaft extending from a second enlarged head, and one or more second barbs at least partially extending radially from the second elongated shaft;

a soft-tissue fastener tool (SFT) configured to couple with and deliver the soft-tissue fastener through the implant and soft-tissue;

a bone fastener tool (BFT) configured to couple with and deliver the bone fastener and the implant, the BFT further configured to facilitate positioning of at least a portion of the implant over the targeted area after delivery thereto; and

a cannula comprising a cannula distal end, a cannula proximal end, and a cannula passage extending through and between the cannula proximal end and the cannula distal end, the cannula passage configured to receive the BFT coupled with the bone fastener and implant therethrough, such that the BFT is configured to secure the implant about the targeted area via passing through the cannula distal end to insert the bone fastener to a bone about the targeted area with the implant located therebetween.

CLAUSE 2. The system of clause 1, wherein the implant comprises a nonwoven medical textile, a woven medical textile, a braided construction, a weft-knit medical textile, a warp knit medical textile, or a combination thereof.

CLAUSE 3. The system of clause 1 or clause 2, wherein the implant comprises a woven layer and a nonwoven layer coupled to the woven layer.

CLAUSE 4. The system of any one of clause 1 to clause 3, wherein the implant comprises a first end edge, a second edge, and a pair of intermediary edges that each extend between the first and second edges.

CLAUSE 5. The system of any one of clause 1 to clause 4, wherein the implant comprises a substantially planar configuration when in a flattened state, optionally in which the first end edge is separated from the second end edge under a first tension of 5 Newtons (N) applied perpendicular to each of the end edges, and optionally in which the intermediate edges are separated from each other under a tension of 5 N applied perpendicular to each of the intermediate edges.

CLAUSE 6. The system of any one of clause 1 to clause 5, wherein the bioresorbable hyaluronan-based fibers and the non-resorbable fibers are joined together by at least one selected from the group consisting of braided, knitted, adhered, intermeshed, weaved, interlocked, twisted, and heat set.

CLAUSE 7. The system of any one of clause 1 to clause 6, wherein the hyaluronan-based fibers comprise at least one selected from the group consisting of hyaluronic acid, sodium hyaluronate, and esters of hyaluronic acid.

CLAUSE 8. The system of clause 7, wherein the esters of hyaluronic acid comprise benzyl esters of hyaluronic acid.

CLAUSE 9. The system of any one of clause 1 to clause 8, wherein the implant comprises from 10% to 98% non-resorbable fibers, based on the weight of the non-resorbable fibers to the total weight of the non-resorbable fibers and bioresorbable hyaluronan-based fibers.

CLAUSE 10. The system of any one of clause 1 to clause 9, wherein the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

CLAUSE 11. The system of any one of clause 1 to clause 10, wherein the implant further comprises bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxybutyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

CLAUSE 12. The system of any one of clause 1 to clause 11, wherein the hyaluronan-based bioresorbable fibers are prepared by at least one selected from the group consisting of ring spinning, air-jet spinning, open-end spinning, mule spinning, wet spinning, dry spinning, electrospinning, pneumatospinning, pultrusion, and extrusion.

CLAUSE 13. The system of clause 12, wherein the extrusion comprises at least one selected from the group consisting of solvent exchange extrusion, precipitation extrusion, phase exchange extrusion, and phase change extrusion.

CLAUSE 14. The system of any one of clause 1 to clause 13, wherein the bioresorbable hyaluronan-based fibers comprise at least one selected from the group consisting of mono- and multifilaments.

CLAUSE 15. The system of any one of clause 1 to clause 14, further comprising at least one active agent.

CLAUSE 16. The system of clause 15, wherein the active agent is an antimicrobial agent.

CLAUSE 17. The system of clause 16, wherein the antimicrobial agent comprises at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

CLAUSE 18. The system of clause 15, wherein the active agent is an analgesic.

CLAUSE 19. The system of clause 18, wherein the analgesic comprises at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine.

CLAUSE 20. The system of clause 15, wherein the active agent comprises a vasoconstrictive agent.

CLAUSE 21. The system of clause 20, wherein the vasoconstrictive agent comprises at least one selected from the group consisting of epinephrine, alpha-adrenoreceptor antagonists, vasopressin analogues, norepinephrine, phenylephrine, dopamine, dobutamine, serotonin agonists, and triptans.

CLAUSE 22. The system of any one of clause 15 to clause 21, wherein at least one selected from the group consisting of the non-resorbable fibers and the bioresorbable hyaluronan-based fibers are wetted with the active agent.

CLAUSE 23. The system of any one of clause 15 to clause 21, wherein at least one selected from the group consisting of the non-resorbable fibers and the bioresorbable hyaluronan-based fibers are coated with the active agent.

CLAUSE 24. The system of any one of clause 15 to clause 21, wherein at least one selected from the group consisting of the non-resorbable fibers and the bioresorbable hyaluronan-based fibers have the active agent embedded within the fiber.

CLAUSE 25. The system of any one of clause 1 to clause 24, wherein the BFT comprises:

an elongated body extending from a body proximal end to a body distal end, the body defining a body proximal opening through the body proximal end, a body recess in a peripheral surface of the elongated body at point that is offset from the body distal end, and a body passage extending between the body proximal opening and a body distal opening within the body recess; and
one or more spikes each having a spike proximal end coupled to the body distal end, and a spike distal end extending from the body distal end and configured to be at least partially received within the first elongated shaft of the bone fastener, body distal end having a dimension larger than a transverse dimension of the one or more spikes, such that a shoulder is defined about the body distal end that is configured to abut the first enlarged head of the bone fastener;
and a handle coupled to the body proximal end.

CLAUSE 26. The system of clause 25, where the one or more spikes comprise a pair of spikes, where each spike of the pair of spikes are parallel to and radially spaced from the other.

CLAUSE 27. The system of clause 25 or clause 26, wherein the BFT further comprises:

a hub having a hub proximal end and a hub distal end, the hub configured to receive the proximal end of the elongated body through the hub distal end;
an pusher having a pusher proximal end and a pusher distal end, the pusher disposed in the body passage such that the pusher distal end is disposed beyond the body distal opening;
a slider coupled to the pusher proximal end, the slider configured to move longitudinally relative to the handle from a retracted position to a deployed position to move the pusher distal end longitudinally away from the body distal opening and radially outward from the elongated body.

CLAUSE 28. The system of clause 27, where the hub is coupled in fixed relation to the elongated body, and the handle is rotatable relative to the hub to cause the pusher distal end to rotate relative to the elongate body.

CLAUSE 29. The system of clause 27 or clause 28, where the body recess defines a sloped surface spaced from the body distal opening, and where the apparatus is configured such that, when the slider is moved from the retracted position to the deployed position, a flexible portion of the pusher contacts the sloped surface to cause the pusher distal end to extend radially outward from the body.

CLAUSE 30. The system of any one of clause 27 to clause 29, where the pusher comprises a roller coupled to the pusher distal end.

CLAUSE 31. The system of any one of clause 25 to clause 30, further comprising a seal assembly, comprising:

an annular seal body having a seal proximal end and a seal distal end, the seal body defining a seal passage extending through and between the seal proximal end and the seal distal end, and a tapered distal surface disposed around the seal passage at the seal distal end; and
a resilient membrane disposed at least around an interior periphery of the seal passage, the resilient membrane configured to cooperate with the elongated body of the BFT to substantially seal the seal passage when the elongated body of the BFT extends through the resilient membrane and the seal passage.

CLAUSE 32. The system of clause 31, where the elongated body of the BFT is disposed through the resilient membrane of the seal and the seal passage with the seal distal end facing the body distal end.

CLAUSE 33. The system of clause 31 or clause 32, wherein the cannula proximal end is configured to be coupled to the seal body, the cannula further defining a cannula proximal surface tapered distally and inward toward the cannula passage, the cannula proximal surface configured to cooperate with the seal distal surface to provide a seal between the cannula proximal surface and the seal distal surface.

CLAUSE 34. The system of clause 33, where an outer surface of the cannula includes one or more threads that are configured to engage soft tissue as the cannula is rotated.

CLAUSE 35. The system of any of clause 1 to clause 34, where a proximal end of the cannula includes an enlarged portion that is configured to be engaged by a user to vary the depth and/or direction of the cannula.

CLAUSE 36. The system of any of clause 27 to clause 35, where the cannula distal end defines a notch configured to permit a portion of the pusher to deflect radially outward through the notch.

CLAUSE 37. The system of any of clause 1 to clause 36, further comprising: an obturator having an obturator body with a obturator proximal end and a tapered obturator distal end, the obturator body configured to extend through the cannula such that the obturator distal end extends distally beyond the cannula distal end to facilitate insertion of the cannula through soft tissue of a patient.

CLAUSE 38. The system of any of clause 1 to clause 37, further comprising a caddy system comprising:

a base defining one or more bone fastener recesses; and
a lid coupled to the base and configured to be rotated relative to the base to selectively cover or expose the bone fastener recess(es).

CLAUSE 39. The system of clause 38, where the base further defines one or more implant recesses each overlapping at least one of the one or more bone fastener recesses, where the lid is configured to be rotated relative to the base to selectively cover or expose the implant.

CLAUSE 40. The system of clause 39, where the one or more implant recesses includes a substantially planar lower surface offset below a substantially planar upper surface of the base.

CLAUSE 41. The system of any of clause 38 to clause 40, wherein the lid is rotatable, relative to the base, over at least one of the bone fastener recesses.

CLAUSE 42. The system of any of clause 38 to clause 41, where the base further defines one or more soft tissue fastener recesses, where the lid is configured to be rotated relative to the base to selectively cover or expose the soft fastener recess(es).

CLAUSE 43. The system of any one of clause 1 to clause 42, wherein the bone fastener comprises a third elongated shaft extending from the first enlarged head, and one or more third barbs at least partially extending radially from the third elongated shaft.

CLAUSE 44. The system of clause 43, wherein the bone fastener further comprises a plurality of fourth and fifth barbs extending at least partially radially from the first elongated shaft and the third elongated shaft respectively, each of the first, third, fourth, and fifth barbs having a leading edge and a trailing edge.

CLAUSE 45. The system of clause 44, wherein the first and third elongated shaft of the bone fastener is configured to extend through the implant such that the trailing edge of each of the fourth and fifth barbs are disposed on a first side of the implant, and at least a portion of the leading edges of each of the first barbs and third barbs are disposed on a second side of the implant.

CLAUSE 46. A method for delivering an implant to a targeted area in a subject, the method comprising:

coupling a bone fastener with an implant by passing the bone fastener therethrough;
inserting the bone fastener at least partially into a bone at the targeted area, thereby securing the implant to said bone;
manipulating a portion of the implant not secured via the bone fastener so as to facilitate placement of said portion of the implant about a soft tissue of the targeted area; and
securing said portion of the implant to the soft tissue by inserting one or more soft tissue fasteners through the implant and the soft tissue;
wherein the implant comprises a plurality of bioresorbable hyaluronan-based fibers and a plurality of non-resorbable fibers.

CLAUSE 47. A method for delivering the implant according to any one of clause 1 to clause 45 to the targeted area using a system according to any one of clauses 1-45, the method comprising:

using the BFT, coupling the bone fastener with the implant by passing the bone fastener therethrough;
aligning the cannula passage about the targeted area;
inserting the BFT through the cannula passage so as to insert the bone fastener at least partially into the bone at the targeted area, thereby securing the implant to said bone;
moving the slider from the retracted position to the deployed position, thereby causing the distal end of the pusher to move distally and radially so as to position a portion of the implant about the soft tissue at the targeted area; and
securing said portion of the implant to the soft tissue by inserting the soft tissue fastener through the implant and the soft tissue.

CLAUSE 48. The method of clause 47, wherein aligning the cannula passage about the targeted area comprises using the obturator to facilitate insertion of the cannula within the subject.

CLAUSE 49. The method of clause 47, wherein the BFT retrieves the bone fastener from the caddy system by inserting the one or more spikes through the first and third elongated shafts of the bone fastener.

CLAUSE 50. The method of clause 47, wherein coupling the bone fastener with the implant comprises placing the implant on the implant recess on the caddy system prior to passing the bone fastener through the implant.

CLAUSE 51. The method of clause 47, wherein inserting the soft tissue fastener through the implant and the soft tissue is via the SFT.

CLAUSE 52. The method of clause 51, further comprises inserting the SFT through the cannula passage for insertion of the soft tissue fastener.

CLAUSE 53. The method of clause 47, further comprising rotating the handle of the BFT so as to cause the pusher to sweep clockwise and/or counterclockwise to further facilitate positioning of the implant about the targeted area.

**Claims**

1. A system for delivery of an implant to a targeted area of a subject, the system comprising:

the implant comprising a plurality of bioresorbable hyaluronan-based fibers and a plurality of non-resorbable fibers;

a bone fastener configured to couple the implant to bone, the bone fastener having a first elongated shaft extending from a first enlarged head, and one or more first barbs at least partially extending radially from the first elongated shaft;

a soft-tissue fastener configured to couple the implant to soft tissue, the soft-tissue fastener having a second elongated shaft extending from a second enlarged head, and one or more second barbs at least partially extending radially from the second elongated shaft;

a soft-tissue fastener tool (SFT) configured to couple with and deliver the soft-tissue fastener through the implant and soft-tissue;

a bone fastener tool (BFT) configured to couple with and deliver the bone fastener and the implant, the BFT further configured to facilitate positioning of at least a portion of the implant over the targeted area after delivery thereto; and

a cannula comprising a cannula distal end, a cannula proximal end, and a cannula passage extending through and between the cannula proximal end and the cannula distal end, the cannula passage configured to receive the BFT coupled with the bone fastener and implant therethrough, such that the BFT is configured to secure the implant about the targeted area via passing through the cannula distal end to insert the bone fastener to a bone about the targeted area with the implant located therebetween.

2. The system of claim 1, wherein the implant comprises a nonwoven medical textile, a woven medical textile, a braided construction, a weft-knit medical textile, a warp knit medical textile, or a combination thereof.

3. The system of claim 1 or claim 2, wherein the implant comprises a woven layer and a nonwoven layer coupled to the woven layer.

4. The system of any one of claim 1 to claim 3, wherein the implant comprises a first end edge, a second edge, and a pair of intermediary edges that each extend between the first and second edges.

5. The system of any one of claim 1 to claim 4, wherein the implant comprises a substantially planar configuration when in a flattened state, in which the first end edge is separated from the second end edge under a first tension of 5 Newtons (N) applied perpendicular to each of the end edges, and in which the intermediate edges are separated from each other under a tension of 5 N applied perpendicular to each of the intermediate edges.

6. The system of any one of claim 1 to claim 5, wherein the bioresorbable hyaluronan-based fibers and the non-resorbable fibers are joined together by at least one selected from the group consisting of braided, knitted, adhered, intermeshed, weaved, interlocked, twisted, and heat set.

7. The system of any one of claim 1 to claim 6, wherein the hyaluronan-based fibers comprise at least one selected from the group consisting of hyaluronic acid, sodium hyaluronate, and esters of hyaluronic acid, optionally wherein the esters of hyaluronic acid comprise benzyl esters of hyaluronic acid.

8. The system of any one of claim 1 to claim 7, wherein the implant comprises from 10% to 98% non-resorbable fibers, based on the weight of the non-resorbable fibers to the total weight of the non-resorbable fibers and bioresorbable hyaluronan-based fibers.

9. The system of any one of claim 1 to claim 8, wherein the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

10. The system of any one of claim 1 to claim 9, wherein the implant further comprises bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxybutyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

11. The system of any one of claim 1 to claim 10, wherein the hyaluronan-based bioresorbable fibers are prepared by at least one selected from the group consisting of ring spinning, air-jet spinning, open-end spinning, mule spinning, wet spinning, dry spinning, electrospinning, pneumatospinning, pultrusion, and extrusion.

**12.** The system of claim 11, wherein the extrusion comprises at least one selected from the group consisting of solvent exchange extrusion, precipitation extrusion, phase exchange extrusion, and phase change extrusion.

**13.** The system of any one of claim 1 to claim 12, wherein the bioresorbable hyaluronan-based fibers comprise at least one selected from the group consisting of mono- and multifilaments.

**14.** The system of any one of claim 1 to claim 13, further comprising at least one active agent.

**15.** The system of claim 14, wherein:

(a) the active agent is an antimicrobial agent, optionally wherein the antimicrobial agent comprises at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides; or
(b) the active agent is an analgesic, optionally wherein the analgesic comprises at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine; or
(c) the active agent comprises a vasoconstrictive agent.

FIG. 1A

FIG. 1B

FIG. 1C

FIG. 1D

**FIG. 1E**

156

158

164a

162a

112a

160a

126

116

**FIG. 1F**

164b

162b

112b

160b

156

**FIG. 1G**

164c

166

162c

112c

160c

156

**FIG. 1H**

**FIG. 2A**

**FIG. 2B**

**FIG. 2C**

**FIG. 2D**

**FIG. 2E**

**FIG. 2F**

FIG. 3A

FIG. 3B

FIG. 3C

FIG. 3D

FIG. 3E

406

410

408

**FIG. 4A**

C

C

406

**FIG. 4B**

414  412  402  314
312  404

**FIG. 4C**

406

**FIG. 4D**

502

550

554

552

504

506

508

**FIG. 5A**

510

502

554

C

512

C

**FIG. 5B**

502

554

514

556

566

558

516

518

**FIG. 5C**

560

562

564

522

510

560

528

524

526

520

**FIG. 5D**

FIG. 5E

FIG. 5F

FIG. 5G

FIG. 6A

FIG. 6C

FIG. 6B

**FIG. 6D**

**FIG. 6E**

**FIG. 6F**

EP 4 559 410 A1

FIG. 7A

FIG. 7B

FIG. 7C

FIG. 7D

111

FIG. 8A

FIG. 8B

FIG. 8C

FIG. 8D

FIG. 8E

904

902

704

906b
906a
504

**FIG. 9A**

C — C

534
904
526
534

**FIG. 9B**

908

**FIG. 9C**

802

532    526

**FIG. 9D**

802
908

510

**FIG. 9E**

904

802

908

**FIG. 9F**

534

910

534

908

**FIG. 9G**

510    728

**FIG. 10A**

504

1002a    906a

510    728

**FIG. 10B**

510    1002a

1002b    728    906b

**FIG. 10C**

510

728

**FIG. 10D**

510    728

**FIG. 10E**

802    904

510

**FIG. 10F**

728

802    904

728

**FIG. 10G**

904    728

802

510

**FIG. 10H**

802    904    728

**FIG. 10I**

802    510

**FIG. 10J**

802

**FIG. 10K**

FIG. 11

FIG. 12A

FIG. 12B

**FIG. 13A**

**FIG. 13B**

**FIG. 14A**

**FIG. 15A**

**FIG. 14B**

**FIG. 15B**

B

1708
1704
1710
1506b
156
1702

FIG. 17C

B
1702
1704
1506b
1506c
1706
B

FIG. 17A

1404
1602
1604b
1604c
1604a

FIG. 16A

FIG. 17B

1606a
1602
1606c
1406
1606b

FIG. 16B

FIG. 18A

FIG. 19A

FIG. 18B

FIG. 19B

1506b

1506a

2102

**FIG. 21**

1506b

1704

1506b

1506a

1506a

2202

**FIG. 22**

1604b

1604a

1604b

1604a

2002

**FIG. 20**

FIG. 23A

FIG. 23B

FIG. 23C

**FIG. 24**

**FIG. 25**

**FIG. 26A**

**FIG. 26B**

**FIG. 27**

FIG. 28A

FIG. 28B

FIG. 28C

FIG. 29A

FIG. 29B

FIG. 29C

3008

3004

3006

3002

3010

**FIG. 30**

3108

3116

3008

3106

3102   3104

3110   3112

3010   3114

**FIG. 31**

**FIG. 32**

**FIG. 33A**

**FIG. 33B**

**FIG. 34A**

**FIG. 34B**

**FIG. 34C**

FIG. 35

FIG. 36

**FIG. 37A**

**FIG. 37B**

**FIG. 38**

FIG. 39A

FIG. 40A

FIG. 39B

FIG. 40B

**FIG. 41**

**FIG. 42**

**FIG. 43**

4402a

4404

4406

**FIG. 44A**

4408b

4402b

4410b

4412b

4408b

**FIG. 44B**

4402c

4408c

4414c

4410c

4414c

4412c

4408c

**FIG. 44C**

4408d

4402d

4410d

4412d

4408d

**FIG. 44D**

4408e

4402e

4410e

4412e

4408e

**FIG. 44E**

4402f

4408f

4410f

4412f

4408f

**FIG. 44F**

4408g

4402g

4410g

4412g

4408g

**FIG. 44G**

4408h

4402h

4410h

4412h

4408h

**FIG. 44H**

FIG. 45A

FIG. 45B

4602

4604

**FIG. 46**

4706
4708
4710
4712
4720 4714
4704
4718
4702
4716
3320

**FIG. 47**

4704
4708
4802
4708

**FIG. 48**

**FIG. 49**

**FIG. 50**

**FIG. 51A**

**FIG. 51B**

**FIG. 52A**

**FIG. 52B**

5302

5312

5310

5308

5306

5304

**FIG. 53**

5402    5402

5406

5410    5408

5412    5404

5416

5414

**FIG. 54A**

5404

5406

5416

**FIG. 54B**

5402

5404

5406

5416

5412

**FIG. 54C**

FIG. 55

**FIG. 56A**

**FIG. 56F**

**FIG. 56L**

**FIG. 56B**

**FIG. 56G**

**FIG. 56M**

**FIG. 56C**

**FIG. 56H**

**FIG. 56N**

**FIG. 56D**

**FIG. 56I**

**FIG. 56O**

**FIG. 56E**

**FIG. 56J**

**FIG. 56P**

5604

5602

**FIG. 56K**

5702

5704

5706

5708

**FIG. 57A**

5712

5710

5714

**FIG. 57B**

5718

5716

5720

**FIG. 57C**

**FIG. 57D**

**FIG. 57E**

FIG. 58

FIG. 59

FIG. 60

FIG. 61

FIG. 62

FIG. 63

FIG. 64

6502

6504

6506

6508

**FIG. 65**

**FIG. 66A**

**FIG. 66B**

FIG. 67A

FIG. 67B

FIG. 67C

**FIG. 68A**

**FIG. 68D**

**FIG. 68B**

**FIG. 68E**

**FIG. 68C**

6906

6912

6914

6914

6912

**FIG. 69C**

6910

6910

6904

6908

6908

**FIG. 69B**

69C

6902

6906

69B

6904

6904

**FIG. 69A**

7006

7002

7004

7014

7016

**FIG. 70B**

70B

7010

7012

7004

7008

**FIG. 70A**

7004

7010

71B

7006

7012

**FIG. 71A**

7010

7102

7104

**FIG. 71B**

FIG. 72A

FIG. 72B

**FIG. 73C**

**FIG. 73B**

**FIG. 73A**

7302

7306

7304

7312

7310

7308

**FIG. 74**

7502    7504    75B

76  75B

**FIG. 75A**

7504

7506

7508

**FIG. 75B**

7506

7508

**FIG. 76**

FIG. 77

FIG. 78

FIG. 79

FIG. 80

FIG. 81

8206  8204
→ 8202

**FIG. 82**

8404
8406
8408
8402
8410

**FIG. 84**

8306  8304
8308
→ 8302

**FIG. 83**

8506  8504
8508
→ 8502

**FIG. 85**

FIG. 86

**Stress/Strain Relation for Tendon**

FIG. 87

Suture pad
region

Central region

Laterally-locked
edges

**FIG. 88A**

Suture pads

Central region

Suture pads

**FIG. 88B**

**FIG. 88C**

Fabric layer (B)

Nonwoven layer (A)

Fabric layer (B)

D top-view

D side-view

**FIG. 88D**

Swelling study (Thickness)

**FIG. 89A**

Swelling study (volume)

**FIG. 89B**

FIG. 89C

Tensile test (Design 1 bonded ends)

**FIG. 90A**

Tensile test (Design 3)

**FIG. 90C**

Tensile test (Design 2 bonded ends)

**FIG. 90B**

Tensile test (Design 4)

**FIG. 90D**

FIG. 91

Tensile test (Design 1 bonded ends)

FIG. 92A

Tensile test (Design 2 bonded ends)

FIG. 92B

Tensile test (Design 3)

FIG. 92C

Tensile test (Design 4)

FIG. 92D

Tensile test (Hyalonect)

**FIG. 92E**

Tensile test (GraftJacket)

**FIG. 92F**

Fatigue test on Design 1

**FIG. 93A**

Fatigue test on Design 2

**FIG. 93B**

Fatigue test on Design 3

FIG. 93C

Fatigue test on Design 4

FIG. 93D

FIG. 94A

FIG. 94B

FIG. 94C

FIG. 94D

FIG. 95

Tensile strength

| | Max load (N) | St.Dev. | % of Max Load retained |
|---|---|---|---|
| Dry | 48.83 | 8.12 | |
| 1min | 22.55 | 1.82 | 46.18 |
| 5min | 19.08 | 1.44 | 39.06 |
| 10min | 19.80 | 1.66 | 40.55 |
| 1h | 19.67 | 2.26 | 40.29 |
| 5d | 19.80 | 0.57 | 40.55 |
| 12d | 17.69 | 1.44 | 36.23 |
| 20d | 14.43 | 0.83 | 29.55 |
| 28d | 17.15 | 1.09 | 35.12 |
| 42d | 14.85 | 1.44 | 30.42 |
| 152d | 11.97 | 0.76 | 24.51 |

## FIG. 96A

mass loss

## FIG. 96B

**FIG. 96C**

FIG. 97

FIG. 98

**FIG. 99**

**FIG. 100**

**FIG. 101A**

**FIG. 101B**

**FIG. 101C**

FIG. 102

**FIG. 103A**

**FIG. 103B**

**FIG. 104A**

**FIG. 104B**

**FIG. 104C**

Europäisches
Patentamt

European
Patent Office

Office européen
des brevets

## EUROPEAN SEARCH REPORT

**Application Number**

EP 24 21 4878

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/362005 A1 (CHAO NAM [US] ET AL) 17 November 2022 (2022-11-17) * paragraphs [0004], [0005], [0096], [0111], [0128], [0132], [0168]; figures 1-25 * | 1-15 | INV. A61B17/04 A61B17/00 |
| Y | US 2021/402050 A1 (AGNELLO STEFANO [IT] ET AL) 30 December 2021 (2021-12-30) * paragraphs [0006] - [0014], [0020], [0021], [0026], [0067]; figures 3A-E * | 1-15 | |

TECHNICAL FIELDS
SEARCHED      (IPC)

A61B

The present search report has been drawn up for all claims

| Place of search | Date of completion of the search | Examiner |
|---|---|---|
| Munich | 13 March 2025 | Hornung, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 4 559 410 A1

## ANNEX TO THE EUROPEAN SEARCH REPORT
## ON EUROPEAN PATENT APPLICATION NO.

EP 24 21 4878

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2025

| Patent document cited in search report | Publication date | Patent family member(s) | | Publication date |
|---|---|---|---|---|
| US 2022362005 A1 | 17-11-2022 | NONE | | |
| US 2021402050 A1 | 30-12-2021 | EP | 4171671 A1 | 03-05-2023 |
| | | US | 2021402050 A1 | 30-12-2021 |
| | | US | 2025073016 A1 | 06-03-2025 |
| | | WO | 2021262986 A1 | 30-12-2021 |

EPO FORM P0459

For more details about this annex : see Official Journal of the European Patent Office, No. 12/82

189

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 66365122 A
- US 63189400 A
- US 65881722 A
- US 63173623 A
- US 63189351 A
- US 45690121 A
- US 63120232 A
- US 35734521 A
- US 63043517 A
- US 48760123 A
- US 33081916 A
- US 2015029792 W
- US 61989899 A
- US 63511530 A
- US 5939323 A
- US 6872819 B
- EP 9704684 W
- WO 9808876 A
- US 20080188936 A
- US 4965353 A
- WO 9311805 A
- WO 9403212 A
- US 6482231 B
- WO 9961080 A
- WO 9965534 A
- US 10918372 B

### Non-patent literature cited in the description

- **KOWALSKY MS ; DELLENBAUGH SG ; ERLICH-MAN DB et al.** Evaluation of suture abrasion against rotator cuff tendon and proximal humerus bone. *Arthroscopy*, 2008, vol. 24, 329-334
- **GOA, KAREN L. ; PAUL BENFIELD.** Hyaluronic acid. *Drugs*, 1994, vol. 47 (3), 536-566
- **NECAS, J. B. L. B. P. et al.** Hyaluronic acid (hyaluronan): a review. *Veterinarni medicina*, 2008, vol. 53 (8), 397-411
- **CAMPOCCIA D. et al.** *Biomaterials*, 1998, vol. 19, 2101-2127
- **VINDIGNI V. et al.** *Int. J. Mol. Sci.*, 2009, vol. 10, 2972-2985
- **HALBLEIB, M. et al.** *Biomaterials*, 2003, vol. 24, 3125-3132
- **VINDIGNI V. et al.** *Int. J. Mol. Sci*, 2009, vol. 10, 2972-2985
- **PASQUINELLI G. et al.** *J. Anat.*, 2008, vol. 213, 520-530